# EUROPEAN PATENT APPLICATION

(11) **EP 3 970 743 A1**
(43) Date of publication of application: **23.03.2022**
(21) Application number: 20805264.7
(22) Date of filing: 15.05.2020
(51) Int. Cl.: A61K 39/395, A61P 35/00, C07K 16/18, C07K 16/28, C07K 16/46, C12N 5/10, A61K 47/55, A61K 47/56, A61K 47/62, A61K 47/68, A61K 49/00, C12N 1/15, C12N 1/19, C12N 1/21, C12N 15/13, C12N 15/62, C12N 15/63, G01N 33/53, G01N 33/574, C12P 21/08

(54) **BISPECIFIC ANTIBODY BINDING TO CD40 AND FAP**

(30) Priority: 15.05.2019 JP 2019092298
(71) Applicant: Kyowa Kirin Co., Ltd., Tokyo 100-0004 (JP)
(72) Inventor: TEZUKA Yuta, Tokyo 100-0004 (JP); OSANAI Aya, Tokyo 100-0004 (JP); USAMI Katsuaki, Tokyo 100-0004 (JP); NISHIYA Harue, Tokyo 100-0004 (JP)
(74) Representative: Hoffmann Eitle
(86) International application number: PCT/JP2020/019545
(87) International publication number: WO 2020/230899

(57) **Abstract**

An object of the present invention is to provide a bispecific antibody, which binds to CD40 and FAP and exhibits a strong CD40 agonistic activity in a FAP dependent manner or a bispecific antibody fragment thereof, and the like. The present invention relates to a bispecific antibody, which is a bispecific antibody containing an IgG portion including a first antigen binding domain and also containing a second antigen binding domain, and binding to human CD40 and human FAP, wherein the C terminus of a heavy chain of the IgG portion binds to the second antigen binding domain either directly or via a linker, or a bispecific antibody fragment thereof, and the like.

## Description

### TECHNICAL FIELD

The present invention relates to a bispecific antibody comprising an antigen binding domain that binds to CD40 and an antigen binding domain that binds to fibroblast activation protein alpha (FAP) or a bispecific antibody fragment thereof, a DNA encoding the bispecific antibody or the bispecific antibody fragment thereof, a vector including the DNA, a hybridoma and a transformant strain that produce the bispecific antibody, a method for producing the bispecific antibody or the bispecific antibody fragment thereof, a therapeutic agent and a diagnostic agent comprising the bispecific antibody or the bispecific antibody fragment thereof, a therapeutic method and a diagnostic method using the bispecific antibody or the bispecific antibody fragment thereof, and a reagent for detection or measurement comprising the bispecific antibody.

### BACKGROUND ART

CD40 is a type I membrane-associated glycoprotein identified as an antigen expressed on the surface of a human B cell, and is known to be expressed on various cell types such as B lymphocytes, dendritic cells, monocytes, epithelial cells, and fibroblasts, or also a certain type of tumor cells such as neoplastic human B cells. In a CD40-deficient mouse, it has been confirmed that thymus-dependent immunoglobulin class switching or germinal center formation is impaired, and an important role of CD40 in cellular and humoral immune responses has been demonstrated. CD40 signaling is involved in immunoglobulin class switching or induction of cytotoxic T lymphocytes (CTLs), and therefore, activation of tumor immunity or application to a pharmaceutical product as an adjuvant for a cancer vaccine is also expected (NPL 1).

FAP is a transmembrane glycoprotein of 85 kDa and forms a dimer. FAP belongs to the serine protease superfamily and has a peptidase activity. FAP is expressed at a high level in fibroblasts infiltrating into tumors and cancer-associated fibroblasts (CAFs) in various cancer types such as pancreatic cancer, breast cancer, and stomach cancer, and some cancer cells such as melanomas. In a normal tissue of a healthy subject, FAP is expressed at only a few parts such as fibroblasts present in a lymphoid tissue or mesenchymal stem cells in the bone marrow. By focusing on this property of localized expression, FAP has been expected as a target molecule for a cancer molecular targeted drug, a diagnostic marker, or a cancer vaccine target (NPL 2).

As a conventional antibody targeting CD40, Chi-Lob 7/4, HCD-122, APX005M, SEA-CD40, CP870,893 (21.4.1), and the like can be exemplified (NPL 3). Among them, CP870,893 has a strong CD40 signaling inducing ability, and a clinical trial was conducted for solid tumors using systemic immune activation as a drug efficacy mechanism. However, effectiveness has not yet been demonstrated, and expression of toxicity derived from systemic immune activation such as cytokine syndrome, elevation of a thrombus marker, or elevation of a liver parameter has been reported (NPL 4).

As a bispecific protein that recognizes CD40, a multivalent antibody that recognizes CD28 and CD40 is known (PTL 1). An IgG-type anti-human GPC3/anti-mouse CD40 bispecific antibody having a heterodimerized heavy chain is known (PTL 2). In addition, a bispecific protein that binds to a cancer antigen such as nectin-4, PSMA, EGFR, HER2, or MSLN and to CD40 and activates CD40 is known (PTLs 3 and 4). Any of these bispecific antibodies has a CD40 binding site derived from an agonistic anti-CD40 antibody.

Further, a method for activating a CD40-expressing cell in the vicinity of a cancer cell by a bispecific molecule such as a diabody having specificity for CD40 and a cancer cell surface antigen is known (PTL 5).

Further, a bispecific protein that has a CD40 binding site derived from an agonistic anti-CD40 antibody SGN-40 and a FAP binding site, and activates CD40 in a FAP-dependent manner has been reported (PTL 6). A bispecific protein that has a CD40 binding module and a FAP binding module derived from an ankyrin repeat protein is known, and it is known that the bispecific protein induces CD40 signaling specifically for FAP, and activates dendritic cells, B cells, and macrophages (NPLs 5 and 6).

### CITATION LIST

### PATENT LITERATURE

PTL 1: WO 2009/131239
PTL 2: WO 2015/156268
PTL 3: WO 2017/205738
PTL 4: WO 2018/140831
PTL 5: WO 99/61057
PTL 6: WO 2018/185045

### NON PATENT LITERATURE

NPL 1: Diehl L et al., Nat Med. 1999 Jul; 5(7): 774-9
NPL 2: Guan-Min Jiang et al., Oncotarget. 2016 May 31; 7(22): 33472-82
NPL 3: Beatty GL et al., Expert Rev Anticancer Ther. 2017 Feb; 17(2): 175-186
NPL 4: Vonderheide RH et al., Oncoimmunology. 2013 Jan 1; 2(1): e23033
NPL 5: Fiedler U. et al., AACR Annual Meeting 2018, poster 4552. (April 14-18, 2018)
NPL 6: Rigamonti N. et al., SITC 33rd Annual Meeting 2018, poster 637 (November 9-11, 2018)

### SUMMARY OF INVENTION

### TECHNICAL PROBLEM

An object of the present invention is to provide a bispecific antibody that binds to CD40 and FAP and exhibits a strong CD40 agonistic activity only in the presence of FAP or a bispecific antibody fragment thereof, a DNA encoding the bispecific antibody or the bispecific antibody fragment thereof, a vector including the DNA, a hybridoma and a transformant strain that produce the bispecific antibody or the bispecific antibody fragment thereof, a method for producing the bispecific antibody or the bispecific antibody fragment thereof, a therapeutic agent and a diagnostic agent comprising the bispecific antibody or the bispecific antibody fragment thereof, a therapeutic method and a diagnostic method using the bispecific antibody or the bispecific antibody fragment thereof, and a reagent for detection or measurement comprising the bispecific antibody or the bispecific antibody fragment thereof.

### SOLUTION TO PROBLEM

As a means for solving the above problems, a bispecific antibody, which is a bispecific antibody comprising an IgG portion comprising a first antigen binding domain and also comprising a second antigen binding domain, and binding to human CD40 and human FAP, wherein the C terminus of a heavy chain of the IgG portion binds to the second antigen binding domain either directly or via a linker, and a bispecific antibody fragment thereof, and the like are provided.

That is, the present invention relates to the following.
1. A bispecific antibody, which is a bispecific antibody comprising an IgG portion comprising a first antigen binding domain and also comprising a second antigen binding domain, and binding to human CD40 and human fibroblast activation protein alpha (FAP), wherein the C terminus of a heavy chain of the IgG portion binds to the second antigen binding domain either directly or via a linker.
2. The bispecific antibody according to the above 1, wherein either one of the first antigen binding domain and the second antigen binding domain is derived from a non-agonistic anti-CD40 antibody.
3. The bispecific antibody according to the above 1 or 2, which divalently binds to each of human CD40 and human FAP.
4. The bispecific antibody according to any one of the above 1 to 3, wherein the C terminus of the heavy chain of the IgG portion directly binds to the second antigen binding domain.
5. The bispecific antibody according to any one of the above 1 to 4, which has a CD40 agonistic activity.
6. The bispecific antibody according to any one of the above 1 to 5, which does not exhibit a CD40 agonistic activity in the absence of a FAP-expressing cell, and exhibits a CD40 agonistic activity only in the presence of a FAP-expressing cell.
7. The bispecific antibody according to any one of the above 1 to 6, wherein either one of the first antigen binding domain and the second antigen binding domain is Fab of an anti-CD40 IgG antibody (anti-CD40 Fab).
8. The bispecific antibody according to any one of the above 1 to 7, wherein either one of the first antigen binding domain and the second antigen binding domain is Fab of an anti-FAP antibody (anti-FAP Fab).
9. The bispecific antibody according to any one of the above 1 to 8, wherein the first antigen binding domain is anti-CD40 Fab and the second antigen binding domain is anti-FAP Fab.
10. The bispecific antibody according to any one of the above 1 to 8, wherein the first antigen binding domain is anti-FAP Fab and the second antigen binding domain is anti-CD40 Fab.
11. The bispecific antibody according to the above 7, 9, or 10, wherein the anti-CD40 Fab is Fab comprising a heavy chain variable region (VH) comprising complementarity determining regions (CDRs) 1 to 3 comprising the amino acid sequences of SEQ ID NOS: 24 to 26, respectively, and a light chain variable region (VL) comprising CDRs 1 to 3 comprising the amino acid sequences of SEQ ID NOS: 19 to 21, respectively.
12. The bispecific antibody according to any one of the above 7, and 9 to 11, wherein the anti-CD40 Fab is Fab comprising VH comprising the amino acid sequence of SEQ ID NO: 23, and VL comprising the amino acid sequence of SEQ ID NO: 18.
13. The bispecific antibody according to any one of the above 8 to 12, wherein the anti-FAP Fab is Fab comprising VH comprising heavy chain CDRs 1 to 3 depicted in any one selected from the following (i) to (v) and VL comprising CDRs 1 to 3 comprising the amino acid sequences of SEQ ID NOS: 19 to 21, respectively:
   (i) heavy chain CDRs 1 to 3 comprising the amino acid sequences of SEQ ID NOS: 33 to 35, respectively;
   (ii) heavy chain CDRs 1 to 3 comprising the amino acid sequences of SEQ ID NOS: 38 to 40, respectively;
   (iii) heavy chain CDRs 1 to 3 comprising the amino acid sequences of SEQ ID NOS: 43 to 45, respectively;
   (iv) heavy chain CDRs 1 to 3 comprising the amino acid sequences of SEQ ID NOS: 48 to 50, respectively; and
   (v) heavy chain CDRs 1 to 3 comprising the amino acid sequences of SEQ ID NOS: 53 to 55, respectively.
14. The bispecific antibody according to any one of the above 8 to 13, wherein the anti-FAP Fab is Fab comprising VH comprising the amino acid sequence of any one selected from SEQ ID NOS: 32, 37, 42, 47, 52, 75, 77, 79, 81, and 83, and VL comprising the amino acid sequence of SEQ ID NO: 18.
15. The bispecific antibody according to any one of the above 1 to 14, wherein the IgG portion comprises a heavy chain constant region comprising the amino acid sequence of SEQ ID NO: 61.
16. The bispecific antibody according to any one of the above 1 to 9 and 11 to 15, which consists of two heavy chains comprising the amino acid sequence of any one selected from SEQ ID NOS: 65, 67, 69, 71, 73, 85, 87, 89, 91, and 93, and four light chains comprising VL comprising the amino acid sequence of SEQ ID NO: 18.
17. The bispecific antibody according to any one of the above 1 to 8 and 10 to 15, which consists of two heavy chains comprising the amino acid sequence of any one selected from SEQ ID NOS: 95, 97, 99, 101, 103, 105, 107, 109, and 111, and four light chains comprising VL comprising the amino acid sequence of SEQ ID NO: 18.
18. A bispecific antibody fragment of the bispecific antibody according to any one of the above 1 to 17.
19. A DNA encoding the bispecific antibody according to any one of the above 1 to 17 or the bispecific antibody fragment according to the above 18.
20. A recombinant vector, containing the DNA according to the above 19.
21. A transformant strain obtained by introducing the recombinant vector according to the above 20 into a host cell.
22. A method for producing the bispecific antibody according to any one of the above 1 to 17 or the bispecific antibody fragment according to the above 18, characterized by culturing the transformant strain according to the above 21 in a culture medium to produce and accumulate the bispecific antibody according to any one of the above 1 to 17 or the bispecific antibody fragment according to the above 18 in a culture, and collecting the bispecific antibody or the bispecific antibody fragment according to the above 18 from the culture.
23. A therapeutic agent and/or a diagnostic agent for a disease associated with at least one of CD40 and FAP, containing the bispecific antibody according to any one of the above 1 to 17 or the bispecific antibody fragment according to the above 18 as an active ingredient.
24. The therapeutic agent and/or the diagnostic agent according to the above 23, wherein the disease associated with at least one of CD40 and FAP is a cancer.
25. A therapeutic method and/or a diagnostic method for a disease associated with at least one of CD40 and FAP, using the bispecific antibody according to any one of the above 1 to 17 or the bispecific antibody fragment according to the above 18.
26. The method according to the above 25, wherein the disease associated with at least one of CD40 and FAP is a cancer.
27. The bispecific antibody according to any one of the above 1 to 17 or the bispecific antibody fragment according to the above 18 for use in therapy and/or diagnosis for a disease associated with at least one of CD40 and FAP.
28. The bispecific antibody or the bispecific antibody fragment according to the above 27, wherein the disease associated with at least one of CD40 and FAP is a cancer.
29. Use of the bispecific antibody according to any one of the above 1 to 17 or the bispecific antibody fragment according to the above 18 for producing a therapeutic agent and/or a diagnostic agent for a disease associated with at least one of CD40 and FAP.
30. The use according to the above 28, wherein the disease associated with at least one of CD40 and FAP is a cancer.
31. A reagent for detecting or measuring at least one of CD40 and FAP, containing the bispecific antibody according to any one of the above 1 to 17 or the bispecific antibody fragment according to the above 18.
32. A derivative of a bispecific antibody or a bispecific antibody fragment thereof, in which a radioisotope, a low-molecular weight drug, a high-molecular weight drug, a protein, or an antibody drug is bound chemically or through genetic engineering to the bispecific antibody according to any one of the above 1 to 17 or the bispecific antibody fragment according to the above 18.

### ADVANTAGEOUS EFFECTS OF INVENTION

According to the present invention, a bispecific antibody that binds to CD40 and FAP and exhibits a strong CD40 agonistic activity only in the presence of FAP or a bispecific antibody fragment thereof, a DNA encoding the bispecific antibody or the bispecific antibody fragment thereof, a vector comprising the DNA, a hybridoma and a transformant strain that produce the bispecific antibody or the bispecific antibody fragment thereof, a method for producing the bispecific antibody or the bispecific antibody fragment thereof, a therapeutic agent and a diagnostic agent comprising the bispecific antibody, a therapeutic method and a diagnostic method using the bispecific antibody or the bispecific antibody fragment thereof, and a reagent for detection or measurement comprising the bispecific antibody or the bispecific antibody fragment thereof can be provided.

### BRIEF DESCRIPTION OF DRAWINGS

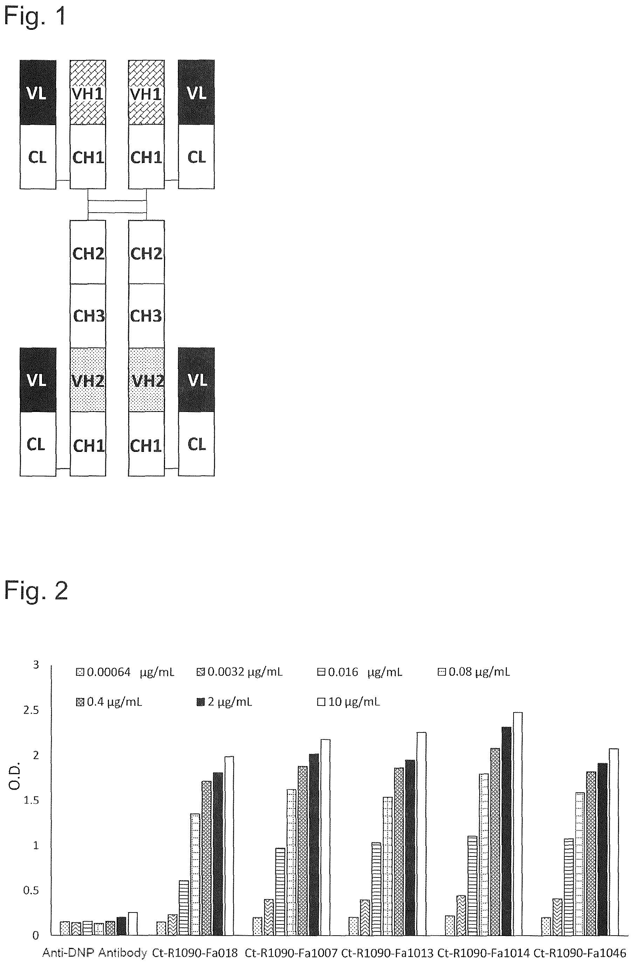

[Fig. 1] Fig. 1 shows a bispecific antibody in which Fab is directly bound to the C terminus of a heavy chain of an IgG portion as an example of a structure of a bispecific antibody of the present invention.
[Fig. 2] Fig. 2 shows the results of evaluating the binding of each of various CD40-FAP bispecific antibodies to human FAP-GST by an ELISA method. The vertical axis represents an absorbance, and each of the CD40-FAP bispecific antibodies was added at 10, 2, 0.4, 0.08, 0.016, 0.0032, or 0.00064 µg/mL. For comparison, an anti-DNP antibody was used as the negative control antibody.

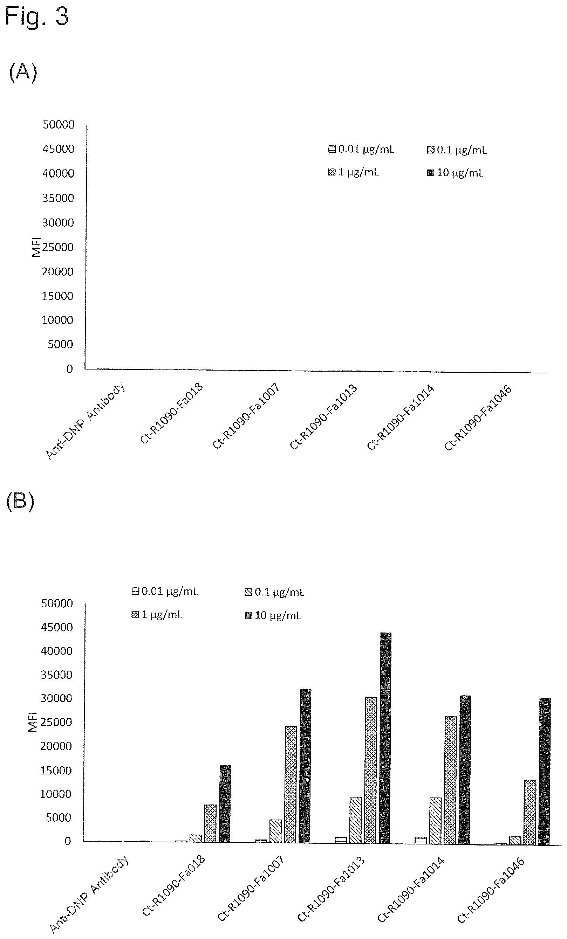

[Fig. 3] Fig. 3(A) shows the results of evaluating the binding activity of each of various CD40-FAP bispecific antibodies to HEK 293 cells by flow cytometry. Fig. 3(B) shows the results of evaluating the binding of each of various CD40-FAP bispecific antibodies to HEK 293 cells having expressed human FAP by flow cytometry. The vertical axis of each drawing represents the binding activity expressed as a mean fluorescence intensity (MFI). Each of the bispecific antibodies was added at 10, 1, 0.1, or 0.01 µg/mL. For comparison, an anti-DNP antibody was used as the negative control antibody.

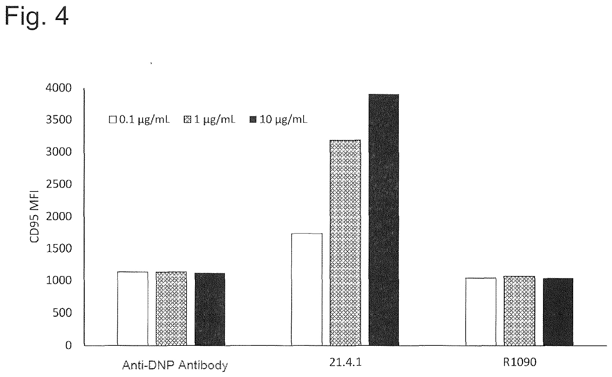

[Fig. 4] Fig. 4 shows a CD40 signaling inducing activity using an increase in the expression level of CD95 by R1090S55A against Ramos cells as an index. The vertical axis represents a mean fluorescence intensity (MFI), which indicates the expression level of CD95. Each of the bispecific antibodies was added at 10, 1, or 0.1 µg/mL. For comparison, an anti-DNP antibody was used as the negative control antibody, and 21.4.1 which is an agonistic anti-CD40 antibody was used as the positive control antibody.

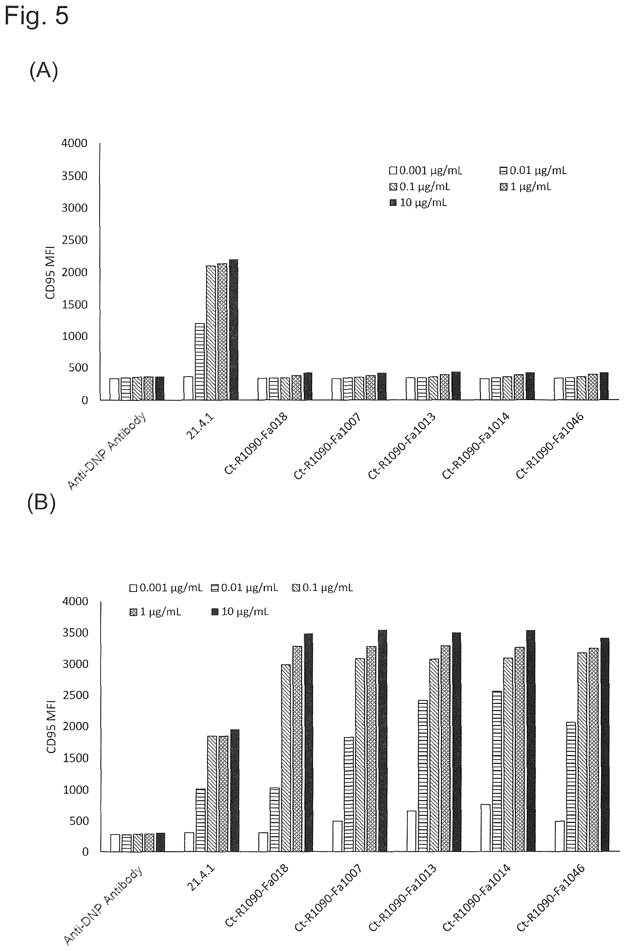

[Fig. 5] Fig. 5(A) shows a CD40 signaling inducing activity using an increase in the expression level of CD95 by each of various CD40-FAP bispecific antibodies against Ramos cells cocultured with HEK 293 as an index. Fig. 5(B) shows a CD40 signaling inducing activity using an increase in the expression level of CD95 by each of various CD40-FAP bispecific antibodies against Ramos cells cocultured with HEK 293 having expressed human FAP as an index. The vertical axis of each drawing represents a mean fluorescence intensity (MFI), which indicates the expression level of CD95. Each of the bispecific antibodies was added at 10, 1, 0.1, 0.01 or 0.001 µg/mL. For comparison, an anti-DNP antibody was used as the negative control antibody, and 21.4.1 which is an agonistic anti-CD40 antibody was used as the positive control antibody.

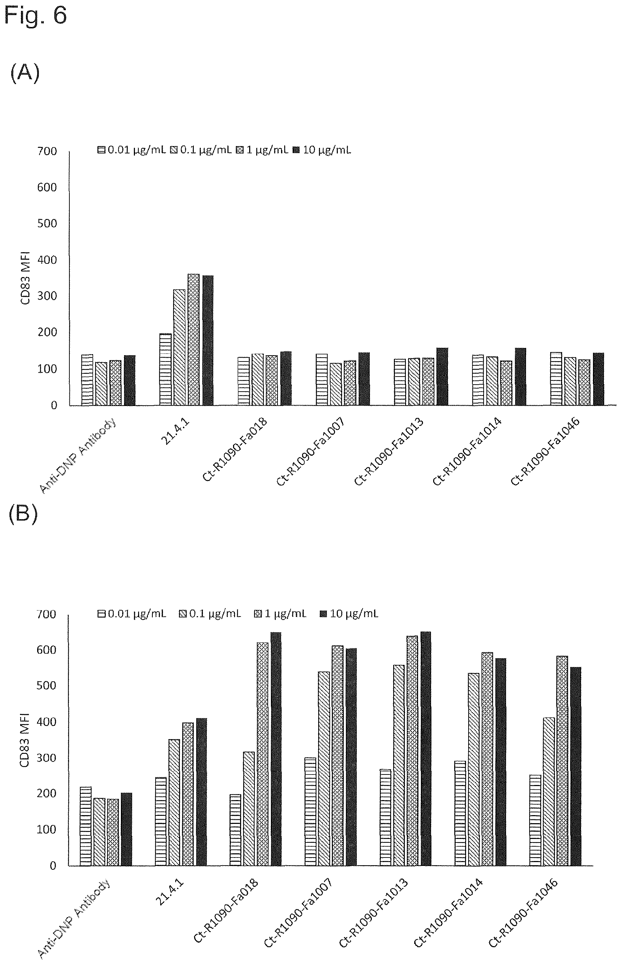

[Fig. 6] Fig. 6(A) shows a CD40 signaling inducing activity using an increase in the expression level of CD83 by each of various CD40-FAP bispecific antibodies against dendritic cells cocultured with HEK 293 as an index. Fig. 6(B) shows a CD40 signaling inducing activity using an increase in the expression level of CD83 by each of various CD40-FAP bispecific antibodies against dendritic cells cocultured with HEK 293 having expressed human FAP as an index. The vertical axis of each drawing represents a mean fluorescence intensity (MFI), which indicates the expression level of CD83. Each of the bispecific antibodies was added at 10, 1, 0.1, or 0.01 µg/mL. For comparison, an anti-DNP antibody was used as the negative control antibody, and 21.4.1 which is an agonistic anti-CD40 antibody was used as the positive control antibody.

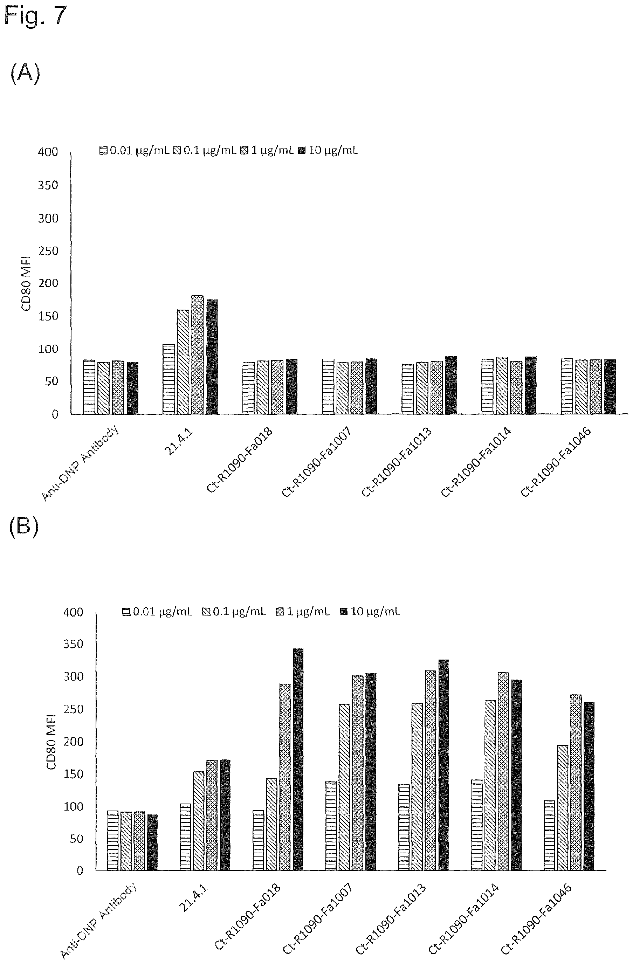

[Fig. 7] Fig. 7(A) shows a CD40 signaling inducing activity by each of various CD40-FAP bispecific antibodies using an increase in the expression level of CD80 against dendritic cells cocultured with HEK 293 as an index. The vertical axis represents a mean fluorescence intensity (MFI), which indicates the expression level of CD80. Fig. 7(B) shows a CD40 signaling inducing activity by each of various CD40-FAP bispecific antibodies using an increase in the expression level of CD80 against dendritic cells cocultured with HEK 293 having expressed human FAP as an index. The vertical axis of each drawing represents a fluorescence intensity, which indicates the expression level of CD80. Each of the bispecific antibodies was added at 10, 1, 0.1, or 0.01 µg/mL. For comparison, an anti-DNP antibody was used as the negative control antibody, and 21.4.1 which is an agonistic anti-CD40 antibody was used as the positive control antibody.

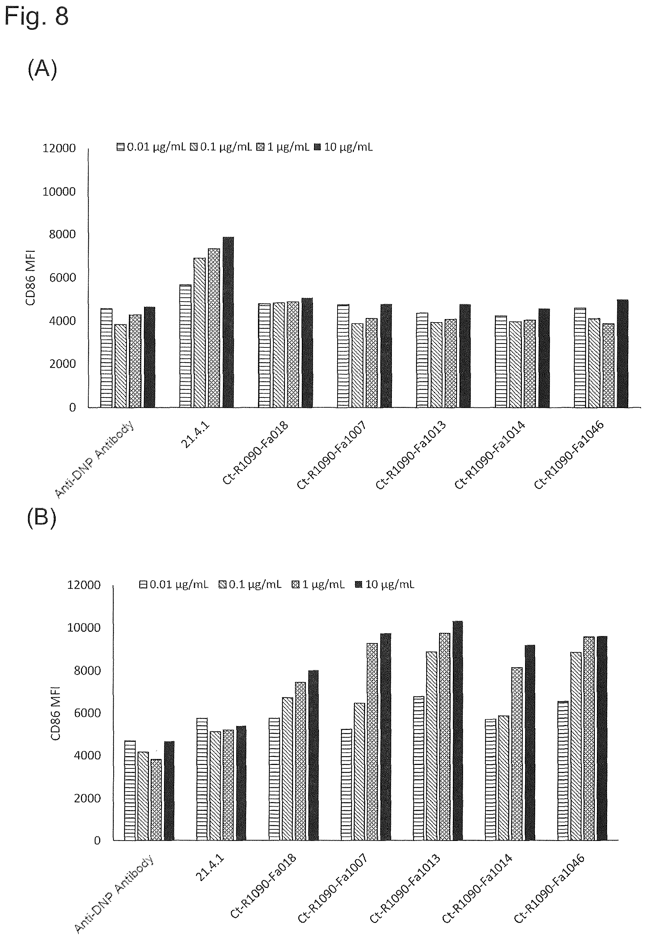

[Fig. 8] Fig. 8(A) shows a CD40 signaling inducing activity using an increase in the expression level of CD86 by each of various CD40-FAP bispecific antibodies against dendritic cells cocultured with HEK 293 as an index. Fig. 8(B) shows a CD40 signaling inducing activity using an increase in the expression level of CD86 by each of various CD40-FAP bispecific antibodies against dendritic cells cocultured with HEK 293 having expressed human FAP as an index. The vertical axis of each drawing represents a mean fluorescence intensity (MFI), which indicates the expression level of CD86. Each of the bispecific antibodies was added at 10, 1, 0.1, or 0.01 µg/mL. For comparison, an anti-DNP antibody was used as the negative control antibody, and 21.4.1 which is an agonistic anti-CD40 antibody was used as the positive control antibody.

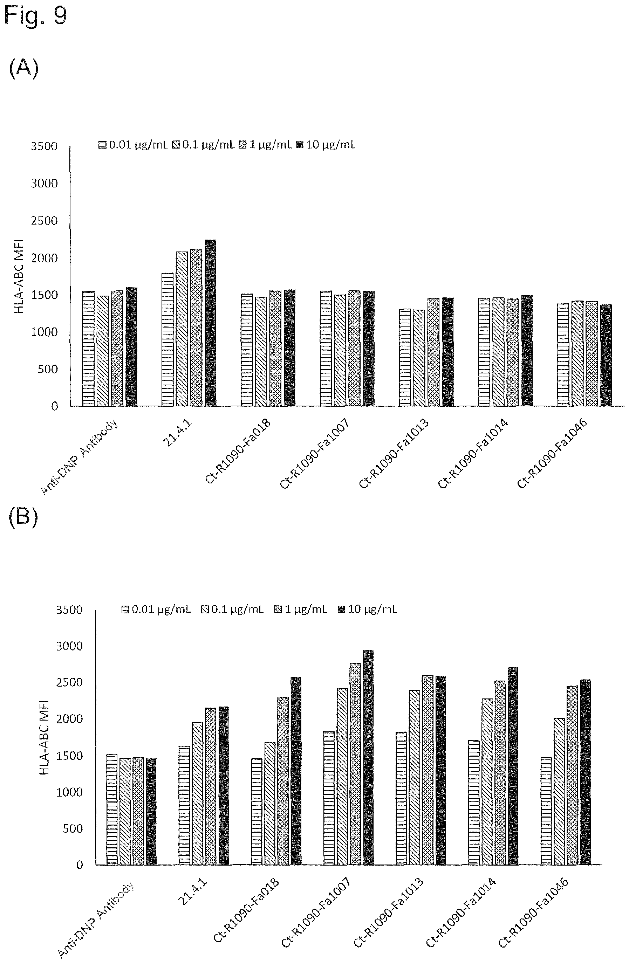

[Fig. 9] Fig. 9(A) shows a CD40 signaling inducing activity using an increase in the expression level of HLA-ABC by each of various CD40-FAP bispecific antibodies against dendritic cells cocultured with HEK 293 as an index. Fig. 9(B) shows a CD40 signaling inducing activity using an increase in the expression level of HLA-ABC by each of various CD40-FAP bispecific antibodies against dendritic cells cocultured with HEK 293 having expressed human FAP as an index. The vertical axis of each drawing represents a mean fluorescence intensity (MFI), which indicates the expression level of HLA-ABC. Each of the bispecific antibodies was added at 10, 1, 0.1, or 0.01 µg/mL. For comparison, an anti-DNP antibody was used as the negative control antibody, and 21.4.1 which is an agonistic anti-CD40 antibody was used as the positive control antibody.

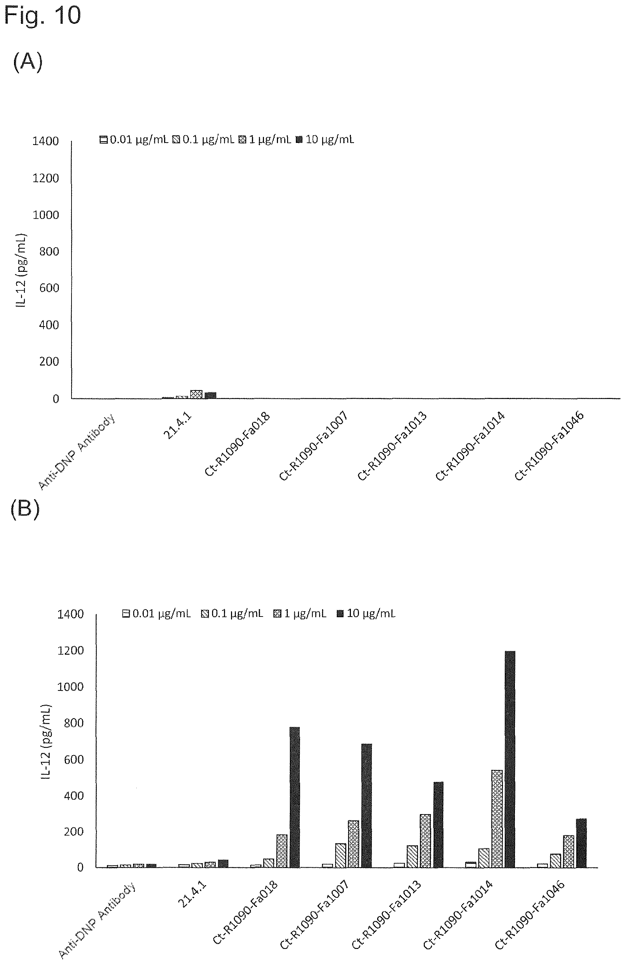

[Fig. 10] Fig. 10(A) shows a CD40 signaling inducing activity using an increase in the concentration (an increase in the expression level) of IL-12 by each of various CD40-FAP bispecific antibodies against dendritic cells cocultured with HEK 293 as an index. Fig. 10(B) shows a CD40 signaling inducing activity using an increase in the concentration (an increase in the expression level) of IL-12 by each of various CD40-FAP bispecific antibodies against dendritic cells cocultured with HEK 293 having expressed human FAP as an index. The vertical axis of each drawing represents the concentration of IL-12 (pg/mL). Each of the bispecific antibodies was added at 10, 1, 0.1, or 0.01 µg/mL. For comparison, an anti-DNP antibody was used as the negative control antibody, and 21.4.1 which is an agonistic anti-CD40 antibody was used as the positive control antibody.

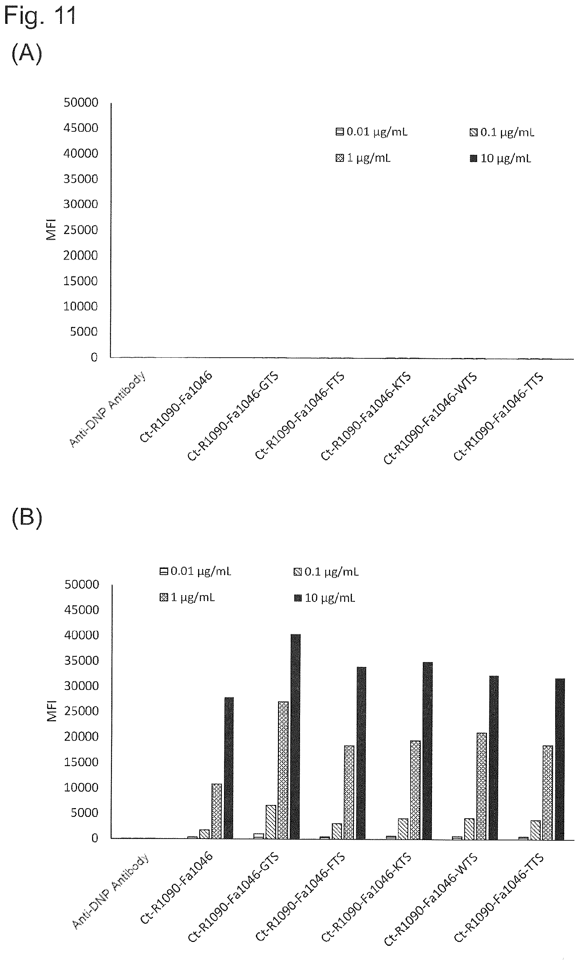

[Fig. 11] Fig. 11(A) shows the results of evaluating the binding activity of each of various CD40-FAP bispecific antibodies to HEK 293 cells by flow cytometry. Fig. 11(B) shows the results of evaluating the binding of each of various CD40-FAP bispecific antibodies to HEK 293 cells having expressed human FAP by flow cytometry. The vertical axis of each drawing represents a mean fluorescence intensity (MFI) which was used as the index of the binding activity. Each of the bispecific antibodies was added at 10, 1, 0.1, or 0.01 µg/mL. For comparison, an anti-DNP antibody was used as the negative control antibody.

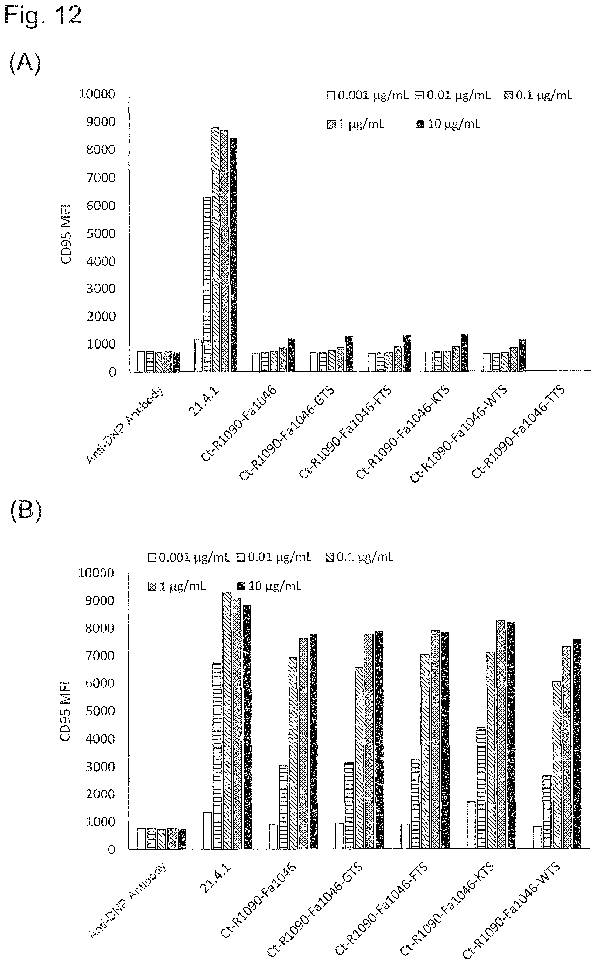

[Fig. 12] Fig. 12(A) shows a CD40 signaling inducing activity using an increase in the expression level of CD95 by each of various CD40-FAP bispecific antibodies against Ramos cells cocultured with HEK 293 as an index. Fig. 12(B) shows a CD40 signaling inducing activity using an increase in the expression level of CD95 by each of various CD40-FAP bispecific antibodies against Ramos cells cocultured with HEK 293 having expressed human FAP as an index. The vertical axis of each drawing represents a mean fluorescence intensity (MFI), which indicates the expression level of CD95. Each of the bispecific antibodies was added at 10, 1, 0.1, 0.01 or 0.001 µg/mL. For comparison, an anti-DNP antibody was used as the negative control antibody, and 21.4.1 which is an agonistic anti-CD40 antibody was used as the positive control antibody.

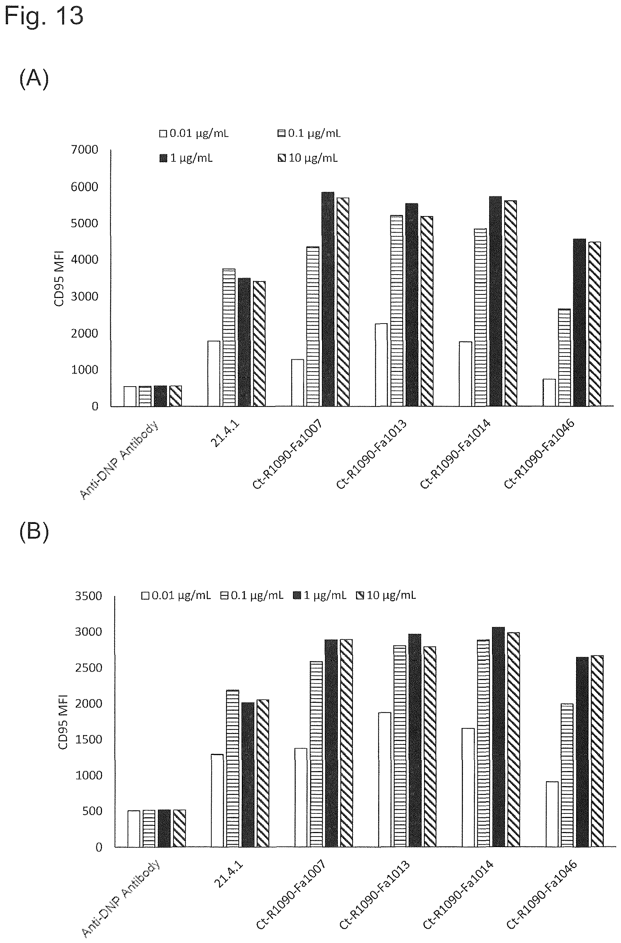

[Fig. 13] Fig. 13(A) and Fig. 13(B) each show a CD40 signaling inducing activity using an increase in the expression level of CD95 by each of various CD40-FAP bispecific antibodies against Ramos cells cocultured with a FAP-positive cell line as an index. As the FAP-positive cell line, a fibroblast cell line IMR90 was used in Fig. 13(A), and a melanoma cell line RPMI 7951 was used in Fig. 13(B). The vertical axis of each drawing represents a mean fluorescence intensity (MFI), which indicates the expression level of CD95. Each of the bispecific antibodies was added at 10, 1, 0.1, or 0.01 µg/mL. For comparison, an anti-DNP antibody was used as the negative control antibody, and 21.4.1 which is an agonistic anti-CD40 antibody was used as the positive control antibody.

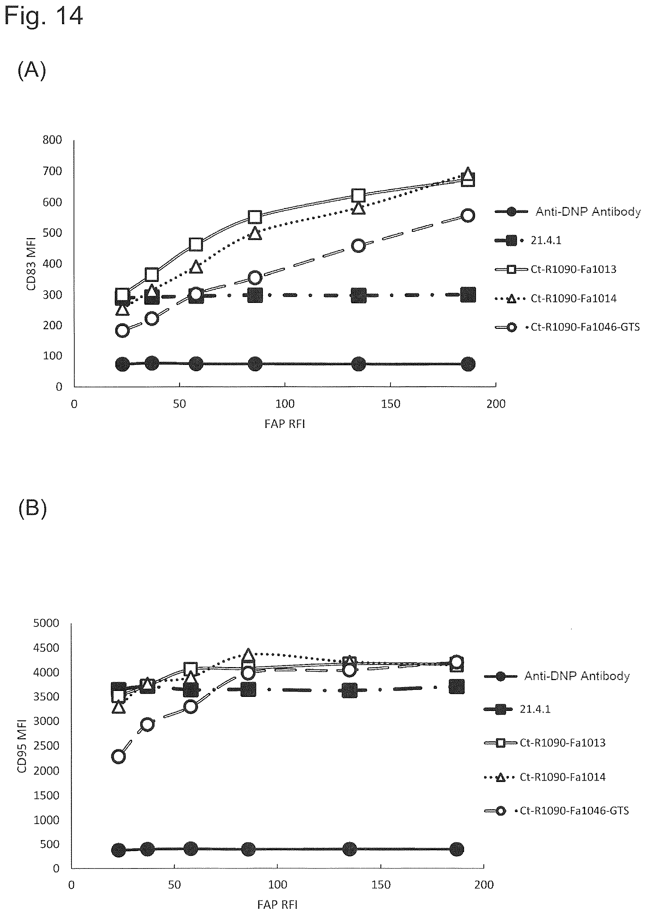

[Fig. 14] Fig. 14(A) and Fig. 14(B) each show a CD40 signaling inducing activity by each of various CD40-FAP bispecific antibodies against Ramos cells cocultured with beads with immobilized FAP. In Fig. 14(A) and Fig. 14(B), an increase in the expression levels of CD83 and CD95, respectively, was used as an index of the CD40 signaling inducing activity. The vertical axis of each drawing represents a mean fluorescence intensity (MFI), which indicates the expression levels of CD83 or CD95. The horizontal axis of each drawing represents a relative fluorescence intensity (RFI) of an anti-FAP antibody to an anti-DNP antibody serving as the negative control antibody, which indicates the expression level of FAP in each of the cocultured beads. Each of the bispecific antibodies was added at 1 µg/mL. For comparison, an anti-DNP antibody was used as the negative control antibody, and 21.4.1 which is an agonistic anti-CD40 antibody was used as the positive control antibody.

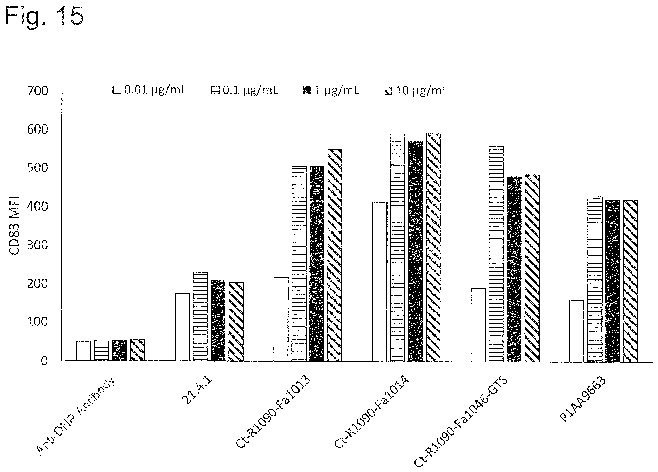

[Fig. 15] Fig. 15 shows a CD40 signaling inducing activity using an increase in the expression level of CD83 by each of various CD40-FAP bispecific antibodies against Ramos cells cocultured with a fibroblast cell line SC00A05 as an index. The vertical axis of the drawing represents a mean fluorescence intensity (MFI), which indicates the expression level of CD83. Each of the bispecific antibodies was added at 10, 1, 0.1, or 0.01 µg/mL. For comparison, an anti-DNP antibody was used as the negative control antibody, and 21.4.1 which is an agonistic anti-CD40 antibody was used as the positive control antibody.

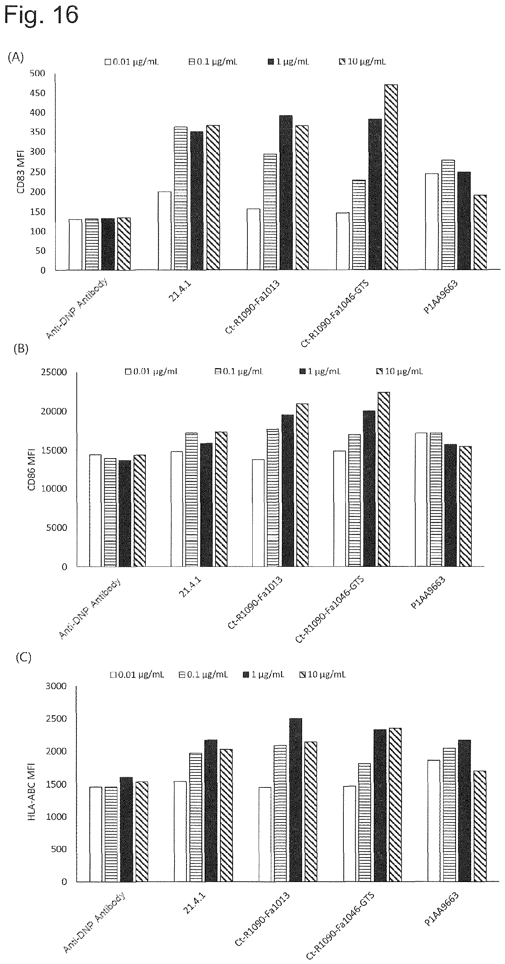

[Fig. 16] Figs. 16(A) to 16(C) each show a CD40 signaling inducing activity by each of various CD40-FAP bispecific antibodies against dendritic cells cocultured with a fibroblast cell line SC00A05. In Figs. 16(A) to 16(C), an increase in the expression levels of CD83, CD86, and HLA-ABC, respectively, was used as an index of the CD40 signaling inducing activity. The vertical axes of the respective drawings each represent a mean fluorescence intensity (MFI), which indicates the expression levels of CD83, CD86, and HLA-ABC, respectively. Each of the bispecific antibodies was added at 10, 1, 0.1, or 0.01 µg/mL. For comparison, an anti-DNP antibody was used as the negative control antibody, and 21.4.1 which is an agonistic anti-CD40 antibody was used as the positive control antibody.

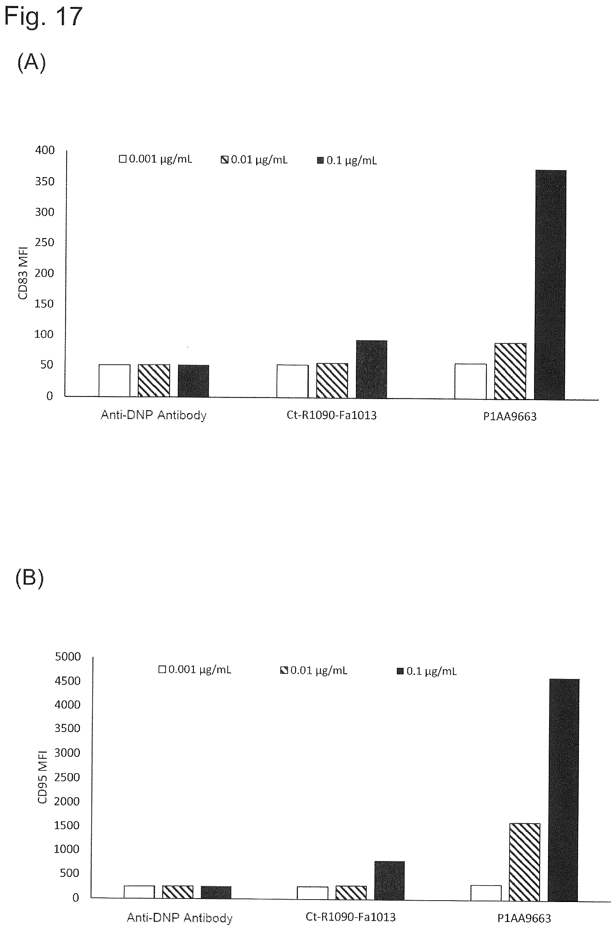

[Fig. 17] Fig. 17(A) and Fig. 17(B) each show a CD40 signaling inducing activity independent of FAP-expressing cells by each of various CD40-FAP bispecific antibodies against Ramos cells under the condition that a crosslinking antibody was added. In Fig. 17(A) and Fig. 17(B), an increase in the expression levels of CD83 and CD95, respectively, was used as an index of the CD40 signaling inducing activity. The vertical axis of each drawing represents a mean fluorescence intensity (MFI), which indicates the expression level of CD83 or CD95. Each of the bispecific antibodies was added at 0.1, 0.01, or 0.001 µg/mL. For comparison, an anti-DNP antibody was used as the negative control antibody.

### DESCRIPTION OF EMBODIMENTS

The present invention relates to a bispecific antibody that specifically binds to human CD40 and human FAP (hereinafter referred to as the bispecific antibody of the present invention).

The CD40 in the present invention is used synonymously with TNF receptor superfamily member 5 (TNFRSF5), Bp50, CDW40, MGC9013, and p50. As the CD40, for example, human CD40 containing the amino acid sequence represented by GenBank Accession No. NP_001241 in NCBI (http://www.ncbi.nlm.nih.gov/) or SEQ ID NO: 6, monkey CD40 containing the amino acid sequence represented by GenBank Accession No. XP_005569274 or SEQ ID NO: 8, and the like are exemplified. Further, for example, a polypeptide that is composed of an amino acid sequence in which one or more amino acids are deleted, substituted, or added in the amino acid sequence represented by SEQ ID NO: 6, GenBank Accession No. NP_001241, GenBank Accession No. XP_005569274, or SEQ ID NO: 8 and that has the function of CD40 is exemplified.

A polypeptide comprising an amino acid sequence having generally 70% or more, preferably 80% or more, and more preferably 90% or more homology with the amino acid sequence represented by SEQ ID NO: 6, GenBank Accession No. NP_001241, GenBank Accession No. XP_005569274, or SEQ ID NO: 8, and most preferably, a polypeptide that is composed of an amino acid sequence having 95%, 96%, 97%, 98%, and 99% or more homology and that has the function of CD40 are also included in the CD40 of the present invention.

The polypeptide comprising an amino acid sequence in which one or more amino acid residues are deleted, substituted, or added in the amino acid sequence represented by SEQ ID NO: 6, GenBank Accession No. NP_001241, GenBank Accession No. XP_005569274, or SEQ ID NO: 8 can be obtained by, for example, introducing a site-specific mutation into a DNA encoding the amino acid sequence represented by SEQ ID NO: 6, GenBank Accession No. NP_001241, GenBank Accession No. XP_005569274, or SEQ ID NO: 8 using a site-specific mutagenesis method [Molecular Cloning, A Laboratory Manual, Second Edition, Cold Spring Harbor Laboratory Press (1989), Current Protocols in Molecular Biology, John Wiley & Sons (1987-1997), Nucleic Acids Research, 10, 6487 (1982), Proc. Natl. Acad. Sci. USA, 79, 6409 (1982), Gene, 34, 315 (1985), Nucleic Acids Research, 13, 4431 (1985), Proceeding of the National Academy of Sciences in USA, 82, 488 (1985)], or the like. The number of amino acids to be deleted, substituted, or added is not particularly limited, but is preferably one to several tens, for example, 1 to 20, and more preferably one to several, for example, 1 to 5 amino acids.

As a gene encoding CD40, for example, the nucleotide sequence of human CD40 represented by SEQ ID NO: 5 or GenBank Accession No. NM_001250, the nucleotide sequence of monkey CD40 represented by SEQ ID NO: 7 or GenBank Accession No. XM_011766922, and the like are exemplified. Further, for example, a gene that is composed of a nucleotide sequence in which one or more nucleotides are deleted, substituted, or added in the nucleotide sequence represented by SEQ ID NO: 5, GenBank Accession No. NM _001250, SEQ ID NO: 7, or GenBank Accession No. XM_011766922 and that contains a DNA encoding a polypeptide having the function of CD40, a gene that is composed of preferably a nucleotide sequence having 60% or more homology, more preferably a nucleotide sequence having 80% or more homology, and further more preferably a nucleotide sequence having 95% or more homology with the nucleotide sequence of SEQ ID NO: 5, GenBank Accession No. NM_001250, SEQ ID NO: 7, or GenBank Accession No. XM_011766922 and that contains a DNA encoding a polypeptide having the function of CD40, a gene that is composed of a DNA which hybridizes with a DNA composed of the nucleotide sequence represented by SEQ ID NO: 5, GenBank Accession No. NM_001250, SEQ ID NO: 7, or GenBank Accession No. XM_011766922 under stringent conditions, and that contains a DNA encoding a polypeptide having the function of CD40, and the like are also included in the gene encoding the CD40 of the present invention.

The DNA which hybridizes under stringent conditions means, for example, a hybridizable DNA obtained by a colony hybridization method, a plaque hybridization method, a southern blot hybridization method, a DNA microarray method, or the like using a DNA having the nucleotide sequence represented by SEQ ID NO: 5, GenBank Accession No. NM_001250, SEQ ID NO: 7, or GenBank Accession No. XM_011766922 as a probe. Specifically, a DNA that can be identified by performing hybridization at 65°C in the presence of 0.7 to 1.0 mol/L sodium chloride [Molecular Cloning, A Laboratory Manual, Second Edition, Cold Spring Harbor Laboratory Press (1989), Current Protocols in Molecular Biology, John Wiley & Sons (1987-1997), DNA Cloning 1: Core Techniques, A Practical Approach, Second Edition, Oxford University (1995)] using a filter or a microscope slide on which a DNA derived from a hybridized colony or plaque, or a PCR product or an oligo DNA having the nucleotide sequence is immobilized, and then washing the filter or the microscope slide under the condition of 65°C using an SSC solution having a concentration of 0.1 to 2 times (a composition of the SSC solution having a concentration of 1 time is composed of 150 mmol/L sodium chloride and 15 mmol/L sodium citrate) can be exemplified. As the hybridizable DNA, for example, a DNA preferably having 60% or more homology, more preferably a DNA having 80% or more homology, and further more preferably a DNA having 95% or more homology with the nucleotide sequence of SEQ ID NO: 5, GenBank Accession No. NM_001250, SEQ ID NO: 7, or GenBank Accession No. XM_011766922 can be exemplified.

A gene polymorphism is often observed in a nucleotide sequence of a gene encoding a protein of a eukaryote. A gene in which a small-scale mutation has occurred in a nucleotide sequence due to such a polymorphism among genes used in the present invention is also included in the gene encoding the CD40 of the present invention.

The value of homology in the present invention may be a value calculated using a homology search program known to those skilled in the art unless otherwise particularly specified, however, with respect to a nucleotide sequence, a value calculated using a default parameter in BLAST [J. Mol. Biol., 215, 403 (1990)], and the like are exemplified, and with respect to an amino acid sequence, a value calculated using a default parameter in BLAST 2 [Nucleic Acids Research, 25, 3389 (1997), Genome Research, 7, 649 (1997), http://www.ncbi.nlm.nih.gov/Education/BLASTinfo/information3.html], and the like are exemplified.

As for the default parameters, G (Cost to open gap) is 5 in the case of a nucleotide sequence and 11 in the case of an amino acid sequence, -E (Cost to extend gap) is 2 in the case of a nucleotide sequence and 1 in the case of an amino acid sequence, -q (Penalty for nucleotide mismatch) is -3, -r (reward for nucleotide match) is 1, -e (expect value) is 10, -W (wordsize) is 11 residues in the case of a nucleotide sequence and 3 residues in the case of an amino acid sequence, -y [Dropoff (X) for blast extensions in bits] is 20 in the case of blastn and 7 in the case of programs other than blastn, -X (X dropoff value for gapped alignment in bits) is 15, and -Z (final X dropoff value for gapped alignment in bits) is 50 in the case of blastn and 25 in the case of programs other than blastn (http://www.ncbi.nlm.nih.gov/blast/html/blastcgihelp.html).

A polypeptide composed of a partial sequence of the amino acid sequence of CD40 can be produced by a method known to those skilled in the art, and can be produced by, for example, deleting part of the DNA encoding the amino acid sequence of SEQ ID NO: 6, GenBank Accession No. NP_001241, GenBank Accession No. XP_005569274, or SEQ ID NO: 8 and culturing a transformant transfected with an expression vector including the resulting DNA. In addition, for example, a polypeptide having an amino acid sequence in which one or more amino acids are deleted, substituted, or added in the partial sequence of the amino acid sequence of SEQ ID NO: 6, GenBank Accession No. NP_001241, GenBank Accession No. XP_005569274, or SEQ ID NO: 8 can be obtained by the same method as described above based on the polypeptide or the DNA produced by the above method. Further, a polypeptide composed of the partial sequence of the amino acid sequence of CD40, or a polypeptide having an amino acid sequence in which one or more amino acids are deleted, substituted, or added in the partial sequence of the amino acid sequence of CD40 can also be produced using a chemical synthesis method such as a fluorenylmethyloxycarbonyl (Fmoc) method or a t-butyloxycarbonyl (tBoc) method.

As an extracellular domain of the CD40 in the present invention, for example, a region in which the amino acid sequence of human CD40 represented by GenBank Accession No. NP_001241 is predicted using a known transmembrane region prediction program SOSUI (http://sosui.proteome.bio.tuat.ac.jp/sosuiframe0.html), TMHMM ver. 2 (http://www.cbs.dtu.dk/services/TMHMM-2.0/), ExPASy Proteomics Server (http://Ca.expasy.org/), or the like is exemplified. Specifically, the amino acid sequence of positions 21 to 194 of SEQ ID NO: 6 or GenBank Accession No. NP_001241, and the amino acid sequence of SEQ ID NO: 2 are exemplified.

Examples of the function of CD40 include induction of CD40 signaling when a CD40 ligand or agonist binds it to cause various effects. For example, when CD40 signaling is induced in a cancer cell, cell death or growth inhibition of the cancer cell, or the like is caused. When CD40 signaling is induced in a B lymphocyte, for example, activation of the B lymphocyte, promotion of expression of CD95, class switch recombination, somatic hypermutation, or the like is caused to induce production of an antibody with high antigen affinity or the like. When CD40 signaling is induced in a dendritic cell, for example, maturation of the dendritic cell or production of IL-12 is caused. When CD40 signaling is induced in a macrophage, for example, reduction in a surface marker of an M2 macrophage, induction of expression of a surface marker of an M1 macrophage, or pro-inflammatory cytokine production is caused.

The CD40 signaling refers to signaling transmitted into cells through CD40.

The FAP in the present invention is used synonymously with FAPA, SIMP, DPPIV, FAPalpha, and Separase.

As the FAP, for example, human FAP containing the amino acid sequence represented by GenBank Accession No. NP_004451 or SEQ ID NO: 14, mouse FAP containing the amino acid sequence represented by GenBank Accession No. NP_032012 or SEQ ID NO: 16, and the like are exemplified. Further, for example, a polypeptide that is composed of an amino acid sequence in which one or more amino acids are deleted, substituted, or added in the amino acid sequence represented by SEQ ID NO: 14 or 16, GenBank Accession No. NP_004451, or GenBank Accession No. NP_032012, and that has the function of FAP is exemplified.

A polypeptide containing an amino acid sequence having preferably 70% or more, more preferably 80% or more, and further more preferably 90% or more homology with the amino acid sequence of SEQ ID NO: 14 or 16, GenBank Accession No. NP_004451, or GenBank Accession No. NP_032012, and most preferably, a polypeptide that is composed of an amino acid sequence having 95%, 96%, 97%, 98%, and 99% or more homology and that has the function of FAP are also included in the FAP of the present invention.

The polypeptide having an amino acid sequence in which one or more amino acid residues are deleted, substituted, or added in the amino acid sequence of SEQ ID NO: 14 or 16, GenBank Accession No. NP_004451, or GenBank Accession No. NP_032012 can be obtained by, for example, introducing a site-specific mutation into a DNA encoding the amino acid sequence of SEQ ID NO: 14 or 16, GenBank Accession No. NP_004451, or GenBank Accession No. NP_032012 using the above-mentioned site-specific mutagenesis method, or the like. The number of amino acids to be deleted, substituted, or added is not particularly limited, but is preferably one to several tens, for example, 1 to 20, and more preferably one to several, for example, 1 to 5 amino acids.

As a gene encoding the FAP in the present invention, for example, a gene of human FAP containing the nucleotide sequence of GenBank Accession No. NM_004460 or SEQ ID NO: 13 or a gene of mouse FAP containing the nucleotide sequence of GenBank Accession No. NM_007986 or SEQ ID NO: 15 is exemplified.

Further, for example, a gene that is composed of a nucleotide sequence in which one or more nucleotides are deleted, substituted, or added in the nucleotide sequence of SEQ ID NO: 13 or 15, GenBank Accession No. NM_004460, or GenBank Accession No. NM_007986, and that contains a DNA encoding a polypeptide having the function of FAP, a gene that is composed of a nucleotide sequence having 60% or more homology, preferably a nucleotide sequence having 80% or more homology, and more preferably a nucleotide sequence having 95% or more homology with the nucleotide sequence of SEQ ID NO: 13 or 15, GenBank Accession No. NM_004460, or GenBank Accession No. NM_007986, and that contains a DNA encoding a polypeptide having the function of FAP, a gene that is composed of a DNA which hybridizes with a DNA containing the nucleotide sequence of SEQ ID NO: 13 or 15, GenBank Accession No. NM_004460, or GenBank Accession No. NM_007986 under stringent conditions, and that contains a DNA encoding a polypeptide having the function of FAP, and the like are also included in the gene encoding the FAP of the present invention.

As an extracellular domain of the FAP in the present invention, for example, a region in which the amino acid sequence of human FAP of GenBank Accession No. NP_004451 is predicted using a known transmembrane region prediction program SOSUI (http://sosui.proteome.bio.tuat.ac.jp/sosuiframe0.html), TMHMM ver. 2 (http://www.cbs.dtu.dk/services/TMHMM-2.0/), ExPASy Proteomics Server (http://Ca.expasy.org/), or the like can be exemplified. Specifically, the amino acid sequence of positions 27 to 760 of SEQ ID NO: 14 or GenBank Accession No. NP_004451, and the amino acid sequence of SEQ ID NO: 10 are exemplified.

As the function of FAP, for example, reconstruction of an extracellular matrix caused by the protease activity of the molecule, and the like are exemplified.

An antibody is a protein derived from a gene (referred to as "antibody gene") encoding all or part of a heavy chain variable region, a heavy chain constant region, a light chain variable region, and a light chain constant region constituting an immunoglobulin. The antibody of the present invention also includes an antibody or an antibody fragment having any immunoglobulin class and subclass.

The heavy chain (H chain) refers to a polypeptide having a higher molecular weight of the two types of polypeptides constituting an immunoglobulin molecule. The heavy chain determines the antibody class and subclass. IgA, IgD, IgE, IgG, and IgM include an α chain, a δ chain, an ε chain, a γ chain, and a µ chain as the heavy chain, respectively, and the heavy chain constant region is characterized by a different amino acid sequence. The light chain (L chain) refers to a polypeptide having a lower molecular weight of the two types of polypeptides constituting an immunoglobulin molecule. In the case of a human antibody, there exist two types, a κ chain and a λ chain, in the light chain.

The variable region (V region) generally refers to a region that is present in an amino acid sequence at the N-terminal side of an immunoglobulin and is rich in diversity. Because a part other than the variable region has a structure with less diversity, it is called a constant region (C region). The respective variable regions of the heavy chain and the light chain are associated to form an antigen binding site and determine the binding property of the antibody to the antigen. Hereinafter, the heavy chain variable region or the light chain variable region is abbreviated as VH or VL.

The antigen binding site is a site that recognizes and binds to an antigen in an antibody, and refers to a site that forms a complementary conformation with an antigenic determinant (epitope). At the antigen binding site, a strong intermolecular interaction occurs between the antigenic determinants. The antigen binding site is constituted by VH and VL including at least three complementarity determining regions (CDRs). In the case of a human antibody, VH and VL each include three CDRs. These CDRs are referred to as CDR1, CDR2, and CDR3, respectively, in order from the N-terminal side.

In the constant region, the heavy chain constant region and the light chain constant region are denoted as CH and CL, respectively. The CH is classified into an α chain, a δ chain, an ε chain, a γ chain, and a µ chain which are subclasses of the heavy chain. The CH is constituted by a CH1 domain, a hinge domain, a CH2 domain, and a CH3 domain arranged in order from the N-terminal side, and the CH2 domain and the CH3 domain together are called an Fc region. On the other hand, the CL is classified into two subclasses called a Cλ chain and a Cκ chain.

In the case of human IgG, in the heavy chain, a variable region corresponds to the amino acid sequence at positions 1 to 117 numbered according to the EU index of Kabat et al. (Kabat et al., Sequences of proteins of immunological interest, 1991 Fifth edition), and a constant region corresponds to the amino acid sequence downstream of position 118. In the light chain of a human antibody, the amino acid sequence at positions 1 to 107 numbered according to Kabat et al. (Kabat numbering) corresponds to a variable region, and the amino acid sequence downstream of position 108 corresponds to a constant region.

In the case of human IgG, CH1 refers to a region having the amino acid sequence at positions 118 to 215 indicated by the EU index. Similarly, CH2 refers to a region having the amino acid sequence at positions 231 to 340 indicated by the EU index of Kabat et al., and CH3 refers to a region having the amino acid sequence at positions 341 to 447 indicated by the EU index of Kabat et al. Between CH1 and CH2, an amino acid region rich in flexibility called a hinge region (hereinafter sometimes referred to as a hinge) is present. The hinge region refers to a region having the amino acid sequence at positions 216 to 230 indicated by the EU index of Kabat et al.

The CL refers to a region having the amino acid sequence at positions 108 to 214 indicated by Kabat numbering in the case of the κ chain of a human antibody, and refers to a region having the amino acid sequence at positions 108 to 215 in the case of the λ chain.

A monoclonal antibody is an antibody secreted by an antibody-producing cell maintaining monoclonality, and recognizes a single epitope (also referred to as an antigenic determinant). The monoclonal antibody molecules have the same amino acid sequence (primary structure) and have a single structure. A polyclonal antibody refers to a population of antibody molecules secreted by antibody-producing cells of different clones. An oligoclonal antibody refers to a population of antibody molecules in which multiple different monoclonal antibodies are mixed.

The epitope refers to a structural part of an antigen that an antibody recognizes and binds to. Examples of the epitope include a single amino acid sequence, a conformation composed of an amino acid sequence, an amino acid sequence to which a sugar chain is bound, and a conformation composed of an amino acid sequence to which a sugar chain is bound, and the like, each of which a monoclonal antibody recognizes and binds to.

Examples of the monoclonal antibody in the present invention can include an antibody produced by a hybridoma, and a genetically recombinant antibody produced by a transformant transformed with an expression vector comprising an antibody gene.

The hybridoma can be prepared by, for example, preparing an antigen, obtaining an antibody-producing cell having antigen specificity from an animal immunized with the antigen, and then fusing the antibody-producing cell with a myeloma cell. A desired monoclonal antibody can be obtained by culturing the hybridoma or by administering the hybridoma to an animal to convert the hybridoma into an ascites tumor, separating the culture solution or the ascites, followed by purification. As the animal to be immunized with the antigen, any animal can be used as long as it can produce a hybridoma, however, a mouse, a rat, a hamster, a rabbit, or the like is preferably used. In addition, the hybridoma can also be produced by obtaining a cell having an antibody-producing ability from such an immunized animal, subjecting the cell to in vitro immunization, and then fusing the cell with a myeloma cell.

Examples of the genetically recombinant antibody in the present invention include antibodies produced using a gene recombinant technique such as a recombinant mouse antibody, a recombinant rat antibody, a recombinant hamster antibody, a recombinant rabbit antibody, a human chimeric antibody (also referred to as a chimeric antibody), a humanized antibody (also referred to as a CDR-grafted antibody), and a human antibody. In the genetically recombinant antibody, it is possible to determine which animal species the heavy chain and the light chain variable regions and constant regions derived from are applied according to the animal species to be used as a target and the purpose. For example, when the animal species to be used as a target is a human, as the variable region, one derived from a human or a non-human animal such as a mouse can be adopted, and as the constant region and the linker, those derived from a human can be adopted.

The chimeric antibody refers to an antibody composed of VH and VL of an antibody of an animal other than a human (non-human animal) and CH and CL of a human antibody. As the non-human animal, any animal such as a mouse, a rat, a hamster, or a rabbit can be used as long as it can produce a hybridoma. The chimeric antibody can be produced by obtaining cDNAs encoding VH and VL from a hybridoma derived from a non-human animal that produces a monoclonal antibody, inserting each of the cDNAs into an expression vector for an animal cell having DNAs encoding CH and CL of a human antibody, thereby constructing a chimeric antibody expression vector, and then introducing the vector into an animal cell to cause expression.

The humanized antibody refers to an antibody in which CDRs of VH and VL of a non-human animal antibody are grafted in the corresponding CDRs of VH and VL of a human antibody. A region other than the CDRs of VH and VL is referred to as a framework region (hereinafter referred to as FR). The humanized antibody can be produced by constructing a cDNA encoding the amino acid sequence of VH composed of the amino acid sequence of CDR of VH of a non-human animal antibody and the amino acid sequence of FR of VH of an arbitrary human antibody, and a cDNA encoding the amino acid sequence of VL composed of the amino acid sequence of CDR of VL of a non-human animal antibody and the amino acid sequence of FR of VL of an arbitrary human antibody, inserting each of the cDNAs into an expression vector for an animal cell having DNAs encoding CH and CL of a human antibody, thereby constructing a humanized antibody expression vector, and then introducing the vector into an animal cell to cause expression.

The human antibody originally refers to an antibody that is naturally present in the human body, but also includes an antibody that is obtained from a human antibody phage library and a human antibody-producing transgenic animal, each of which is produced by recent advancement of genetic engineering, cellular engineering, or developmental engineering technology, and the like.

The antibody that is naturally present in the human body can be obtained by, for example, infecting human peripheral blood lymphocytes with an EB virus or the like so as to immortalize the lymphocytes, followed by cloning to culture a lymphocyte that produces the antibody, and then purifying the antibody from the culture supernatant.

The human antibody phage library is a library in which an antibody fragment such as Fab or scFv is expressed on phage surfaces by inserting an antibody gene prepared from a human B cell into a phage gene. It is possible to collect a phage having expressed an antibody fragment having a desired antigen binding activity on the surface from the library using a binding activity to a substrate on which an antigen is immobilized as an index. The antibody fragment can be further converted into a human antibody molecule consisting of two complete H chains and two complete L chains using a genetic engineering technique.

The human antibody-producing transgenic animal means an animal in which a human antibody gene is incorporated into a cell. Specifically, for example, a human antibody-producing transgenic mouse can be produced by introducing a human antibody gene into a mouse ES cell, implanting the ES cell to a mouse early embryo, and then allowing the embryo to develop into an individual. A human antibody derived from the human antibody-producing transgenic animal can be prepared by obtaining a hybridoma using a conventional hybridoma production method that is performed for a non-human animal, and culturing the hybridoma to produce and accumulate the antibody in the culture supernatant.

In the present invention, the anti-CD40 antibody refers to a monoclonal antibody that specifically recognizes and binds to the extracellular domain of CD40. In addition, in the present invention, the anti-FAP antibody refers to a monoclonal antibody that specifically recognizes and binds to the extracellular domain of FAP.

In the present invention, the bispecific antibody refers to an antibody that specifically binds to each of two types of epitopes. The bispecific antibody of the present invention comprises an IgG portion comprising a first antigen binding domain and also comprises a second antigen binding domain, and has a structure in which the C terminus of a heavy chain of the IgG portion binds to the second antigen binding domain either directly or via a linker.

The IgG portion in the present invention refers to a partial structure of the bispecific antibody composed of IgG or IgG with a modified Fc moiety, and has a heterotetramer structure obtained by assembling two heterodimers composed of one light chain and one heavy chain.

The heavy chain constant region of the IgG may be in any subclass such as IgG1, IgG2, IgG3, or IgG4. Further, part of the amino acid sequence thereof may be deleted, added, substituted, and/or inserted. In addition, all or part of the fragments of the amino acid sequence composed of CH1, a hinge, CH2, and CH3 of the heavy chain of IgG can be appropriately combined and used. Further, the amino acid sequences thereof can also be used by partially deleting or changing the order. In addition, the subclass of IgG used for the IgG portion is not particularly limited, but is preferably IgG4, or an IgG4 mutant obtained by substituting a Ser residue at position 228 in the heavy chain constant region of IgG4 with Pro, and a Leu residue at position 235 therein with Asn (hereinafter referred to as IgG4PE), or an IgG4 mutant obtained by substituting a Ser residue at position 228 in the heavy chain constant region of IgG4 with Pro, a Leu residue at position 235 therein with Asn, and an Arg residue at position 409 therein with Lys (hereinafter referred to as IgG4PE R409K).

For example, an IgG portion in which the heavy chain constant region (CH1-hinge-CH2-CH3 in this order from the N-terminal side) is composed of IgG4PE R409K comprising the amino acid sequence of SEQ ID NO: 61 is preferred.

The antigen binding domain in the present invention is a partial structure having a function of specifically recognizing and binding to an antigen. As the antigen binding domain, for example, a recombinant protein or a polypeptide utilizing a protein having a binding ability to an antigen such as an antibody or an antibody fragment thereof, a ligand or a receptor is exemplified.

In the present invention, the binding of a recombinant protein, a polypeptide, an antibody or an antibody fragment thereof, a bispecific antibody, or the like to any one of CD40 and FAP can be confirmed by measuring the binding affinity between a cell having expressed CD40 or FAP and a recombinant protein, a polypeptide, an antibody or an antibody fragment thereof, or a bispecific antibody using, for example, a known immunological detection method, preferably a fluorescent cell staining method, or the like. Further, it is also possible to use known immunological detection methods [Monoclonal Antibodies - Principles and Practice, Third Edition, Academic Press (1996), Antibodies - A Laboratory Manual, Cold Spring Harbor Laboratory (1988), Monoclonal Antibody Experimental Manual, Kodansha scientific books (1987)], and the like in combination.

In the bispecific antibody of the present invention, the first antigen binding domain is a partial structure constituting a part of the IgG portion. Further, the second antigen binding domain is a partial structure that binds to the heavy chain C-terminal side of the IgG portion either directly or via a linker. One of the first and second antigen binding domains binds to CD40, and the other binds to FAP. Among the bispecific antibodies of the present invention, an antibody in which the first antigen binding domain and the second antigen binding domain bind CD40 and FAP, respectively, is referred to as a CD40-FAP bispecific antibody. Further, an antibody in which the first antigen binding domain and the second antigen binding domain bind FAP and CD40, respectively, is referred to as a FAP-CD40 bispecific antibody.

The bispecific antibody of the present invention may be either a CD40-FAP bispecific antibody or a FAP-CD40 bispecific antibody, but is preferably a CD40-FAP bispecific antibody.

In the present invention, the antigen binding domain that binds to FAP may be any as long as it specifically recognizes and binds to FAP. The antigen binding domain that binds to FAP may be in any form: for example, an anti-FAP antibody, a ligand for FAP, or a naturally occurring interacting molecule which is a polypeptide, a protein molecule or a fragment thereof that can be produced by a gene recombination technique, a conjugate of the protein molecule with a low-molecular weight molecule or a natural product, or the like.

Specific examples of the antigen binding domain that binds to FAP include a polypeptide comprising CDR of an anti-FAP antibody, an antibody fragment of an anti-FAP antibody, a polypeptide comprising a variable region of an anti-FAP antibody, a polypeptide comprising a binding domain of a ligand that binds to FAP, and the like. As the antigen binding domain that binds to FAP, a polypeptide comprising a variable region of an anti-FAP antibody is preferred, and Fab of an anti-FAP antibody is more preferred. The Fab of an anti-FAP antibody is sometimes referred to as anti-FAP Fab.

In the present invention, the antigen binding domain that binds to CD40 may be any as long as it specifically recognizes and binds to CD40.

Specific examples of the antigen binding domain that binds to CD40 include a polypeptide comprising CDR of an anti-CD40 antibody, an antibody fragment of an anti-CD40 antibody, a polypeptide comprising a variable region of an anti-CD40 antibody, a polypeptide comprising a binding domain of a ligand that binds to CD40, and the like. As the antigen binding domain that binds to CD40, a polypeptide comprising a variable region of an anti-CD40 antibody is preferred, and Fab of an anti-CD40 antibody is more preferred. The Fab of an anti-CD40 antibody is sometimes referred to as anti-CD40 Fab.

In the present invention, as a specific example of the antigen binding domain that binds to CD40, one that is derived from a non-agonistic anti-CD40 antibody and specifically recognizes and binds to CD40 is also exemplified.

The non-agonistic anti-CD40 antibody refers to an antibody that specifically recognizes and binds to CD40, but does not have an agonistic activity.

Examples of the antigen binding domain that is derived from a non-agonistic anti-CD40 antibody and specifically binds to CD40 include a polypeptide comprising CDR of a non-agonistic anti-CD40 antibody, an antibody fragment of a non-agonistic anti-CD40 antibody, a polypeptide comprising a variable region of a non-agonistic anti-CD40 antibody, and the like.

When the non-agonistic anti-CD40 antibody is used as the antigen binding domain to CD40, a polypeptide comprising a variable region of the non-agonistic anti-CD40 antibody is preferred, and Fab of the non-agonistic anti-CD40 antibody is more preferred.

In the bispecific antibody of the present invention, when the second antigen binding domain is Fab, the heavy chain C terminus of the IgG portion may be bound to the heavy chain N terminus of the Fab either directly or via a linker or may be bound to the light chain N terminus, but is preferably bound to the heavy chain N terminus of the Fab.

In the bispecific antibody of the present invention, the C terminus of the heavy chain of the IgG portion and the second antigen binding domain may be bound directly or may be bound via a linker. In the present invention, the linker refers to a chemical structure for binding the C terminus of the heavy chain of the IgG portion to the second antigen binding domain, and is preferably a polypeptide. As the linker, for example, a single-stranded peptide is exemplified, and as a specific example, a single-stranded peptide having an amino acid sequence composed of ES, ESKYG, ESKYGPP, GGGGS, or a repetitive sequence of GGGGS is exemplified.

The bispecific antibody of the present invention has, for example, a structure shown in Fig. 1, that is, two polypeptide chains (also referred to as heavy chains of the bispecific antibody) in which VH1-CH1-hinge-CH2-CH3-VH2-CH1 are bound in this order from the N-terminal side, and four polypeptide chains (also referred to as light chains of the bispecific antibody) in which VL-CL are bound from the N-terminal side. Here, VH1 represents VH at the N-terminal side of the heavy chain, and VH2 represents VH at the C-terminal side thereof.

The two heavy chains of the bispecific antibody are associated with each other by linking cysteine residues in the hinge regions via a disulfide bond. The heavy chains and the light chains of the bispecific antibody are associated with each other by linking cysteine residues in CH1 and CL via a disulfide bond. The two heavy chains of the bispecific antibody may be the same or different, but are preferably the same. Further, the four light chains of the bispecific antibody may be mutually different or the same, but it is preferred that the four are all the same. In addition, CL may be either of a Cλ chain and a Cκ chain.

As the bispecific antibody of the present invention, for example, a bispecific antibody having a CD40 agonistic activity is exemplified. As the bispecific antibody of the present invention, a bispecific antibody that does not exhibit a CD40 agonistic activity in the absence of a FAP molecule or a cell that expresses FAP (also referred to as a FAP-expressing cell), but exhibits a CD40 agonistic activity only in the presence of a FAP molecule or a cell that expresses FAP is preferred. Such a bispecific antibody activates CD40 only in a lesion site such as a cancer or a lymph node in which a cell that expresses FAP is present, and therefore is preferred from the viewpoint that an adverse effect caused by systemic activation of CD40 does not occur.

In humans, examples of the cell that expresses FAP include fibroblasts in a local tumor area or a local lymph node area, tumor cells, and the like.

The CD40 agonistic activity of the bispecific antibody of the present invention refers to an activity of inducing activation of an antigen-presenting cell, an activity of inducing cell death of a tumor cell, or the like occurring by binding of the bispecific antibody to CD40 on a cell to cause signaling through the CD40.

In the present invention, whether or not an antibody or a bispecific antibody has the CD40 agonistic activity can be confirmed by, for example, evaluating an increase in the expression level of CD95, CD80, CD83, CD86, and/or HLA-ABC on a cell that expresses CD40 such as a B cell or a human Burkitt's lymphoma cell line Ramos cell (JCRB cell bank, Cell No: JCRB9119) in accordance with the method described in Lagresle et al., J Immunol 1995; 154: 5746-5756.

In the present invention, if the expression level of CD95, CD80, CD83, CD86, and/or HLA-ABC, or the like is increased as compared with the negative control when an antibody or a bispecific antibody binds to a cell that expresses CD40, it is determined that the antibody or the bispecific antibody has an agonistic activity. On the other hand, if the expression level of CD95, CD80, CD83, CD86, and/or HLA-ABC, or the like is not increased as compared with the negative control when an antibody or a bispecific antibody binds to a cell that expresses CD40, it is determined that the antibody or the bispecific antibody does not have an agonistic activity.

As the bispecific antibody of the present invention, specifically, a bispecific antibody that induces activation of an antigen-presenting cell which expresses CD40 and/or cell death of a tumor cell, when the bispecific antibody binds to FAP and CD40 in the presence of a cell that expresses FAP, or the like is exemplified.

In the present invention, the CD40 antagonistic activity refers to an activity of inhibiting activation of CD40 by a CD40 ligand or a CD40 agonist, or the like. For example, it refers to an activity of inhibiting signaling induction by binding of a CD40 ligand or a CD40 agonist to CD40, or the like.

An antibody that binds to CD40, but does not have a CD40 antagonistic activity is referred to as a non-antagonistic anti-CD40 antibody.

The CD40 antagonistic activity of an antibody can be confirmed by, for example, inhibition of induction of the expression of CD95 by a CD40 ligand against a cell that expresses CD40 such as a Ramos cell by adding the antibody.

The bispecific antibody of the present invention may bind to CD40 and FAP expressed on the same cell, or may bind to CD40 and FAP expressed on two different cells.

In the present invention, the number of binding domains to a certain antigen included in a single molecule of a bispecific antibody refers to a binding valence. For example, in the present invention, when a single molecule of a bispecific antibody has two antigen binding domains that bind to CD40 and two antigen binding domains that bind to FAP, the bispecific antibody can be said to divalently bind to each of CD40 and FAP.

The bispecific antibody of the present invention can be produced by a conventional production technique ([Nature Protocols, 9, 2450-2463 (2014)], WO 1998/050431, WO 2001/7734, WO 2002/002773, and WO 2009/131239) or the like.

The bispecific antibody of the present invention may have the second antigen binding domains at the C terminus of each of the two heavy chains included in the IgG portion, or may have one second antigen binding domain only at the C terminus of one heavy chain, but preferably have the second antigen binding domains at the C terminus of each of the two heavy chains. When the bispecific antibody has the second antigen binding domains at the C terminus of each of the two heavy chains, these may be the same or different, but are preferably the same second antigen binding domain.

A bispecific antibody, in which one or more of amino acid residues are deleted, added, substituted, or inserted in the amino acid sequence constituting the bispecific antibody of the present invention, and which has the same activity as that of the bispecific antibody described above, is also included in the bispecific antibody of the present invention.

The number of amino acids to be deleted, substituted, inserted, and/or added is one or more, and is not particularly limited, and is a number such that deletion, substitution, insertion, or addition can be carried out using a well-known technique such as a site-specific mutagenesis method described in Molecular Cloning, The Second Edition, Cold Spring Harbor Laboratory Press (1989), Current Protocols in Molecular Biology, John Willy & Sons (1987-1997), Nucleic Acids Research, 10, 6487 (1982), Proc. Natl. Acad. Sci., USA, 79, 6409 (1982), Gene, 34, 315 (1985), Nucleic Acids Research, 13, 4431 (1985), Proc. Natl. Acad. Sci USA, 82, 488 (1985), or the like. For example, it is generally one to several tens, preferably 1 to 20, more preferably 1 to 10, and further more preferably 1 to 5.

The above description that one or more of amino acid residues in the amino acid sequence of the bispecific antibody of the present invention are deleted, substituted, inserted, or added indicates as follows. The description means that there is a deletion, substitution, insertion, or addition of one or multiple amino acid residues in arbitrary one or multiple amino acid sequences in the same sequence. Further, such a deletion, substitution, insertion, or addition may sometimes occur simultaneously, and the amino acid residues to be substituted, inserted, or added may be either a natural type or an unnatural type.

Examples of the natural amino acid residue include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, L-cysteine, and the like.

Hereinafter, preferred examples of mutually substitutable amino acid residues are shown. Amino acid residues included in the same group can be mutually substituted.

group A: leucine, isoleucine, norleucine, valine, norvaline, alanine, 2-aminobutanoic acid, methionine, O-methylserine, t-butyl glycine, t-butyl alanine, and cyclohexylalanine
group B: aspartic acid, glutamic acid, isoaspartic acid, isoglutamic acid, 2-aminoadipic acid, and 2-aminosuberic acid
group C: asparagine and glutamine
group D: lysine, arginine, ornithine, 2,4-diaminobutanoic acid, and 2,3-diaminopropionic acid
group E: proline, 3-hydroxyproline, and 4-hydroxyproline
group F: serine, threonine, and homoserine
group G: phenylalanine and tyrosine

The bispecific antibody of the present invention also includes an antibody containing any amino acid residue subjected to a post-translational modification. Examples of the post-translational modification include a substitution of a glutamine residue at the N terminus of a polypeptide with pyroglutamate (pyroGlu), and the like [Beck et al, Analytical Chemistry, 85, 715-736 (2013)].

As the bispecific antibody of the present invention, for example, a bispecific antibody in which either one of the first antigen binding domain and the second antigen binding domain is anti-CD40 Fab derived from a non-agonistic anti-CD40 antibody, and a bispecific antibody in which either one of the first antigen binding domain and the second antigen binding domain is anti-FAP Fab are also exemplified.

Further, as the bispecific antibody of the present invention, for example, a bispecific antibody in which the first antigen binding domain is anti-CD40 Fab and the second antigen binding domain is anti-FAP Fab, and a bispecific antibody in which the first antigen binding domain is anti-FAP Fab and the second antigen binding domain is anti-CD40 Fab are exemplified.

Specific examples of the bispecific antibody of the present invention include bispecific antibodies described in the following (1) to (4), and the like:
(1) a bispecific antibody, in which the C terminus of a heavy chain of an IgG portion including anti-CD40 Fab derived from an anti-CD40 antibody is directly bound to anti-FAP Fab, and which binds to human CD40 and human FAP;
(2) a bispecific antibody, in which the C terminus of a heavy chain of an IgG portion including anti-FAP Fab is directly bound to anti-CD40 Fab derived from an anti-CD40 antibody, and which binds to human CD40 and human FAP;
(3) a bispecific antibody, in which the C terminus of a heavy chain of an IgG portion including anti-CD40 Fab derived from a non-agonistic anti-CD40 antibody is directly bound to anti-FAP Fab, and which binds to human CD40 and human FAP; and
(4) a bispecific antibody, in which the C terminus of a heavy chain of an IgG portion including anti-FAP Fab is directly bound to anti-CD40 Fab derived from a non-agonistic anti-CD40 antibody, and which binds to human CD40 and human FAP.

Here, a constant region (CH1-hinge-CH2-CH3 in this order from the N-terminal side) of the heavy chain of the IgG portion preferably contains the amino acid sequence of SEQ ID NO: 61. Further, the bispecific antibody or the bispecific antibody fragment thereof of the present invention is preferably the above (1) and (3).

Among the above bispecific antibodies (1) to (4), a bispecific antibody that comprises anti-CD40 Fab comprising VH comprising complementarity determining regions (CDRs) 1 to 3 comprising the amino acid sequences of SEQ ID NOS: 24 to 26, respectively, and VL comprising CDRs 1 to 3 comprising the amino acid sequences of SEQ ID NOS: 19 to 21, respectively, is preferred, and a bispecific antibody that comprises anti-CD40 Fab comprising VH comprising the amino acid sequence of SEQ ID NO: 23, and VL comprising the amino acid sequence of SEQ ID NO: 18 is more preferred.

Further, among the above bispecific antibodies (1) to (4), a bispecific antibody that comprises anti-FAP Fab comprising VH comprising heavy chain CDRs 1 to 3 depicted in any one selected from the following (i) to (v), preferably any one selected from (iii) to (v), and more preferably (iii), and VL comprising CDRs 1 to 3 comprising the amino acid sequences of SEQ ID NOS: 19 to 21, respectively:
(i) heavy chain CDRs 1 to 3 comprising the amino acid sequences of SEQ ID NOS: 33 to 35, respectively;
(ii) heavy chain CDRs 1 to 3 comprising the amino acid sequences of SEQ ID NOS: 38 to 40, respectively;
(iii) heavy chain CDRs 1 to 3 comprising the amino acid sequences of SEQ ID NOS: 43 to 45, respectively;
(iv) heavy chain CDRs 1 to 3 comprising the amino acid sequences of SEQ ID NOS: 48 to 50, respectively; and
(v) heavy chain CDRs 1 to 3 comprising the amino acid sequences of SEQ ID NOS: 53 to 55, respectively.

Further, among the above bispecific antibodies (1) to (4), a bispecific antibody that comprises anti-FAP Fab comprising VH comprising the amino acid sequence of any one selected from SEQ ID NOS: 32, 37, 42, 47, 52, 75, 77, 79, 81, and 83, preferably SEQ ID NOS: 42, 47, and 75, and more preferably the amino acid sequence of SEQ ID NO: 42 and VL comprising the amino acid sequence of SEQ ID NO: 18 is more preferred.

Among the above bispecific antibodies (1) to (4), a bispecific antibody that comprises anti-CD40 Fab comprising VH comprising CDRs 1 to 3 comprising the amino acid sequences of SEQ ID NOS: 24 to 26, respectively, and VL comprising CDRs 1 to 3 comprising the amino acid sequences of SEQ ID NOS: 19 to 21, respectively, and that comprises anti-FAP Fab comprising VH comprising heavy chain CDRs 1 to 3 depicted in any one selected from the following (i) to (v), preferably any one selected from (iii) to (v), and more preferably (iii), and VL comprising CDRs 1 to 3 comprising the amino acid sequences of SEQ ID NOS: 19 to 21, respectively is more preferred:
(i) heavy chain CDRs 1 to 3 comprising the amino acid sequences of SEQ ID NOS: 33 to 35, respectively;
(ii) heavy chain CDRs 1 to 3 comprising the amino acid sequences of SEQ ID NOS: 38 to 40, respectively;
(iii) heavy chain CDRs 1 to 3 comprising the amino acid sequences of SEQ ID NOS: 43 to 45, respectively;
(iv) heavy chain CDRs 1 to 3 comprising the amino acid sequences of SEQ ID NOS: 48 to 50, respectively; and
(v) heavy chain CDRs 1 to 3 comprising the amino acid sequences of SEQ ID NOS: 53 to 55, respectively.

Among the above bispecific antibodies (1) to (4), a bispecific antibody that comprises anti-CD40 Fab comprising VH comprising the amino acid sequence of SEQ ID NO: 23 and VL comprising the amino acid sequence of SEQ ID NO: 18, and that comprises anti-FAP Fab comprising VH comprising the amino acid sequence of any one selected from SEQ ID NOS: 32, 37, 42, 47, 52, 75, 77, 79, 81, and 83, preferably SEQ ID NOS: 42, 47, and 75, and more preferably the amino acid sequence of SEQ ID NO: 42 and VL comprising the amino acid sequence of SEQ ID NO: 18 is further more preferred.

Further, as the bispecific antibody of the present invention, a bispecific antibody consisting of two heavy chains comprising the amino acid sequence of any one selected from SEQ ID NOS: 65, 67, 69, 71, 73, 85, 87, 89, 91, 93, 95, 97, 99, 101, 103, 105, 107, 109, and 111, preferably SEQ ID NOS: 69, 71, and 85, and more preferably the amino acid sequence of SEQ ID NO: 69 and four light chains comprising VL comprising the amino acid sequence of SEQ ID NO: 18 is exemplified.

Further, the bispecific antibody of the present invention also includes a bispecific antibody composed of a heavy chain comprising an amino acid sequence having at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, or at least 99% homology with the amino acid sequence of any one selected from SEQ ID NOS: 65, 67, 69, 71, 73, 85, 87, 89, 91, 93, 95, 97, 99, 101, 103, 105, 107, 109, and 111, and four light chains comprising an amino acid sequence having at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, or at least 99% homology with the amino acid sequence of SEQ ID NO: 18.

Examples of the bispecific antibody of the present invention include Ct-R1090-Fa018, Ct-R1090-Fa1007, Ct-R1090-Fa1013, Ct-R1090-Fa1014, Ct-R1090-Fa1046, Ct-R1090-Fa1046-GTS, Ct-R1090-Fa1046-FTS, Ct-R1090-Fa1046-KTS, Ct-R1090-Fa1046-WTS, Ct-R1090-Fa1046-TTS, Ct-Fa018-R1090, Ct-Fa1007-R1090, Ct-Fa1013-R1090, Ct-Fa1014-R1090, Ct-Fa1046-GTS-R1090, Ct-Fa1046-FTS-R1090, Ct-Fa1046-KTS-R1090, Ct-Fa1046-WTS-R1090, and Ct-Fa1046-TTS-R1090. Among these, Ct-R1090-Fa1013, Ct-R1090-Fa1014, and Ct-R1090-Fa1046-GTS are preferred, and Ct-R1090-Fa1013 is more preferred.

The bispecific antibody of the present invention also includes an antibody having an effector activity.

The effector activity refers to an antibody-dependent cellular cytotoxicity activity that is caused via the Fc region of the antibody, and examples thereof include an antibody-dependent cellular cytotoxicity activity (ADCC activity), a complement-dependent cytotoxicity activity (CDC activity), an antibody-dependent cellular phagocytosis activity (ADCP activity) that is caused by phagocytes such as macrophages or dendritic cells, an opsonin effect, and the like.

In the present invention, the ADCC activity and the CDC activity can be measured using a known measurement method [Cancer Immunol. Immunother., 36, 373 (1993)].

The ADCC activity refers to an activity that activates an immune cell (a natural killer cell or the like) when an antibody having bound to an antigen on a target cell binds to an Fc receptor of the immune cell via the Fc region of the antibody so as to damage the target cell.

The Fc receptor (FcR) is a receptor that binds to the Fc region of the antibody, and induces various effector activities by binding of the antibody. Each FcR corresponds to the subclass of an antibody, and IgG, IgE, IgA, and IgM bind specifically to FcγR, FcεR, FcαR, and FcµR, respectively. Further, in the FcγR, there are subtypes of FcyRI (CD64), FcγRII (CD32), and FcyRIII (CD16), and the subtypes have isoforms of FcyRIA, FcyRIB, FcγRIC, FcyRIIA, FcyRIIB, FcyRIIC, FcyRIIIA, and FcyRIIIB, respectively. The different types of FcyRs are present on different cells [Annu. Rev. Immunol. 9: 457-492 (1991)]. In humans, FcyRIIIB is expressed specifically in neutrophils, and FcγRIIIA is expressed in monocytes, natural killer cells (NK cells), macrophages, and some T cells. An NK cell-dependent ADCC activity is induced through the binding of the antibody to FcγRIIIA.

The CDC activity refers to an activity that activates a series of cascades (complement activation pathways) composed of complement-related protein groups in the blood by an antibody having bound to an antigen on a target cell so as to damage the target cell. In addition, a protein fragment generated by the activation of the complement induces the migration and activation of an immune cell. The cascade of CDC activity starts when C1q first binds to the Fc region, and subsequently binds to C1r and C1s that are two serine proteases so as to form a C1 complex.

The CDC activity or the ADCC activity of the bispecific antibody of the present invention against an antigen-expressing cell can be evaluated by a known measurement method [Cancer Immunol. Immunother., 36, 373 (1993)].

As a method for controlling the effector activity of the bispecific antibody of the present invention, a method for controlling the amount of fucose (also referred to as core fucose) that is α-1,6-linked to N-acetylglucosamine (GlcNAc) present at the reducing end of an N-linked complex sugar chain that binds to asparagine (Asn) at position 297 of the Fc region (a constant region composed of CH2 and CH3 domains) of the IgG antibody portion (WO 2005/035586, WO 2002/31140, and WO 00/61739), a method for controlling by modifying an amino acid residue of the Fc region of the antibody (WO 00/42072), and the like are known.

The ADCC activity of the antibody can be increased or decreased by controlling the amount of fucose to be added to the bispecific antibody. For example, as a method for decreasing the content of fucose that binds to the N-linked complex sugar chain bound to Fc of the antibody, by expressing the bispecific antibody using an α1,6-fucosyltransferase gene-deficient host cell, the bispecific antibody having high ADCC can be obtained. On the other hand, as a method for increasing the content of fucose that binds to the N-linked complex sugar chain bound to Fc of the bispecific antibody, by expressing the antibody using a host cell transfected with an α1,6-fucosyltransferase gene, the bispecific antibody having a low ADCC activity can be obtained.

In addition, the ADCC activity or the CDC activity can be increased or decreased by modifying an amino acid residue in the Fc region of the bispecific antibody. For example, by using the amino acid sequence of the Fc region described in US Patent Application Publication No. 2007/0148165, the CDC activity of the bispecific antibody can be increased. Further, by performing an amino acid modification described in US Patent No. 6,737,056, US Patent No. 7,297,775, US Patent No. 7,317,091, or the like, the ADCC activity or the CDC activity can be increased or decreased.

Further, a bispecific antibody in which the effector activity is controlled may be obtained by combining the above-mentioned methods.

The stability of the bispecific antibody of the present invention can be evaluated by measuring the amount of an aggregate (oligomer) formed in a sample stored during a purification process or under certain conditions. In the case of the bispecific antibody of the present invention, when the amount of the aggregate decreases under the same conditions as compared with that of the control, it is evaluated that the bispecific antibody of the present invention has higher stability than the control. The amount of the aggregate can be measured by separating an aggregated antibody and a non-aggregated antibody using appropriate chromatography including gel filtration chromatography.

The productivity of a cell that produces the bispecific antibody of the present invention can be evaluated by measuring the amount of an antibody produced from the antibody-producing cell in a culture solution. More specifically, the productivity can be evaluated by measuring the amount of the antibody contained in a culture supernatant obtained by removing the producing cell from the culture solution using an appropriate method such as an HPLC method or an ELISA method.

In the present invention, the antibody fragment is an immunoglobulin fragment and is a protein that includes an antigen binding site and has an antigen binding activity to the antigen. For example, Fab, Fab', F(ab')₂, scFv, a diabody, dsFv, VHH, a peptide including CDR, or the like is exemplified.

The Fab is an antibody fragment, which has a molecular weight of about 50,000 and has an antigen binding activity, and in which about a half of an H chain at the N-terminal side and the entire L chain are bound via a disulfide bond (S-S bond) among the fragments obtained by treating an IgG antibody with a protease papain (cleaved at an amino acid residue at position 224 in the H chain). The H chain of Fab including VH and CH1 is referred to as VH-CH1. Further, the L chain of Fab including VL and CL is referred to as VL-CL. The Fab derived from an anti-CD40 antibody is referred to as anti-CD40 Fab, and the Fab derived from an anti-FAP antibody is referred to as anti-FAP Fab.

The F(ab')₂ is an antibody fragment, which has a molecular weight of about 100,000 and has an antigen binding activity, and is slightly larger than a molecule obtained by binding Fabs via an S-S bond in the hinge region among the fragments obtained by treating IgG with a protease pepsin (cleaved at an amino acid residue at position 234 in the H chain).

The Fab' is an antibody fragment, which has a molecular weight of about 50,000 and has an antigen binding activity, and in which an S-S bond in the hinge region of the above F(ab')₂ is cleaved.

The scFv is a VH-P-VL or VL-P-VH polypeptide in which one VH and one VL are linked using an appropriate peptide linker (P) of 12 or more residues, and is an antibody fragment having an antigen binding activity.

The diabody is an antibody fragment in which scFvs having the same or different antigen binding specificity form a dimer, and is an antibody fragment having a divalent antigen binding activity to the same antigen or antigen binding activities each specific for different antigens.

The dsFv refers to a molecule obtained by binding polypeptides in which one amino acid residue in each of VH and VL is substituted with a cysteine residue via an S-S bond between the cysteine residues.

The VHH (also referred to as a nanobody) refers to a heavy chain variable region in a variable domain of heavy chain of heavy chain antibody (VHH antibody), and can bind to an antigen without the presence of another polypeptide.

The VHH antibody is an antibody present in an animal of the family Camelidae such as an alpaca and an elasmobranch such as a shark, and does not include a light chain or CH1, and is composed only of a heavy chain.

The peptide comprising CDR is configured to comprise at least one region of CDRs of VH or VL. A peptide comprising multiple CDRs can be produced by binding CDRs either directly or via an appropriate peptide linker. The peptide comprising CDR can be produced by constructing DNAs encoding CDRs of VH and VL of the bispecific antibody of the present invention, inserting the DNAs into an expression vector for a prokaryote or an expression vector for a eukaryote, and then introducing the expression vector into a prokaryote or a eukaryote to cause expression. In addition, the peptide comprising CDR can also be produced by a chemical synthesis method such as an Fmoc method or a tBoc method.

An Fc region that includes an amino acid residue modification aiming at enhancing or eliminating the effector activity of the antibody, stabilizing the antibody, and controlling the blood half-life can also be used for the bispecific antibody of the present invention.

The bispecific antibody of the present invention includes a derivative of the bispecific antibody in which a radioisotope, a low-molecular weight drug, a high-molecular weight drug, a protein, an antibody drug, or the like is bound chemically or in a genetic engineering manner to the bispecific antibody of the present invention.

The derivative of the bispecific antibody in the present invention can be produced by binding a radioisotope, a low-molecular weight drug, a high-molecular weight drug, an immunostimulant, a protein, an antibody drug, or the like to the N-terminal side or the C-terminal side of an H chain or an L chain of the bispecific antibody of the present invention, a sugar chain in the bispecific antibody, or the like using a chemical method [Introduction to Antibody Engineering, Chijin Shokan Co., Ltd. (1994)].

Further, the derivative of the bispecific antibody in the present invention can be produced by a genetic engineering technique in which a DNA encoding the bispecific antibody of the present invention is ligated to a DNA encoding a desired protein or antibody drug, the resultant is inserted into an expression vector, and the expression vector is introduced into an appropriate host cell to cause expression.

Examples of the radioisotope include ¹¹¹In, ¹³¹I, ¹²⁵I, ⁹⁰Y, ⁶⁴Cu, ⁹⁹Tc, ⁷⁷Lu, ²¹¹At, and the like. The radioisotope can be directly bound to the antibody by a chloramine T method or the like. In addition, a substance that chelates the radioisotope may be bound to the antibody. Examples of the chelating agent include 1-isothiocyanatobenzyl-3-methyldiethylenetriaminepentaacetic acid (MX-DTPA) and the like.

Examples of the low-molecular weight drug include anticancer agents such as an alkylating agent, a nitrosourea agent, an antimetabolite, an antibiotic, a plant alkaloid, a topoisomerase inhibitor, a hormonal therapy agent, a hormone antagonist, an aromatase inhibitor, a P-glycoprotein inhibitor, a platinum complex derivative, an M-phase inhibitor, or a kinase inhibitor [Clinical oncology, Japanese Journal of Cancer and Chemotherapy (1996)], anti-inflammatory agents such as a steroidal agent such as hydrocortisone or prednisone, a nonsteroidal agent such as aspirin or indomethacin, an immunomodulatory agent such as gold thiomalate or penicillamine, an immunosuppressive agent such as cyclophosphamide or azathioprine, an antihistamine agent such as chlorpheniramine maleate or clemastine [Inflammation and anti-inflammatory therapy, Ishiyaku Publishers, Inc. (1982)], and the like.

Examples of the anticancer agent include amifostine (Ethyol), cisplatin, dacarbazine (DTIC), dactinomycin, mechlorethamine (nitrogen mustard), streptozocin, cyclophosphamide, ifosfamide, carmustine (BCNU), lomustine (CCNU), doxorubicin (Adriamycin), epirubicin, gemcitabine (Gemzar), daunorubicin, procarbazine, mitomycin, cytarabine, etoposide, 5-fluorouracil, fluorouracil, vinblastine, vincristine, bleomycin, daunomycin, peplomycin, estramustine, paclitaxel (Taxol), docetaxel (Taxotere), aldesleukin, asparaginase, busulfan, carboplatin, oxaliplatin, nedaplatin, cladribine, camptothecin, 10-hydroxy-7-ethyl-camptothecin (SN38), floxuridine, fludarabine, hydroxyurea, idarubicin, mesna, irinotecan (CPT-11), nogitecan, mitoxantrone, topotecan, leuprolide, megestrol, melphalan, mercaptopurine, hydroxycarbamide, plicamycin, mitotane, pegaspargase, pentostatin, pipobroman, streptozocin, tamoxifen, goserelin, leuprorelin, flutamide, teniposide, testolactone, thioguanine, thiotepa, uracil mustard, vinorelbine, chlorambucil, hydrocortisone, prednisolone, methylprednisolone, vindesine, nimustine, semustine, capecitabine, Tomudex, azacitidine, UFT, oxaloplatin, gefitinib (Iressa), imatinib (STI571), erlotinib, an FMS-like tyrosine kinase 3 (Flt3) inhibitor, a vascular endothelial growth facotr receptor (VEGFR) inhibitor, a fibroblast growth factor receptor (FGFR) inhibitor, an epidermal growth factor receptor (EGFR) inhibitor such as Tarceva, radicicol, 17-allylamino-17-demethoxygeldanamycin, rapamycin, amsacrine, alltrans retinoic acid, thalidomide, lenalidomide, anastrozole, fadrozole, letrozole, exemestane, gold thiomalate, D-penicillamine, bucillamine, azathioprine, mizoribine, cyclosporine, rapamycin, hydrocortisone, bexarotene (Targretin), tamoxifen, dexamethasone, a progestin, an estrogen, anastrozole (Arimidex), Leuplin, aspirin, indomethacin, celecoxib, azathioprine, penicillamine, gold thiomalate, chlorpheniramine maleate, chlorpheniramine, clemastine, tretinoin, bexarotene, arsenic, bortezomib, allopurinol, calicheamicin, ibritumomab tiuxetan, targretin, ozogamine, clarithromycin, leucovorin, ketoconazole, aminoglutethimide, suramin, methotrexate, or maytansinoid, or a derivative thereof, and the like.

Examples of a method for binding a low-molecular weight drug to the bispecific antibody of the present invention include a method for binding the drug to an amino group of the antibody via glutaraldehyde, a method for binding an amino group of the drug to a carboxyl group of the antibody via a water-soluble carbodiimide, and the like.

Examples of the high-molecular weight drug include polyethylene glycol (PEG), albumin, dextran, polyoxyethylene, a styrene-maleic acid copolymer, polyvinylpyrrolidone, a pyran copolymer, hydroxypropyl methacrylamide, and the like. By binding such a high-molecular weight compound to the bispecific antibody of the present invention, an effect such as (1) improvement of the stability against various chemical, physical, or biological factors, (2) significant extension of the blood half-life, or (3) elimination of immunogenicity or suppression of antibody production is expected [Bioconjugate pharmaceutical, Hirokawa-Shoten Ltd. (1993)].

Examples of a method for binding PEG to the bispecific antibody of the present invention include a method for reacting with a PEGylation reagent, and the like [Bioconjugate pharmaceutical, Hirokawa-Shoten Ltd. (1993)]. Examples of the PEGylation reagent include a modifying agent to an ε-amino group of lysine (JP-A-S61-178926), a modifying agent to a carboxyl group of aspartic acid and glutamic acid (JP-AS56-23587), a modifying agent to a guanidino group of arginine (JP-A-H2-117920), and the like.

The immunostimulant may be a natural product known as an immunoadjuvant, and specific examples thereof include a drug that enhances immunity such as a β(1→3) glucan (for example, lentinan or schizophyllan) or α-galactosylceramide (KRN7000), and the like.

Examples of the protein include a cytokine or a growth factor that activates immunocompetent cells such as NK cells, macrophages, or neutrophils, or a toxic protein, and the like.

Examples of the cytokine or the growth factor include interferon (hereinafter referred to as IFN)-α, IFN-β, and IFN-y, interleukin (hereinafter referred to as IL)-2, IL-12, IL-15, IL-18, IL-21, and IL-23, a granulocyte colony stimulating factor (G-CSF), a granulocyte-macrophage colony stimulating factor (GM-CSF), a macrophage colony stimulating factor (M-CSF), and the like.

Examples of the toxic protein include ricin, diphtheria toxin, ONTAK, and the like, and also include a protein toxin in which a mutation is introduced into a protein for regulating toxicity.

A fusion antibody with a protein or an antibody drug can be produced by ligating a cDNA encoding the protein to a cDNA encoding the bispecific antibody of the present invention to construct a DNA encoding the fusion antibody, inserting the DNA into an expression vector for a prokaryote or a eukaryote, and then introducing the expression vector into a prokaryote or a eukaryote to cause expression.

When the derivative of the bispecific antibody is used for a detection method or a quantitative determination method, or as a reagent for detection, a reagent for quantitative determination, or a diagnostic agent, examples of the drug to be bound to the bispecific antibody of the present invention include a labeling substance to be used for a general immunological detection or measurement method. Examples of the labeling substance include an enzyme such as alkaline phosphatase, peroxidase, or luciferase, a luminescent substance such as acridinium ester or lophine, or a fluorescent substance such as fluorescein isothiocyanate (FITC) or tetramethylrhodamine isothiocyanate (RITC), Alexa (registered trademark) Fluor 488, or R-phycoerythrin (R-PE), and the like.

In the present invention, the bispecific antibody having a cytotoxicity activity such as a CDC activity or an ADCC activity is included. The CDC activity or the ADCC activity of the bispecific antibody of the present invention against an antigen-expressing cell can be evaluated by a known measurement method [Cancer Immunol. Immunother., 36, 373 (1993)].

Further, the present invention relates to a composition containing a bispecific antibody that specifically recognizes and binds to CD40 and FAP or a therapeutic agent for a disease associated with at least one of CD40 and FAP containing the bispecific antibody as an active ingredient.

The disease associated with at least one of CD40 and FAP may be any as long as it is a disease associated with at least one of CD40 and FAP, and for example, a malignant tumor, a cancer, and the like are exemplified.

In the present invention, examples of the malignant tumor and the cancer include, large intestine cancer, colorectal cancer, lung cancer, breast cancer, glioma, malignant melanoma (melanoma), thyroid cancer, renal cell carcinoma, leukemia, lymphoma, T cell lymphoma, stomach cancer, pancreatic cancer, cervical cancer, endometrial cancer, ovarian cancer, bile duct cancer, esophageal cancer, liver cancer, head and neck cancer, skin cancer, urinary tract cancer, bladder cancer, prostate cancer, choriocarcinoma, pharyngeal cancer, laryngeal cancer, mesothelioma, pleural tumor, arrhenoblastoma, endometrial hyperplasia, endometriosis, embryoma, fibrosarcoma, Kaposi's sarcoma, angioma, cavernous hemangioma, angioblastoma, retinoblastoma, astrocytoma, neurofibroma, oligodendroglioma, medulloblastoma, neuroblastoma, glioma, rhabdomyosarcoma, glioblastoma, osteogenic sarcoma, leiomyosarcoma, Wilms tumor, and the like.

The therapeutic agent containing the bispecific antibody of the present invention, or a derivative thereof may contain only the bispecific antibody or a derivative thereof as an active ingredient, however, in general, it is preferably provided as a pharmaceutical preparation produced using an arbitrary method known in the technical field of pharmaceutics by mixing it together with one or more pharmacologically acceptable carriers.

As the route of administration, it is preferred to use the most effective route for the treatment, and for example, oral administration or parenteral administration such as intraoral, intra-airway, intrarectal, subcutaneous, intramuscular, or intravenous administration is exemplified. Above all, intravenous administration is preferred.

Examples of a dosage form include a spray, a capsule, a tablet, a powder, a granule, a syrup, an emulsion, a suppository, an injection, an ointment, a tape, and the like.

A dose or administration frequency varies depending on a desired therapeutic effect, an administration method, a treatment duration, an age, a body weight, etc., but is generally 10 µg/kg to 10 mg/kg per day for an adult.

Further, the present invention relates to a reagent for immunological detection or measurement of at least one of CD40 and FAP, or a diagnostic agent for a disease associated with at least one of CD40 and FAP, each of which contains the bispecific antibody of the present invention. In addition, the present invention relates to a method for immunological detection or measurement of at least one of CD40 and FAP, a therapeutic method for a disease associated with at least one of CD40 and FAP, or a diagnostic method for a disease associated with at least one of CD40 and FAP, preferably a disease involved in a CD40 and FAP-expressing cell, each of which uses the bispecific antibody of the present invention.

Examples of a method for detecting or measuring the amount of at least one of CD40 and FAP in the present invention include known arbitrary methods. For example, an immunological detection or measurement method and the like are exemplified.

The immunological detection or measurement method is a method for detecting or measuring the amount of an antibody or the amount of an antigen using a labeled antigen or antibody. Examples of the immunological detection or measurement method include a radioimmunoassay method (RIA), an enzyme immunoassay method (EIA or ELISA), a fluorescence immunoassay method (FIA), a luminescent immunoassay method, a Western blotting method, a physicochemical method, and the like.

By detecting or measuring a cell expressing at least one of CD40 and FAP using the bispecific antibody of the present invention, it is possible to diagnose a disease associated with at least one of CD40 and FAP, preferably a disease involved in a CD40 and FAP-expressing cell.

It is possible to use a known immunological detection method for detecting a cell expressing at least one of CD40 and FAP. As the known immunological detection method, an immunoprecipitation method, an immunocytological staining method, an immunohistological staining method, or a fluorescent antibody staining method, and the like are exemplified. In addition, in the fluorescent antibody staining method, for example, an FMAT 8100 HTS system (manufactured by Applied Biosystems, Inc.), or the like can be used.

A biological sample to be subjected to detection or measurement of at least one of CD40 and FAP in the present invention is not particularly limited as long as the sample may contain a cell expressing at least one of CD40 and FAP, for example, a tissue cell, blood, plasma, serum, pancreatic juice, urine, feces, a tissue fluid, a culture solution, or the like.

The diagnostic agent containing the bispecific antibody of the present invention, or a derivative thereof may contain a reagent for performing an antigen-antibody reaction or a reagent for detecting the reaction in accordance with a desired diagnostic method. Examples of the reagent for performing an antigen-antibody reaction include a buffer, a salt, and the like.

Examples of the reagent for detection include a reagent, which is used for a general immunological detection or measurement method, such as a labeled secondary antibody that binds to the bispecific antibody, or a derivative thereof, or a substrate corresponding to a label.

Hereinafter, a method for producing the bispecific antibody of the present invention, a method for evaluating the activity of the bispecific antibody, and a therapeutic method and a diagnostic method for a disease using the bispecific antibody will be specifically described.

### 1. Method for Producing Monoclonal Antibody

A method for producing a monoclonal antibody in the present invention includes the following operation steps. That is, (1) at least one of the purification of an antigen to be used as an immunogen and the production of a cell in which the antigen is overexpressed on the cell surface, (2) a step of preparing an antibody-producing cell by immunizing an animal with the antigen, followed by collecting the blood and examining an antibody titer thereof to determine when to resect the spleen or the like, (3) preparing a myeloma cell (myeloma), (4) fusing the antibody-producing cell with the myeloma, (5) screening a hybridoma group that produces a target antibody, (6) separating (cloning) a monoclonal cell from the hybridoma group, (7) in some cases, culturing the hybridoma for producing a monoclonal antibody in a large amount, or breeding an animal implanted with the hybridoma, (8) investigating the bioactivity of the monoclonal antibody produced in this manner, and the antigen binding specificity thereof, or examining the characteristics as a labeling reagent, and the like.

Hereinafter, a method for producing a monoclonal antibody that binds to CD40 and a monoclonal antibody that binds to FAP, which are used for producing the bispecific antibody that binds to CD40 and FAP in the present invention, will be described in detail in accordance with the above-mentioned steps. The method for producing the antibodies is not limited thereto, and for example, an antibody-producing cell other than a spleen cell, and a myeloma can also be used.

### (1) Purification of Antigen

A cell that expresses CD40 or FAP can be obtained by introducing an expression vector containing a cDNA encoding the full length of CD40 or FAP or a partial length thereof into E. coli, yeast, an insect cell, an animal cell, or the like. In addition, at least one of CD40 and FAP is purified from various human cultured tumor cells or human tissues or the like in which at least one of CD40 and FAP is expressed in a large amount and can be used as an antigen. In addition, the cultured tumor cell or the tissue or the like can also be used as an antigen as it is. Further, a synthetic peptide having a partial sequence of CD40 or FAP is prepared by a chemical synthesis method such as an Fmoc method or a tBoc method and can also be used as an antigen.

CD40 and FAP used in the present invention can be produced using a method described in Molecular Cloning, A Laboratory Manual, Second Edition, Cold Spring Harbor Laboratory Press (1989), or Current Protocols In Molecular Biology, John Wiley & Sons (1987-1997), or the like, for example, by expressing a DNA encoding CD40 or FAP in a host cell using the following method.

A recombinant vector is produced by inserting a full-length cDNA containing a region encoding CD40 or FAP downstream of a promoter in an appropriate expression vector. A DNA fragment that has been prepared based on the full-length cDNA and has an appropriate length and contains a region encoding a polypeptide may be used in place of the full-length cDNA. Subsequently, by introducing the obtained recombinant vector into a host cell suitable for the expression vector, a transformant that produces CD40 or FAP can be obtained.

As the expression vector, any vector can be used as long as it can autonomously replicate or can be integrated into a chromosome in a host cell to be used, and contains an appropriate promoter at a position capable of transcribing a DNA encoding CD40 or FAP.

As the host cell, any cell, for example, a microorganism belonging to the genus Escherichia such as E. coli or the like, yeast, an insect cell, an animal cell, or the like, can be used as long as it can express a target gene.

When a prokaryote such as E. coli is used as the host cell, the recombinant vector is preferably a vector that can autonomously replicate in the prokaryote, and also contains a promoter, a ribosomal binding sequence, a DNA containing a region encoding CD40 or FAP, and a transcription termination sequence. In addition, the transcription termination sequence is not necessarily needed for the recombinant vector, however, it is preferred that the transcription termination sequence is located immediately downstream of a structural gene. Further, the recombinant vector may contain a gene that controls the promoter.

As the recombinant vector, it is preferred to use a plasmid in which the distance between a Shine-Dalgarno sequence, which is a ribosomal binding sequence, and a start codon is adjusted to an appropriate distance (for example, 6 to 18 nucleotides).

In addition, in the nucleotide sequence of the DNA encoding CD40 or FAP, it is possible to substitute a nucleotide so that a codon becomes most suitable for expression in a host, and as a result, the production rate of the target CD40 or FAP can be improved.

As the expression vector, any vector can be used as long as it can exhibit its function in a host cell to be used, and examples thereof include pBTrp2, pBTacl, pBTac2 (each of which is manufactured by Roche Diagnostics K.K.), pKK233-2 (manufactured by Pharmacia Corporation), pSE280 (manufactured by Invitrogen, Inc.), pGEMEX-1 (manufactured by Promega Corporation), pQE-8 (manufactured by QIAGEN, Inc.), pKYP10 (JP-A-S58-110600), pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene Corporation), pTrs30 [prepared from E. coli JM109/pTrS30 (FERM BP-5407)], pTrs32 [prepared from E. coli JM109/pTrS32 (FERM BP-5408)], pGHA2 [prepared from E. coli IGHA2 (FERM BP-400), JP-A-S60-221091], pGKA2 [prepared from E. coli IGKA2 (FERM BP-6798), JP-AS60-221091], pTerm2 (US Patent No. 4,686,191, US Patent No. 4,939,094, or US Patent No. 5,160,735), pSupex, pUB110, pTP5, pC194, pEG400 [J. Bacteriol., 172, 2392 (1990)], pGEX (manufactured by Pharmacia Corporation), pET System (manufactured by Novagen, Inc.), pME18SFL3 (manufactured by Toyobo Co., Ltd.), and the like.

The promoter may be any as long as it functions in a host cell to be used. Examples thereof include promoters derived from E. coli, a phage, or the like such as a trp promoter (Ptrp), a lac promoter, a PL promoter, a PR promoter, or a T7 promoter. In addition, examples thereof also include artificially designed and modified promoters such as a tandem promoter in which two Ptrp promoters are linked in tandem, a tac promoter, a lacT7 promoter, or a let I promoter, and the like.

Examples of the host cell include E. coli XL1-Blue, E. coli XL2-Blue, E. coli DH1, E. coli MC1000, E. coli KY3276, E. coli W1485, E. coli JM109, E. coli HB101, E. coli No. 49, E. coli W3110, E. coli NY49, E. coli DH5α, and the like.

As a method for introducing a recombinant vector into a host cell, any method can be used as long as it is a method for introducing a DNA into a host cell to be used, and examples thereof include a method using calcium ions [Proc. Natl. Acad. Sci. USA, 69, 2110 (1972), Gene, 17, 107 (1982), Molecular & General Genetics, 168, 111 (1979)].

When an animal cell is used as a host, as the expression vector, any vector can be used as long as it functions in an animal cell, and examples thereof include pcDNAI (manufactured by Invitrogen, Inc.), pcDM8 (manufactured by Funakoshi Co., Ltd.), pAGE107 [JP-A-H3-22979; Cytotechnology, 3, 133 (1990)], pAS3-3 (JP-A-H2-227075), pCDM8 [Nature, 329, 840 (1987)], pcDNAI/Amp (manufactured by Invitrogen, Inc.), pcDNA3.1 (manufactured by Invitrogen, Inc.), pREP4 (manufactured by Invitrogen, Inc.), pAGE103 [J. Biochemistry, 101, 1307 (1987)], pAGE210, pME18SFL3, pKANTEX93 (WO 97/10354), and the like.

As the promoter, any promoter can be used as long as it can exhibit its functions in an animal cell, and examples thereof include a cytomegalovirus (CMV) immediate early (IE) gene promoter, an SV40 early promoter, a retrovirus promoter, a metallothionein promoter, a heat-shock promoter, an SRα promoter, or a Moloney murine leukemia virus promoter or enhancer. In addition, a human CMV IE gene enhancer may be used together with the promoter.

Examples of the host cell include a human Burkitt's lymphoma cell Namalwa, an African Green Monkey kidney-derived cell COS, a Chinese hamster ovary-derived cell CHO, a human leukemia cell HBT5637 (JP-A-S63-000299), and the like.

As a method for introducing a recombinant vector into a host cell, any method can be used as long as it is a method for introducing a DNA into an animal cell, and examples thereof include an electroporation method [Cytotechnology, 3, 133 (1990)], a calcium phosphate method (JP-A-H2-227075), a lipofection method [Proc. Natl. Acad. Sci. USA, 84, 7413 (1987)], and the like.

CD40 or FAP can be produced by culturing a microorganism having a recombinant vector incorporating a DNA encoding CD40 or FAP, or a transformant derived from an animal cell or the like obtained as described above in a culture medium, producing and accumulating at least one of the CD40 and FAP in the culture, and then collecting it from the culture. A method for culturing the transformant in a culture medium can be carried out according to a usual method used for culturing a host.

In the case of being expressed in a cell derived from a eukaryote, it is possible to obtain CD40 or FAP to which a sugar or a sugar chain is added.

When culturing a microorganism transformed with a recombinant vector using an inducible promoter, an inducer may be added to a culture medium as needed. For example, when a microorganism transformed with a recombinant vector using a lac promoter is cultured, isopropyl-β-D-thiogalactopyranoside or the like may be added to a culture medium, and when a microorganism transformed with a recombinant vector using a trp promoter is cultured, indoleacrylic acid or the like may be added to a culture medium.

Examples of the culture medium in which the transformant obtained using an animal cell as a host is cultured include RPMI 1640 medium [The Journal of the American Medical Association, 199, 519 (1967)], Eagle's MEM medium [Science, 122, 501 (1952)], Dulbecco's modified MEM medium [Virology, 8, 396 (1959)], Medium 199 [Proc. Soc. Exp. Biol. Med., 73, 1 (1950)], Iscove's modified Dulbecco's medium (IMDM), which are generally used, or a culture medium in which fetal bovine serum (FBS) or the like is added to any of these culture media, and the like. The culture is usually carried out under the conditions of pH 6 to 8 and 30 to 40°C in the presence of 5% CO₂ for 1 to 7 days. In addition, during the culture, an antibiotic such as kanamycin or penicillin may be added to the culture medium as needed.

As a method for expressing a gene encoding CD40 or FAP, a method of secretory production, fused protein expression, or the like [Molecular Cloning, A Laboratory Manual, Second Edition, Cold Spring Harbor Laboratory Press (1989)] can be used in addition to direct expression. Examples of a method for producing CD40 or FAP include a method for producing it in a host cell, a method for secreting it out of a host cell, and a method for producing it on an outer membrane of a host cell, and an appropriate method can be selected by changing a host cell to be used or the structure of CD40 or FAP to be produced.

For example, an antigen-fusion protein can be produced by preparing a DNA in which a DNA encoding an Fc region of an antibody, a DNA encoding glutathione S-transferase (GST), a DNA encoding a FLAG tag or a DNA encoding a Histidine tag, or the like is ligated to a DNA encoding an amino acid sequence of an extracellular domain, followed by expression and purification. Specific examples thereof include an Fc-fusion protein in which an extracellular domain of CD40 or FAP is bound to an Fc region of human IgG, and a fusion protein of an extracellular domain of CD40 or FAP with glutathione S-transferase (GST).

When CD40 or FAP is produced in a host cell or on an outer membrane of a host cell, CD40 or FAP can be actively secreted out of the host cell using the method of Paulson et al. [J. Biol. Chem., 264, 17619 (1989)], the method of Lowe et al. [Proc. Natl. Acad. Sci., USA, 86, 8227 (1989), Genes Develop., 4, 1288 (1990)], or a method described in JP-A-H05-336963, WO 94/23021, or the like. In addition, the production amount of CD40 or FAP can also be increased by utilizing a gene amplification system using a dihydrofolate reductase gene or the like (JP-A-H2-227075).

The produced CD40 or FAP can be isolated and purified, for example, as follows.

When CD40 or FAP is expressed in cells in a dissolved state, the cells are collected by centrifugation after completion of the culture, suspended in an aqueous buffer solution, followed by homogenization of the cells using an ultrasonic homogenizer, a French press, a Manton Gaulin homogenizer, a Dyno mill, or the like, whereby a cell-free extract solution is obtained. It is possible to obtain a purified protein from a supernatant obtained by centrifugation of the cell-free extract solution using methods such as general protein isolation and purification methods, that is, a solvent extraction method, a salting-out method using ammonium sulfate or the like, a desalting method, a precipitation method using an organic solvent, anion exchange chromatography using a resin such as diethylaminoethyl (DEAE)-Sepharose or DIAION HPA-75 (manufactured by Mitsubishi Chemical Corporation), cation exchange chromatography using a resin such as S-Sepharose FF (manufactured by Pharmacia Corporation), hydrophobic chromatography using a resin such as Butyl Sepharose or Phenyl Sepharose, a gel filtration method using a molecular sieve, affinity chromatography, a chromatofocusing method, electrophoresis such as isoelectric focusing electrophoresis, and the like alone or in combination.

When CD40 or FAP is expressed in cells by forming an insoluble body, the cells are collected and then homogenized in the same manner as described above, followed by centrifugation, whereby the insoluble body of the CD40 or FAP is collected as a precipitated fraction. The collected insoluble body of the CD40 or FAP is solubilized with a protein denaturing agent. The CD40 or FAP is returned to a normal conformation by diluting or dialyzing the solubilized solution, and thereafter, a purified protein of a polypeptide can be obtained by the same isolation and purification methods as described above.

When CD40 or FAP, or a derivative thereof such as a sugar-modified body thereof is extracellularly secreted, the CD40 or FAP, or the derivative thereof such as a sugar-modified body thereof can be collected in a culture supernatant. The culture supernatant is subjected to a treatment using a method such as centrifugation in the same manner as described above, thereby obtaining a soluble fraction, and then by using the same isolation and purification methods as described above, a purified protein can be obtained from the soluble fraction.

In addition, CD40 or FAP used in the present invention can also be produced using a chemical synthesis method such as an Fmoc method or a tBoc method. Specifically, for example, chemical synthesis can be carried out using a peptide synthesizer manufactured by Advanced Chemtech, Inc., PerkinElmer, Inc., Pharmacia Corporation, Protein Technology Instrument, Inc., Synthecell-Vega Biomolecules Corporation, Perceptive, Inc., Shimadzu Corporation, or the like.

### (2) Step of Preparing Antibody-Producing Cell

An animal such as a mouse, a rat, a hamster, a rabbit, cattle, or an alpaca is immunized with the antigen obtained in (1), and an antibody-producing cell in the spleen, the lymph node, or the peripheral blood of the animal is collected. In addition, as the animal, for example, a transgenic mouse that produces a human-derived antibody described in the document of Tomizuka. et al. [Tomizuka. et al., Proc Natl Acad Sci USA., 97, 722 (2000)], a conditional knockout mouse of CD40 or FAP for enhancing immunogenicity, or the like is exemplified as an immunized animal.

The immunization is carried out by administering an antigen together with an appropriate adjuvant such as a Freund's complete adjuvant, an aluminum hydroxide gel, Bordetella pertussis vaccine, or the like. As a method for administration of an immunogen when immunizing a mouse, any method of subcutaneous injection, intraperitoneal injection, intravenous injection, intradermal injection, intramuscular injection, footpad injection, and the like may be used, but intraperitoneal injection, footpad injection, or intravenous injection is preferred. When the antigen is a partial peptide, a conjugate of the antigen with a carrier protein such as BSA (bovine serum albumin) or KLH (Keyhole Limpet hemocyanin) is produced and used as an immunogen.

The administration of the antigen is performed 5 to 10 times every 1 to 2 weeks after the first administration. On day 3 to 7 after each administration, the blood is collected from a venous plexus of the fundus, and the antibody titer of the serum thereof is measured using an enzyme immunoassay method [Antibodies - A Laboratory Manual, Cold Spring Harbor Laboratory (1988)] or the like. If an animal whose serum shows a sufficient antibody titer against the antigen used for the immunization is used as a supply source for the antibody-producing cell for fusion, the effect of the subsequent operation can be enhanced.

On day 3 to 7 after the final administration of the antigen, a tissue including the antibody-producing cell such as the spleen is resected from the immunized animal, and the antibody-producing cell is collected. The antibody-producing cell is a lymphocyte that is a plasma cell and a progenitor cell thereof. The cell may be obtained from any site of an individual and can be generally obtained from the spleen, the lymph node, the bone marrow, the tonsil, the peripheral blood, or an appropriate combination thereof, or the like, but spleen cells are most generally used. When spleen cells are used, the spleen is shredded and loosened, followed by centrifugation, and then red blood cells are removed, whereby the antibody-producing cells for fusion are obtained.

### (3) Step of Preparing Myeloma

As a myeloma, a cell that is derived from a mammal such as a mouse, a rat, a guinea pig, a hamster, a rabbit, or a human, and that has no ability of autoantibody production can be used, however, generally, an established cell line obtained from a mouse, for example, a 8-azaguanine resistant mouse (BALB/c derived) myeloma cell line P3-X63Ag8-U1 (P3-U1) [Current Topics in Microbiology and Immunology, 18, 1 (1978)], P3-NS1/1-Ag41 (NS-1) [European J. Immunology, 6, 511 (1976)], SP2/0-Ag14 (SP-2) [Nature, 276, 269 (1978)], P3-X63-Ag8653 (653) [J. Immunology, 123, 1548 (1979)], P3-X63-Ag8 (X63) [Nature, 256, 495 (1975)], or the like is used. The cell line is subcultured in a suitable culture medium, for example, a culture medium such as an 8-azaguanine medium [RPMl 1640 medium supplemented with glutamine, 2-mercaptoethanol, gentamicin, FCS, and 8-azaguanine], Iscove's modified Dulbecco's medium (hereinafter referred to as "IMDM"), or Dulbecco's modified Eagle medium (hereinafter referred to as "DMEM"). The above-mentioned cell line is subcultured in a normal culture medium (for example, DMEM medium containing 10% FCS) 3 to 4 days before cell fusion, and 2 x 10⁷ or more cells are ensured on the day of performing the fusion.

### (4) Cell Fusion

The antibody-producing cells for fusion obtained in (2) and the myeloma cells obtained in (3) are well washed with Minimum Essential Medium (MEM) or PBS (1.83 g of disodium phosphate, 0.21 g of monopotassium phosphate, 7.65 g of sodium chloride, 1 L of distilled water, pH 7.2), and mixed to give the antibody-producing cells for fusion : the myeloma cells = 5:1 to 10:1, followed by centrifugation, and then the supernatant is removed. After the precipitated cell clusters are well loosened, a mixed solution of polyethylene glycol 1000 (PEG-1000), MEM medium, and dimethylsulfoxide is added thereto while stirring at 37°C. Further, 1 to 2 mL of MEM medium is added thereto every 1 to 2 minutes for several times, and then MEM medium is added so that the total amount becomes 50 mL. After centrifugation, the supernatant is removed, the precipitated cell clusters are gently loosened, and then the cells are gently suspended in HAT medium [a normal culture medium supplemented with hypoxanthine, thymidine, and aminopterin]. The resulting suspension is cultured in a 5% CO₂ incubator at 37°C for 7 to 14 days.

In addition, the cell fusion can also be carried out by the following method. The spleen cells and the myeloma cells are well washed with a serum-free medium (for example, DMEM), or phosphate buffered saline (hereinafter referred to as "phosphate buffer solution"), and mixed so that the ratio of the spleen cells to the myeloma cells becomes about 5:1 to 10:1, followed by centrifugation. The supernatant is removed, and after the precipitated cell clusters are well loosened, 1 mL of a serum-free medium containing 50% (w/v) polyethylene glycol (molecular weight: 1000 to 4000) is dropped thereon while stirring. Thereafter, 10 mL of the serum-free medium is slowly added thereto, followed by centrifugation. The supernatant is removed again, the precipitated cells are suspended in a normal culture medium containing an appropriate amount of a hypoxanthine-aminopterin-thymidine (HAT) solution and human interleukin 2 (IL-2) (hereinafter referred to as HAT medium), and the suspension is dispensed in each well of a culture plate (hereinafter referred to as a plate), and then the cells are cultured in the presence of 5% carbon dioxide gas at 37°C for about 2 weeks. During the course of the culture, the HAT medium is supplemented as appropriate.

### (5) Selection of Hybridoma Group

When the myeloma cells used for the fusion are an 8-azaguanine resistant strain, that is, a hypoxanthine-guanine-phosphoribosyltransferase (HGPRT)-deficient strain, the unfused myeloma cells and the fused cells between the myeloma cells cannot survive in the HAT medium. On the other hand, the fused cells between the antibody-producing cells, and the hybridomas of the antibody-producing cell and the myeloma cell can survive in the HAT medium, however, the life span of the fused cells between the antibody-producing cells is reached shortly. Therefore, by continuing the culture in the HAT medium, only the hybridomas of the antibody-producing cell and the myeloma cell survive, and as a result, the hybridomas can be obtained.

For a hybridoma grown in a colony form, medium replacement with a culture medium obtained by removing aminopterin from the HAT medium (hereinafter referred to as HT medium) is performed. Thereafter, a portion of the culture supernatant is collected, and a hybridoma that produces an antibody can be selected using the below-mentioned antibody titer measurement method. Examples of the antibody titer measurement method include various known techniques such as a radioisotopic immunoassay method (RIA method), a solid-phase enzyme immunoassay method (ELISA method), a fluorescent antibody method, and a passive hemagglutination reaction method, but an RIA method or an ELISA method is preferred from the viewpoint of detection sensitivity, rapidity, accuracy, a possibility of automation of an operation, and the like.

The hybridoma determined to produce a desired antibody by measuring the antibody titer is transferred to another plate, and cloning is performed. Examples of the cloning method include a limiting dilution method in which culture is performed by dilution so that one cell is contained in one well of a plate, a soft agar method in which culture is performed in a soft agar medium to collect colonies, a method in which one cell is isolated using a micromanipulator, a method in which one cell is isolated using a cell sorter, and the like.

For a well in which the antibody titer is observed, for example, cloning by a limiting dilution method is repeated 2 to 4 times, and the cell in which the antibody titer is stably observed is selected as a hybridoma strain that produces a monoclonal antibody against CD40 or FAP.

### (6) Preparation of Monoclonal Antibody

The monoclonal antibody-producing hybridoma obtained in (5) is intraperitoneally injected into a mouse or a nude mouse at the age of 8 to 10 weeks having been subjected to a pristane treatment [0.5 mL of 2,6,10,14-tetramethylpentadecane (Pristane) is intraperitoneally administered, followed by breeding for 2 weeks]. In 10 to 21 days, the hybridoma is converted into an ascites tumor. The ascites is collected from this mouse, followed by centrifugation, removing solids, and then salting out with 40% to 50% ammonium sulfate. Thereafter, purification is performed by a caprylic acid precipitation method, a DEAE-Sepharose column, a protein A column, or a gel filtration column, and then an IgG or IgM fraction is collected and a purified monoclonal antibody is prepared. In addition, by growing the hybridoma in the peritoneal cavity of a mouse of the same strain (for example, BALB/c) or a Nu/Nu mouse, a rat, a guinea pig, a hamster, a rabbit, or the like, ascites containing a large amount of a monoclonal antibody that binds to CD40 or FAP can be obtained.

After culturing the monoclonal antibody-producing hybridoma obtained in (5) in RPMI 1640 medium supplemented with 10% FBS, or the like, the supernatant is removed by centrifugation, and the residue is suspended in GIT medium or Hybridoma-SFM medium supplemented with 5% Daigo's GF21, or the like, and then cultured for 3 to 7 days by flask culture, spinner culture, bag culture, or the like. The obtained cell suspension is centrifuged, and purification from the obtained supernatant is performed by a protein A column or a protein G column, and then an IgG fraction is collected, whereby a purified monoclonal antibody can also be obtained. As a simple method for the purification, it is also possible to use a commercially available monoclonal antibody purification kit (for example, MabTrap GII kit manufactured by Amersham Pharmacia Biotech, Inc.), or the like.

The determination of the subclass of the antibody is carried out by an enzyme immunoassay method using a subclass typing kit. The quantitative determination of a protein content can be carried out by a Lowry method or a method of calculation from the absorbance at 280 nm [1.4 (OD₂₈₀) = 1 mg/mL immunoglobulin].

### (7) Binding Assay of Monoclonal Antibody to CD40 or FAP

The binding activity of the monoclonal antibody to CD40 or FAP can be measured by a binding assay system such as an Ouchterlony method, an ELISA method, an RIA method, a flow cytometry method (FCM), or a surface plasmon resonance method (SPR).

An Ouchterlony method is simple, but a concentration operation is needed when the concentration of the antibody is low. On the other hand, when an ELISA method or an RIA method is used, by allowing a culture supernatant to directly react with an antigen-adsorbed solid phase and further by using antibodies corresponding to various immunoglobulin isotypes and subclasses as secondary antibodies, it is possible to identify the isotype and subclass of the antibody and also to measure the binding activity of the antibody.

As a specific example of the procedure, the purified or partially purified recombinant CD40 or FAP is adsorbed to a solid phase surface of a 96-well plate for ELISA or the like, and then the solid phase surface to which the antigen is not adsorbed is blocked with a protein unrelated to the antigen, for example, bovine serum albumin (BSA). After an ELISA plate is washed with phosphate buffer saline (PBS) and PBS containing 0.05% Tween 20 (Tween-PBS), or the like, a serially diluted first antibody (for example, mouse serum, a culture supernatant, or the like) is reacted therewith, and then the antibody is bound to the antigen immobilized on the plate. Subsequently, as a second antibody, an anti-immunoglobulin antibody labeled with biotin, an enzyme (horse radish peroxidase (HRP), alkaline phosphatase (ALP), or the like), a chemiluminescent substance or a radioactive compound, or the like, is dispensed to allow the second antibody to react with the first antibody bound to the plate. After well washing with Tween-PBS, a reaction according to the labeling substance of the second antibody is performed, and then a monoclonal antibody that specifically reacts with the target antigen is selected.

In an FCM method, the binding activity of an antibody to an antigen-expressing cell can be measured [Cancer Immunol. Immunother., 36, 373 (1993)]. Binding of an antibody to a membrane protein antigen expressed on a cell membrane means that the antibody recognizes the conformation of a naturally occurring antigen and binds thereto.

Examples of an SPR method include a kinetics analysis by Biacore. For example, by using Biacore T100, the kinetics in binding of an antigen and a test substance are measured, and the result is analyzed with an analysis software attached to an instrument. As a specific example of the procedure, after fixing an anti-mouse IgG antibody to a sensor chip CM5 by an amine coupling method, a test substance such as a hybridoma culture supernatant or a purified monoclonal antibody is allowed to flow to bind in an appropriate amount, further the antigen at multiple known concentrations is allowed to flow, and then binding and dissociation are measured. Subsequently, a kinetics analysis by a 1:1 binding model is carried out with respect to the obtained data using the software attached to the instrument to obtain various parameters. Alternatively, after fixing CD40 or FAP onto the sensor chip by, for example, an amine coupling method, a purified monoclonal antibody at multiple known concentrations is allowed to flow, and then binding and dissociation are measured. A kinetics analysis by a bivalent binding model is carried out with respect to the obtained data using the software attached to the instrument to obtain various parameters.

In addition, in the present invention, it is possible to select an antibody that binds to CD40 or FAP competitively with the antibody against CD40 or FAP by allowing a test antibody to coexist in the above binding assay system to cause a reaction. That is, by screening an antibody whose binding to an antigen is inhibited when a test antibody is added, it is possible to obtain an antibody that competes with the antibody obtained above, for binding to CD40 or FAP.

### (8) Identification of Epitope for Monoclonal Antibody against CD40 or FAP

In the present invention, the identification of an epitope which the antibody recognizes and binds to can be carried out as follows.

For example, a partially deficient variant of an antigen, a mutant of an antigen in which an amino acid residue different among species is modified, or a mutant of an antigen in which a specific domain is modified is produced, and if the reactivity of an antibody against the deficient variant or the mutant is lowered, it becomes clear that the deficient site or the amino acid modified site is an epitope for the antibody. Such a partially deficient variant or a mutant of an antigen may be obtained as a secretory protein using a suitable host cell, for example, E. coli, yeast, a plant cell, a mammalian cell, or the like, or may be prepared as an antigen-expressing cell by expressing it on a cell membrane of a host cell. In the case of a membrane-associated antigen, in order to express it while maintaining the conformation of the antigen, it is preferred to express it on the membrane of a host cell. In addition, it is also possible to confirm the reactivity of the antibody by producing a synthetic peptide mimicking the primary structure or the conformation of the antigen. As for a synthetic peptide, a method for producing various partial peptides of the molecule thereof using a known peptide synthesis technique, and the like are exemplified.

For example, with respect to the extracellular domain of human and mouse CD40 or FAP, it is possible to identify an epitope for an antibody by producing a chimeric protein in which domains constituting the respective regions are appropriately combined, and then confirming the reactivity of the antibody with the protein. Thereafter, it is possible to identify the epitope in more detail by variously synthesizing an oligopeptide of the corresponding region or a mutant or the like of the peptide using an oligopeptide synthesis technique well known to those skilled in the art, and then confirming the reactivity of the antibody with the peptide. As a simple method for obtaining various types of oligopeptides, a commercially available kit [for example, SPOTs Kit (manufactured by Genosys Biotechnologies, Inc.), a series of multipin·peptide synthesis kit (manufactured by Chiron Corporation) using a multipin synthesis method, or the like] can also be used.

An antibody that binds to the same epitope as an epitope to which an antibody that binds to CD40 or FAP binds can be obtained by identifying an epitope for an antibody obtained in the above-mentioned binding assay system, producing a partial synthetic peptide of the epitope, a synthetic peptide mimicking the conformation of the epitope, a recombinant of the epitope, or the like, and then performing immunization therewith.

For example, if the epitope is a membrane protein, an antibody specific to the epitope can be more efficiently produced by producing a recombinant fusion protein in which the entire extracellular domain or a part of the extracellular domain is linked to an appropriate tag, for example, a FLAG tag, a Histidine tag, a GST protein or an antibody Fc region, or the like, and performing immunization with the recombinant protein.

### 2. Production of Genetically Recombinant Antibody

As production examples of genetically recombinant antibodies, methods for producing a chimeric antibody, a humanized antibody, and a human antibody will be described below, although the methods are schematically described in P. J. Delves., ANTIBODY PRODUCTION ESSENTIAL TECHNIQUES., 1997 WILEY, P. Shepherd and C. Dean. Monoclonal Antibodies., 2000 OXFORD UNIVERSITY PRESS, and J. W. Goding., Monoclonal Antibodies: principles and practice., 1993 ACADEMIC PRESS, and the like. In addition, genetically recombinant mouse, rat, hamster, and rabbit antibodies can also be produced using the same method.

### (1) Acquisition of cDNA Encoding V Region of Monoclonal Antibody from Hybridoma

Acquisition of cDNAs encoding VH and VL of a monoclonal antibody can be carried out, for example, as follows.

First, mRNA is extracted from a hybridoma that produces a monoclonal antibody, and cDNAs are synthesized. Subsequently, the synthesized cDNAs are each cloned into a vector such as a phage or a plasmid, thereby producing a cDNA library. A recombinant phage or a recombinant plasmid containing a cDNA encoding VH or VL is isolated from the library using a DNA encoding a C region part or a V region part of the antibody as a probe, respectively. The entire nucleotide sequence of VH or VL in the isolated recombinant phage or recombinant plasmid is determined, and then the entire amino acid sequence of VH or VL is deduced from the nucleotide sequence.

As a non-human animal used for producing a hybridoma, a mouse, a rat, a hamster, a rabbit, or the like is used, but any animal can be used as long as a hybridoma can be produced.

In the preparation of the total RNA from a hybridoma, a guanidine thiocyanate-cesium trifluoroacetate method [Methods in Enzymol., 154, 3 (1987)], or a kit such as RNA easy Kit (manufactured by QIAGEN, Inc.), or the like is used.

In the preparation of mRNA from the total RNA, an oligo (dT)-immobilized cellulose column method [Molecular Cloning, A Laboratory Manual, Second Edition, Cold Spring Harbor Laboratory Press (1989)], or a kit such as Oligo-dT30 <Super> mRNA Purification Kit (manufactured by Takara Bio, Inc.), or the like is used. Further, it is also possible to prepare mRNA using a kit such as Fast Track mRNA Isolation Kit (manufactured by Invitrogen, Inc.), or QuickPrep mRNA Purification Kit (manufactured by Pharmacia Corporation).

In the synthesis of cDNAs and the production of a cDNA library, a known method [Molecular Cloning, A Laboratory Manual, Second Edition, Cold Spring Harbor Laboratory Press (1989), Current Protocols in Molecular Biology, Supplement 1, John Wiley & Sons (1987-1997)], or a kit such as SuperScript Plasmid System for cDNA Synthesis and Plasmid Cloning (manufactured by Invitrogen, Inc.) or ZAP-cDNA Synthesis Kit (manufactured by Stratagene Corporation), or the like is used.

When the cDNA library is produced, as the vector into which a cDNA synthesized using mRNA extracted from a hybridoma as a template is incorporated, any vector can be used as long as it can incorporate the cDNA.

For example, ZAP Express [Strategies, 5, 58 (1992)], pBluescript II SK(+) [Nucleic Acids Research, 17, 9494 (1989)], λZAP II (manufactured by Stratagene Corporation), λgt 10, λgt 11 [DNA Cloning: A Practical Approach, I, 49 (1985)], Lambda BlueMid (manufactured by Clontech Laboratories, Inc.), λExCell, pT7T3-18U (manufactured by Pharmacia Corporation), pcD2 [Mol. Cell. Biol., 3, 280 (1983)], pUC18 [Gene, 33, 103 (1985)], or the like is used.

As E. coli into which a cDNA library constructed by a phage or a plasmid vector is introduced, any E. coli can be used as long as it can introduce, express, and maintain the cDNAlibrary. For example, XL1-Blue MRF' [Strategies, 5, 81 (1992)], C600 [Genetics, 39, 440 (1954)], Y1088, Y1090 [Science, 222, 778 (1983)], NM522 [J. Mol. Biol., 166, 1 (1983)], K802 [J. Mol. Biol., 16, 118 (1966)], JM105 [Gene, 38, 275 (1985)], or the like is used.

In the selection of a cDNA clone encoding VH or VL of a non-human antibody from the cDNA library, a colony hybridization method using an isotope or a fluorescently labeled probe, or a plaque hybridization method [Molecular Cloning, A Laboratory Manual, Second Edition, Cold Spring Harbor Laboratory Press (1989)], or the like is used.

In addition, it is also possible to prepare a cDNA encoding VH or VL by preparing a primer and performing a polymerase chain reaction method [hereinafter referred to as a PCR method, Molecular Cloning, A Laboratory Manual, Second Edition, Cold Spring Harbor Laboratory Press (1989), Current Protocols in Molecular Biology, Supplement 1, John Wiley & Sons (1987-1997)] using a cDNA synthesized from mRNA or a cDNA library as a template.

The selected cDNA is cleaved with an appropriate restriction enzyme or the like, and then cloned into a plasmid such as pBluescript SK(-) (manufactured by Stratagene Corporation), and the nucleotide sequence of the cDNA is determined by a commonly used nucleotide sequence analysis method or the like. For example, after performing a reaction such as a dideoxy method [Proc. Natl. Acad. Sci. USA, 74, 5463 (1977)], an analysis is performed using an automatic nucleotide sequence analyzer such as A.L.F. DNA sequencer (manufactured by Pharmacia Corporation).

By deducing the entire amino acid sequence of each of VH and VL from the determined entire nucleotide sequence and comparing it with the entire amino acid sequence of each of VH and VL of a known antibody [Sequences of Proteins of Immunological Interest, US Dept. Health and Human Services (1991)], it is confirmed whether or not the obtained cDNA encodes the complete amino acid sequence of each of VH and VL of the antibody containing a secretion signal sequence.

With respect to the complete amino acid sequence of each of VH and VL of the antibody containing a secretion signal sequence, by comparison with the entire amino acid sequence of each of VH and VL of a known antibody [Sequences of Proteins of Immunological Interest, US Dept. Health and Human Services (1991)], the length of the secretion signal sequence and the N-terminal amino acid sequence can be deduced, and further the subgroup to which these belong can be identified.

In addition, the amino acid sequence of each of CDRs of VH and VL can be deduced by comparison with the amino acid sequence of each of VH and VL of a known antibody [Sequences of Proteins of Immunological Interest, US Dept. Health and Human Services (1991)].

Further, with respect to the obtained complete amino acid sequence of each of VH and VL, it is possible to confirm whether or not the complete amino acid sequence of each of VH and VL is new by, for example, performing a homology search by the BLAST method [J. Mol. Biol., 215, 403 (1990)] or the like using an arbitrary database such as SWISS-PROT or PIR-Protein.

### (2) Construction of Expression Vector for Genetically Recombinant Antibody

An expression vector for a genetically recombinant antibody can be constructed by cloning a DNA encoding at least one of CH and CL of a human antibody into an expression vector for an animal cell.

As a C region of a human antibody, CH and CL of an arbitrary human antibody can be used, and for example, CH of yl subclass and CL of κ class of a human antibody, or the like can be used. As a DNA encoding CH or CL of a human antibody, a cDNA is used, but it is also possible to use a chromosomal DNA composed of an exon and an intron.

As the expression vector for an animal cell, any vector can be used as long as it can incorporate a gene encoding a C region of a human antibody and express the gene, and for example, pAGE107 [Cytotechnol., 3, 133 (1990)], pAGE103 [J. Biochem., 101, 1307 (1987)], pHSG274 [Gene, 27, 223 (1984)], pKCR [Proc. Natl. Acad. Sci. USA, 78, 1527 (1981)], pSG1bd2-4 [Cytotechnol., 4, 173 (1990)], pSE1UK1Sed1-3 [Cytotechnol., 13, 79 (1993)], INPEP4 (manufactured by Biogen-IDEC, Inc.), N5KG1val (US Patent No. 6,001,358), N5KG4PE R409K (described in WO 2006/033386), an N5KG2 vector (described in WO 2003/033538), a transposon vector (WO 2010/143698), or the like can be used.

As a promoter and an enhancer of the expression vector for an animal cell, an SV40 early promoter [J. Biochem., 101, 1307 (1987)], Moloney murine leukemia virus LTR [Biochem. Biophys. Res. Commun., 149, 960 (1987), a CMV promoter (US Patent No. 5,168,062), or a promoter [Cell, 41, 479 (1985)] and an enhancer [Cell, 33, 717 (1983)] of an immunoglobulin H chain, or the like can be used.

In the expression of a genetically recombinant antibody, a vector carrying both genes of the antibody H chain and L chain (tandem-type vector) [J. Immunol. Methods, 167, 271 (1994)] is used from the viewpoints of ease of construction of the vector, ease of introduction into an animal cell, balance of the expression levels of the antibody H chain and L chain in the cell, and the like, however, multiple vectors separately carrying each of the genes of the antibody H chain and L chain (separation-type vectors) can also be used in combination.

As the tandem-type expression vector for a genetically recombinant antibody, pKANTEX93 (WO 97/10354), pEE18 [Hybridoma, 17, 559 (1998)], N5KG1val (US Patent No. 6,001,358), N5KG4PE R409K (described in WO 2006/033386), an N5KG2 vector (described in WO 2003/033538), a Tol2 transposon vector (WO 2010/143698), or the like is used.

### (3) Construction of Chimeric Antibody Expression Vector

By cloning the cDNA encoding VH or VL of a non-human antibody obtained in (1) upstream of each gene encoding CH or CL of a human antibody in the expression vector for a genetically recombinant antibody obtained in (2), a chimeric antibody expression vector can be constructed.

First, in order to ligate the cDNA encoding VH or VL of a non-human antibody at the 3'-end side to CH or CL of a human antibody at the 5'-end side, cDNAs of VH and VL designed so that the nucleotide sequence of a ligation region encodes an appropriate amino acid and to become an appropriate restriction enzyme recognition sequence are produced. Subsequently, the produced cDNAs of VH and VL are each cloned upstream of each gene encoding CH or CL of a human antibody in the expression vector for a genetically recombinant antibody obtained in (2) so that they are expressed in an appropriate form, whereby a chimeric antibody expression vector is constructed.

In addition, each cDNA encoding VH or VL of a non-human antibody is amplified by a PCR method using a synthetic DNA containing an appropriate restriction enzyme recognition sequence at both ends, and is cloned into the expression vector for a genetically recombinant antibody obtained in (2), whereby a chimeric antibody expression vector can also be constructed.

### (4) Production of cDNA Encoding V Region of Humanized Antibody

A cDNA encoding VH or VL of a humanized antibody can be produced as follows. First, each amino acid sequence of a framework region (hereinafter referred to as FR) of VH or VL of a human antibody, to which the amino acid sequence of CDR of VH or VL of a non-human antibody obtained in (1) is to be grafted is selected.

As the amino acid sequence of FR to be selected, any amino acid sequence can be used as long as it is derived from a human antibody. For example, an amino acid sequence of FR of a human antibody registered in a database such as Protein Data Bank, or a common amino acid sequence in each subgroup of FR of a human antibody [Sequences of Proteins of Immunological Interest, US Dept. Health and Human Services (1991)], or the like is used. In order to suppress a decrease in the binding activity of an antibody, an amino acid sequence of human FR having a homology as high as possible (60% or more) with the amino acid sequence of FR of VH or VL of the original non-human antibody is selected.

Subsequently, each of the amino acid sequences of CDRs of the original non-human antibody is grafted to the selected amino acid sequence of FR of VH or VL of a human antibody, and each amino acid sequence of VH or VL of a humanized antibody is designed. By converting the designed amino acid sequence into a DNA sequence in consideration of the usage frequency of codons found in the nucleotide sequence of the antibody gene [Sequences of Proteins of Immunological Interest, US Dept. Health and Human Services (1991)], each cDNA sequence of VH or VL of a humanized antibody is designed.

Based on the designed cDNA sequence, several synthetic DNAs having a length of around 100 to 150 nucleotides are synthesized and PCR is performed using them. In this case, from the viewpoint of the reaction efficiency in the PCR and the length of a synthesizable DNA, preferably 4 to 6 synthetic DNAs are designed for each of the H chain and the L chain. In addition, it is also possible to synthesize and use a synthetic DNA having a full-length variable region.

Further, by introducing an appropriate restriction enzyme recognition sequence at the 5' end of the synthetic DNA located at both ends, a cDNA encoding VH or VL of a humanized antibody can be easily cloned into the expression vector for a genetically recombinant antibody obtained in (2). After PCR, each amplified product is cloned into a plasmid such as pBluescript SK(-) (manufactured by Stratagene Corporation), the nucleotide sequence is determined by the same method as described in (1), and thus a plasmid containing a DNA sequence encoding the amino acid sequence of VH or VL of a desired humanized antibody is obtained.

### (5) Modification of Amino Acid Sequence of V Region of Humanized Antibody

The antigen binding activity of a humanized antibody prepared merely by grafting only CDRs of VH and VL of a non-human antibody to FRs of VH and VL of a human antibody is lowered as compared with that of the original non-human antibody [BIO/TECHNOLOGY, 9, 266 (1991)]. For this reason, the lowered antigen binding activity of the humanized antibody can be increased by identifying amino acid residues directly involved in the binding to an antigen, amino acid residues interacting with the amino acid residues of CDRs, and amino acid residues maintaining the conformation of the antibody and indirectly involved in the binding to an antigen in the amino acid sequence of FRs of VH and VL of the human antibody, and substituting the amino acid residues with the amino acid residues of the original non-human antibody.

In order to identify the amino acid residues of FR involved in the antigen binding activity, it is possible to construct and analyze the conformation of the antibody using X-ray crystallography [J. Mol. Biol., 112, 535 (1977)], or computer modeling [Protein Engineering, 7, 1501 (1994)], or the like. Further, it is possible to obtain a modified humanized antibody having a necessary antigen binding activity by producing several types of modified antibodies for each antibody, and repeatedly examining the correlation with each antigen binding activity thereof through trial and error.

The amino acid residues of FRs of VH and VL of a human antibody can be modified by performing the PCR described in (4) using a synthetic DNA for modification. With respect to the amplification product after the PCR, the nucleotide sequence is determined to confirm that the desired modification has been carried out by the method described in (1).

### (6) Construction of Expression Vector for Humanized Antibody

An expression vector for a humanized antibody can be constructed by cloning each cDNA encoding VH or VL of the constructed humanized antibody upstream of each gene encoding CH or CL of a human antibody of the expression vector for a genetically recombinant antibody obtained in (2).

For example, the cloning is performed upstream of each gene encoding CH or CL of a human antibody in the expression vector for a genetically recombinant antibody obtained in (2) by introducing an appropriate restriction enzyme recognition sequence at the 5' end of the synthetic DNA located at both ends among the synthetic DNAs used when constructing VH or VL of the humanized antibody obtained in (4) and (5) so that they are expressed in an appropriate form.

### (7) Construction of Expression Vector for Human Antibody

When a hybridoma that produces a monoclonal antibody is established using an animal that produces a human antibody as an immunized animal, the amino acid sequences and the cDNA sequences of VH and VL of a human antibody can be obtained in (1). Therefore, by cloning each gene encoding VH or VL of a human antibody obtained in (1) upstream of each gene encoding CH or CL of a human antibody of the expression vector for a genetically recombinant antibody obtained in (2), an expression vector for a human antibody can be constructed.

### (8) Transient Expression of Genetically Recombinant Antibody

By transiently expressing a genetically recombinant antibody using the expression vector for a genetically recombinant antibody obtained in (3), (6), and (7), or an expression vector obtained by modification thereof, the antigen binding activities of many types of genetically recombinant antibodies obtained can be efficiently evaluated.

As a host cell into which the expression vector is introduced, any cell can be used as long as it is a host cell capable of expressing a genetically recombinant antibody, and for example, a COS-7 cell [American Type Culture Collection (ATCC) number: CRL1651] is used. In the introduction of the expression vector into a COS-7 cell, a DEAE-dextran method [Methods in Nucleic Acids Res., CRC press (1991)], a lipofection method [Proc. Natl. Acad. Sci. USA, 84, 7413 (1987)], or the like is used.

After the introduction of the expression vector, the expression level and the antigen binding activity of the genetically recombinant antibody in a culture supernatant are measured using an enzyme immunoassay method [Monoclonal Antibodies-Principles and practice, Third Edition, Academic Press (1996), Antibodies-A Laboratory Manual, Cold Spring Harbor Laboratory (1988), Monoclonal Antibody Experimental Manual, Kodansha scientific books (1987)], or the like.

### (9) Acquisition of Stable Expression Cell Line of Genetically Recombinant Antibody and Preparation of Genetically Recombinant Antibody

A transformant strain that stably expresses a genetically recombinant antibody can be obtained by introducing the expression vector for a genetically recombinant antibody obtained in (3), (6), and (7) into an appropriate host cell.

As the introduction of the expression vector into a host cell, for example, an electroporation method [JP-A-H2-257891, Cytotechnology, 3, 133 (1990)], a calcium ion method, an electroporation method, a spheroplast method, a lithium acetate method, a calcium phosphate method, a lipofection method, and the like are exemplified. In addition, as a method for introducing a gene into an animal described below, for example, a microinjection method, a method for introducing a gene into an ES cell using an electroporation method or a lipofection method, a nuclear transfer method, and the like are exemplified.

As a host cell into which the expression vector for a genetically recombinant antibody is introduced, any cell can be used as long as it is a host cell capable of expressing a genetically recombinant antibody. For example, mouse SP2/0-Ag14 cells (ATCC CRL 1581), mouse P3X63-Ag8.653 cells (ATCC CRL 1580), Chinese hamster CHO-K1 cells (ATCC CCL-61), DUKXB11 (ATCC CCL-9096), Pro-5 cells (ATCC CCL-1781), CHO-S cells (Life Technologies, Cat No. 11619), dihydrofolate reductase gene (dhfr)-deficient CHO cells (CHO/DG44 cells) [Proc. Natl. Acad. Sci. USA, 77, 4216 (1980)], Lec13 cells having acquired lectin resistance [Somatic Cell and Molecular Genetics, 12, 55 (1986)], α1,6-fucosyltransferase gene-deficient CHO cells (WO 2005/035586 and WO 02/31140), Rat YB2/3HL.P2.G11.16Ag.20 cells (ATCC No.: CRL 1662), and the like are used.

In addition, it is also possible to use a host cell in which the activity of a protein such as an enzyme involved in the synthesis of intracellular sugar nucleotide GDP-fucose, a protein such as an enzyme involved in sugar chain modification such that the 1-position of fucose is α-linked to the 6-position of N-acetylglucosamine at the reducing end of an N-glycoside-linked complex sugar chain, a protein involved in the transport of intracellular sugar nucleotide GDP-fucose to the Golgi body, or the like is decreased or lost, for example, α1,6-fucosyltransferase gene-deficient CHO cells (WO 2005/035586 and WO 02/31140), or the like.

After introduction of the expression vector, a transformant strain that stably expresses a genetically recombinant antibody is selected by culturing the transformant strain in a medium for animal cell culture containing a drug such as G418 sulfate (hereinafter referred to as G418) (JP-A-H2-257891).

As the medium for animal cell culture, RPMI 1640 medium (manufactured by Invitrogen, Inc.), GIT medium (manufactured by Nippon Pharmaceutical Co., Ltd.), EX-CELL 301 medium (manufactured by JRH Biosciences, Inc.), EX-CELL 302 medium (manufactured by JRH Bioscience, Inc.), EX-CELL 325 medium (manufactured by JRH Bioscience., Inc.), IMDM medium (manufactured by Invitrogen, Inc.) or Hybridoma-SFM medium (manufactured by Invitrogen, Inc.), or a medium in which any of various additives such as FBS is added to any of these media, or the like is used. By culturing the obtained transformant strain in the medium, a genetically recombinant antibody is expressed and accumulated in the culture supernatant. The expression level and the antigen binding activity of the genetically recombinant antibody in the culture supernatant can be measured by an ELISA method or the like. In addition, the expression level of the genetically recombinant antibody produced by the transformant strain can be increased using a DHFR amplification system (JP-A-H2-257891) or the like.

The genetically recombinant antibody can be purified using a protein A column from the culture supernatant of the transformant strain [Monoclonal Antibodies - Principles and Practice, Third Edition, Academic Press (1996), Antibodies - A Laboratory Manual, Cold Spring Harbor Laboratory (1988)]. In addition, the purification can also be carried out by combining methods used for purifying a protein such as gel filtration, ion exchange chromatography, and ultrafiltration.

The molecular weights of an H chain, an L chain, or the entire antibody molecule of a purified genetically recombinant antibody can be measured using a polyacrylamide gel electrophoresis method [Nature, 227, 680 (1970)], or a Western blotting method [Monoclonal Antibodies - Principles and Practice, Third Edition, Academic Press (1996), Antibodies - A Laboratory Manual, Cold Spring Harbor Laboratory (1988)], or the like.

### 3. Designing of Bispecific Antibody

The bispecific antibody of the present invention can be produced by designing each of the second antigen binding domain and the IgG portion, and designing the bispecific antibody in which these are linked.

3-1. Designing of IgG Portion

The IgG portion can be obtained by obtaining monoclonal antibodies using the method described in the above 1., determining the cDNA sequences of CDR and a variable region of each antibody using the method described in the above 2., and designing the IgG portion including the CDR or the variable region of the antibody.

### 3-2. Designing of Second Antigen Binding Domain

When CDR or a variable region of an antibody is included in the second antigen binding domain, the second antigen binding domain can be produced by determining the DNA sequence of the CDR or the variable region of the antibody using the method described in the above 1. and 2. and designing the second antigen binding domain including them. As such a second antigen binding domain, a single-stranded one obtained by binding VH and VL either directly or via an appropriate linker as scFV or the like, one designed to be expressed in the form of a double strand as Fab, dsFv, or the like, and bound via a disulfide bond after expression, or VHH or the like can also be used. Further, as the second antigen binding domain, a peptide fragment including a ligand for an antigen protein or a portion that binds to an antigen protein for the ligand can also be used. The antigen binding activity of the second antigen binding domain is evaluated by the above-mentioned method, and one retaining the antigen binding activity can be selected.

### 4. Production of Bispecific Antibody

The antigen binding domain can be isolated and obtained by a technique such as a phage display method or a yeast display method other than the method using a hybridoma described in the above 1. [Emmanuelle Laffy et. al., Human Antibodies 14, 33-55, (2005)].

Further, when a bispecific antibody composed of multiple VHs and a single VL is produced, a screening using a phage display method or the like is performed and each VH most suitable for the single VL is selected so that each antigen binding domain contained in the bispecific antibody reacts with each specific antigen.

Specifically, first, an animal is immunized with a first antigen using the method described in the above 1., and a hybridoma is produced from its spleen, and then, a DNA sequence encoding a first antigen binding domain is cloned. Subsequently, an animal is immunized with a second antigen, and a cDNA library is prepared from its spleen, and then, a DNA encoding the amino acid sequence of VH is obtained from the library by PCR.

Subsequently, a phage library expressing scFv in which VH obtained by immunization with the second antigen and VL of the first antigen binding domain are linked is produced, and a phage displaying scFv that specifically binds to the second antigen is selected by panning using the phage library. From the selected phage, a DNA sequence encoding the amino acid sequence of VH of a second antigen binding domain is cloned.

Further, when the second antigen binding domain is Fab, a DNA sequence encoding the amino acid sequence of a polypeptide in which VH of the first antigen binding domain and VH of the second antigen binding domain are linked through a heavy chain constant region is designed, and the DNA sequence is inserted into, for example, the expression vector for a genetically recombinant antibody described in the above 2. (2) together with a DNA sequence encoding the amino acid sequence of the single VL, whereby the expression vector for the bispecific antibody of the present invention can be constructed.

### 5. Evaluation of Activity of Bispecific Antibody of the Present Invention

The evaluation of the activity of the purified bispecific antibody can be carried out as follows.

The binding activity of the bispecific antibody of the present invention to a cell line expressing at least one of CD40 and FAP can be measured using the binding assay system described in the above 1. (7).

The CDC activity or the ADCC activity against a cell expressing at least one of CD40 and FAP can be measured by a known measurement method [Cancer Immunol. Immunother., 36, 373 (1993)].

The cell death-inducing activity (also referred to as anticellular activity) of the bispecific antibody of the present invention can be measured by the following method. For example, cells are seeded in a 96-well plate, and after adding an antibody and culturing the cells for a certain period of time, WST-8 reagent (manufactured by Dojindo Molecular Technologies, Inc.) is allowed to react, and then an absorbance at 450 nm is measured with a plate reader to measure the viability of the cells.

The signal transduction into a cell from CD40 or FAP can be evaluated by detecting phosphorylation of a protein in the cell through Western blotting or the like.

### 6. Therapeutic Method for Disease Using Bispecific Antibody of the Present Invention

The bispecific antibody of the present invention can be used for a treatment of a disease associated with at least one of CD40 and FAP, preferably a disease involved in a cell that expresses CD40 or FAP. As the disease associated with at least one of CD40 and FAP, for example, a malignant tumor, a cancer, and the like are exemplified.

Examples of the malignant tumor and the cancer include, large intestine cancer, colorectal cancer, lung cancer, breast cancer, glioma, malignant melanoma (melanoma), thyroid cancer, renal cell carcinoma, leukemia, lymphoma, T cell lymphoma, stomach cancer, pancreatic cancer, cervical cancer, endometrial cancer, ovarian cancer, bile duct cancer, esophageal cancer, liver cancer, head and neck cancer, squamous cell cancer, skin cancer, urinary tract cancer, bladder cancer, prostate cancer, choriocarcinoma, pharyngeal cancer, laryngeal cancer, pleural tumor, arrhenoblastoma, endometrial hyperplasia, endometriosis, embryoma, fibrosarcoma, Kaposi's sarcoma, angioma, cavernous hemangioma, angioblastoma, retinoblastoma, astrocytoma, neurofibroma, oligodendroglioma, medulloblastoma, neuroblastoma, glioma, rhabdomyosarcoma, glioblastoma, osteogenic sarcoma, leiomyosarcoma, Wilms tumor, and the like.

A therapeutic agent containing the bispecific antibody of the present invention or a derivative thereof may contain only the bispecific antibody or a derivative thereof as an active ingredient, however, in general, it is provided as a pharmaceutical preparation produced using a method known in the technical field of pharmaceutics by mixing it together with one or more pharmacologically acceptable carriers.

Examples of the route of administration include oral administration or parenteral administration such as intraoral, intra-airway, intrarectal, subcutaneous, intramuscular, and intravenous administration. Examples of the dosage form include a spray, a capsule, a tablet, a powder, a granule, a syrup, an emulsion, a suppository, an injection, an ointment, a tape, and the like. Various pharmaceutical preparations can be produced by a conventional method using an excipient, a filler, a binder, a wetting agent, a disintegrating agent, a surfactant, a lubricant, a dispersant, a buffer, a preservative, a solubilizing agent, an antiseptic, a coloring agent, a flavoring agent, a stabilizer, and the like that are generally used.

Examples of the excipient include lactose, fructose, glucose, corn starch, sorbit, crystalline cellulose, sterile water, ethanol, glycerol, physiological saline, a buffer solution, and the like. Examples of the disintegrating agent include starch, sodium alginate, gelatin, calcium carbonate, calcium citrate, dextrin, magnesium carbonate, synthetic magnesium silicate, and the like.

Examples of the binder include methyl cellulose or a salt thereof, ethyl cellulose, gum arabic, gelatin, hydroxypropyl cellulose, polyvinylpyrrolidone, and the like. Examples of the lubricant include talc, magnesium stearate, polyethylene glycol, hydrogenated vegetable oil, and the like.

Examples of the stabilizer include amino acids such as arginine, histidine, lysine and methionine, human serum albumin, gelatin, dextran 40, methyl cellulose, sodium sulfite, sodium metasulfite, and the like.

Examples of other additives include syrup, vaseline, glycerin, ethanol, propylene glycol, citric acid, sodium chloride, sodium nitrite, sodium phosphate, and the like.

Examples of the pharmaceutical preparation suitable for oral administration include an emulsion, a syrup, a capsule, a tablet, a powder, a granule, and the like.

A liquid preparation such as an emulsion or a syrup is produced using water, a saccharide such as sucrose, sorbitol, or fructose, a glycol such as polyethylene glycol or propylene glycol, an oil such as sesame oil, olive oil, or soybean oil, a preservative such as p-hydroxybenzoic acid ester, a flavor such as strawberry flavor or peppermint, or the like, as an additive.

A capsule, a tablet, a powder, a granule, or the like can be produced using an excipient such as lactose, glucose, sucrose, or mannitol, a disintegrating agent such as starch or sodium alginate, a lubricant such as magnesium stearate or talc, a binder such as polyvinyl alcohol, hydroxypropyl cellulose, or gelatin, a surfactant such as a fatty acid ester, a plasticizer such as glycerin, or the like as an additive.

Examples of the pharmaceutical preparation suitable for parenteral administration include an injection, a suppository, a spray, and the like. An injection is produced using a carrier composed of a salt solution, a glucose solution, or a mixture of both, or the like.

A suppository is produced using a carrier such as cacao butter, a hydrogenated fat, or carboxylic acid. A spray is produced using a carrier which does not stimulate the buccal or airway mucous membrane of a recipient and disperses the monoclonal antibody of the present invention as fine particles so as to facilitate absorption thereof, or the like. Examples of the carrier include lactose, glycerin, and the like. In addition, it can also be produced as an aerosol or a dry powder. Further, a component exemplified as the additive for the pharmaceutical preparation suitable for oral administration can also be added in the above-mentioned parenteral preparation.

An effective amount of the bispecific antibody of the present invention and an effective amount to be administered as a combination with a suitable diluent and a pharmacologically usable carrier is 0.0001 mg to 100 mg per kg of the body weight at one time, and is administered at intervals of 2 days to 8 weeks.

### 7. Diagnostic Method for Disease Using Bispecific Antibody of the Present Invention

By detecting or measuring a cell having expressed at least one of CD40 and FAP using the bispecific antibody of the present invention, it is possible to diagnose a disease associated with at least one of CD40 and FAP, preferably a disease involved in a cell that expresses CD40 or FAP.

The diagnosis of a malignant tumor or a cancer that is a disease associated with at least one of CD40 and FAP can be carried out by, for example, detecting or measuring at least one of CD40 and FAP as follows.

First, with respect to biological samples collected from the bodies of multiple healthy subjects, at least one of CD40 and FAP is detected or measured by the following immunological method using the bispecific antibody of the present invention, or a derivative thereof, and then the abundance of at least one of CD40 and FAP in the biological samples of the healthy subjects is examined.

Subsequently, the abundance of at least one of CD40 and FAP in a biological sample of a test subject is also examined in the same manner, and then, the abundance is compared with the abundance of the healthy subjects. When the abundance of at least one of CD40 and FAP of the test subject increases as compared with that of the healthy subjects, the test subject is diagnosed as having a cancer. With respect also to the diagnosis of the other diseases associated with at least one of CD40 and FAP, the diagnosis can be carried out in the same manner.

The immunological method is a method for detecting or measuring the amount of an antibody or the amount of an antigen using a labeled antigen or antibody. Examples thereof include a radioactive material labeled immune antibody method, an enzyme immunoassay method, a fluorescence immunoassay method, a luminescence immunoassay method, a Western blotting method, a physicochemical method, and the like.

Examples of the radioactive material labeled immune antibody method include a method in which the bispecific antibody of the present invention is allowed to react with an antigen or a cell or the like having expressed an antigen, and further an anti-immunoglobulin antibody or a binding fragment subjected to radiolabeling is allowed to react therewith, followed by measurement with a scintillation counter or the like.

Examples of the enzyme immunoassay method include a method in which the bispecific antibody of the present invention is allowed to react with an antigen or a cell or the like having expressed an antigen, and further an anti-immunoglobulin antibody or a binding fragment subjected to labeling is allowed to react therewith, followed by measurement of a coloring dye with an absorptiometer. For example, a sandwich ELISA method and the like are exemplified.

As a labeling substance used in the enzyme immunoassay method, a known enzyme label [Enzyme Immunoassay Method, IGAKU-SHOIN Ltd. (1987)] can be used. For example, an alkaline phosphatase label, a peroxidase label, a luciferase label, a biotin label, or the like is used.

The sandwich ELISA method is a method in which after binding an antibody to a solid phase, an antigen that is a detection or measurement target is trapped, and then a second antibody is allowed to react with the trapped antigen. In the ELISA method, two types of antibodies that are antibodies or antibody fragments binding to an antigen desired to be detected or measured and have different antigen binding sites are prepared, and among them, a first antibody or antibody fragment is adsorbed onto a plate (for example, a 96-well plate) in advance, and subsequently, a second antibody or antibody fragment is labeled with a fluorescent substance such as FITC, an enzyme such as peroxidase, biotin, or the like beforehand. Cells separated from the inside of the living body or a homogenate liquid thereof, tissues or a homogenate liquid thereof, a cell culture supernatant, serum, pleural effusion, ascites, intraocular fluid, or the like is allowed to react with the plate on which the antibody is adsorbed, and thereafter the labeled antibody or antibody fragment is allowed to react therewith, and then, a detection reaction is performed according to the labeling substance. From a calibration curve prepared by serially diluting an antigen at a known concentration, the antigen concentration in the test sample is calculated.

As the antibody used in the sandwich ELISA method, either a polyclonal antibody or a monoclonal antibody may be used, and an antibody fragment such as Fab, Fab', or F(ab)₂ may be used. The combination of the two types antibodies used in the sandwich ELISA method may be a combination of monoclonal antibodies or antibody fragments thereof that bind to different epitopes, or may be a combination of a polyclonal antibody and a monoclonal antibody or an antibody fragment thereof.

As the fluorescence immunoassay method, measurement is performed by, for example, a method described in the document [Monoclonal Antibodies - Principles and Practice, Third Edition, Academic Press (1996), Monoclonal Antibody Experimental Manual, Kodansha scientific books (1987)], or the like. As a labeling substance used in the fluorescence immunoassay method, a known fluorescent label [Fluorescent Antibody Method, Soft Science, Inc. (1983)] can be used. For example, FITC, RITC, or the like is used.

As the luminescence immunoassay method, measurement is performed by, for example, a method described in the document [Bioluminescence and Chemiluminescence Clinical Test 42, Hirokawa-Shoten Ltd. (1998)], or the like. As a labeling substance used in the luminescence immunoassay method, a known luminescent label is exemplified, and for example, an acridinium ester, lophine, or the like is used.

As the Western blotting method, measurement is performed by fractionating an antigen or a cell or the like having expressed an antigen by SDS (sodium dodecyl sulfate) - PAGE [Antibodies - A Laboratory Manual Cold Spring Harbor Laboratory (1988)], thereafter blotting the gel onto a polyvinylidene fluoride (PVDF) membrane or a nitrocellulose membrane, allowing an antibody or an antibody fragment that binds to the antigen to react with the membrane, and then further allowing an anti-IgG antibody or an antibody fragment thereof labeled with a fluorescent substance such as FITC, labeled with an enzyme such as peroxidase, or labeled with biotin, or the like to react therewith, followed by visualizing the label. One example is shown below.

First, cells or tissues having expressed a polypeptide containing a desired amino acid sequence are lysed, and 0.1 to 30 µg in terms of protein amount per lane is electrophoresed by an SDS-PAGE method under reducing conditions. Subsequently, the electrophoresed protein is transferred to a PVDF membrane and is allowed to react with PBS containing 1 to 10% BSA (hereinafter referred to as BSA-PBS) for 30 minutes at room temperature to perform a blocking operation. Then, the bispecific antibody of the present invention is allowed to react therewith, and the membrane is washed with PBS containing 0.05 to 0.1% Tween 20 (Tween-PBS), and then a goat anti-mouse IgG labeled with peroxidase is allowed to react therewith for 2 hours at room temperature. By performing washing with Tween-PBS, and detecting a band to which the antibody is bound using ECL Western Blotting Detection Reagents (manufactured by Amersham, Inc.) or the like, an antigen is detected. As the antibody used for detection by Western blotting, an antibody capable of binding to a polypeptide that does not retain a natural conformation is used.

As the physicochemical method, for example, by binding at least one of CD40 and FAP which are antigens to the bispecific antibody of the present invention, an aggregate is formed, and the aggregate is detected. As another physicochemical method, a capillary tube method, a one-dimensional immunodiffusion method, an immunoturbidimetric method, a latex immunoturbidimetric method [Outline of Clinical Examination Method, KANEHARA & Co., LTD. (1998)], or the like can also be used.

In the latex immunoturbidimetric method, when a carrier such as a polystyrene latex having a particle diameter of about 0.1 to 1 µm sensitized with an antibody or an antigen is used to cause an antigen-antibody reaction with a corresponding antigen or antibody, the scattered light is increased in a reaction solution and the transmitted light is decreased. The antigen concentration or the like in a test sample is measured by detecting this change as an absorbance or an integrating sphere turbidity.

On the other hand, for the detection or measurement of a cell having expressed at least one of CD40 and FAP, a known immunological detection method can be used, but it is preferred to use an immunoprecipitation method, an immunocytological staining method, an immunohistological staining method, a fluorescent antibody staining method, or the like.

As the immunoprecipitation method, a cell or the like having expressed at least one of CD40 and FAP is allowed to react with the bispecific antibody of the present invention, and then a carrier having a specific binding ability to an immunoglobulin such as Protein G Sepharose is added thereto, thereby precipitating an antigen-antibody complex.

Alternatively, the detection or measurement of cells having expressed at least one of CD40 and FAP can also be carried out by the following method. First, the bispecific antibody of the present invention is immobilized on a 96-well plate for ELISA, followed by blocking with BSA-PBS. Subsequently, BSA-PBS is discarded, and the plate is well washed with PBS, and then, a lysate solution of cells or tissues having expressed at least one of CD40 and FAP is allowed to react therewith. From the plate after being well washed, an immunoprecipitated material is extracted with a sample buffer for SDS-PAGE, and then detected by the above-mentioned Western blotting.

The immunocytological staining method or the immunohistological staining method is a method in which a cell or a tissue having expressed an antigen or the like is treated with a surfactant or methanol, or the like for enhancing the permeability of the antibody in some cases, and then allowed to react with the bispecific antibody of the present invention, and further react with an anti-immunoglobulin antibody or a binding fragment thereof fluorescently labeled with FITC or the like, labeled with an enzyme such as peroxidase, or labeled with biotin, or the like, and thereafter the label is visualized, and then observed with a microscope. In addition, detection can be carried out by a fluorescent antibody staining method in which a fluorescently labeled antibody is allowed to react with a cell and analyzed with a flow cytometer [Monoclonal Antibodies - Principles and Practice, Third Edition, Academic Press (1996), Monoclonal Antibody Experimental Manual, Kodansha scientific books (1987)]. In particular, the bispecific antibody of the present invention enables detection of at least one of CD40 and FAP expressed on a cell membrane by a fluorescent antibody staining method.

In addition, when the FMAT 8100 HTS system (manufactured by Applied Biosystems, Inc.) or the like is used among the fluorescent antibody staining methods, it is possible to measure the amount of an antigen or the amount of an antibody without separating the formed antibody-antigen complex from a free antibody or antigen not involved in the formation of the antibody-antigen complex.

### EXAMPLES

Hereinafter, the present invention will be more specifically described by way of Examples, however, the present invention is not limited to the following Examples. [Example 1] Preparation of Soluble Human and Monkey CD40 Antigens and Soluble Human FAP Antigen

### 1. Preparation of Soluble Antigens of Human CD40 and Monkey CD40

Each of the extracellular domain proteins of human and monkey CD40 in which FLAG-Fc was added to the C terminus was produced by a method described below. The nucleotide sequence encoding the extracellular domain of human CD40 is shown in SEQ ID NO: 1, the amino acid sequence deduced from the nucleotide sequence is shown in SEQ ID NO: 2, the nucleotide sequence encoding the extracellular domain of monkey CD40 is shown in SEQ ID NO: 3, and the amino acid sequence deduced from the nucleotide sequence is shown in SEQ ID NO: 4.

### (1) Production of Human and Monkey CD40-FLAG-Fc Vectors

A gene fragment of the extracellular domain of human CD40 composed of the nucleotide sequence represented by SEQ ID NO: 1 was produced based on the nucleotide sequence of a human CD40 gene (Genbank Accession Number: NM_001250, SEQ ID NO: 5, an amino acid sequence encoded by the gene is represented by SEQ ID NO: 6).

An INPEP4 vector (manufactured by Biogen-IDEC GmbH) containing a FLAG-tag and an Fc region of human IgG was digested with restriction enzymes KpnI and XbaI, and a gene fragment of the extracellular domain to which a human CD40 signal sequence coding region composed of the nucleotide sequence at positions 1 to 60 of the nucleotide sequence represented by SEQ ID NO: 1 was added was inserted at an appropriate site, whereby a human CD40-FLAG-Fc expression vector was produced.

In the same manner, a monkey CD40-FLAG-Fc expression vector containing a gene fragment of the extracellular domain of monkey CD40 composed of the nucleotide sequence represented by SEQ ID NO: 3 was produced based on the nucleotide sequence of a monkey CD40 gene (SEQ ID NO: 7, an amino acid sequence encoded by the gene is represented by SEQ ID NO: 8) cloned from a monkey peripheral blood mononuclear cell (PBMC).

### (2) Production of Human and Monkey CD40-FLAG-Fc Proteins

The human CD40-FLAG-Fc expression vector produced in 1-(1) was introduced into HEK 293 cells using FreeStyle (trademark) 293 Expression System (manufactured by Thermo Fisher, Inc.) and the cells were cultured to express a protein in a transient expression system. The culture supernatant was collected 5 days after introduction of the vector, and filtered through a membrane filter (manufactured by Millipore Corporation) having a pore diameter of 0.22 µm.

The culture supernatant was subjected to affinity purification using a Protein A resin (MabSelect, manufactured by GE Healthcare, Inc.). The antibody adsorbed to the Protein A resin was washed with Dulbecco's phosphate buffered saline [D-PBS(-) without Ca and Mg, liquid; hereinafter referred to as D-PBS(-), manufactured by Nacalai Tesque, Inc.], eluted with a 20 mM sodium citrate and 50 mM NaCl buffer solution (pH 3.4) and collected in a tube containing a 1 M sodium phosphate buffer solution (pH 7.0).

Subsequently, the buffer solution was replaced with D-PBS(-) by ultrafiltration using VIVASPIN (manufactured by Sartrius stealin), followed by filter sterilization with a membrane filter Millex-Gv (manufactured by Millipore Corporation) having a pore diameter of 0.22 µm, whereby a human CD40-FLAG-Fc protein was produced. In the same manner, a monkey CD40-FLAG-Fc protein was produced using the monkey CD40-FLAG-Fc expression vector produced in 1-(1). The concentration of the obtained protein was determined by measuring an absorbance at a wavelength of 280 nm and performing calculation using an extinction coefficient estimated from the amino acid sequence of each protein.

### (3) Production of Human and Monkey CD40-GST Vectors

An N5 vector (manufactured by Biogen-IDEC GmbH) containing a GST region was digested with restriction enzymes BglII and KpnI, and a gene fragment of the extracellular domain of human CD40 composed of the nucleotide sequence represented by SEQ ID NO: 1 described in 1-(1) was inserted at an appropriate site, whereby a human CD40-GST expression vector was produced. In the same manner, a monkey CD40-GST expression vector containing a gene fragment of the extracellular domain composed of the nucleotide sequence represented by SEQ ID NO: 3 was produced.

### (4) Production of Human and Monkey CD40-GST Proteins

The human CD40-GST vector produced in 1-(3) was introduced into HEK 293 cells in the same manner as in 1-(2), and the cells were cultured, and then, the culture supernatant was filtered through a membrane filter. The culture supernatant was reacted with Glutathione Sepharose 4B (manufactured by GE Healthcare), and washed with D-PBS(-), and then subjected to affinity purification using 10 mM Glutathione in 50 mM Tris-HCl (pH 8.0) as an elution buffer solution.

The eluted fusion protein solution was subjected to ultrafiltration and filter sterilization with a membrane filter in the same manner as in 1-(2), whereby a human CD40-GST protein was obtained. Further, by using the monkey CD40-GST vector, a monkey CD40-GST protein was obtained in the same manner. The concentration of the obtained protein was determined by measuring an absorbance at a wavelength of 280 nm and performing calculation using an extinction coefficient estimated from the amino acid sequence of each protein.

### 2. Preparation of Soluble Antigens of Human and Mouse FAP

Each of the extracellular domain proteins of human and mouse FAP in which FLAG-Fc or GST was added was produced by a method described below. The nucleotide sequence encoding the extracellular domain of human FAP is represented by SEQ ID NO: 9, and the amino acid sequence deduced from the nucleotide sequence is represented by SEQ ID NO: 10. The nucleotide sequence encoding the extracellular domain of mouse FAP is represented by SEQ ID NO: 11, and the amino acid sequence deduced from the nucleotide sequence is represented by SEQ ID NO: 12.

Human and mouse FAP-FLAG-Fc proteins were obtained in the same manner as described in 1-(1) and (2) based on a human FAP gene represented by SEQ ID NO: 13 (Genbank Accession Number: NM_004460, an amino acid sequence encoded by the gene is represented by SEQ ID NO: 14), and a mouse FAP gene represented by SEQ ID NO: 15 (Genbank Accession Number: NM_007986, an amino acid sequence encoded by the gene is represented by SEQ ID NO: 16), respectively.

Further, each of human and mouse FAP-GST proteins was obtained in the same manner as described in 1-(3) and (4). The concentration of each of the obtained proteins was determined by measuring an absorbance at a wavelength of 280 nm and performing calculation using an extinction coefficient estimated from the amino acid sequence of each protein.

### [Example 2] Production of Human FAP-Expressing HEK 293 Cells

A target FAP expression vector Tn-pMug-Hygro-human FAP was obtained by inserting a human FAP gene represented by SEQ ID NO: 9 into a pUC vector (manufactured by Takara Bio, Inc.) using In-Fusion HD Cloning Kit (manufactured by Takara Bio, Inc.).

The obtained human FAP expression vector Tn-pMug-Hygro-human FAP was introduced into HEK 293 cells using FuGENE HD Transfection Reagent (manufactured by Promega Corporation). In order to select cells transfected with the expression vector, the culture medium was replaced with a 1 mg/mL hygromycin-containing medium, and drug-resistant cells were obtained.

From the obtained drug selected cells, a FAP high expression fraction that binds to an anti-FAP antibody sibrotuzumab was subjected to single cell isolation using a cell sorter SH800 (manufactured by Sony Corporation). The expression of FAP in the obtained clone was confirmed by FACS Canto (trademark) II (BD Bioscience). The obtained human FAP-expressing cell is hereinafter referred to as human FAP-HEK 293.

### [Example 3] Acquisition of Anti-CD40 Monoclonal Antibody and Expression Vector

### 1. Production of CD40-Immunized Human Antibody M13 Phage Library

As an immunogen, the human CD40-FLAG-Fc produced in Example 1 was intraperitoneally administered to a human antibody-producing mouse [Ishida & Lonberg, IBC's 11th Antibody Engineering, Abstract 2000; Ishida, I. et al., Cloning & Stem Cells 4, 85-96 (2002) and Isamu Ishida (2002) Experimental medicine 20, 6, 846-851] a total of 4 times. Only at the first immunization, Alum gel (2 mg/mouse) and pertussis vaccine (1 × 10⁹ vaccines/mouse) were added as adjuvants.

The second immunization was performed two weeks after the first immunization, the third immunization was performed 1 week thereafter, the final immunization was performed 10 days after the third immunization, and the mouse was dissected 4 days after the final immunization and the spleen was surgically excised. The excised spleen was placed on a cell strainer (manufactured by Falcon, Inc.), and cells were transferred to a tube while gently smashing with a silicon rod and centrifuged. Thereafter, the precipitated cells were reacted with a red blood cell depletion reagent (manufactured by Sigma-Aldrich Co. LLC) in ice for 3 minutes, followed by further centrifugation.

RNA was extracted from the obtained spleen cells using RNeasy Mini kit (manufactured by QIAGEN, Inc.), cDNAs were amplified using a SMARTer RACE cDNA amplification kit (manufactured by Clontech Laboratories, Inc.), and further, a VH gene fragment was amplified by PCR. The VH gene fragment was inserted into a phagemid pCANTAB 5E (manufactured by Amersham Pharmacia, Inc.) together with a VL gene fragment that is a human antibody germ-line sequence and contains the nucleotide sequence represented by SEQ ID NO: 17 encoding the amino acid sequence (VL) of an L6 sequence, and E. coli TG1 (manufactured by Lucigen Corporation) was transformed with the phagemid, whereby plasmids were obtained.

Note that the L6 sequence encodes a light chain variable region (VL) of a human antibody composed of the amino acid sequence represented by SEQ ID NO: 18, and the amino acid sequences of CDRs 1, 2, and 3 of the VL (also represented by LCDRs 1, 2, and 3, respectively) are represented by SEQ ID NOS: 19, 20, and 21, respectively.

By infecting VCSM13 Interference Resistant Helper Phage (manufactured by Agilent Technologies, Inc.) with the obtained plasmids, a CD40-immunized human antibody M13 phage library that has the VL gene composed of the L6 sequence and includes a library of VH genes was obtained.

### 2. Acquisition of Anti-CD40 Monoclonal Antibody

By using the CD40-immunized human antibody M13 phage library, an anti-CD40 monoclonal antibody including VL containing the L6 amino acid sequence and VH containing a desired amino acid sequence was obtained by the following phage display method. MAXISORP STARTUBE (manufactured by NUNC, Inc.) in which the human CD40-GST obtained in Example 1 was immobilized and a portion to which the human CD40-GST is not bound was blocked using SuperBlock Blockig Buffer (manufactured by Thermo Fisher, Inc.), and the human antibody M13 phage library were allowed to react at room temperature for 1 to 2 hours, and washing was performed 3 times each with D-PBS(-) and PBS containing 0.1% Tween 20 (hereinafter referred to as PBS-T, manufactured by Wako Pure Chemical Industries, Ltd.), and thereafter, the phage was eluted with 0.1 M Gly-HCl (pH 2.2).

The eluted phage was used to infect TG1 competent cells to amply the phage, which was reacted again with human CD40-GST immobilized on MAXISORP STARTUBE, followed by washing 5 times each with D-PBS(-) and PBS-T, and thereafter, the phage was eluted with 0.1 M Gly-HCl (pH 2.2).

This operation was repeated twice or three times to concentrate the phage displaying scFv that specifically binds to human CD40. The concentrated phage was used to infect TG1, which was then inoculated in a SOBAG plate (2.0% tryptone, 0.5% Yeast extract, 0.05% NaCl, 2.0% glucose, 10 mM MgCl₂, 100 µg/mL ampicillin, and 1.5% agar) to form a colony.

The colony was inoculated and cultured, and then infected with VCSM13 Interference Resistant Helper Phage, and cultured again, whereby a monoclonal phage was obtained. By using the obtained monoclonal phage, a clone that binds to both human and monkey CD40-GST was selected by ELISA.

In the ELISA, MAXISORP (manufactured by NUNC, Inc.) in which the human or monkey CD40-GST in Example 1 was immobilized on each well and a portion to which the human or monkey CD40-GST is not bound was blocked using SuperBlock Blockig Buffer (manufactured by Thermo Fisher, Inc.) was used. To each well, each phage clone was added and reacted at room temperature for 30 minutes, and thereafter, each well was washed 3 times with PBS-T.

Subsequently, an anti-M13 antibody (manufactured by GE Healthcare, Inc.) labeled with horseradish peroxidase was diluted 5000 times with PBS-T containing 10% Block Ace (manufactured by Dainippon Pharmaceutical Co., Ltd.), and the resultant was added in an amount of 50 µL to each well, and incubated at room temperature for 30 minutes. After the microplate was washed four times with PBS-T, a TMB chromogenic substrate solution (manufactured by DAKO, Inc.) was added in an amount of 50 µL to each well and incubated at room temperature for 10 minutes. The coloring reaction was stopped by adding a 2 N HCl solution to each well (50 µL/well), and an absorbance at a wavelength of 450 nm (reference wavelength: 570 nm) was measured using a plate reader (Emax, Molecular Devices, Inc.).

A sequence analysis was performed for clones bound to both human and monkey CD40, whereby an anti-CD40 antibody R1090S55A having VL containing the amino acid sequence of L6 was obtained. The entire nucleotide sequence encoding VH of the obtained CD40 antibody R1090S55A is represented by SEQ ID NO: 22, the amino acid sequence deduced from the nucleotide sequence is represented by SEQ ID NO: 23, and the amino acid sequences of CDRs 1 to 3 of VH (hereinafter sometimes referred to as HCDRs 1 to 3) are represented by SEQ ID NOS: 24, 25, and 26, respectively.

### 3. Acquisition of Anti-CD40 Monoclonal Antibody Expression Vector

A soluble IgG expression vector into which the gene of the obtained anti-CD40 antibody R1090S55A was integrated was produced. First, the L6 gene encoding VL of R1090S55A was subcloned into the BglII-BsiWI site of the N5KG4PE R409K vector (described in WO 2006/033386).

Thereafter, the VH gene of R1090S55A was subcloned into the SalI-NheI site of the N5KG4PE R409K vector, whereby N5KG4PE R409K_R1090S55A that is an expression vector for the anti-CD40 monoclonal antibody R1090S55A having a constant region of human IgG4PE R409K was obtained.

### 4. Acquisition of Positive Control Antibody Expression Vector

In order to produce an anti-CD40 monoclonal antibody 21.4.1 (CP870,893) described in WO 2003/040170 as the positive control antibody of the anti-CD40 antibody, an expression vector was produced. The nucleotide sequence of VH of 21.4.1 is represented by SEQ ID NO: 27 and the amino acid sequence of the VH deduced from the sequence is represented by SEQ ID NO: 28. Further, the nucleotide sequence of VL of 21.4.1 is represented by SEQ ID NO: 29 and the amino acid sequence of the VL deduced from the sequence is represented by SEQ ID NO: 30.

The genes encoding VH and VL of 21.4.1 were synthesized and subcloned into the SalI-NheI and BglII-BsiWI sites of an N5KG2 vector (described in WO 2003/033538), respectively, whereby an expression vector N5KG2_21.4.1 for the anti-CD40 monoclonal antibody 21.4.1 having a constant region of human IgG2 was obtained.

### [Example 4] Acquisition of Anti-FAP Monoclonal Antibody and Expression Vector 1. Production of Human Naive Antibody M13 Phage Library

A VH gene fragment was amplified by PCR from a cDNA derived from human PBMC. The VH gene fragment was inserted into a vector in which a tag sequence of a phagemid pCANTAB 5E (manufactured by Amersham Pharmacia, Inc.) was changed to FLAG-His tag and a trypsin recognition sequence (Nature Biotechnoloy 2001, 19, 75-78) together with the VL gene fragment containing the nucleotide sequence of L6 represented by SEQ ID NO: 17, and E. coli TG1 (manufactured by Lucigen Corporation) was transformed with the vector, whereby plasmids were obtained.

By infecting VCSM13 Interference Resistant Helper Phage (manufactured by Agilent Technologies, Inc.) with the obtained plasmids, a human naive antibody M13 phage library that has the VL gene containing the L6 nucleotide sequence and includes a library of VH genes was obtained.

### 2. Production of FAP-Immunized Human Antibody M13 Phage Library

As an immunogen, the GST-human FAP, the GST-mouse FAP, or the Fc-human FAP produced in Example 1 was administered to a human antibody-producing mouse [Ishida & Lonberg, IBC's 11th Antibody Engineering, Abstract 2000; Ishida, I. et al., Cloning & Stem Cells 4, 85-96 (2002) and Isamu Ishida (2002) Experimental medicine 20, 6, 846-851] at 20 µg/mouse or 50 µg/mouse a total of 4 times. Only at the first immunization, Alum gel (0.25 mg/mouse or 2 mg/mouse) and an inactivated Bordetella pertussis suspension (manufactured by Nacalai Tesque, Inc.) (1 × 10⁹ cells/mouse) were added as adjuvants.

The second immunization was performed two weeks after the first immunization, the third immunization was performed 1 week thereafter, the final immunization was performed 2 weeks after the third immunization, and the mouse was dissected 4 days after the final immunization and the lymph node or the spleen was surgically excised. The excised lymph node or spleen was homogenized, and thereafter, the cells were transferred to a tube through a cell strainer (manufactured by Falcon, Inc.), and centrifuged to precipitate the cells. The obtained cells were mixed with a red blood cell depletion reagent (manufactured by Sigma-Aldrich Co. LLC) and reacted with the reagent for 1 minute in a warm water bath at 37°C, followed by dilution with DMEM medium (manufactured by Sigma-Aldrich Co. LLC), and further the resultant was centrifuged.

RNA was extracted from the obtained lymph node cells or spleen cells using RNeasy Mini Plus kit (manufactured by QIAGEN, Inc.), cDNAs were amplified using a SMARTer RACE cDNA amplification kit (manufactured by Clontech Laboratories, Inc.), and further, a VH gene fragment was amplified by PCR. The VH gene fragment was inserted into a vector in which a tag sequence of a phagemid pCANTAB 5E (manufactured by Amersham Pharmacia, Inc.) was changed to FLAG-His tag and a trypsin recognition sequence together with the VL gene fragment containing the nucleotide sequence of L6 represented by SEQ ID NO: 17, and E. coli TG1 (manufactured by Lucigen Corporation) was transformed with the vector, whereby plasmids were obtained.

By infecting VCSM13 Interference Resistant Helper Phage (manufactured by Agilent Technologies, Inc.) with the obtained plasmids, a FAP-immunized human antibody M13 phage library that has the VL gene composed of the L6 sequence and includes a library of VH genes was obtained.

### 3. Acquisition of Anti-FAP Monoclonal Antibody

By using the human naive antibody M13 phage library and the FAP-immunized human antibody M13 phage library, an anti-FAP monoclonal antibody including VL containing the amino acid sequence of L6 was obtained by the following phage display method. MAXISORP STARTUBE in which human FAP-His (manufactured by BioLegend Co., Ltd.), mouse FAP (manufactured by R&D Systems), or the Fc-human FAP or the Fc-mouse FAP produced in Example 1 was immobilized and blocking was performed using SuperBlock Blockig Buffer, and the human naive antibody M13 phage library or the FAP-immunized human antibody M13 phage library were allowed to react at room temperature for 1 to 2 hours, and washing was performed with D-PBS(-) and PBS-T, and thereafter, the phage was eluted with 0.25% trypsin (manufactured by Nacalai Tesque, Inc.). The eluted phage was used to infect TG1 competent cells to amply the phage. Thereafter, the resultant was reacted again with Fc-human FAP, Fc-mouse FAP, human FAP-His, or mouse FAP immobilized on MAXISORP STARTUBE, and then, washing and elution were performed. This operation was repeated to concentrate the phage displaying an antibody molecule that specifically binds to human FAP and mouse FAP. The concentrated phage was used to infect TG1, which was then inoculated in a SOBAG plate (2.0% tryptone, 0.5% Yeast extract, 0.05% NaCl, 2.0% glucose, 10 mM MgCl₂, 100 µg/mL ampicillin, and 1.5% agar) to form a colony.

The colony was inoculated and cultured, and then infected with VCSM13 Interference Resistant Helper Phage, and cultured again, whereby a monoclonal phage solution was obtained. By using the obtained monoclonal phage solution, a clone that binds to human and mouse FAP was selected by ELISA.

In the ELISA, MAXISORP (manufactured by NUNC, Inc.) in which an anti-GST antibody (manufactured by Rockland Immunochemicals, Inc.) was immobilized on each well and blocking was performed using SuperBlock Blockig Buffer (manufactured by Thermo Fisher, Inc.), and thereafter, human FAP-His (manufactured by BioLegend Co., Ltd.), mouse FAP (manufactured by R&D Systems), or the GST-human FAP or the GST-mouse FAP produced in Example 1 was bound thereto was used. To each well, each phage solution was added and reacted at room temperature for 60 minutes, and thereafter, each well was washed 3 times with PBS-T.

Subsequently, an anti-M13 antibody (manufactured by GE Healthcare, Inc.) labeled with horseradish peroxidase was diluted 1000 times with PBS-T containing 10% Block Ace (manufactured by Dainippon Pharmaceutical Co., Ltd.), and the resultant was added in an amount of 50 µL to each well, and incubated at room temperature for 30 minutes. After the microplate was washed three times with PBS-T, a TMB chromogenic substrate solution (manufactured by DAKO, Inc.) was added in an amount of 50 µL to each well and incubated at room temperature for 10 minutes. The coloring reaction was stopped by adding a 2 N HCl solution to each well (50 µL/well), and an absorbance at a wavelength of 450 nm (reference wavelength: 570 nm) was measured using a plate reader (EnSpire, manufactured by PerkinElmer, Inc.).

A sequence analysis was performed for clones bound to human and mouse FAP, whereby anti-FAP antibodies having VL containing the amino acid sequence of L6 were obtained. In Table 1, the entire nucleotide sequence encoding VH of each of the obtained anti-FAP antibodies and the amino acid sequence deduced from the nucleotide sequence, and the amino acid sequences of HCDRs 1 to 3 are shown.

**[Table 1]**

| Sequence Information of VH of Anti-FAP Antibody | | | | | |
|---|---|---|---|---|---|
| Clone name | Nucleotide sequence of VH | Amino acid sequence of VH | Amino acid sequence of HCDR1 | Amino acid sequence of HCDR2 | Amino acid sequence of HCDR3 |
| Fa018 | SEQ ID NO: 31 | SEQ ID NO: 32 | SEQ ID NO: 33 | SEQ ID NO: 34 | SEQ ID NO: 35 |
| Fal007 | SEQ ID NO: 36 | SEQ ID NO: 37 | SEQ ID NO: 38 | SEQ ID NO: 39 | SEQ ID NO: 40 |
| Fal013 | SEQ ID NO: 41 | SEQ ID NO: 42 | SEQ ID NO: 43 | SEQ ID NO: 44 | SEQ ID NO: 45 |
| Fal014 | SEQ ID NO: 46 | SEQ ID NO: 47 | SEQ ID NO: 48 | SEQ ID NO: 49 | SEQ ID NO: 50 |
| Fa1046 | SEQ ID NO: 51 | SEQ ID NO: 52 | SEQ ID NO: 53 | SEQ ID NO: 54 | SEQ ID NO: 55 |

### 4. Acquisition of Positive Control Antibody Expression Vector

As the positive control antibody of the anti-FAP antibody, an antibody having a variable region of an anti-FAP monoclonal antibody sibrotuzumab described in WO 2001/68708 was produced. The nucleotide sequence of VH of sibrotuzumab is represented by SEQ ID NO: 56 and the amino acid sequence of the VH deduced from the sequence is represented by SEQ ID NO: 57. Further, the nucleotide sequence of VL of sibrotuzumab is represented by SEQ ID NO: 58 and the amino acid sequence of the VL deduced from the sequence is represented by SEQ ID NO: 59.

Gene fragments of the nucleotide sequence of the VH represented by SEQ ID NO: 56 and the nucleotide sequence of the VL represented by SEQ ID NO: 58 of sibrotuzumab were synthesized, and inserted into appropriate restriction enzyme sites of a pCI vector (Promega Corporation), whereby a sibrotuzumab expression vector was obtained.

### [Example 5] Production of Bispecific Antibody that Binds to CD40 and FAP

A bispecific antibody that has a structure shown in Fig. 1 and binds to human and monkey CD40 and human FAP was produced by the following method. As the form of the bispecific antibody, the form described in WO 2009/131239 was adopted.

The bispecific antibody produced by the following steps has CH (the nucleotide sequence is represented by SEQ ID NO: 60 and the amino acid sequence is represented by SEQ ID NO: 61) of IgG4PE R409K as the heavy chain constant region of the IgG portion, and CH1 (the nucleotide sequence is represented by SEQ ID NO: 62 and the amino acid sequence is represented by SEQ ID NO: 63) of IgG4 as CH1 of Fab that binds to the C terminus of the heavy chain of the IgG portion. Further, the bispecific antibody has a light chain including VL encoded by L6.

The name of the bispecific antibody, the clone of the anti-CD40 antibody and the clone of the anti-FAP antibody used for the production of the bispecific antibody are shown in Table 2.

**[Table 2]**

| Antibody Clone included in CD40-FAP Bispecific Antibody | | |
|---|---|---|
| Name of bispecific antibody | Clone of VH1 | Clone of VH2 |
| Ct-R1090-Fa018 | R1090S55A | Fa018 |
| Ct-R1090-Fa1007 | R1090S55A | Fa1007 |
| Ct-R1090-Fa1013 | R1090S55A | Fa1013 |
| Ct-R1090-Fa1014 | R1090S55A | Fa1014 |
| Ct-R1090-Fa1046 | R1090S55A | Fa1046 |

### 1. Production of Expression Vector for Bispecific Antibody

As for the bispecific antibodies shown in Table 2, an expression vector was produced by a method described below.

The nucleotide sequence (SEQ ID NO: 17) encoding the L6 amino acid sequence was obtained by performing codon conversion optimized for expression in a mammalian cell based on the amino acid sequence (SEQ ID NO: 18) of VL (L6) common to the anti-CD40 antibody R1090S55A obtained in Example 3 and the anti-FAP antibody clones shown in Table 2. The nucleotide sequence represented by SEQ ID NO: 17 was artificially synthesized, and cloned into an appropriate restriction enzyme site of a pCI vector (Promega Corporation) containing a signal sequence, whereby a light chain expression vector was obtained.

Subsequently, the nucleotide sequence encoding VH of the anti-CD40 antibody R1090S55A represented by SEQ ID NO: 22, the nucleotide sequence encoding CH of IgG4PE R409K represented by SEQ ID NO: 60, an artificially synthesized gene containing the nucleotide sequence encoding CH1 of IgG4 represented by SEQ ID NO: 62, and the nucleotide sequence encoding VH of the anti-FAP antibody clone shown in Table 1 amplified by PCR were inserted into a pCI vector (Promega Corporation), whereby a heavy chain expression vector was obtained. In Table 3, the nucleotide sequence encoding the heavy chain of the obtained CD40-FAP bispecific antibody, and the amino acid sequence deduced from the nucleotide sequence are shown.

**[Table 3]**

| Nucleotide Sequence and Amino Acid Sequence of Heavy Chain Included in CD40-FAP Bispecific Antibody | | |
|---|---|---|
| Name of bispecific antibody | Nucleotide sequence of heavy chain | Amino acid sequence of heavy chain |
| Ct-R1090-Fa018 | SEQ ID NO: 64 | SEQ ID NO: 65 |
| Ct-R1090-Fa1007 | SEQ ID NO: 66 | SEQ ID NO: 67 |
| Ct-R1090-Fa1013 | SEQ ID NO: 68 | SEQ ID NO: 69 |
| Ct-R1090-Fa1014 | SEQ ID NO: 70 | SEQ ID NO: 71 |
| Ct-R1090-Fa1046 | SEQ ID NO: 72 | SEQ ID NO: 73 |

### 2. Preparation of Bispecific Antibody

By using the expression vector for the bispecific antibody prepared in Example 5.1, each of the bispecific antibodies was expressed and purified by the following method.

The heavy chain expression vector for the CD40-FAP bispecific antibody and the light chain expression vector containing the nucleotide sequence encoding L6 were co-transfected into Expi293F (trademark) cells by Expi293F Expression System (manufactured by Thermo Fisher, Inc.), and Transfection Enhancer was added thereto after 16 hours, whereby a bispecific antibody was expressed in a transient expression system.

The culture supernatant was collected 4 days after introduction of the vector, and filtered through a membrane filter (manufactured by Millipore Corporation) having a pore diameter of 0.22 µm, and thereafter, the antibody was subjected to affinity purification using a Protein A resin (MabSelect, manufactured by GE Healthcare, Inc.). As the washing solution, D-PBS(-) was used. The antibody adsorbed to the Protein A was eluted with a 20 mM sodium citrate-50 mM NaCl buffer solution (pH 3.0) and collected in a tube containing a 200 mM sodium phosphate buffer solution (pH 7.0).

Subsequently, the buffer solution was replaced with D-PBS(-) by ultrafiltration using VIVASPIN (manufactured by Sartrius stealin), followed by filter sterilization with a membrane filter Millex-Gv (manufactured by Millipore Corporation) having a pore diameter of 0.22 µm, whereby a CD40-FAP bispecific antibody was obtained. The concentration of the purified antibody was determined by measuring an absorbance at a wavelength of 280 nm of the antibody solution and performing calculation using an extinction coefficient estimated from the amino acid sequence of each antibody.

### [Example 6] Production of Control Antibody

An anti-CD40 monoclonal antibody R1090S55A having CH of IgG4PE R409K and 21.4.1, and an anti-FAP monoclonal antibody sibrotuzumab were produced in the same manner as in Example 5-2 using the respective expression vectors of N5KG4PE R409K_R1090S55A produced in Example 3-3, N5KG2_21.4.1 produced in Example 3-4, and the sibrotuzumab expression vector produced in Example 4-4, respectively.

### [Example 7] Evaluation of Binding Affinity for FAP of Bispecific Antibody by ELISA

The binding affinity for human FAP of the CD40-FAP bispecific antibodies obtained in Example 5 was evaluated by ELISA according to the following procedure.

The human FAP-GST produced in Example 1 was diluted to a concentration of 10 µg/mL with D-PBS(-), and the resultant was dispensed in a 96-well Nunc-Immuno Plate (manufactured by Thermo Fisher, Inc.) at 50 µL/well, and the plate was left to stand overnight at 4°C. After each well was washed three times with 200 µL/well of D-PBS(-), in order to perform blocking, 1% (w/v) BSA-PBS(-) pH 7.0 (manufactured by Nacalai Tesque, Inc.) was added thereto at 50 µL/well, and the plate was left to stand at room temperature for 1 hour.

The solution in the well was discarded, and each of the CD40-FAP bispecific antibodies diluted to 0.00064, 0.0032, 0.016, 0.08, 0.4, 2, 10, or 50 µg/mL was added to each well at 50 µL/well. After the plate was left to stand at room temperature for 2 hours, washing was performed three times with 200 µL/well of 0.05 w/v%-tPBS (1x) without KCl (pH7.2) (manufactured by Nacalai Tesque, Inc.), and as a secondary antibody, a solution obtained by diluting Peroxidase AffiniPure Goat Anti-Human IgG, Fcγ Fragment specific (manufactured by Jackson ImmunoReseach, Inc.) 1/1000 with 1% (w/v) BSA-PBS(-) pH 7.0 (manufactured by Nacalai Tesque, Inc.) was added at 50 µL/well, and the plate was left to stand at room temperature for 1 hour.

After each well was washed three times with 200 µL/well of 0.05 w/v%-tPBS (1x) without KCl (pH7.2) (manufactured by Nacalai Tesque, Inc.), a mixed liquid obtained by mixing equal amounts of liquid A and liquid B of Substrate Reagent Pack (manufactured by R&D Systems) was added at 100 µL/well, and the plate was left to stand for 5 minutes. The reaction was stopped by adding Stop solution (manufactured by R&D Systems) at 50 µL/well, and absorbances at 450 nm and 570 nm were measured using EPOCH 2 microplate reader (manufactured by BioTek Instruments), and a value obtained by subtracting the absorbance at 570 nm from the absorbance at 450 nm was calculated.

Note that as the negative control, an IgG4 antibody R409K mutant (an antibody obtained by substituting R at position 409 with K in a human IgG4 antibody) (hereinafter referred to as an anti-DNP antibody) produced in accordance with the method described in Example 5 using a vector encoding an isotype control antibody [anti-2,4-dinitrophenol (DNP) IgG4 antibody described in Clin Cancer Res 2005, 11(8), 3126-3135] was used.

The results of measuring the binding affinity for human FAP of the CD40-FAP bispecific antibodies are shown in Fig. 2. As shown in Fig. 2, it could be confirmed that any of the CD40-FAP bispecific antibodies produced in Example 5 binds to the immobilized human FAP-GST.

### [Example 8] Evaluation of Binding Affinity for FAP of Bispecific Antibody Using Flow Cytometer

The evaluation of the binding affinity of the CD40-FAP bispecific antibodies produced in Example 5 for human embryonic kidney cells HEK 293 cells (ATCC No. CRL-1573) and human FAP/HEK 293 cells obtained in Example 2 was performed as follows.

HEK 293 cells or FAP/HEK 293 cells were suspended in Staining Buffer (D-PBS(-) containing 1% BSA and 2% FBS, also referred to as SB) at a cell density of 2 × 10⁶ cells/mL, and the suspension was dispensed in a 96-well round bottom plate (manufactured by Falcon, Inc.) at 50 µL/well. After centrifugation (1500 rpm, 4°C, 3 minutes), the supernatant was removed, and to the resulting pellet, the antibody obtained in Example 5 was added and suspended at 50 µL/well to give a final concentration of 0.01, 0.1, 1, or 10 µg/mL, and the plate was left to stand at ice temperature for 30 minutes.

After further centrifugation (2000 rpm, 4°C, 2 minutes), the supernatant was removed, the resulting pellet was washed three times with 200 µL/well of SB, and thereafter, 1 µg/mL of Goat F(ab')₂ Anti-Human IgG (γ chain specific) Allophycocyanin (APC) Conjugate (manufactured by Southern Biotech, Inc.) was added at 50 µL/well, and the resultant was incubated at ice temperature for 30 minutes. After washing twice with SB, the cells were suspended in 200 µL/well of SB, and the fluorescence intensity of each cell was measured using a flow cytometer FACSCANTO II (manufactured by Becton, Dickinson and Company). As the negative control, the DNP antibody was used.

The measurement results for HEK 293 are shown in Fig. 3(A), and the measurement results for human FAP/HEK 293 cells are shown in Fig. 3(B). From Fig. 3(A) and Fig. 3(B), any of the test antibodies did not bind to the HEK 293 cells or exhibited an extremely low binding affinity for the HEK 293 cells. On the other hand, the test antibodies bound to the human FAP/HEK 293 cells from a concentration as low as 0.01 µg/mL.

From the above results, it was demonstrated that any of the CD40-FAP bispecific antibodies produced in Example 5 is an antibody that specifically binds to FAP.

### [Example 9] Evaluation of CD40 Signaling Inducing Activity of CD40 Monoclonal Antibody by Analysis of Expression Level of CD95 Using Flow Cytometry

The CD40 signaling inducing activity of each of the CD40 monoclonal antibodies R1090S55A and 21.4.1 obtained in Example 6 was evaluated by an FCM method as follows using an increase in the expression level of CD95 on Ramos cells as an index.

Ramos cells (2 × 10⁶ cells/mL) were seeded in a U-bottom 96-well plate (manufactured by Falcon, Inc.) at 50 µL/well, and a test antibody diluted to 0.2, 2, or 20 µg/mL (final concentration of 0.1, 1, or 10 µg/mL) with RPMI 1640 medium (manufactured by Sigma-Aldrich Co. LLC) containing 10% FBS was added thereto at 50 µg/mL, and the cells were cultured at 37°C in the presence of 5.0% carbon dioxide gas for 16 hours.

After centrifugation (2000 rpm, 4°C, 2 minutes), the supernatant was removed, and the pellet was washed 3 times with 200 µL/well of SB. After centrifugation (2000 rpm, 4°C, 2 minutes), the supernatant was removed, and a PE mouse anti-human CD95 antibody (manufactured by Becton, Dickinson and Company) was added to the pellet to suspend the pellet, and then, the plate was left to stand at ice temperature for 30 minutes.

After further centrifugation (2000 rpm, 4°C, 2 minutes), the supernatant was removed, and the pellet was washed 3 times with 200 µL/well of SB. Thereafter, 7-aminoactinomycin (7AAD) (manufactured by Becton, Dickinson and Company) diluted 100 times was suspended in 200 µL/well of SB, and the fluorescence intensity of CD95 on Ramos cells was measured with a flow cytometer FACSCANTO II (manufactured by Becton, Dickinson and Company). As the negative control, the anti-DNP antibody was used.

The evaluation results of the anti-CD40 antibodies 21.4.1 and R1090S55A are shown in Fig. 4. From Fig. 4, the agonistic anti-CD40 monoclonal antibody 21.4.1 increased the expression level of CD95 on the Ramos cells as compared with the anti-DNP antibody. On the other hand, the anti-CD40 monoclonal antibody R1090S55A showed the same expression level of CD95 as that of the anti-DNP antibody.

That is, it was demonstrated that only 21.4.1 is an agonistic antibody that induces CD40 signaling, and the anti-CD40 monoclonal antibody R1090S55A obtained in Example 6 is a non-agonistic antibody that does not induce CD40 signaling. Further, when a similar test was performed in the presence of a CD40 ligand, the anti-CD40 monoclonal antibody R1090S55A did not inhibit the increase in the expression level of CD95 on the Ramos cells by the CD40 ligand (data not shown). That is, it was demonstrated that the anti-CD40 monoclonal antibody R1090S55A is a non-antagonistic antibody that does not inhibit the CD40 signaling induction by the CD40 ligand.

### [Example 10] Evaluation of CD40 Signaling Inducing Activity of CD40-FAP Bispecific Antibody by Analysis of Expression Level of CD95 Using Flow Cytometry

The CD40 signaling inducing activity of each of the CD40-FAP bispecific antibodies obtained in Example 5 against Ramos cells in the coexistence with FAP-positive or negative cells was evaluated by an FCM method as follows using an increase in the expression level of CD95 on the Ramos cells as an index.

Ramos cells (4 × 10⁶ cells/mL) were seeded in a U-bottom 96-well plate (manufactured by Falcon, Inc.) at 25 µL/well, and a test antibody diluted to 0.002, 0.02, 0.2, 2, or 20 µg/mL (final concentration of 0.001, 0.01, 0.1, 1, or 10 µg/mL) with RPMI 1640 medium (manufactured by Sigma-Aldrich Co. LLC) containing 10% FBS was added thereto at 50 µg/well, and further, HEK 293 cells or human FAP/HEK 293 cells (4 × 10⁶ cells/mL) were added thereto at 25 µL/well, and the cells were cultured at 37°C in the presence of 5.0% carbon dioxide gas for 16 hours.

After centrifugation (2000 rpm, 4°C, 2 minutes), the supernatant was removed, and the pellet was washed 3 times with 200 µL/well of SB. After centrifugation (2000 rpm, 4°C, 2 minutes), the supernatant was removed, and a PE mouse anti-human CD95 antibody (manufactured by Becton, Dickinson and Company) and FITC mouse anti-human CD20 (manufactured by Becton, Dickinson and Company) were added to the pellet to suspend the pellet, and then, the plate was left to stand at ice temperature for 30 minutes.

After further centrifugation (2000 rpm, 4°C, 2 minutes), the supernatant was removed, and the pellet was washed 3 times with 200 µL/well of SB. Thereafter, 7AAD (manufactured by Becton, Dickinson and Company) diluted 100 times was suspended in 200 µL/well of SB, and the fluorescence intensity of CD95 on Ramos cells present in a CD20-positive fraction was measured with a flow cytometer FACSCANTO II (manufactured by Becton, Dickinson and Company). As the negative control, the anti-DNP antibody was used, and as the positive control, 21.4.1 was used.

The results of the Ramos cells cocultured with the HEK 293 cells are shown in Fig. 5(A), and the results of the Ramos cells cocultured with the human FAP/HEK 293 cells are shown in Fig. 5(B).

From Fig. 5(A) and Fig. 5(B), in either of the coculture system experiments, the anti-DNP antibody being the negative control did not increase the expression level of CD95 on the Ramos cells, but the anti-CD40 antibody 21.4.1 being the positive control increased the expression level of CD95 on the Ramos cells.

Further, as shown in Fig. 5(A), when the HEK 293 cells negative for FAP and the Ramos cells were cocultured, any of the CD40-FAP bispecific antibodies did not induce CD40 signaling. On the other hand, as shown in Fig. 5(B), it was demonstrated that when the human FAP/HEK 293 cells positive for FAP and the Ramos cells are cocultured, the CD40-FAP bispecific antibodies increase the expression level of CD95 on the Ramos cells, and induce CD40 signaling.

These results indicate that as shown in Fig. 5(A), when the CD40-FAP bispecific antibody binds to CD40 on the Ramos cell alone, the CD40-FAP bispecific antibody does not induce CD40 signaling, and as shown in Fig. 5(B), only when FAP-positive cells coexist, the CD40-FAP bispecific antibody induces CD40 signaling.

As shown in Fig. 4, it has been confirmed that R1090S55A being a parent antibody does not induce CD40 signaling in Ramos cells, however, it was found that the CD40-FAP bispecific antibody has a CD40 signaling inducing activity which is not possessed by the parent antibody.

The results suggested that the CD40-FAP bispecific antibody of the present invention induces signaling in a CD40-positive cell such as a B cell specifically to a site where a cell that expresses FAP is present such as a tumor or a lymphoid tissue.

### [Example 11] Evaluation of CD40 Signaling Inducing Activity Against Dendritic Cells

The CD40 signaling inducing activity of each of the CD40-FAP bispecific antibodies obtained in Example 5 against dendritic cells in the coexistence with FAP-positive or negative cells was evaluated by an FCM method and an ELISA method using an increase in the expression level of an activation marker on the dendritic cells or a cytokine produced by the dendritic cells as an index. As the negative control, the anti-DNP antibody was used, and as the positive control, 21.4.1 was used.

### 1. Preparation of Cells

After Untouched Frozen NPB-CD14+ Monocytes (manufactured by AllCells, Inc.) were thawed, the cells were suspended in RPMI 1640 (manufactured by Nacalai Tesque, Inc.) containing 10% serum, penicillin-streptomycin (manufactured by Nacalai Tesque, Inc.), 100 ng/mL recombinant human GM-CSF (manufactured by R&D Systems), and 100 ng/mL recombinant human IL4 (manufactured by R&D Systems) at a cell density of 1 × 10⁶ cells/mL, and the suspension was added to 6-well Flat Bottom Ultra-Low Attachment Surface (manufactured by Corning Incorporated) at 3 mL/well.

The culture medium was replaced with a fresh culture medium 2 days and 4 days after culturing. The cells were collected 7 days after the start of culturing and centrifuged at 1500 rpm for 5 minutes at room temperature. The precipitated cells were prepared in RPMI 1640 medium containing 100 ng/mL IL-4 and 100 ng/mL GM-CSF at 2 × 10⁵ cells/mL, and added to 24-well Flat Bottom Ultra-Low Attachment Surface (manufactured by Corning Incorporated) at 250 µL/well. Further, human FAP/HEK 293 cells or HEK 293 cells were prepared at 4 × 10⁵ cells/mL, and added thereto at 125 µL/well. The evaluation target antibody prepared to a final concentration of 0.01, 0.1, 1, or 10 µg/mL was added thereto at 125 µL/well and mixed therewith, and the cells were cultured at 37°C in the presence of 5% CO₂ for 2 days.

### 2. Measurement of Expression of Activation Marker on Dendritic Cells by FCM Method

The cells cultured for 2 days were centrifuged (2000 rpm, 4°C, 5 minutes). The supernatant was removed, and 1% (w/v) BSA-PBS(-) pH 7.0 without KCl (manufactured by Nacalai Tesque, Inc.) (also referred to as FACS Buffer) was added thereto in an amount of 150 µL each. BD Fc Block Reagent for Human (manufactured by BD Pharmingen, Inc.) was added thereto to suspend the cells, and the suspension was left to stand on ice for 5 minutes.

Thereafter, the cell suspension was added to a 96-well U-bottom plate (manufactured by Falcon, Inc.) in an amount of 50 µL each. A labeled antibody Brilliant Violet 421 anti-human CD80 antibody (manufactured by Biolegend Co., Ltd.), PE Human CD83 antibody (manufactured by Beckman Coulter, Inc.), PE Mouse Anti-Human CD86 (manufactured by BD Pharmingen, Inc.), FITC mouse anti-human HLA-ABC (manufactured by BD Pharmingen, Inc.), or APC anti-human CD45 (manufactured by Biolegend Co., Ltd.) suspended in FACS Buffer was added thereto at 50 µL/well, followed by incubation at 4°C for 30 minutes.

After centrifugation (1200 rpm, 4°C, 2 minutes), the supernatant was decanted. The precipitated cells were washed twice with 200 µL of FACS Buffer. Thereafter, the cells were resuspended in 100 µL of FACS Buffer, and the fluorescence intensity was measured using FACS Canto II (manufactured by BD Biosciences Company). In the analysis, data analysis software FlowJo 9.6.4 was used, and with respect to CD80, CD83, CD86, and HLA-ABC on the CD45-positive cells, the expression level was evaluated based on the mean fluorescence intensity (MFI) of the antibody bound thereto.

### 3. Measurement of Expression of IL-12 Produced by Dendritic Cells by ELISA Method

A change in the expression level of IL-12 produced was verified using the activation of dendritic cells as an index. The culture supernatant of the cells after culturing for 2 days in Example 11-1 was collected, and the expression level of IL-12 was evaluated using Human IL-12 p70 ELISA Ready-SET-Go! (manufactured by Affymetrix, Inc.) according to the recommended method.

### 4. Evaluation Results

The results of the increase in the expression levels of CD83, CD80, CD86, HLA-ABC, and IL-12 of the dendritic cells cocultured with HEK 293 cells are shown in Fig. 6(A), Fig. 7(A), Fig. 8(A), Fig. 9(A), and Fig. 10(A), respectively. Further, the results of the expression levels of CD83, CD80, CD86, HLA-ABC, and IL-12 of the dendritic cells cocultured with human FAP/HEK 293 cells are shown in Fig. 6(B), Fig. 7(B), Fig. 8(B), Fig. 9(B), and Fig. 10(B), respectively.

In any of the drawings, the anti-CD40 monoclonal antibody 21.4.1 being the positive control increased the expression of the marker and the cytokine expressed by the dendritic cells as compared with the anti-DNP antibody being the negative control.

Further, as shown in Fig. 6(A), Fig. 7(A), Fig. 8(A), Fig. 9(A), and Fig. 10(A), when the HEK 293 cells negative for FAP and the dendritic cells were cocultured, any of the CD40-FAP bispecific antibodies did not increase the expression levels of CD83, CD80, CD86, and HLA-ABC on the dendritic cells, and also soluble IL-12, or did not induce CD40 signaling. On the other hand, as shown in Fig. 6(B), Fig. 7(B), Fig. 8(B), Fig. 9(B), and Fig. 10(B), it was demonstrated that when the human FAP/HEK 293 cells positive for FAP and the Ramos cells are cocultured, the CD40-FAP bispecific antibodies increase the expression levels of CD83, CD80, CD86, and HLA-ABC on the dendritic cells, and also soluble IL-12, and induce CD40 signaling.

The results suggested that the CD40-FAP bispecific antibody of the present invention induces signaling in a CD40-positive cell such as a dendritic cell specifically to a site where a cell that expresses FAP is present such as a tumor or a lymphoid tissue. By combining these results with the results of Example 10, it was demonstrated that the CD40-FAP bispecific antibody of the present invention induces CD40 signaling in CD40-positive immune cells regardless of cell types.

### [Example 12] Production of CD40-FAP Bispecific Antibody in which N-Linked Glycosylated Amino Acid Residue was Substituted

The heavy chain amino acid sequence of Fa1046 represented by SEQ ID NO: 52 among the anti-FAP antibody clones obtained in Example 4 has an N-linked glycosylated amino acid residue in the framework, and therefore, modified antibodies in which the amino acid residue was substituted were produced by the following method.

In order to substitute the N-linked glycosylated amino acid residue contained in the VH sequence of the FAP antibody clone Fa1046 represented by SEQ ID NO: 52 with an alternative amino acid residue, a corresponding nucleotide sequence was obtained by performing codon conversion optimized for expression in a mammalian cell. The clone name of the modified antibody produced, and the nucleotide sequence encoding VH and the amino acid sequence thereof are shown in Table 4.

**[Table 4]**

| FAP Antibody Clone in which N-Linked Glycosylated Amino Acid Residue was Substituted | | |
|---|---|---|
| Clone name | Nucleotide sequence of VH | Amino acid sequence of VH |
| Fa1046-GTS | SEQ ID NO: 74 | SEQ ID NO: 75 |
| Fa1046-FTS | SEQ ID NO: 76 | SEQ ID NO: 77 |
| Fa1046-KTS | SEQ ID NO: 78 | SEQ ID NO: 79 |
| Fa1046-WTS | SEQ ID NO: 80 | SEQ ID NO: 81 |
| Fa1046-TTS | SEQ ID NO: 82 | SEQ ID NO: 83 |

With respect to each clone shown in Table 4, a CD40-FAP bispecific antibody was produced in the same manner as in Example 5. The name of the bispecific antibody, and the clone of the anti-CD40 antibody and the clone of the anti-FAP antibody used for the production of the bispecific antibody (hereinafter also referred to as a modified bispecific antibody) are shown in Table 5. The nucleotide sequence encoding the heavy chain of the obtained CD40-FAP bispecific antibody containing the VH of each anti-FAP antibody and the amino acid sequence deduced from the nucleotide sequence are shown in Table 6.

**[Table 5]**

| Clone used for Modified Bispecific Antibody | | |
|---|---|---|
| Name of bispecific antibody | Clone of VH1 | Clone of VH2 |
| Ct-R1090-Fa1046-GTS | R1090S55A | Fa1046-GTS |
| Ct-R1090-Fa1046-FTS | R1090S55A | Fa1046-FTS |
| Ct-R1090-Fa1046-KTS | R1090S55A | Fa1046-KTS |
| Ct-R1090-Fa1046-WTS | R1090S55A | Fa1046-WTS |
| Ct-R1090-Fa1046-TTS | R1090S55A | Fa1046-TTS |

**[Table 6]**

| Sequence Information of Heavy Chain of Modified Bispecific Antibody | | |
|---|---|---|
| Name of bispecific antibody | Nucleotide sequence of heavy chain | Amino acid sequence of heavy chain |
| Ct-R1090-Fa1046-GTS | SEQ ID NO: 84 | SEQ ID NO: 85 |
| Ct-R1090-Fa1046-FTS | SEQ ID NO: 86 | SEQ ID NO: 87 |
| Ct-R1090-Fa1046-KTS | SEQ ID NO: 88 | SEQ ID NO: 89 |
| Ct-R1090-Fa1046-WTS | SEQ ID NO: 90 | SEQ ID NO: 91 |
| Ct-R1090-Fa1046-TTS | SEQ ID NO: 92 | SEQ ID NO: 93 |

### [Example 13] Evaluation of Binding Affinity for Human FAP of CD40-FAP Bispecific Antibody in which N-Linked Glycosylated Amino Acid Residue was Substituted Using Flow Cytometry

The binding affinity of the modified bispecific antibodies of R1090S55A-Fa1046 obtained in Example 12 for human FAP/HEK 293 cells and HEK 293 cells was evaluated in the same manner as in Example 7.

The measurement results for HEK 293 cells are shown in Fig. 11(A), and the measurement results for human FAP/HEK 293 cells are shown in Fig. 11(B). As shown in Fig. 11(A), any of the test antibodies did not bind to the HEK 293 cells negative for FAP. On the other hand, as shown in Fig. 11(B), any of the test antibodies bound to the human FAP/HEK 293 cells positive for FAP from a concentration as low as 0.01 µg/mL, and had an activity equal to or higher than that of R1090S55A-Fa1046 before modification.

From the above results, it was demonstrated that any of the modified bispecific antibodies of R1090S55A-Fa1046 produced in Example 12 is an antibody that specifically binds to FAP.

### [Example 14] Evaluation of CD40 Signaling Inducing Activity of CD40-FAP Bispecific Antibody in which N-Linked Glycosylated Amino Acid Residue was Substituted by Analysis of Expression Level of CD95 Using Flow Cytometry

The CD40 signaling inducing activity of each of the CD40-FAP bispecific antibodies obtained in Example 12 against Ramos cells in the coexistence with FAP-positive or negative cells was evaluated in the same manner as in Example 10 using an increase in the expression level of CD95 on the Ramos cells as an index. As the negative control, the anti-DNP antibody was used, and as the positive control, 21.4.1 was used.

The results of the Ramos cells cocultured with the HEK 293 cells are shown in Fig. 12(A), and the results of the Ramos cells cocultured with the human FAP/HEK 293 cells are shown in Fig. 12(B).

As shown in Fig. 12(A), when the HEK 293 cells negative for FAP and the Ramos cells were cocultured, any of the modified bispecific antibodies showed the same expression level of CD95 as that of the anti-DNP antibody, and did not induce CD40 signaling. On the other hand, as shown in Fig. 12(B), when the human FAP/HEK 293 cells positive for FAP and the Ramos cells were cocultured, the modified bispecific antibodies increased the expression level of CD95 to the same level or higher than R1090S55A-Fa1046 before modification. Therefore, it was demonstrated that the modified bispecific antibodies induce CD40 signaling to the same level or higher than R1090S55A-Fa1046 before modification.

The results suggested that the modified bispecific antibodies produced in Example 12 induce signaling in a CD40-positive cell such as a B cell or a dendritic cell specifically to a site where a cell that expresses FAP is present such as a tumor or a lymphoid tissue.

### [Example 15] Production of Bispecific Antibody that Binds to CD40 and FAP

A bispecific antibody that has a heavy chain including VH1 and VH2 shown in Table 7 and a light chain including VL containing the amino acid sequence of L6 was produced in accordance with the method described in Example 5. The nucleotide sequence encoding the heavy chain of each FAP-CD40 bispecific antibody and the amino acid sequence deduced from the nucleotide sequence are shown in Table 8.

**[Table 7]**

| VH Clone included in FAP-CD40 Bispecific Antibody | | |
|---|---|---|
| Name of bispecific antibody | Clone of VH1 | Clone of VH2 |
| Ct-Fa018-R1090 | Fa018 | R1090S55A |
| Ct-Fa1007-R1090 | Fa1007 | R1090S55A |
| Ct-Fa1013-R1090 | Fa1013 | R1090S55A |
| Ct-Fa1014-R1090 | Fa1014 | R1090S55A |
| Ct-Fa1046-GTS-R1090 | Fa1046-GTS | R1090S55A |
| Ct-Fa1046-FTS-R1090 | Fa1046-FTS | R1090S55A |
| Ct-Fa1046-KTS-R1090 | Fa1046-KTS | R1090S55A |
| Ct-Fa1046-WTS-R1090 | Fa1046-WTS | R1090S55A |
| Ct-Fa1046-TTS-R1090 | Fa1046-TTS | R1090S55A |

**[Table 8]**

| Nucleotide Sequence and Amino Acid Sequence Included in CD40-FAP Bispecific Antibody | | |
|---|---|---|
| Name of bispecific antibody | Nucleotide sequence of heavy chain | Amino acid sequence of heavy chain |
| Ct-Fa018-R1090 | SEQ ID NO: 94 | SEQ ID NO: 95 |
| Ct-Fa1007-R1090 | SEQ ID NO: 96 | SEQ ID NO: 97 |
| Ct-Fa1013-R1090 | SEQ ID NO: 98 | SEQ ID NO: 99 |
| Ct-Fa1014-R1090 | SEQ ID NO: 100 | SEQ ID NO: 101 |
| Ct-Fa1046-GTS-R1090 | SEQ ID NO: 102 | SEQ ID NO: 103 |
| Ct-Fa1046-FTS-R1090 | SEQ ID NO: 104 | SEQ ID NO: 105 |
| Ct-Fa1046-KTS-R1090 | SEQ ID NO: 106 | SEQ ID NO: 107 |
| Ct-Fa1046-WTS-R1090 | SEQ ID NO: 108 | SEQ ID NO: 109 |
| Ct-Fa1046-TTS-R1090 | SEQ ID NO: 110 | SEQ ID NO: 111 |

### [Example 16] Evaluation of CD40 Signaling Inducing Activity of CD40-FAP Bispecific Antibody by Analysis of Expression Level of CD95 Using Flow Cytometry

The CD40 signaling inducing activity of each of the CD40-FAP bispecific antibodies obtained in Example 15 was confirmed in the same manner as in Example 9.

### [Example 17] Evaluation of CD40 Signaling Inducing Activity of CD40-FAP Bispecific Antibody by Analysis of Expression Level of CD95 Using Flow Cytometry

The CD40 signaling inducing activity of each of the CD40-FAP bispecific antibodies obtained in Example 5 (Ct-R1090-Fa1007, Ct-R1090-Fa1013, Ct-R1090-Fa1014, and Ct-R1090-Fa1046) against Ramos cells in FAP-positive fibroblasts and FAP-positive cancer cells was evaluated in the same manner as in Example 10 using an increase in the expression level of CD95 on the Ramos cells as an index.

However, the test antibody was added at a concentration of 0.02, 0.2, 2, or 20 µg/mL (final concentration of 0.01, 0.1, 1, or 10 µg/mL). Further, in place of the HEK 293 cells or the human FAP/HEK 293 cells used in Example 10, a FAP-positive fibroblast cell line IMR-90 (ATCC No. CCL-186) or a FAP-positive cancer cell line RPMI-7951 (DSMZ No. ACC 66) was used.

The results of the Ramos cells cocultured with IMR-90 cells are shown in Fig. 13(A), and the results of the Ramos cells cocultured with RPMI-7951 cells are shown in Fig. 13(B).

As a result, in either of the coculture system experiments, the anti-DNP antibody being the negative control did not increase the expression of CD95 on the Ramos cells, but the anti-CD40 monoclonal antibody 21.4.1 being the positive control increased the expression of CD95 on the Ramos cells.

As shown in Fig. 13(A) and Fig. 13(B), it was demonstrated that when the FAP-positive fibroblasts or the FAP-positive cancer cells and the Ramos cells are cocultured, the CD40-FAP bispecific antibodies increase the expression level of CD95 on the Ramos cells and induce CD40 signaling.

The results suggested that the CD40-FAP bispecific antibody of the present invention induces signaling in a CD40-positive cell such as a B cell specifically to a site where a fibroblast or a cancer cell that expresses FAP is present such as a tumor or a lymphoid tissue.

### [Example 18] Evaluation of CD40 Signaling Inducing Activity of CD40-FAP Bispecific Antibody by Analysis of Expression Levels of CD83 and CD95 Using Flow Cytometry

The CD40 signaling inducing activity of each of the CD40-FAP bispecific antibodies obtained in Example 5 and Example 12 (Ct-R1090-Fa1013, Ct-R1090-Fa1014, and Ct-R1090-Fa1046-GTS) against Ramos cells in human FAP-immobilized beads was evaluated in the same manner as in Example 10 using an increase in the expression levels of CD83 and CD95 on the Ramos cells as an index.

However, the test antibody was added at 2 µg/mL (final concentration of 1 µg/mL), and in place of the HEK 293 cells or the human FAP/HEK 293 cells used in Example 10, human FAP-immobilized beads produced by the following method were added. Further, as the antibody for detecting CD83, an APC mouse anti-human CD83 antibody (manufactured by Biolegend Co., Ltd.) was used.

### (Method for Producing FAP-Immobilized Beads)

Dynabeads M-280 Streptavidin (manufactured by Invitrogen, Inc.) with immobilized human FAP were prepared as follows. The human FAP-FLAG-Fc obtained in Example 1 was labeled with biotin using Biotin Labeling Kit-NH2 (manufactured by Dojindo Laboratories Co., Ltd.), whereby human FAP-FLAG-Fc-Biotin was obtained. The human FAP-FLAG-Fc-Biotin (1 µg) was serially diluted 1.5-fold, and each dilute solution was used to bind to 6.5 × 10⁶ Dynabeads M-280 Streptavidin. By doing this, beads with a different amount of immobilized human FAP were obtained.

### (Method for Measuring Amount of FAP Immobilized on Beads)

The amount of FAP immobilized on the beads was calculated as a relative fluorescence intensity value (RFI) by measuring the binding of the anti-DNP antibody being the negative control and the anti-FAP antibody to the FAP-immobilized beads using a flow cytometer FACSCANTO II (manufactured by Becton, Dickinson and Company).

The results of the expression level of CD83 on the Ramos cells are shown in Fig. 14(A) and the results of the expression level of CD95 on the Ramos cells are shown in Fig. 14(B).

As a result, regardless of the amount of human FAP immobilized on the beads cocultured, the anti-DNP antibody being the negative control did not increase the expression levels of CD83 and CD95 on the Ramos cells, but the anti-CD40 monoclonal antibody 21.4.1 being the positive control increased the expression levels of CD83 and CD95 on the Ramos cells.

As shown in Fig. 14(A) and Fig. 14(B), it was demonstrated that the CD40-FAP bispecific antibodies Ct-R1090-Fa1013, Ct-R1090-Fa1014,and Ct-R1090-Fa1046-GTS increase the expression levels of CD83 and CD95 on the Ramos cells in correlation with the amount of FAP and induce CD40 signaling in the beads with immobilized FAP.

The results suggested that the CD40-FAP bispecific antibody of the present invention induces signaling in a CD40-positive cell such as a B cell or a dendritic cell specifically to a site where a cell that expresses FAP on the surface thereof is present regardless of the cell type, and the intensity of the signaling tends to correlate with the expression level of FAP.

### [Example 19] Production of CD40-FAP Bispecific Antibody as Control

A CD40-FAP bispecific antibody described in WO 2018/185045 was produced by the following method.

### 1. Production of Expression Vector for Bispecific Antibody

An antibody clone P1AA9663 described in WO 2018/185045 is a CD40-FAP bispecific antibody obtained by combining an anti-FAP antibody 28H1 that binds to FAP and an anti-CD40 antibody SGN-40, and has been reported to induce signaling in CD40-positive cells (WO 2018/185045, Beatty GL et al., Expert Rev Anticancer Ther. 2017 Feb;17(2): 175-186). Nucleotide sequences (SEQ ID NOS: 115, 116, and 117) were obtained by performing codon conversion optimized for expression in a mammalian cell based on amino acid sequences (SEQ ID NOS: 112, 113, and 114) constituting P1AA9663. A gene fragment containing the nucleotide sequences represented by SEQ ID NOS: 115, 116, and 117 was synthesized, and cloned into an appropriate restriction enzyme site of a pCI vector (Promega Corporation) containing a nucleotide sequence encoding a signal sequence, whereby an expression vector was obtained.

### 2. Preparation of Bispecific Antibody

The bispecific antibody was expressed and purified by the following method using the expression vector for the bispecific antibody produced in Example 19.1.

The expression vector for a molecule constituting the bispecific antibody was co-transfected into Expi293F (trademark) cells by Expi293F Expression System (manufactured by Thermo Fisher, Inc.), and after 16 hours, Transfection Enhancer was added thereto, whereby a bispecific antibody was expressed in a transient expression system.

The culture supernatant was collected 4 days after introduction of the vector, and filtered through a membrane filter (manufactured by Millipore Corporation) having a pore diameter of 0.22 µm, and thereafter, the antibody was subjected to affinity purification using a Protein A resin (manufactured by MabSelect, GE Healthcare, Inc.). As the washing solution, D-PBS(-) was used. The antibody adsorbed to the Protein A was eluted with a 20 mM sodium citrate-50 mM NaCl buffer solution (pH 3.0) and collected in a tube containing a 200 mM sodium phosphate buffer solution (pH 7.0).

Subsequently, the buffer solution was replaced with D-PBS(-) by ultrafiltration using VIVASPIN (manufactured by Sartrius stealin), followed by filter sterilization with a membrane filter Millex-Gv (manufactured by Millipore Corporation) having a pore diameter of 0.22 µm, whereby the CD40-FAP bispecific antibody P1AA9663 was obtained. An absorbance at a wavelength of 280 nm of the antibody solution was measured, and the concentration of the purified antibody was calculated using an extinction coefficient estimated from the amino acid sequence of each antibody.

### [Example 20] Evaluation of CD40 Signaling Inducing Activity of CD40-FAP Bispecific Antibody by Analysis of Expression Level of CD83 Using Flow Cytometry

The CD40 signaling inducing activity of each of the CD40-FAP bispecific antibodies obtained in Example 5, Example 12, and Example 19 (Ct-R1090-Fa1013, Ct-R1090-Fa1014, Ct-R1090-Fa1046-GTS, and PIAA9663) against Ramos cells in the presence of FAP-positive cells was evaluated in the same manner as in Example 10 and Example 19 using an increase in the expression level of CD83 on the Ramos cells as an index.

However, the test antibody was added at a concentration of 0.02, 0.2, 2, or 20 µg/mL (final concentration of 0.01, 0.1, 1, or 10 µg/mL), and in place of the HEK 293 cells or the human FAP/HEK 293 cells used in Example 10, a FAP-positive fibroblast cell line SC-00A05 (manufactured by Neuromics, Inc.) was used.

The results of the change in the expression level of CD83 in the Ramos cells are shown in Fig. 15. As a result, in either of the coculture system experiments, the anti-DNP antibody being the negative control did not increase the expression level of CD83 on the Ramos cells, but the anti-CD40 antibody 21.4.1 being the positive control increased the expression level of CD83 on the Ramos cells.

It was demonstrated that any of the CD40-FAP bispecific antibodies Ct-R1090-Fa1013, Ct-R1090-Fa1014,and Ct-R1090-Fa1046-GTS increases the expression level of CD83 on the Ramos cells and induces CD40 signaling. Further, it was demonstrated that the antibodies increase the expression level of CD83 more and induce CD40 signaling more strongly than the control CD40-FAP bispecific antibody PIAA9663.

The results suggested that the CD40-FAP bispecific antibody of the present invention induces signaling in a CD40-positive cell such as a B cell more strongly than the conventional CD40-FAP bispecific antibody.

### [Example 21] Evaluation of CD40 Signaling Inducing Activity of CD40-FAP Bispecific Antibody against Dendritic Cells

The CD40 signaling inducing activity of each of the CD40-FAP bispecific antibodies obtained in Example 5, Example 12, and Example 19 (Ct-R1090-Fa1013, Ct-R1090-Fa1046-GTS, and PIAA9663) against dendritic cells in the coexistence with FAP-positive cells was evaluated in the same manner as in Example 11 using an increase in the expression levels of CD83, CD86, and HLA-ABC being the activation markers of dendritic cells as an index.

However, in place of the human FAP/HEK 293 cells or the HEK 293 cells in Example 11, a FAP-positive fibroblast cell line SC-00A05 (manufactured by Neuromics, Inc.) was used. Further, as the antibody for detecting CD86, PE-Cy7 Mouse Anti-Human CD86 (manufactured by Biolegend Co., Ltd.) was used.

The results of the change in the expression levels of CD83, CD86, and HLA-ABC on the dendritic cells are shown in Fig. 16(A), Fig. 16(B), and Fig. 16(C), respectively.

From Fig. 16(A), Fig. 16(B), and Fig. 16(C), with respect to any of the markers, the anti-DNP antibody being the negative control did not increase the expression level, but the anti-CD40 monoclonal antibody 21.4.1 being the positive control increased the expression level.

In addition, it was demonstrated that with respect to any of the markers, Ct-R1090-Fa1013 and Ct-R1090-Fa1046-GTS increase the expression level as compared with P1AA9663, and Ct-R1090-Fa1013 and Ct-R1090-Fa1046-GTS induce CD40 signaling strongly as compared with P1AA9663.

The results suggested that the CD40-FAP bispecific antibody of the present invention induces CD40 signaling against a CD40-positive cell such as a dendritic cell strongly as compared with the conventional CD40-FAP bispecific antibody.

### [Example 22] Evaluation of CD40 Signaling Inducing Activity of CD40-FAP Bispecific Antibody by Analysis of Expression Levels of CD83 and CD95 Using Flow Cytometry in Presence of Crosslinking Antibody

In order to confirm the presence or absence of the CD40 signaling inducing activity by antibody crosslinking independent of FAP-expressing cells, the CD40 signaling inducing activity of each of the CD40-FAP bispecific antibodies obtained in Example 5 and Example 19 (Ct-R1090-Fa1013 and PIAA9663) against Ramos cells in the presence of a crosslinking antibody was evaluated in the same manner as in Example 10 using an increase in the expression levels of CD83 and CD95 on the Ramos cells as an index.

However, the HEK 293 cells or the human FAP/HEK 293 cells in Example 10 were not used, and after the test antibody was added, a crosslinking antibody anti-human IgG (FcyR specific) (manufactured by Jackson ImmunoResearch Laboratories, Inc.) prepared at 80 µg/mL (final concentration of 20 µg/mL) was added at 25 µL/well.

Further, as the antibody for detecting CD83, an APC mouse anti-human CD83 antibody (manufactured by Biolegend Co., Ltd.) was used.

The results of the change in the expression level of CD83 on the Ramos cells are shown in Fig. 17(A), and the results of expression level of CD95 are shown in Fig. 17(B).

From Fig. 17(A) and Fig. 17(B), the anti-DNP antibody being the negative control did not increase the expression levels of CD83 and CD95 on the Ramos cells.

In addition, P1AA9663 being the control clearly increased the expression levels of CD83 and CD95 at a concentration of 0.1 µg/mL at which a FAP-dependent agonistic activity is observed, but Ct-R1090-Fa1013 did not clearly increase the expression levels of CD83 and CD95 at the same concentration. Therefore, it was demonstrated that in the presence of the crosslinking antibody, P1AA9663 strongly induces FAP-expressing cell-independent CD40 signaling, but Ct-R1090-Fa1013 does not clearly induce FAP-expressing cell-independent CD40 signaling.

The results suggested that when crosslinking occurs by antibodies or the like in a living body, the conventional CD40-FAP bispecific antibody may strongly induce CD40 signaling even at a site where a cell that expresses FAP is not present, but the CD40-FAP bispecific antibody of the present invention may not induce CD40 signaling at a site where a cell that expresses FAP is not present.

The present invention has been explained in detail using the specific aspects, but it is obvious to those skilled in the art that various changes and modifications can be made without departing from the spirit and scope of the present invention. The present application is based on a Japanese patent application filed on May 15, 2019 (Patent Application No. 2019-092298), which is incorporated by reference in its entirety.

### SEQUENCE LISTING FREE TEXT

SEQ ID NO: 19: Description of artificial sequence: amino acid sequence of LCDR1 of L6
SEQ ID NO: 20: Description of artificial sequence: amino acid sequence of LCDR2 of L6
SEQ ID NO: 21: Description of artificial sequence: amino acid sequence of LCDR3 of L6
SEQ ID NO: 24: Description of artificial sequence: amino acid sequence of HCDR1 of R1090
SEQ ID NO: 25: Description of artificial sequence: amino acid sequence of HCDR2 of R1090
SEQ ID NO: 26: Description of artificial sequence: amino acid sequence of HCDR3 of R1090
SEQ ID NO: 33: Description of artificial sequence: amino acid sequence of HCDR1 of Fa018
SEQ ID NO: 34: Description of artificial sequence: amino acid sequence of HCDR2 of Fa018
SEQ ID NO: 35: Description of artificial sequence: amino acid sequence of HCDR3 of Fa018
SEQ ID NO: 38: Description of artificial sequence: amino acid sequence of HCDR1 of Fa1007
SEQ ID NO: 39: Description of artificial sequence: amino acid sequence of HCDR2 of Fa1007
SEQ ID NO: 40: Description of artificial sequence: amino acid sequence of HCDR3 of Fa1007
SEQ ID NO: 43: Description of artificial sequence: amino acid sequence of HCDR1 of Fa1013
SEQ ID NO: 44: Description of artificial sequence: amino acid sequence of HCDR2 of Fa1013
SEQ ID NO: 45: Description of artificial sequence: amino acid sequence of HCDR3 of Fa1013
SEQ ID NO: 48: Description of artificial sequence: amino acid sequence of HCDR1 of Fa1014
SEQ ID NO: 49: Description of artificial sequence: amino acid sequence of HCDR2 of Fa1014
SEQ ID NO: 50: Description of artificial sequence: amino acid sequence of HCDR3 of Fa1014
SEQ ID NO: 53: Description of artificial sequence: amino acid sequence of HCDR1 of Fa1046
SEQ ID NO: 54: Description of artificial sequence: amino acid sequence of HCDR2 of Fa1046
SEQ ID NO: 55: Description of artificial sequence: amino acid sequence of HCDR3 of Fa1046
SEQ ID NO: 56: Description of artificial sequence: nucleotide sequence of VH of Sibrotuzumab
SEQ ID NO: 57: Description of artificial sequence: amino acid sequence of VH of Sibrotuzumab
SEQ ID NO: 58: Description of artificial sequence: nucleotide sequence of VL of Sibrotuzumab
SEQ ID NO: 59: Description of artificial sequence: amino acid sequence of VL of Sibrotuzumab
SEQ ID NO: 60: Description of artificial sequence: nucleotide sequence of CH of IgG4PE R409K
SEQ ID NO: 61: Description of artificial sequence: amino acid sequence of CH of IgG4PE R409K
SEQ ID NO: 64: Description of artificial sequence: nucleotide sequence of heavy chain of Ct-R1090-Fa018
SEQ ID NO: 65: Description of artificial sequence: amino acid sequence of heavy chain of Ct-R1090-Fa018
SEQ ID NO: 66: Description of artificial sequence: nucleotide sequence of heavy chain of Ct-R1090-Fa1007
SEQ ID NO: 67: Description of artificial sequence: amino acid sequence of heavy chain of Ct-R1090-Fa1007
SEQ ID NO: 68: Description of artificial sequence: nucleotide sequence of heavy chain of Ct-R1090-Fa1013
SEQ ID NO: 69: Description of artificial sequence: amino acid sequence of heavy chain of Ct-R1090-Fa1013
SEQ ID NO: 70: Description of artificial sequence: nucleotide sequence of heavy chain of Ct-R1090-Fa1014
SEQ ID NO: 71: Description of artificial sequence: amino acid sequence of heavy chain of Ct-R1090-Fa1014
SEQ ID NO: 72: Description of artificial sequence: nucleotide sequence of heavy chain of Ct-R1090-Fa1046
SEQ ID NO: 73: Description of artificial sequence: amino acid sequence of heavy chain of Ct-R1090-Fa1046
SEQ ID NO: 74: Description of artificial sequence: nucleotide sequence of VH of Fa1046-GTS
SEQ ID NO: 75: Description of artificial sequence: amino acid sequence of VH of Fa1046-GTS
SEQ ID NO: 76: Description of artificial sequence: nucleotide sequence of VH of Fa1046-FTS
SEQ ID NO: 77: Description of artificial sequence: amino acid sequence of VH of Fa1046-FTS
SEQ ID NO: 78: Description of artificial sequence: nucleotide sequence of VH of Fa1046-KTS
SEQ ID NO: 79: Description of artificial sequence: amino acid sequence of VH of Fa1046-KTS
SEQ ID NO: 80: Description of artificial sequence: nucleotide sequence of VH of Fa1046-WTS
SEQ ID NO: 81: Description of artificial sequence: amino acid sequence of VH of Fa1046-WTS
SEQ ID NO: 82: Description of artificial sequence: nucleotide sequence of VH of Fa1046-TTS
SEQ ID NO: 83: Description of artificial sequence: amino acid sequence of VH of Fa1046-TTS
SEQ ID NO: 84: Description of artificial sequence: nucleotide sequence of heavy chain of Ct-R1090-Fa1046-GTS
SEQ ID NO: 85: Description of artificial sequence: amino acid sequence of heavy chain of Ct-R1090-Fa1046-GTS
SEQ ID NO: 86: Description of artificial sequence: nucleotide sequence of heavy chain of Ct-R1090-Fa1046-FTS
SEQ ID NO: 87: Description of artificial sequence: amino acid sequence of heavy chain of Ct-R1090-Fa1046-FTS
SEQ ID NO: 88: Description of artificial sequence: nucleotide sequence of heavy chain of Ct-R1090-Fa1046-KTS
SEQ ID NO: 89: Description of artificial sequence: amino acid sequence of heavy chain of Ct-R1090-Fa1046-KTS
SEQ ID NO: 90: Description of artificial sequence: nucleotide sequence of heavy chain of Ct-R1090-Fa1046-WTS
SEQ ID NO: 91: Description of artificial sequence: amino acid sequence of heavy chain of Ct-R1090-Fa1046-WTS
SEQ ID NO: 92: Description of artificial sequence: nucleotide sequence of heavy chain of Ct-R1090-Fa1046-TTS
SEQ ID NO: 93: Description of artificial sequence: amino acid sequence of heavy chain of Ct-R1090-Fa1046-TTS
SEQ ID NO: 94: Description of artificial sequence: nucleotide sequence of heavy chain of Ct-Fa018-R1090
SEQ ID NO: 95: Description of artificial sequence: amino acid sequence of heavy chain of Ct-Fa018-R1090
SEQ ID NO: 96: Description of artificial sequence: nucleotide sequence of heavy chain of Ct-Fa1007-R1090
SEQ ID NO: 97: Description of artificial sequence: amino acid sequence of heavy chain of Ct-Fa1007-R1090
SEQ ID NO: 98: Description of artificial sequence: nucleotide sequence of heavy chain of Ct-Fa1013-R1090
SEQ ID NO: 99: Description of artificial sequence: amino acid sequence of heavy chain of Ct-Fa1013-R1090
SEQ ID NO: 100: Description of artificial sequence: sequence of heavy chain of Ct-Fa1014-R1090
SEQ ID NO: 101: Description of artificial sequence: amino acid sequence of heavy chain of Ct-Fa1014-R1090
SEQ ID NO: 102: Description of artificial sequence: nucleotide sequence of heavy chain of Ct-Fa1046-GTS-R1090
SEQ ID NO: 103: Description of artificial sequence: amino acid sequence of heavy chain of Ct-Fa1046-GTS-R1090
SEQ ID NO: 104: Description of artificial sequence: nucleotide sequence of heavy chain of Ct-Fa1046-FTS-R1090
SEQ ID NO: 105: Description of artificial sequence: amino acid sequence of heavy chain of Ct-Fa1046-FTS-R1090
SEQ ID NO: 106: Description of artificial sequence: nucleotide sequence of heavy chain of Ct-Fa1046-KTS-R1090
SEQ ID NO: 107: Description of artificial sequence: amino acid sequence of heavy chain of Ct-Fa1046-KTS-R1090
SEQ ID NO: 108: Description of artificial sequence: nucleotide sequence of heavy chain of Ct-Fa1046-WTS-R1090
SEQ ID NO: 109: Description of artificial sequence: amino acid sequence of heavy chain of Ct-Fa1046-WTS-R1090
SEQ ID NO: 110: Description of artificial sequence: nucleotide sequence of heavy chain of Ct-Fa1046-TTS-R1090
SEQ ID NO: 111: Description of artificial sequence: amino acid sequence of heavy chain of Ct-Fa1046-TTS-R1090
SEQ ID NO: 112: Description of artificial sequence: amino acid sequence of constituent molecule 1 of P1AA9663
SEQ ID NO: 113: Description of artificial sequence: amino acid sequence of constituent molecule 2 of P1AA9663
SEQ ID NO: 114: Description of artificial sequence: amino acid sequence of constituent molecule 3 of P1AA9663
SEQ ID NO: 115: Description of artificial sequence: nucleotide sequence of constituent molecule 1 of P1AA9663
SEQ ID NO: 116: Description of artificial sequence: nucleotide sequence of constituent molecule 2 of P1AA9663
SEQ ID NO: 117: Description of artificial sequence: nucleotide sequence of constituent molecule 3 of P1AA9663

## Claims

1. A bispecific antibody, comprising an IgG portion comprising a first antigen binding domain and also comprising a second antigen binding domain, and binding to human CD40 and human fibroblast activation protein alpha (FAP), wherein the C terminus of a heavy chain of the IgG portion binds to the second antigen binding domain either directly or via a linker.

2. The bispecific antibody according to claim 1, wherein either one of the first antigen binding domain and the second antigen binding domain is derived from a non-agonistic anti-CD40 antibody.

3. The bispecific antibody according to claim 1 or 2, which divalently binds to each of human CD40 and human FAP.

4. The bispecific antibody according to any one of claims 1 to 3, wherein the C terminus of the heavy chain of the IgG portion directly binds to the second antigen binding domain.

5. The bispecific antibody according to any one of claims 1 to 4, which has a CD40 agonistic activity.

6. The bispecific antibody according to any one of claims 1 to 5, which does not exhibit a CD40 agonistic activity in the absence of a FAP-expressing cell, and exhibits a CD40 agonistic activity only in the presence of a FAP-expressing cell.

7. The bispecific antibody according to any one of claims 1 to 6, wherein either one of the first antigen binding domain and the second antigen binding domain is Fab of an anti-CD40 IgG antibody (anti-CD40 Fab).

8. The bispecific antibody according to any one of claims 1 to 7, wherein either one of the first antigen binding domain and the second antigen binding domain is Fab of an anti-FAP antibody (anti-FAP Fab).

9. The bispecific antibody according to any one of claims 1 to 8, wherein the first antigen binding domain is anti-CD40 Fab and the second antigen binding domain is anti-FAP Fab.

10. The bispecific antibody according to any one of claims 1 to 8, wherein the first antigen binding domain is anti-FAP Fab and the second antigen binding domain is anti-CD40 Fab.

11. The bispecific antibody according to claim 7, 9, or 10, wherein the anti-CD40 Fab is Fab comprising a heavy chain variable region (VH) comprising complementarity determining regions (CDRs) 1 to 3 comprising the amino acid sequences of SEQ ID NOS: 24 to 26, respectively, and a light chain variable region (VL) comprising CDRs 1 to 3 comprising the amino acid sequences of SEQ ID NOS: 19 to 21, respectively.

12. The bispecific antibody according to any one of claims 7, and 9 to 11, wherein the anti-CD40 Fab is Fab comprising VH comprising the amino acid sequence of SEQ ID NO: 23, and VL comprising the amino acid sequence of SEQ ID NO: 18.

13. The bispecific antibody according to any one of claims 8 to 12, wherein the anti-FAP Fab is Fab comprising VH comprising heavy chain CDRs 1 to 3 depicted in any one selected from the following (i) to (v) and VL comprising CDRs 1 to 3 comprising the amino acid sequences of SEQ ID NOS: 19 to 21, respectively:
(i) heavy chain CDRs 1 to 3 comprising the amino acid sequences of SEQ ID NOS: 33 to 35, respectively;
(ii) heavy chain CDRs 1 to 3 comprising the amino acid sequences of SEQ ID NOS: 38 to 40, respectively;
(iii) heavy chain CDRs 1 to 3 comprising the amino acid sequences of SEQ ID NOS: 43 to 45, respectively;
(iv) heavy chain CDRs 1 to 3 comprising the amino acid sequences of SEQ ID NOS: 48 to 50, respectively; and
(v) heavy chain CDRs 1 to 3 comprising the amino acid sequences of SEQ ID NOS: 53 to 55, respectively.

14. The bispecific antibody according to any one of claims 8 to 13, wherein the anti-FAP Fab is Fab comprising VH comprising the amino acid sequence of any one selected from SEQ ID NOS: 32, 37, 42, 47, 52, 75, 77, 79, 81, and 83, and VL comprising the amino acid sequence of SEQ ID NO: 18.

15. The bispecific antibody according to any one of claims 1 to 14, wherein the IgG portion comprises a heavy chain constant region comprising the amino acid sequence of SEQ ID NO: 61.

16. The bispecific antibody according to any one of claims 1 to 9 and 11 to 15, which consists of two heavy chains comprising the amino acid sequence of any one selected from SEQ ID NOS: 65, 67, 69, 71, 73, 85, 87, 89, 91, and 93, and four light chains comprising VL comprising the amino acid sequence of SEQ ID NO: 18.

17. The bispecific antibody according to any one of claims 1 to 8 and 10 to 15, which consists of two heavy chains comprising the amino acid sequence of any one selected from SEQ ID NOS: 95, 97, 99, 101, 103, 105, 107, 109, and 111, and four light chains comprising VL comprising the amino acid sequence of SEQ ID NO: 18.

18. A bispecific antibody fragment of the bispecific antibody according to any one of claims 1 to 17.

19. A DNA encoding the bispecific antibody according to any one of claims 1 to 17 or the bispecific antibody fragment according to claim 18.

20. A recombinant vector, comprising the DNA according to claim 19.

21. A transformant strain obtained by introducing the recombinant vector according to claim 20 into a host cell.

22. A method for producing the bispecific antibody according to any one of claims 1 to 17 or the bispecific antibody fragment according to claim 18, **characterized by** culturing the transformant strain according to claim 21 in a culture medium to produce and accumulate the bispecific antibody according to any one of claims 1 to 17 or the bispecific antibody fragment according to claim 18 in a culture, and collecting the bispecific antibody or the bispecific antibody fragment according to claim 18 from the culture.

23. A therapeutic agent and/or a diagnostic agent for a disease associated with at least one of CD40 and FAP, comprising the bispecific antibody according to any one of claims 1 to 17 or the bispecific antibody fragment according to claim 18 as an active ingredient.

24. The therapeutic agent and/or the diagnostic agent according to claim 23, wherein the disease associated with at least one of CD40 and FAP is a cancer.

25. A therapeutic method and/or a diagnostic method for a disease associated with at least one of CD40 and FAP, using the bispecific antibody according to any one of claims 1 to 17 or the bispecific antibody fragment according to claim 18.

26. The method according to claim 25, wherein the disease associated with at least one of CD40 and FAP is a cancer.

27. The bispecific antibody according to any one of claims 1 to 17 or the bispecific antibody fragment according to claim 18 for use in therapy and/or diagnosis for a disease associated with at least one of CD40 and FAP.

28. The bispecific antibody or the bispecific antibody fragment according to claim 27, wherein the disease associated with at least one of CD40 and FAP is a cancer.

29. Use of the bispecific antibody according to any one of claims 1 to 17 or the bispecific antibody fragment according to claim 18 for producing a therapeutic agent and/or a diagnostic agent for a disease associated with at least one of CD40 and FAP.

30. The use according to claim 28, wherein the disease associated with at least one of CD40 and FAP is a cancer.

31. A reagent for detecting or measuring at least one of CD40 and FAP, comprising the bispecific antibody according to any one of claims 1 to 17 or the bispecific antibody fragment according to claim 18.

32. A derivative of a bispecific antibody or a bispecific antibody fragment thereof, in which a radioisotope, a low-molecular weight drug, a high-molecular weight drug, a protein, or an antibody drug is bound chemically or through genetic engineering to the bispecific antibody according to any one of claims 1 to 17 or the bispecific antibody fragment according to claim 18.


SEQUENCE LISTING

<110>  Kyowa Kirin Co., Ltd.

<120>  Bispecific antibody binding to CD40 and FAP

<130>  W529460

<150>  JP2019-092298
<151>  2019-05-15

<160>  117

<170>  PatentIn version 3.5

<210>  1
<211>  522
<212>  DNA
<213>  Homo sapiens

<400>  1
gaaccaccca ctgcatgcag agaaaaacag tacctaataa acagtcagtg ctgttctttg      60

tgccagccag gacagaaact ggtgagtgac tgcacagagt tcactgaaac ggaatgcctt     120

ccttgcggtg aaagcgaatt cctagacacc tggaacagag agacacactg ccaccagcac     180

aaatactgcg accccaacct agggcttcgg gtccagcaga agggcacctc agaaacagac     240

accatctgca cctgtgaaga aggctggcac tgtacgagtg aggcctgtga gagctgtgtc     300

ctgcaccgct catgctcgcc cggctttggg gtcaagcaga ttgctacagg ggtttctgat     360

accatctgcg agccctgccc agtcggcttc ttctccaatg tgtcatctgc tttcgaaaaa     420

tgtcaccctt ggacaagctg tgagaccaaa gacctggttg tgcaacaggc aggcacaaac     480

aagactgatg ttgtctgtgg tccccaggat cggctgagag cc                        522


<210>  2
<211>  174
<212>  PRT
<213>  Homo sapiens

<400>  2

Glu Pro Pro Thr Ala Cys Arg Glu Lys Gln Tyr Leu Ile Asn Ser Gln
1               5                   10                  15


Cys Cys Ser Leu Cys Gln Pro Gly Gln Lys Leu Val Ser Asp Cys Thr
            20                  25                  30


Glu Phe Thr Glu Thr Glu Cys Leu Pro Cys Gly Glu Ser Glu Phe Leu
        35                  40                  45


Asp Thr Trp Asn Arg Glu Thr His Cys His Gln His Lys Tyr Cys Asp
    50                  55                  60

Pro Asn Leu Gly Leu Arg Val Gln Gln Lys Gly Thr Ser Glu Thr Asp
65                  70                  75                  80

Thr Ile Cys Thr Cys Glu Glu Gly Trp His Cys Thr Ser Glu Ala Cys
                85                  90                  95

Glu Ser Cys Val Leu His Arg Ser Cys Ser Pro Gly Phe Gly Val Lys
            100                 105                 110

Gln Ile Ala Thr Gly Val Ser Asp Thr Ile Cys Glu Pro Cys Pro Val
        115                 120                 125

Gly Phe Phe Ser Asn Val Ser Ser Ala Phe Glu Lys Cys His Pro Trp
    130                 135                 140

Thr Ser Cys Glu Thr Lys Asp Leu Val Val Gln Gln Ala Gly Thr Asn
145                 150                 155                 160

Lys Thr Asp Val Val Cys Gly Pro Gln Asp Arg Leu Arg Ala
                165                 170

<210>  3
<211>  522
<212>  DNA
<213>  Macaca fascicularis

<400>  3
gaaccaccca ctgcatgcag agaaaaacag tacctaataa acagtcagtg ctgttctttg      60

tgccagccag gacagaaact ggtgagtgac tgcacagagt tcaccgaaac agaatgcctt     120

ccttgcggtg aaagcgaatt cctagacacc tggaatagag agacacgctg ccaccagcac     180

aaatactgcg accccaacct agggcttcgg gtccagcaga agggcacctc agaaacagac     240

accatctgca cctgtgaaga aggcctgcac tgtacgagtg agtcctgtga gagctgtgtc     300

ccgcaccgct catgcttgcc tggctttggg gtcaagcaga ttgctacagg ggtttctgat     360

accatctgtg agccctgccc ggtcggcttc ttctccaatg tgtcatctgc ttttgaaaag     420

tgtcgccctt ggacaagctg tgagaccaaa gacctggttg tgcaacaggc aggcacaaac     480

aagactgatg ttgtctgtgg tccccaggat cggcagagag cc                        522

<210>  4
<211>  174
<212>  PRT
<213>  Macaca fascicularis

<400>  4

Glu Pro Pro Thr Ala Cys Arg Glu Lys Gln Tyr Leu Ile Asn Ser Gln
1               5                   10                  15

Cys Cys Ser Leu Cys Gln Pro Gly Gln Lys Leu Val Ser Asp Cys Thr
            20                  25                  30

Glu Phe Thr Glu Thr Glu Cys Leu Pro Cys Gly Glu Ser Glu Phe Leu
        35                  40                  45

Asp Thr Trp Asn Arg Glu Thr Arg Cys His Gln His Lys Tyr Cys Asp
    50                  55                  60

Pro Asn Leu Gly Leu Arg Val Gln Gln Lys Gly Thr Ser Glu Thr Asp
65                  70                  75                  80

Thr Ile Cys Thr Cys Glu Glu Gly Leu His Cys Thr Ser Glu Ser Cys
                85                  90                  95

Glu Ser Cys Val Pro His Arg Ser Cys Leu Pro Gly Phe Gly Val Lys
            100                 105                 110

Gln Ile Ala Thr Gly Val Ser Asp Thr Ile Cys Glu Pro Cys Pro Val
        115                 120                 125

Gly Phe Phe Ser Asn Val Ser Ser Ala Phe Glu Lys Cys Arg Pro Trp
    130                 135                 140

Thr Ser Cys Glu Thr Lys Asp Leu Val Val Gln Gln Ala Gly Thr Asn
145                 150                 155                 160

Lys Thr Asp Val Val Cys Gly Pro Gln Asp Arg Gln Arg Ala
                165                 170

<210>  5
<211>  834
<212>  DNA
<213>  Homo sapiens

<400>  5
atggttcgtc tgcctctgca gtgcgtcctc tggggctgct tgctgaccgc tgtccatcca      60

gaaccaccca ctgcatgcag agaaaaacag tacctaataa acagtcagtg ctgttctttg     120

tgccagccag gacagaaact ggtgagtgac tgcacagagt tcactgaaac ggaatgcctt     180

ccttgcggtg aaagcgaatt cctagacacc tggaacagag agacacactg ccaccagcac     240

aaatactgcg accccaacct agggcttcgg gtccagcaga agggcacctc agaaacagac     300

accatctgca cctgtgaaga aggctggcac tgtacgagtg aggcctgtga gagctgtgtc     360

ctgcaccgct catgctcgcc cggctttggg gtcaagcaga ttgctacagg ggtttctgat     420

accatctgcg agccctgccc agtcggcttc ttctccaatg tgtcatctgc tttcgaaaaa     480

tgtcaccctt ggacaagctg tgagaccaaa gacctggttg tgcaacaggc aggcacaaac 540

aagactgatg ttgtctgtgg tccccaggat cggctgagag ccctggtggt gatccccatc 600

atcttcggga tcctgtttgc catcctcttg gtgctggtct ttatcaaaaa ggtggccaag 660

aagccaacca ataaggcccc ccaccccaag caggaacccc aggagatcaa ttttcccgac 720

gatcttcctg gctccaacac tgctgctcca gtgcaggaga ctttacatgg atgccaaccg 780

gtcacccagg aggatggcaa agagagtcgc atctcagtgc aggagagaca gtga 834

<210> 6
<211> 277
<212> PRT
<213> Homo sapiens

<400> 6

Met Val Arg Leu Pro Leu Gln Cys Val Leu Trp Gly Cys Leu Leu Thr
1               5                   10                  15

Ala Val His Pro Glu Pro Pro Thr Ala Cys Arg Glu Lys Gln Tyr Leu
            20                  25                  30

Ile Asn Ser Gln Cys Cys Ser Leu Cys Gln Pro Gly Gln Lys Leu Val
        35                  40                  45

Ser Asp Cys Thr Glu Phe Thr Glu Thr Glu Cys Leu Pro Cys Gly Glu
    50                  55                  60

Ser Glu Phe Leu Asp Thr Trp Asn Arg Glu Thr His Cys His Gln His
65                  70                  75                  80

Lys Tyr Cys Asp Pro Asn Leu Gly Leu Arg Val Gln Gln Lys Gly Thr
                85                  90                  95

Ser Glu Thr Asp Thr Ile Cys Thr Cys Glu Glu Gly Trp His Cys Thr
            100                 105                 110

Ser Glu Ala Cys Glu Ser Cys Val Leu His Arg Ser Cys Ser Pro Gly
        115                 120                 125

Phe Gly Val Lys Gln Ile Ala Thr Gly Val Ser Asp Thr Ile Cys Glu
    130                 135                 140

Pro Cys Pro Val Gly Phe Phe Ser Asn Val Ser Ser Ala Phe Glu Lys
145                 150                 155                 160

Cys His Pro Trp Thr Ser Cys Glu Thr Lys Asp Leu Val Val Gln Gln
                165                 170                 175

Ala Gly Thr Asn Lys Thr Asp Val Val Cys Gly Pro Gln Asp Arg Leu
            180                 185                 190

Arg Ala Leu Val Val Ile Pro Ile Ile Phe Gly Ile Leu Phe Ala Ile
        195                 200                 205

Leu Leu Val Leu Val Phe Ile Lys Lys Val Ala Lys Lys Pro Thr Asn
    210                 215                 220

Lys Ala Pro His Pro Lys Gln Glu Pro Gln Glu Ile Asn Phe Pro Asp
225                 230                 235                 240

Asp Leu Pro Gly Ser Asn Thr Ala Ala Pro Val Gln Glu Thr Leu His
                245                 250                 255

Gly Cys Gln Pro Val Thr Gln Glu Asp Gly Lys Glu Ser Arg Ile Ser
            260                 265                 270

Val Gln Glu Arg Gln
        275

<210>  7
<211>  849
<212>  DNA
<213>  Macaca fascicularis

<400>  7
atggttcgtc tgcctctgca gtgcgtcctc tggggctgct tgctgaccgc tgtctatcca      60

gaaccaccca ctgcatgcag agaaaaacag tacctaataa acagtcagtg ctgttctttg     120

tgccagccag gacagaaact ggtgagtgac tgcacagagt tcaccgaaac agaatgcctt     180

ccttgcggtg aaagcgaatt cctagacacc tggaatagag agacacgctg ccaccagcac     240

aaatactgcg accccaacct agggcttcgg gtccagcaga agggcacctc agaaacagac     300

accatctgca cctgtgaaga aggcctgcac tgtacgagtg agtcctgtga gagctgtgtc     360

ccgcaccgct catgcttgcc tggctttggg gtcaagcaga ttgctacagg ggtttctgat     420

accatctgtg agccctgccc ggtcggcttc ttctccaatg tgtcatctgc ttttgaaaag     480

tgtcgccctt ggacaagctg tgagaccaaa gacctggttg tgcaacaggc aggcacaaac     540

aagactgatg ttgtctgtgg tccccaggat cggcagagag ccctggtggt gatccccatc     600

tgcttgggga tcctgtttgt catcctcctc ttggtgctgg tctttatcag tgagtcctca     660

gaaaaggtgg ccaagaagcc aaacgataag gcccccaccc ccaagcagga accccaggag     720

atcaattttc tggacgatct tcctggctcc aaccctgccg ctccagtgca ggagacttta     780

catggatgcc aaccggtcac ccaggaggat ggcaaagaga gtcgcatctc agtgcaggag     840

agacagtga                                                              849

<210>  8
<211>  282
<212>  PRT
<213>  Macaca fascicularis

<400>  8

Met Val Arg Leu Pro Leu Gln Cys Val Leu Trp Gly Cys Leu Leu Thr
1               5                   10                  15

Ala Val Tyr Pro Glu Pro Pro Thr Ala Cys Arg Glu Lys Gln Tyr Leu
            20                  25                  30

Ile Asn Ser Gln Cys Cys Ser Leu Cys Gln Pro Gly Gln Lys Leu Val
        35                  40                  45

Ser Asp Cys Thr Glu Phe Thr Glu Thr Glu Cys Leu Pro Cys Gly Glu
    50                  55                  60

Ser Glu Phe Leu Asp Thr Trp Asn Arg Glu Thr Arg Cys His Gln His
65                  70                  75                  80

Lys Tyr Cys Asp Pro Asn Leu Gly Leu Arg Val Gln Gln Lys Gly Thr
                85                  90                  95

Ser Glu Thr Asp Thr Ile Cys Thr Cys Glu Glu Gly Leu His Cys Thr
            100                 105                 110

Ser Glu Ser Cys Glu Ser Cys Val Pro His Arg Ser Cys Leu Pro Gly
        115                 120                 125

Phe Gly Val Lys Gln Ile Ala Thr Gly Val Ser Asp Thr Ile Cys Glu
    130                 135                 140

Pro Cys Pro Val Gly Phe Phe Ser Asn Val Ser Ser Ala Phe Glu Lys
145                 150                 155                 160

Cys Arg Pro Trp Thr Ser Cys Glu Thr Lys Asp Leu Val Val Gln Gln
                165                 170                 175

Ala Gly Thr Asn Lys Thr Asp Val Val Cys Gly Pro Gln Asp Arg Gln
            180                 185                 190

Arg Ala Leu Val Val Ile Pro Ile Cys Leu Gly Ile Leu Phe Val Ile
        195                 200                 205

Leu Leu Leu Val Leu Val Phe Ile Ser Glu Ser Ser Glu Lys Val Ala

210                 215                 220

Lys Lys Pro Asn Asp Lys Ala Pro His Pro Lys Gln Glu Pro Gln Glu
225                 230                 235                 240

Ile Asn Phe Leu Asp Asp Leu Pro Gly Ser Asn Pro Ala Ala Pro Val
                245                 250                 255

Gln Glu Thr Leu His Gly Cys Gln Pro Val Thr Gln Glu Asp Gly Lys
            260                 265                 270

Glu Ser Arg Ile Ser Val Gln Glu Arg Gln
        275                 280

<210>  9
<211>  2202
<212>  DNA
<213>  Homo sapiens

<400>  9
cgcccttcaa gagttcataa ctctgaagaa aatacaatga gagcactcac actgaaggat      60

attttaaatg gaacattttc ttataaaaca ttttttccaa actggatttc aggacaagaa      120

tatcttcatc aatctgcaga taacaatata gtactttata atattgaaac aggacaatca      180

tataccattt tgagtaatag aaccatgaaa agtgtgaatg cttcaaatta cggcttatca      240

cctgatcggc aatttgtata tctagaaagt gattattcaa agctttggag atactcttac      300

acagcaacat attacatcta tgaccttagc aatggagaat ttgtaagagg aaatgagctt      360

cctcgtccaa ttcagtattt atgctggtcg cctgttggga gtaaattagc atatgtctat      420

caaaacaata tctatttgaa acaaagacca ggagatccac cttttcaaat aacatttaat      480

ggaagagaaa ataaaatatt taatggaatc ccagactggg tttatgaaga ggaaatgctt      540

gctacaaaat atgctctctg gtggtctcct aatggaaaat ttttggcata tgcggaattt      600

aatgatacgg atataccagt tattgcctat tcctattatg gcgatgaaca atatcctaga      660

acaataaata ttccataccc aaaggctgga gctaagaatc ccgttgttcg gatatttatt      720

atcgatacca cttaccctgc gtatgtaggt ccccaggaag tgcctgttcc agcaatgata      780

gcctcaagtg attattattt cagttggctc acgtgggtta ctgatgaacg agtatgtttg      840

cagtggctaa aaagagtcca gaatgtttcg gtcctgtcta tatgtgactt cagggaagac      900

tggcagacat gggattgtcc aaagacccag gagcatatag aagaaagcag aactggatgg      960

gctggtggat tctttgtttc aacaccagtt ttcagctatg atgccatttc gtactacaaa      1020

atatttagtg acaaggatgg ctacaaacat attcactata tcaaagacac tgtggaaaat      1080

gctattcaaa ttacaagtgg caagtgggag gccataaata tattcagagt aacacaggat      1140

tcactgtttt attctagcaa tgaatttgaa gaataccctg gaagaagaaa catctacaga 1200

attagcattg gaagctatcc tccaagcaag aagtgtgtta cttgccatct aaggaaagaa 1260

aggtgccaat attacacagc aagtttcagc gactacgcca agtactatgc acttgtctgc 1320

tacggcccag gcatccccat ttccaccctt catgatggac gcactgatca agaaattaaa 1380

atcctggaag aaaacaagga attggaaaat gctttgaaaa atatccagct gcctaaagag 1440

gaaattaaga aacttgaagt agatgaaatt actttatggt acaagatgat tcttcctcct 1500

caatttgaca gatcaaagaa gtatcccttg ctaattcaag tgtatggtgg tccctgcagt 1560

cagagtgtaa ggtctgtatt tgctgttaat tggatatctt atcttgcaag taaggaaggg 1620

atggtcattg ccttggtgga tggtcgagga acagctttcc aaggtgacaa actcctctat 1680

gcagtgtatc gaaagctggg tgtttatgaa gttgaagacc agattacagc tgtcagaaaa 1740

ttcatagaaa tgggtttcat tgatgaaaaa agaatagcca tatggggctg gtcctatgga 1800

ggatacgttt catcactggc ccttgcatct ggaactggtc ttttcaaatg tggtatagca 1860

gtggctccag tctccagctg ggaatattac gcgtctgtct acacagagag attcatgggt 1920

ctcccaacaa aggatgataa tcttgagcac tataagaatt caactgtgat ggcaagagca 1980

gaatatttca gaaatgtaga ctatcttctc atccacggaa cagcagatga taatgtgcac 2040

tttcaaaact cagcacagat tgctaaagct ctggttaatg cacaagtgga tttccaggca 2100

atgtggtact ctgaccagaa ccacggctta tccggcctgt ccacgaacca cttatacacc 2160

cacatgaccc acttcctaaa gcagtgtttc tctttgtcag ac 2202

<210> 10
<211> 734
<212> PRT
<213> Homo sapiens

<400> 10

Arg Pro Ser Arg Val His Asn Ser Glu Glu Asn Thr Met Arg Ala Leu
1               5                   10                  15

Thr Leu Lys Asp Ile Leu Asn Gly Thr Phe Ser Tyr Lys Thr Phe Phe
            20                  25                  30

Pro Asn Trp Ile Ser Gly Gln Glu Tyr Leu His Gln Ser Ala Asp Asn
        35                  40                  45

Asn Ile Val Leu Tyr Asn Ile Glu Thr Gly Gln Ser Tyr Thr Ile Leu
    50                  55                  60

Ser Asn Arg Thr Met Lys Ser Val Asn Ala Ser Asn Tyr Gly Leu Ser
65                  70                  75                  80

Pro Asp Arg Gln Phe Val Tyr Leu Glu Ser Asp Tyr Ser Lys Leu Trp
                85                  90                  95

Arg Tyr Ser Tyr Thr Ala Thr Tyr Tyr Ile Tyr Asp Leu Ser Asn Gly
            100                 105                 110

Glu Phe Val Arg Gly Asn Glu Leu Pro Arg Pro Ile Gln Tyr Leu Cys
        115                 120                 125

Trp Ser Pro Val Gly Ser Lys Leu Ala Tyr Val Tyr Gln Asn Asn Ile
    130                 135                 140

Tyr Leu Lys Gln Arg Pro Gly Asp Pro Pro Phe Gln Ile Thr Phe Asn
145                 150                 155                 160

Gly Arg Glu Asn Lys Ile Phe Asn Gly Ile Pro Asp Trp Val Tyr Glu
                165                 170                 175

Glu Glu Met Leu Ala Thr Lys Tyr Ala Leu Trp Trp Ser Pro Asn Gly
            180                 185                 190

Lys Phe Leu Ala Tyr Ala Glu Phe Asn Asp Thr Asp Ile Pro Val Ile
        195                 200                 205

Ala Tyr Ser Tyr Tyr Gly Asp Glu Gln Tyr Pro Arg Thr Ile Asn Ile
    210                 215                 220

Pro Tyr Pro Lys Ala Gly Ala Lys Asn Pro Val Val Arg Ile Phe Ile
225                 230                 235                 240

Ile Asp Thr Thr Tyr Pro Ala Tyr Val Gly Pro Gln Glu Val Pro Val
                245                 250                 255

Pro Ala Met Ile Ala Ser Ser Asp Tyr Tyr Phe Ser Trp Leu Thr Trp
            260                 265                 270

Val Thr Asp Glu Arg Val Cys Leu Gln Trp Leu Lys Arg Val Gln Asn
        275                 280                 285

Val Ser Val Leu Ser Ile Cys Asp Phe Arg Glu Asp Trp Gln Thr Trp
    290                 295                 300

Asp Cys Pro Lys Thr Gln Glu His Ile Glu Glu Ser Arg Thr Gly Trp
305                 310                 315                 320

Ala Gly Gly Phe Phe Val Ser Thr Pro Val Phe Ser Tyr Asp Ala Ile
                325                 330                 335

Ser Tyr Tyr Lys Ile Phe Ser Asp Lys Asp Gly Tyr Lys His Ile His
        340                 345                 350

Tyr Ile Lys Asp Thr Val Glu Asn Ala Ile Gln Ile Thr Ser Gly Lys
    355                 360                 365

Trp Glu Ala Ile Asn Ile Phe Arg Val Thr Gln Asp Ser Leu Phe Tyr
370                 375                 380

Ser Ser Asn Glu Phe Glu Glu Tyr Pro Gly Arg Arg Asn Ile Tyr Arg
385                 390                 395                 400

Ile Ser Ile Gly Ser Tyr Pro Pro Ser Lys Lys Cys Val Thr Cys His
                405                 410                 415

Leu Arg Lys Glu Arg Cys Gln Tyr Tyr Thr Ala Ser Phe Ser Asp Tyr
            420                 425                 430

Ala Lys Tyr Tyr Ala Leu Val Cys Tyr Gly Pro Gly Ile Pro Ile Ser
        435                 440                 445

Thr Leu His Asp Gly Arg Thr Asp Gln Glu Ile Lys Ile Leu Glu Glu
    450                 455                 460

Asn Lys Glu Leu Glu Asn Ala Leu Lys Asn Ile Gln Leu Pro Lys Glu
465                 470                 475                 480

Glu Ile Lys Lys Leu Glu Val Asp Glu Ile Thr Leu Trp Tyr Lys Met
                485                 490                 495

Ile Leu Pro Pro Gln Phe Asp Arg Ser Lys Lys Tyr Pro Leu Leu Ile
            500                 505                 510

Gln Val Tyr Gly Gly Pro Cys Ser Gln Ser Val Arg Ser Val Phe Ala
        515                 520                 525

Val Asn Trp Ile Ser Tyr Leu Ala Ser Lys Glu Gly Met Val Ile Ala
    530                 535                 540

Leu Val Asp Gly Arg Gly Thr Ala Phe Gln Gly Asp Lys Leu Leu Tyr
545                 550                 555                 560

Ala Val Tyr Arg Lys Leu Gly Val Tyr Glu Val Glu Asp Gln Ile Thr
                565                 570                 575

Ala Val Arg Lys Phe Ile Glu Met Gly Phe Ile Asp Glu Lys Arg Ile

580                 585                 590

Ala Ile Trp Gly Trp Ser Tyr Gly Gly Tyr Val Ser Ser Leu Ala Leu
        595                 600                 605

Ala Ser Gly Thr Gly Leu Phe Lys Cys Gly Ile Ala Val Ala Pro Val
    610                 615                 620

Ser Ser Trp Glu Tyr Tyr Ala Ser Val Tyr Thr Glu Arg Phe Met Gly
625                 630                 635                 640

Leu Pro Thr Lys Asp Asp Asn Leu Glu His Tyr Lys Asn Ser Thr Val
                645                 650                 655

Met Ala Arg Ala Glu Tyr Phe Arg Asn Val Asp Tyr Leu Leu Ile His
            660                 665                 670

Gly Thr Ala Asp Asp Asn Val His Phe Gln Asn Ser Ala Gln Ile Ala
        675                 680                 685

Lys Ala Leu Val Asn Ala Gln Val Asp Phe Gln Ala Met Trp Tyr Ser
    690                 695                 700

Asp Gln Asn His Gly Leu Ser Gly Leu Ser Thr Asn His Leu Tyr Thr
705                 710                 715                 720

His Met Thr His Phe Leu Lys Gln Cys Phe Ser Leu Ser Asp
                725                 730

<210>  11
<211>  2205
<212>  DNA
<213>  Mus musculus

<400>  11
cgtccctcaa gagtttacaa acctgaagga aacacaaaga gagctcttac cttgaaggat      60

attttaaatg gaacattctc atataaaaca tattttccca actggatttc agaacaagaa     120

tatcttcatc aatctgagga tgataacata gtattttata atattgaaac aagagaatca     180

tatatcattt tgagtaatag caccatgaaa agtgtgaatg ctacagatta tggtttgtca     240

cctgatcggc aatttgtgta tctagaaagt gattattcaa agctctggcg atattcatac     300

acagcgacat actacatcta cgaccttcag aatggggaat ttgtaagagg atacgagctc     360

cctcgtccaa ttcagtatct atgctggtcg cctgttggga gtaaattagc atatgtatat     420

caaaacaata tttatttgaa acaaagacca ggagatccac cttttcaaat aacttatact     480

ggaagagaaa atagaatatt taatggaata ccagactggg tttatgaaga ggaaatgctt     540

gccacaaaat atgctctttg gtggtctcca gatggaaaat ttttggcata tgtagaattt 600

aatgattcag atataccaat tattgcctat tcttattatg gtgatggaca gtatcctaga 660

actataaata ttccatatcc aaaggctggg gctaagaatc cggttgttcg tgtttttatt 720

gttgacacca cctaccctca ccacgtgggc ccaatggaag tgccagttcc agaaatgata 780

gcctcaagtg actattattt cagctggctc acatgggtgt ccagtgaacg agtatgcttg 840

cagtggctaa aaagagtgca gaatgtctca gtcctgtcta tatgtgattt cagggaagac 900

tggcatgcat gggaatgtcc aaagaaccag gagcatgtag aagaaagcag aacaggatgg 960

gctggtggat tctttgtttc gacaccagct tttagccagg atgccacttc ttactacaaa 1020

atatttagcg acaaggatgg ttacaaacat attcactaca tcaaagacac tgtggaaaat 1080

gctattcaaa ttacaagtgg caagtgggag gccatatata tattccgcgt aacacaggat 1140

tcactgtttt attctagcaa tgaatttgaa ggttaccctg gaagaagaaa catctacaga 1200

attagcattg gaaactctcc tccgagcaag aagtgtgtta cttgccatct aaggaaagaa 1260

aggtgccaat attacacagc aagtttcagc tacaaagcca agtactatgc actcgtctgc 1320

tatggccctg gcctccccat ttccaccctc catgatggcc gcacagacca agaaatacaa 1380

gtattagaag aaaacaaaga actggaaaat tctctgagaa atatccagct gcctaaagtg 1440

gagattaaga agctcaaaga cgggggactg actttctggt acaagatgat tctgcctcct 1500

cagtttgaca gatcaaagaa gtaccctttg ctaattcaag tgtatggtgg tccttgcagc 1560

cagagtgtta agtctgtgtt tgctgttaat tggataactt atctcgcaag taaggagggg 1620

atagtcattg ccctggtaga tggtcggggc actgctttcc aaggtgacaa attcctgcat 1680

gccgtgtatc gaaaactggg tgtatatgaa gttgaggacc agctcacagc tgtcagaaaa 1740

ttcatagaaa tgggtttcat tgatgaagaa agaatagcca tatggggctg gtcctacgga 1800

ggttatgttt catccctggc ccttgcatct ggaactggtc ttttcaaatg tggcatagca 1860

gtggctccag tctccagctg ggaatattac gcatctatct actcagagag attcatgggc 1920

ctcccaacaa aggacgacaa tctcgaacac tataaaaatt caactgtgat ggcaagagca 1980

gaatatttca gaaatgtaga ctatcttctc atccacggaa cagcagatga taatgtgcac 2040

tttcagaact cagcacagat tgctaaagct ttggttaatg cacaagtgga tttccaggcg 2100

atgtggtact ctgaccagaa ccatggtata tcatctgggc gctcccagaa tcatttatat 2160

acccacatga cgcacttcct caagcaatgc ttttctttat cagac 2205

<210> 12
<211> 735
<212> PRT
<213> Mus musculus

<400> 12

Arg Pro Ser Arg Val Tyr Lys Pro Glu Gly Asn Thr Lys Arg Ala Leu
1               5                   10                  15

Thr Leu Lys Asp Ile Leu Asn Gly Thr Phe Ser Tyr Lys Thr Tyr Phe
            20                  25                  30

Pro Asn Trp Ile Ser Glu Gln Glu Tyr Leu His Gln Ser Glu Asp Asp
        35                  40                  45

Asn Ile Val Phe Tyr Asn Ile Glu Thr Arg Glu Ser Tyr Ile Ile Leu
    50                  55                  60

Ser Asn Ser Thr Met Lys Ser Val Asn Ala Thr Asp Tyr Gly Leu Ser
65                  70                  75                  80

Pro Asp Arg Gln Phe Val Tyr Leu Glu Ser Asp Tyr Ser Lys Leu Trp
                85                  90                  95

Arg Tyr Ser Tyr Thr Ala Thr Tyr Tyr Ile Tyr Asp Leu Gln Asn Gly
            100                 105                 110

Glu Phe Val Arg Gly Tyr Glu Leu Pro Arg Pro Ile Gln Tyr Leu Cys
        115                 120                 125

Trp Ser Pro Val Gly Ser Lys Leu Ala Tyr Val Tyr Gln Asn Asn Ile
    130                 135                 140

Tyr Leu Lys Gln Arg Pro Gly Asp Pro Pro Phe Gln Ile Thr Tyr Thr
145                 150                 155                 160

Gly Arg Glu Asn Arg Ile Phe Asn Gly Ile Pro Asp Trp Val Tyr Glu
                165                 170                 175

Glu Glu Met Leu Ala Thr Lys Tyr Ala Leu Trp Trp Ser Pro Asp Gly
            180                 185                 190

Lys Phe Leu Ala Tyr Val Glu Phe Asn Asp Ser Asp Ile Pro Ile Ile
        195                 200                 205

Ala Tyr Ser Tyr Tyr Gly Asp Gly Gln Tyr Pro Arg Thr Ile Asn Ile
    210                 215                 220

Pro Tyr Pro Lys Ala Gly Ala Lys Asn Pro Val Val Arg Val Phe Ile
225                 230                 235                 240

Val Asp Thr Thr Tyr Pro His His Val Gly Pro Met Glu Val Pro Val
                245                 250                 255

Pro Glu Met Ile Ala Ser Ser Asp Tyr Tyr Phe Ser Trp Leu Thr Trp
            260                 265                 270

Val Ser Ser Glu Arg Val Cys Leu Gln Trp Leu Lys Arg Val Gln Asn
        275                 280                 285

Val Ser Val Leu Ser Ile Cys Asp Phe Arg Glu Asp Trp His Ala Trp
    290                 295                 300

Glu Cys Pro Lys Asn Gln Glu His Val Glu Glu Ser Arg Thr Gly Trp
305                 310                 315                 320

Ala Gly Gly Phe Phe Val Ser Thr Pro Ala Phe Ser Gln Asp Ala Thr
                325                 330                 335

Ser Tyr Tyr Lys Ile Phe Ser Asp Lys Asp Gly Tyr Lys His Ile His
            340                 345                 350

Tyr Ile Lys Asp Thr Val Glu Asn Ala Ile Gln Ile Thr Ser Gly Lys
        355                 360                 365

Trp Glu Ala Ile Tyr Ile Phe Arg Val Thr Gln Asp Ser Leu Phe Tyr
    370                 375                 380

Ser Ser Asn Glu Phe Glu Gly Tyr Pro Gly Arg Arg Asn Ile Tyr Arg
385                 390                 395                 400

Ile Ser Ile Gly Asn Ser Pro Pro Ser Lys Lys Cys Val Thr Cys His
                405                 410                 415

Leu Arg Lys Glu Arg Cys Gln Tyr Tyr Thr Ala Ser Phe Ser Tyr Lys
            420                 425                 430

Ala Lys Tyr Tyr Ala Leu Val Cys Tyr Gly Pro Gly Leu Pro Ile Ser
        435                 440                 445

Thr Leu His Asp Gly Arg Thr Asp Gln Glu Ile Gln Val Leu Glu Glu
    450                 455                 460

Asn Lys Glu Leu Glu Asn Ser Leu Arg Asn Ile Gln Leu Pro Lys Val
465                 470                 475                 480

Glu Ile Lys Lys Leu Lys Asp Gly Gly Leu Thr Phe Trp Tyr Lys Met
                485                 490                 495

Ile Leu Pro Pro Gln Phe Asp Arg Ser Lys Lys Tyr Pro Leu Leu Ile

500                 505                 510

Gln Val Tyr Gly Gly Pro Cys Ser Gln Ser Val Lys Ser Val Phe Ala
        515                 520                 525

Val Asn Trp Ile Thr Tyr Leu Ala Ser Lys Glu Gly Ile Val Ile Ala
    530                 535                 540

Leu Val Asp Gly Arg Gly Thr Ala Phe Gln Gly Asp Lys Phe Leu His
545                 550                 555                 560

Ala Val Tyr Arg Lys Leu Gly Val Tyr Glu Val Glu Asp Gln Leu Thr
                565                 570                 575

Ala Val Arg Lys Phe Ile Glu Met Gly Phe Ile Asp Glu Glu Arg Ile
            580                 585                 590

Ala Ile Trp Gly Trp Ser Tyr Gly Gly Tyr Val Ser Ser Leu Ala Leu
        595                 600                 605

Ala Ser Gly Thr Gly Leu Phe Lys Cys Gly Ile Ala Val Ala Pro Val
    610                 615                 620

Ser Ser Trp Glu Tyr Tyr Ala Ser Ile Tyr Ser Glu Arg Phe Met Gly
625                 630                 635                 640

Leu Pro Thr Lys Asp Asp Asn Leu Glu His Tyr Lys Asn Ser Thr Val
                645                 650                 655

Met Ala Arg Ala Glu Tyr Phe Arg Asn Val Asp Tyr Leu Leu Ile His
            660                 665                 670

Gly Thr Ala Asp Asp Asn Val His Phe Gln Asn Ser Ala Gln Ile Ala
        675                 680                 685

Lys Ala Leu Val Asn Ala Gln Val Asp Phe Gln Ala Met Trp Tyr Ser
    690                 695                 700

Asp Gln Asn His Gly Ile Ser Ser Gly Arg Ser Gln Asn His Leu Tyr
705                 710                 715                 720

Thr His Met Thr His Phe Leu Lys Gln Cys Phe Ser Leu Ser Asp
                725                 730                 735

<210>  13
<211>  2280
<212>  DNA
<213>  Homo sapiens

<400>  13
atgaagactt gggtaaaaat cgtatttgga gttgccacct ctgctgtgct tgccttattg      60

gtgatgtgca ttgtcttacg cccttcaaga gttcataact ctgaagaaaa tacaatgaga     120

gcactcacac tgaaggatat tttaaatgga acattttctt ataaaacatt ttttccaaac     180

tggatttcag gacaagaata tcttcatcaa tctgcagata acaatatagt actttataat     240

attgaaacag gacaatcata taccattttg agtaatagaa ccatgaaaag tgtgaatgct     300

tcaaattacg gcttatcacc tgatcggcaa tttgtatatc tagaaagtga ttattcaaag     360

ctttggagat actcttacac agcaacatat tacatctatg accttagcaa tggagaattt     420

gtaagaggaa atgagcttcc tcgtccaatt cagtatttat gctggtcgcc tgttgggagt     480

aaattagcat atgtctatca aaacaatatc tatttgaaac aaagaccagg agatccacct     540

tttcaaataa catttaatgg aagagaaaat aaaatattta atggaatccc agactgggtt     600

tatgaagagg aaatgcttgc tacaaaatat gctctctggt ggtctcctaa tggaaaattt     660

ttggcatatg cggaatttaa tgatacggat ataccagtta ttgcctattc ctattatggc     720

gatgaacaat atcctagaac aataaatatt ccatacccaa aggctggagc taagaatccc     780

gttgttcgga tatttattat cgataccact taccctgcgt atgtaggtcc ccaggaagtg     840

cctgttccag caatgatagc ctcaagtgat tattatttca gttggctcac gtgggttact     900

gatgaacgag tatgtttgca gtggctaaaa agagtccaga atgtttcggt cctgtctata     960

tgtgacttca gggaagactg gcagacatgg gattgtccaa agacccagga gcatatagaa    1020

gaaagcagaa ctggatgggc tggtggattc tttgtttcaa caccagtttt cagctatgat    1080

gccatttcgt actacaaaat atttagtgac aaggatggct acaaacatat tcactatatc    1140

aaagacactg tggaaaatgc tattcaaatt acaagtggca agtgggaggc cataaatata    1200

ttcagagtaa cacaggattc actgttttat tctagcaatg aatttgaaga ataccctgga    1260

agaagaaaca tctacagaat tagcattgga agctatcctc caagcaagaa gtgtgttact    1320

tgccatctaa ggaaagaaag gtgccaatat tacacagcaa gtttcagcga ctacgccaag    1380

tactatgcac ttgtctgcta cggcccaggc atccccattt ccacccttca tgatggacgc    1440

actgatcaag aaattaaaat cctggaagaa aacaaggaat tggaaaatgc tttgaaaaat    1500

atccagctgc ctaaagagga aattaagaaa cttgaagtag atgaaattac tttatggtac    1560

aagatgattc ttcctcctca atttgacaga tcaaagaagt atcccttgct aattcaagtg    1620

tatggtggtc cctgcagtca gagtgtaagg tctgtatttg ctgttaattg gatatcttat    1680

cttgcaagta aggaagggat ggtcattgcc ttggtggatg gtcgaggaac agctttccaa    1740

ggtgacaaac tcctctatgc agtgtatcga aagctgggtg tttatgaagt tgaagaccag    1800

attacagctg tcagaaaatt catagaaatg ggtttcattg atgaaaaaag aatagccata    1860

tggggctggt cctatggagg atacgtttca tcactggccc ttgcatctgg aactggtctt 1920

ttcaaatgtg gtatagcagt ggctccagtc tccagctggg aatattacgc gtctgtctac 1980

acagagagat tcatgggtct cccaacaaag gatgataatc ttgagcacta taagaattca 2040

actgtgatgg caagagcaga atatttcaga aatgtagact atcttctcat ccacggaaca 2100

gcagatgata atgtgcactt tcaaaactca gcacagattg ctaaagctct ggttaatgca 2160

caagtggatt tccaggcaat gtggtactct gaccagaacc acggcttatc cggcctgtcc 2220

acgaaccact tatacaccca catgacccac ttcctaaagc agtgtttctc tttgtcagac 2280

<210> 14
<211> 760
<212> PRT
<213> Homo sapiens

<400> 14

Met Lys Thr Trp Val Lys Ile Val Phe Gly Val Ala Thr Ser Ala Val
1               5                   10                  15

Leu Ala Leu Leu Val Met Cys Ile Val Leu Arg Pro Ser Arg Val His
            20                  25                  30

Asn Ser Glu Glu Asn Thr Met Arg Ala Leu Thr Leu Lys Asp Ile Leu
        35                  40                  45

Asn Gly Thr Phe Ser Tyr Lys Thr Phe Phe Pro Asn Trp Ile Ser Gly
    50                  55                  60

Gln Glu Tyr Leu His Gln Ser Ala Asp Asn Asn Ile Val Leu Tyr Asn
65                  70                  75                  80

Ile Glu Thr Gly Gln Ser Tyr Thr Ile Leu Ser Asn Arg Thr Met Lys
                85                  90                  95

Ser Val Asn Ala Ser Asn Tyr Gly Leu Ser Pro Asp Arg Gln Phe Val
            100                 105                 110

Tyr Leu Glu Ser Asp Tyr Ser Lys Leu Trp Arg Tyr Ser Tyr Thr Ala
        115                 120                 125

Thr Tyr Tyr Ile Tyr Asp Leu Ser Asn Gly Glu Phe Val Arg Gly Asn
    130                 135                 140

Glu Leu Pro Arg Pro Ile Gln Tyr Leu Cys Trp Ser Pro Val Gly Ser
145                 150                 155                 160

Lys Leu Ala Tyr Val Tyr Gln Asn Asn Ile Tyr Leu Lys Gln Arg Pro
                165                 170                 175

Gly Asp Pro Pro Phe Gln Ile Thr Phe Asn Gly Arg Glu Asn Lys Ile
            180                 185                 190

Phe Asn Gly Ile Pro Asp Trp Val Tyr Glu Glu Glu Met Leu Ala Thr
        195                 200                 205

Lys Tyr Ala Leu Trp Trp Ser Pro Asn Gly Lys Phe Leu Ala Tyr Ala
    210                 215                 220

Glu Phe Asn Asp Thr Asp Ile Pro Val Ile Ala Tyr Ser Tyr Tyr Gly
225                 230                 235                 240

Asp Glu Gln Tyr Pro Arg Thr Ile Asn Ile Pro Tyr Pro Lys Ala Gly
                245                 250                 255

Ala Lys Asn Pro Val Val Arg Ile Phe Ile Ile Asp Thr Thr Tyr Pro
            260                 265                 270

Ala Tyr Val Gly Pro Gln Glu Val Pro Val Pro Ala Met Ile Ala Ser
        275                 280                 285

Ser Asp Tyr Tyr Phe Ser Trp Leu Thr Trp Val Thr Asp Glu Arg Val
    290                 295                 300

Cys Leu Gln Trp Leu Lys Arg Val Gln Asn Val Ser Val Leu Ser Ile
305                 310                 315                 320

Cys Asp Phe Arg Glu Asp Trp Gln Thr Trp Asp Cys Pro Lys Thr Gln
                325                 330                 335

Glu His Ile Glu Glu Ser Arg Thr Gly Trp Ala Gly Gly Phe Phe Val
            340                 345                 350

Ser Thr Pro Val Phe Ser Tyr Asp Ala Ile Ser Tyr Tyr Lys Ile Phe
        355                 360                 365

Ser Asp Lys Asp Gly Tyr Lys His Ile His Tyr Ile Lys Asp Thr Val
    370                 375                 380

Glu Asn Ala Ile Gln Ile Thr Ser Gly Lys Trp Glu Ala Ile Asn Ile
385                 390                 395                 400

Phe Arg Val Thr Gln Asp Ser Leu Phe Tyr Ser Ser Asn Glu Phe Glu
                405                 410                 415

Glu Tyr Pro Gly Arg Arg Asn Ile Tyr Arg Ile Ser Ile Gly Ser Tyr
            420                 425                 430

Pro Pro Ser Lys Lys Cys Val Thr Cys His Leu Arg Lys Glu Arg Cys
        435                 440                 445

Gln Tyr Tyr Thr Ala Ser Phe Ser Asp Tyr Ala Lys Tyr Tyr Ala Leu
    450                 455                 460

Val Cys Tyr Gly Pro Gly Ile Pro Ile Ser Thr Leu His Asp Gly Arg
465                 470                 475                 480

Thr Asp Gln Glu Ile Lys Ile Leu Glu Glu Asn Lys Glu Leu Glu Asn
                485                 490                 495

Ala Leu Lys Asn Ile Gln Leu Pro Lys Glu Glu Ile Lys Lys Leu Glu
            500                 505                 510

Val Asp Glu Ile Thr Leu Trp Tyr Lys Met Ile Leu Pro Pro Gln Phe
        515                 520                 525

Asp Arg Ser Lys Lys Tyr Pro Leu Leu Ile Gln Val Tyr Gly Gly Pro
    530                 535                 540

Cys Ser Gln Ser Val Arg Ser Val Phe Ala Val Asn Trp Ile Ser Tyr
545                 550                 555                 560

Leu Ala Ser Lys Glu Gly Met Val Ile Ala Leu Val Asp Gly Arg Gly
                565                 570                 575

Thr Ala Phe Gln Gly Asp Lys Leu Leu Tyr Ala Val Tyr Arg Lys Leu
            580                 585                 590

Gly Val Tyr Glu Val Glu Asp Gln Ile Thr Ala Val Arg Lys Phe Ile
        595                 600                 605

Glu Met Gly Phe Ile Asp Glu Lys Arg Ile Ala Ile Trp Gly Trp Ser
    610                 615                 620

Tyr Gly Gly Tyr Val Ser Ser Leu Ala Leu Ala Ser Gly Thr Gly Leu
625                 630                 635                 640

Phe Lys Cys Gly Ile Ala Val Ala Pro Val Ser Ser Trp Glu Tyr Tyr
                645                 650                 655

Ala Ser Val Tyr Thr Glu Arg Phe Met Gly Leu Pro Thr Lys Asp Asp
            660                 665                 670

Asn Leu Glu His Tyr Lys Asn Ser Thr Val Met Ala Arg Ala Glu Tyr
        675                 680                 685

Phe Arg Asn Val Asp Tyr Leu Leu Ile His Gly Thr Ala Asp Asp Asn
    690                 695                 700

Val His Phe Gln Asn Ser Ala Gln Ile Ala Lys Ala Leu Val Asn Ala
705                 710                 715                 720

Gln Val Asp Phe Gln Ala Met Trp Tyr Ser Asp Gln Asn His Gly Leu
                725                 730                 735

Ser Gly Leu Ser Thr Asn His Leu Tyr Thr His Met Thr His Phe Leu
            740                 745                 750

Lys Gln Cys Phe Ser Leu Ser Asp
        755                 760

<210>  15
<211>  2283
<212>  DNA
<213>  Mus musculus

<400>  15
atgaagacat ggctgaaaac tgtctttgga gttaccaccc tggctgcgct tgctttagtg      60

gtgatatgca ttgtcttacg tccctcaaga gtttacaaac ctgaaggaaa cacaaagaga     120

gctcttacct tgaaggatat tttaaatgga acattctcat ataaaacata ttttcccaac     180

tggatttcag aacaagaata tcttcatcaa tctgaggatg ataacatagt attttataat     240

attgaaacaa gagaatcata tatcattttg agtaatagca ccatgaaaag tgtgaatgct     300

acagattatg gtttgtcacc tgatcggcaa tttgtgtatc tagaaagtga ttattcaaag     360

ctctggcgat attcatacac agcgacatac tacatctacg accttcagaa tggggaattt     420

gtaagaggat acgagctccc tcgtccaatt cagtatctat gctggtcgcc tgttgggagt     480

aaattagcat atgtatatca aaacaatatt tatttgaaac aaagaccagg agatccacct     540

tttcaaataa cttatactgg aagagaaaat agaatattta atggaatacc agactgggtt     600

tatgaagagg aaatgcttgc cacaaaatat gctctttggt ggtctccaga tggaaaattt     660

ttggcatatg tagaatttaa tgattcagat ataccaatta ttgcctattc ttattatggt     720

gatggacagt atcctagaac tataaatatt ccatatccaa aggctggggc taagaatccg     780

gttgttcgtg tttttattgt tgacaccacc taccctcacc acgtgggccc aatggaagtg     840

ccagttccag aaatgatagc ctcaagtgac tattatttca gctggctcac atgggtgtcc     900

agtgaacgag tatgcttgca gtggctaaaa agagtgcaga atgtctcagt cctgtctata     960

tgtgatttca gggaagactg gcatgcatgg gaatgtccaa agaaccagga gcatgtagaa 1020

gaaagcagaa caggatgggc tggtggattc tttgtttcga caccagcttt tagccaggat 1080

gccacttctt actacaaaat atttagcgac aaggatggtt acaaacatat tcactacatc 1140

aaagacactg tggaaaatgc tattcaaatt acaagtggca agtgggaggc catatatata 1200

ttccgcgtaa cacaggattc actgttttat tctagcaatg aatttgaagg ttaccctgga 1260

agaagaaaca tctacagaat tagcattgga aactctcctc cgagcaagaa gtgtgttact 1320

tgccatctaa ggaaagaaag gtgccaatat tacacagcaa gtttcagcta caaagccaag 1380

tactatgcac tcgtctgcta tggccctggc ctccccattt ccaccctcca tgatggccgc 1440

acagaccaag aaatacaagt attagaagaa aacaaagaac tggaaaattc tctgagaaat 1500

atccagctgc ctaaagtgga gattaagaag ctcaaagacg ggggactgac tttctggtac 1560

aagatgattc tgcctcctca gtttgacaga tcaaagaagt accctttgct aattcaagtg 1620

tatggtggtc cttgcagcca gagtgttaag tctgtgtttg ctgttaattg gataacttat 1680

ctcgcaagta aggaggggat agtcattgcc ctggtagatg gtcggggcac tgctttccaa 1740

ggtgacaaat tcctgcatgc cgtgtatcga aaactgggtg tatatgaagt tgaggaccag 1800

ctcacagctg tcagaaaatt catagaaatg ggtttcattg atgaagaaag aatagccata 1860

tggggctggt cctacggagg ttatgtttca tccctggccc ttgcatctgg aactggtctt 1920

ttcaaatgtg gcatagcagt ggctccagtc tccagctggg aatattacgc atctatctac 1980

tcagagagat tcatgggcct cccaacaaag gacgacaatc tcgaacacta taaaaattca 2040

actgtgatgg caagagcaga atatttcaga aatgtagact atcttctcat ccacggaaca 2100

gcagatgata atgtgcactt tcagaactca gcacagattg ctaaagcttt ggttaatgca 2160

caagtggatt tccaggcgat gtggtactct gaccagaacc atggtatatc atctgggcgc 2220

tcccagaatc atttatatac ccacatgacg cacttcctca agcaatgctt ttctttatca 2280

gac 2283

<210> 16
<211> 761
<212> PRT
<213> Mus musculus

<400> 16

Met Lys Thr Trp Leu Lys Thr Val Phe Gly Val Thr Thr Leu Ala Ala
1               5                   10                  15

Leu Ala Leu Val Val Ile Cys Ile Val Leu Arg Pro Ser Arg Val Tyr
            20                  25                  30

Lys Pro Glu Gly Asn Thr Lys Arg Ala Leu Thr Leu Lys Asp Ile Leu
        35                  40                  45

Asn Gly Thr Phe Ser Tyr Lys Thr Tyr Phe Pro Asn Trp Ile Ser Glu
    50                  55                  60

Gln Glu Tyr Leu His Gln Ser Glu Asp Asp Asn Ile Val Phe Tyr Asn
65                  70                  75                  80

Ile Glu Thr Arg Glu Ser Tyr Ile Ile Leu Ser Asn Ser Thr Met Lys
                85                  90                  95

Ser Val Asn Ala Thr Asp Tyr Gly Leu Ser Pro Asp Arg Gln Phe Val
            100                 105                 110

Tyr Leu Glu Ser Asp Tyr Ser Lys Leu Trp Arg Tyr Ser Tyr Thr Ala
        115                 120                 125

Thr Tyr Tyr Ile Tyr Asp Leu Gln Asn Gly Glu Phe Val Arg Gly Tyr
    130                 135                 140

Glu Leu Pro Arg Pro Ile Gln Tyr Leu Cys Trp Ser Pro Val Gly Ser
145                 150                 155                 160

Lys Leu Ala Tyr Val Tyr Gln Asn Asn Ile Tyr Leu Lys Gln Arg Pro
                165                 170                 175

Gly Asp Pro Pro Phe Gln Ile Thr Tyr Thr Gly Arg Glu Asn Arg Ile
            180                 185                 190

Phe Asn Gly Ile Pro Asp Trp Val Tyr Glu Glu Glu Met Leu Ala Thr
        195                 200                 205

Lys Tyr Ala Leu Trp Trp Ser Pro Asp Gly Lys Phe Leu Ala Tyr Val
    210                 215                 220

Glu Phe Asn Asp Ser Asp Ile Pro Ile Ile Ala Tyr Ser Tyr Tyr Gly
225                 230                 235                 240

Asp Gly Gln Tyr Pro Arg Thr Ile Asn Ile Pro Tyr Pro Lys Ala Gly
                245                 250                 255

Ala Lys Asn Pro Val Val Arg Val Phe Ile Val Asp Thr Thr Tyr Pro
            260                 265                 270

His His Val Gly Pro Met Glu Val Pro Val Pro Glu Met Ile Ala Ser
        275                 280                 285

Ser Asp Tyr Tyr Phe Ser Trp Leu Thr Trp Val Ser Ser Glu Arg Val
    290                 295                 300

Cys Leu Gln Trp Leu Lys Arg Val Gln Asn Val Ser Val Leu Ser Ile
305                 310                 315                 320

Cys Asp Phe Arg Glu Asp Trp His Ala Trp Glu Cys Pro Lys Asn Gln
                325                 330                 335

Glu His Val Glu Glu Ser Arg Thr Gly Trp Ala Gly Gly Phe Phe Val
            340                 345                 350

Ser Thr Pro Ala Phe Ser Gln Asp Ala Thr Ser Tyr Tyr Lys Ile Phe
        355                 360                 365

Ser Asp Lys Asp Gly Tyr Lys His Ile His Tyr Ile Lys Asp Thr Val
    370                 375                 380

Glu Asn Ala Ile Gln Ile Thr Ser Gly Lys Trp Glu Ala Ile Tyr Ile
385                 390                 395                 400

Phe Arg Val Thr Gln Asp Ser Leu Phe Tyr Ser Ser Asn Glu Phe Glu
                405                 410                 415

Gly Tyr Pro Gly Arg Arg Asn Ile Tyr Arg Ile Ser Ile Gly Asn Ser
            420                 425                 430

Pro Pro Ser Lys Lys Cys Val Thr Cys His Leu Arg Lys Glu Arg Cys
        435                 440                 445

Gln Tyr Tyr Thr Ala Ser Phe Ser Tyr Lys Ala Lys Tyr Tyr Ala Leu
    450                 455                 460

Val Cys Tyr Gly Pro Gly Leu Pro Ile Ser Thr Leu His Asp Gly Arg
465                 470                 475                 480

Thr Asp Gln Glu Ile Gln Val Leu Glu Glu Asn Lys Glu Leu Glu Asn
                485                 490                 495

Ser Leu Arg Asn Ile Gln Leu Pro Lys Val Glu Ile Lys Lys Leu Lys
            500                 505                 510

Asp Gly Gly Leu Thr Phe Trp Tyr Lys Met Ile Leu Pro Pro Gln Phe
        515                 520                 525

Asp Arg Ser Lys Lys Tyr Pro Leu Leu Ile Gln Val Tyr Gly Gly Pro
    530                 535                 540

Cys Ser Gln Ser Val Lys Ser Val Phe Ala Val Asn Trp Ile Thr Tyr
545                 550                 555                 560

Leu Ala Ser Lys Glu Gly Ile Val Ile Ala Leu Val Asp Gly Arg Gly
                565                 570                 575

Thr Ala Phe Gln Gly Asp Lys Phe Leu His Ala Val Tyr Arg Lys Leu
            580                 585                 590

Gly Val Tyr Glu Val Glu Asp Gln Leu Thr Ala Val Arg Lys Phe Ile
        595                 600                 605

Glu Met Gly Phe Ile Asp Glu Glu Arg Ile Ala Ile Trp Gly Trp Ser
    610                 615                 620

Tyr Gly Gly Tyr Val Ser Ser Leu Ala Leu Ala Ser Gly Thr Gly Leu
625                 630                 635                 640

Phe Lys Cys Gly Ile Ala Val Ala Pro Val Ser Ser Trp Glu Tyr Tyr
                645                 650                 655

Ala Ser Ile Tyr Ser Glu Arg Phe Met Gly Leu Pro Thr Lys Asp Asp
            660                 665                 670

Asn Leu Glu His Tyr Lys Asn Ser Thr Val Met Ala Arg Ala Glu Tyr
        675                 680                 685

Phe Arg Asn Val Asp Tyr Leu Leu Ile His Gly Thr Ala Asp Asp Asn
    690                 695                 700

Val His Phe Gln Asn Ser Ala Gln Ile Ala Lys Ala Leu Val Asn Ala
705                 710                 715                 720

Gln Val Asp Phe Gln Ala Met Trp Tyr Ser Asp Gln Asn His Gly Ile
                725                 730                 735

Ser Ser Gly Arg Ser Gln Asn His Leu Tyr Thr His Met Thr His Phe
            740                 745                 750

Leu Lys Gln Cys Phe Ser Leu Ser Asp
        755                 760

<210>  17
<211>  321
<212>  DNA
<213>  Homo sapiens

<400>  17
gaaatagtgt tgacgcagtc tccagccacc ctgtctttgt ctccagggga aagagccacc      60

ctctcctgca gggccagtca gagtgttagc agctacttag cctggtacca acagaaacct     120

ggccaggctc ccaggctcct catctatgat gcatccaaca gggccactgg catcccagcc     180

aggttcagtg gcagtgggtc tgggacagac ttcactctca ccatcagcag cctagagcct     240

gaagattttg cagtttatta ctgtcagcag cgtagcaact ggcctccgac gttcggccaa     300

gggaccaagg tggaaatcaa a                                               321

<210>  18
<211>  107
<212>  PRT
<213>  Homo sapiens

<400>  18

<210>  19
<211>  11
<212>  PRT
<213>  Artificial Sequence

<220>
<223>  description of the artificial sequence: the amino acid sequence
       of L6 CDR1

<400>  19

Arg Ala Ser Gln Ser Val Ser Ser Tyr Leu Ala
1               5                   10

<210>  20
<211>  7
<212>  PRT
<213>  Artificial Sequence

<220>
<223>  description of the artificial sequence: the amino acid sequence
       of L6 CDR2

<400>  20

Asp Ala Ser Asn Arg Ala Thr
1               5


<210>  21
<211>  9
<212>  PRT
<213>  Artificial Sequence

<220>
<223>  description of the artificial sequence: the amino acid sequence
       of L6 CDR3

<400>  21

Gln Gln Arg Ser Asn Trp Pro Pro Thr
1               5


<210>  22
<211>  354
<212>  DNA
<213>  Homo sapiens

<400>  22
gaagtgcagc ttgtgcagtc cggggctgaa gtgaagaagc ccggggaatc cctgaagatc        60

tcctgtaagg gctccggcta ctccttcatc atctactgga tcggctgggt tcgccagatg       120

cctgggaagg ggctggagtg gatggggatc atctatccca atgatgccga tactcgctac       180

tccccctcct tccaggggca ggtgactatc tccgccgaca agtccatctc cactgcctat       240

ctgcagtggt cctccctgaa ggcctccgat actgccatgt actactgcgt gcgcctgggg       300

actcgcgatt ggttcgatcc ttgggggcag gggactcttg tgactgtgtc ctcc             354


<210>  23
<211>  118
<212>  PRT
<213>  Homo sapiens

<400>  23

Glu Val Gln Leu Val Gln Ser Gly Ala Glu Val Lys Lys Pro Gly Glu
1               5                   10                  15


Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser
        115

<210>  24
<211>  5
<212>  PRT
<213>  Artificial Sequence

<220>
<223>  description of the artificial sequence: the amino acid sequence
       of R1090 HCDR1

<400>  24

Ile Tyr Trp Ile Gly
1               5

<210>  25
<211>  17
<212>  PRT
<213>  Artificial Sequence

<220>
<223>  description of the artificial sequence: the amino acid sequence
       of R1090 HCDR2

<400>  25

Ile Ile Tyr Pro Asn Asp Ala Asp Thr Arg Tyr Ser Pro Ser Phe Gln
1               5                   10                  15

Gly

<210>  26
<211>  9
<212>  PRT

<213>  Artificial Sequence

<220>
<223>  description of the artificial sequence: the amino acid sequence
       of R1090 HCDR3

<400>  26

Leu Gly Thr Arg Asp Trp Phe Asp Pro
1               5


<210>  27
<211>  378
<212>  DNA
<213>  Homo sapiens

<400>  27
caggtgcagc tggtgcagtc tggggctgag gtgaagaagc ctggggcctc agtgaaggtc      60

tcctgcaagg cttctggata caccttcacc ggctactata tgcactgggt gcgacaggcc     120

cctggacaag ggcttgagtg gatgggatgg atcaaccctg acagtggtgg cacaaactat     180

gcacagaagt ttcagggcag ggtcaccatg accagggaca cgtccatcag cacagcctac     240

atggagctga acaggctgag atctgacgac acggccgtgt attactgtgc gagagatcag     300

cccctaggat attgtactaa tggtgtatgc tcctactttg actactgggg ccagggaacc     360

ctggtcaccg tctcctca                                                   378


<210>  28
<211>  126
<212>  PRT
<213>  Homo sapiens

<400>  28

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Gly Tyr
            20                  25                  30


Tyr Met His Trp Val Arg Gln Ala Pro Gly Gln Gly Leu Glu Trp Met
        35                  40                  45


Gly Trp Ile Asn Pro Asp Ser Gly Gly Thr Asn Tyr Ala Gln Lys Phe
    50                  55                  60


Gln Gly Arg Val Thr Met Thr Arg Asp Thr Ser Ile Ser Thr Ala Tyr
65                  70                  75                  80


Met Glu Leu Asn Arg Leu Arg Ser Asp Asp Thr Ala Val Tyr Tyr Cys
                85                  90                  95

Ala Arg Asp Gln Pro Leu Gly Tyr Cys Thr Asn Gly Val Cys Ser Tyr
            100                 105                 110

Phe Asp Tyr Trp Gly Gln Gly Thr Leu Val Thr Val Ser Ser
        115                 120                 125

<210>  29
<211>  321
<212>  DNA
<213>  Homo sapiens

<400>  29
gacatccaga tgacccagtc tccatcttcc gtgtctgcat ctgtaggaga cagagtcacc      60

atcacttgtc gggcgagtca gggtatttac agctggttag cctggtatca gcagaaacca     120

gggaaagccc ctaacctcct gatctatact gcatccactt tacaaagtgg ggtcccatca     180

aggttcagcg gcagtggatc tgggacagat ttcactctca ccatcagcag cctgcaacct     240

gaagattttg caacttacta ttgtcaacag gctaacattt tcccgctcac tttcggcgga     300

gggaccaagg tcgagatcaa a                                               321

<210>  30
<211>  107
<212>  PRT
<213>  Homo sapiens

<400>  30

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Tyr Ser Trp
            20                  25                  30

Leu Ala Trp Tyr Gln Gln Lys Pro Gly Lys Ala Pro Asn Leu Leu Ile
        35                  40                  45

Tyr Thr Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Asn Ile Phe Pro Leu
                85                  90                  95

Thr Phe Gly Gly Gly Thr Lys Val Glu Ile Lys
            100                 105

<210>  31
<211>  354
<212>  DNA
<213>  Homo sapiens

<400>  31
gaggtgcagc tggtgcagtc tggggctgag gtgaagaagc ctggggcctc agtgaaggtt      60

tcctgcaagg catctggata caccttcacc agtcactata tacactgggt gcgacaggcc     120

cctggacaag ggcttgagtg gatgggatgg atcagcgctt tcaatggtga cacaaactat     180

gcacagaagt tccaggacag agtcaccatg accacagaca cgtccacgag cacagtgtac     240

atggagctga ggggcctgag atctgaggac acggccgtgt attactgtgc gagagatagc     300

accaggtggt tcattgacta ctggggccag ggaaccacgg tcaccgtctc ctca           354


<210>  32
<211>  118
<212>  PRT
<213>  Homo sapiens

<400>  32

Glu Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Ser His
            20                  25                  30


Tyr Ile His Trp Val Arg Gln Ala Pro Gly Gln Gly Leu Glu Trp Met
        35                  40                  45


Gly Trp Ile Ser Ala Phe Asn Gly Asp Thr Asn Tyr Ala Gln Lys Phe
    50                  55                  60


Gln Asp Arg Val Thr Met Thr Thr Asp Thr Ser Thr Ser Thr Val Tyr
65                  70                  75                  80


Met Glu Leu Arg Gly Leu Arg Ser Glu Asp Thr Ala Val Tyr Tyr Cys
                85                  90                  95


Ala Arg Asp Ser Thr Arg Trp Phe Ile Asp Tyr Trp Gly Gln Gly Thr
            100                 105                 110


Thr Val Thr Val Ser Ser
        115


<210>  33
<211>  5
<212>  PRT
<213>  Artificial Sequence

<220>
<223>  description of the artificial sequence: the amino acid sequence
       of Fa018 HCDR1

<400>  33

Ser His Tyr Ile His
1               5


<210>  34
<211>  17
<212>  PRT
<213>  Artificial Sequence

<220>
<223>  description of the artificial sequence: the amino acid sequence
       of Fa018 HCDR2

<400>  34

Trp Ile Ser Ala Phe Asn Gly Asp Thr Asn Tyr Ala Gln Lys Phe Gln
1               5                   10                  15


Asp




<210>  35
<211>  9
<212>  PRT
<213>  Artificial Sequence

<220>
<223>  description of the artificial sequence: the amino acid sequence
       of Fa018 HCDR3

<400>  35

Asp Ser Thr Arg Trp Phe Ile Asp Tyr
1               5


<210>  36
<211>  360
<212>  DNA
<213>  Homo sapiens

<400>  36
caggtgcagc tgcaggagtc gggcccagga ctggtgaagc cttcggagac cctgtccctc      60

acctgcactg tctctggtga ctccatcagc agtggtagtt actactgggg ctggatccgc     120

cagccccccg ggaaggggct ggagtggatt gggagtatct attctagtgg gagcacctac      180

cacgacccgt ccctcaagaa tcgagtcacc atgtccgtag acacgtccaa gaaccagttc     240

tccctgaggc tgagctctgt gaccgctgcg gacacggccg tgtattattg tgcgagagat     300

aggggctact actacaatat ggacgtctgg ggccaaggga ccacggtcac cgtctcttca     360

<210>  37
<211>  120
<212>  PRT
<213>  Homo sapiens

<400>  37

Gln Val Gln Leu Gln Glu Ser Gly Pro Gly Leu Val Lys Pro Ser Glu
1               5                   10                  15

Thr Leu Ser Leu Thr Cys Thr Val Ser Gly Asp Ser Ile Ser Ser Gly
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Ser Ser Gly Ser Thr Tyr His Asp Pro Ser
    50                  55                  60

Leu Lys Asn Arg Val Thr Met Ser Val Asp Thr Ser Lys Asn Gln Phe
65                  70                  75                  80

Ser Leu Arg Leu Ser Ser Val Thr Ala Ala Asp Thr Ala Val Tyr Tyr
                85                  90                  95

Cys Ala Arg Asp Arg Gly Tyr Tyr Tyr Asn Met Asp Val Trp Gly Gln
            100                 105                 110

Gly Thr Thr Val Thr Val Ser Ser
        115                 120


<210>  38
<211>  7
<212>  PRT
<213>  Artificial Sequence

<220>
<223>  description of the artificial sequence: the amino acid sequence
       of Fa1007 VH

<400>  38

Ser Gly Ser Tyr Tyr Trp Gly
1               5


<210>  39
<211>  16
<212>  PRT
<213>  Artificial Sequence

<220>
<223>  description of the artificial sequence: the amino acid sequence
       of Fa1007 HCDR2 amino acid

<400>  39

Ser Ile Tyr Ser Ser Gly Ser Thr Tyr His Asp Pro Ser Leu Lys Asn
1               5                   10                  15

<210>  40
<211>  10
<212>  PRT
<213>  Artificial Sequence

<220>
<223>  description of the artificial sequence: the amino acid sequence
       of Fa1007 HCDR3

<400>  40

Asp Arg Gly Tyr Tyr Tyr Asn Met Asp Val
1               5                   10

<210>  41
<211>  360
<212>  DNA
<213>  Homo sapiens

<400>  41
caggtgcagc tgcaggagtc gggcccagga ctggtgaagc cttcggagac cctgtccctc      60

acctgcactg tctctggtgg ctccgtcagc agtggtagtt actactggag ctggatccgg     120

cagtccccag ggaagggact ggagtggatt gggtttatct attacagtgg gaacaccaac     180

tacaacccct ccctcaagag tcgagtcacc atatcagtag acacgtccaa gaaccagttc     240

tccctgaagc tgagctctgt gaccgctgcg gacacggccg tgtattactg tgcgagagat     300

aggggctact actacggtat ggacgtctgg ggccaaggga ccacggtcac cgtctcctca     360

<210>  42
<211>  120
<212>  PRT
<213>  Homo sapiens

<400>  42

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Ser Tyr Tyr Trp Ser Trp Ile Arg Gln Ser Pro Gly Lys Gly Leu Glu
        35                  40                  45

Trp Ile Gly Phe Ile Tyr Tyr Ser Gly Asn Thr Asn Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Asp Arg Gly Tyr Tyr Tyr Gly Met Asp Val Trp Gly Gln
            100                 105                 110

Gly Thr Thr Val Thr Val Ser Ser
        115                 120

<210>  43
<211>  7
<212>  PRT
<213>  Artificial Sequence

<220>
<223>  description of the artificial sequence: the amino acid sequence
       of Fa1013 HCDR1

<400>  43

Ser Gly Ser Tyr Tyr Trp Ser
1               5

<210>  44
<211>  16
<212>  PRT
<213>  Artificial Sequence

<220>
<223>  description of the artificial sequence: the amino acid sequence
       of Fa1013 HCDR2

<400>  44

Phe Ile Tyr Tyr Ser Gly Asn Thr Asn Tyr Asn Pro Ser Leu Lys Ser
1               5                   10                  15

<210>  45
<211>  10
<212>  PRT
<213>  Artificial Sequence

<220>
<223>  description of the artificial sequence: the amino acid sequence
       of Fa1013 HCDR3

<400>  45

Asp Arg Gly Tyr Tyr Tyr Gly Met Asp Val
1               5                   10

<210>  46
<211>  360

<212>  DNA
<213>  Homo sapiens

<400>  46
cagctgcagc tgcaggagtc gggcccagga ctggtgaagc cttcggagac cctgtccctc      60

acctgcactg tctctggtgg ctccatcagc agtagtagtt cctactgggg ctggatccgc     120

cagcccccag gaaagggact ggagtggatt gggagtatct attatagtgg gagcaccaac     180

tacaacccct ccctcaagag tcgagtcacc atatcagtag acacgtccaa gaaccagttc     240

tccctgaagc tgaggtctgt gaccgctgcg gacacggccg tgtattactg tgtgagagat     300

aaaggctact actacggtat ggacgtctgg ggccaaggga ccacggtcac cgtctcctca     360


<210>  47
<211>  120
<212>  PRT
<213>  Homo sapiens

<400>  47

Gln Leu Gln Leu Gln Glu Ser Gly Pro Gly Leu Val Lys Pro Ser Glu
1               5                   10                  15


Thr Leu Ser Leu Thr Cys Thr Val Ser Gly Gly Ser Ile Ser Ser Ser
            20                  25                  30


Ser Ser Tyr Trp Gly Trp Ile Arg Gln Pro Pro Gly Lys Gly Leu Glu
        35                  40                  45


Trp Ile Gly Ser Ile Tyr Tyr Ser Gly Ser Thr Asn Tyr Asn Pro Ser
    50                  55                  60


Leu Lys Ser Arg Val Thr Ile Ser Val Asp Thr Ser Lys Asn Gln Phe
65                  70                  75                  80


Ser Leu Lys Leu Arg Ser Val Thr Ala Ala Asp Thr Ala Val Tyr Tyr
                85                  90                  95


Cys Val Arg Asp Lys Gly Tyr Tyr Tyr Gly Met Asp Val Trp Gly Gln
            100                 105                 110


Gly Thr Thr Val Thr Val Ser Ser
        115                 120


<210>  48
<211>  7
<212>  PRT
<213>  Artificial Sequence

<220>
<223>  description of the artificial sequence: the amino acid sequence

of Fa1014 HCDR1

<400>  48

Ser Ser Ser Ser Tyr Trp Gly
1               5


<210>  49
<211>  16
<212>  PRT
<213>  Artificial Sequence

<220>
<223>  description of the artificial sequence: the amino acid sequence
       of Fa1014 HCDR2

<400>  49

Ser Ile Tyr Tyr Ser Gly Ser Thr Asn Tyr Asn Pro Ser Leu Lys Ser
1               5                   10                  15


<210>  50
<211>  10
<212>  PRT
<213>  Artificial Sequence

<220>
<223>  description of the artificial sequence: the amino acid sequence
       of Fa1014 HCDR3

<400>  50

Asp Lys Gly Tyr Tyr Tyr Gly Met Asp Val
1               5                   10


<210>  51
<211>  366
<212>  DNA
<213>  Homo sapiens

<400>  51
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggaaca cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctca                                                                366


<210>  52
<211>  122
<212>  PRT
<213>  Homo sapiens

<400>  52

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser
        115                 120

<210>  53
<211>  5
<212>  PRT
<213>  Artificial Sequence

<220>
<223>  description of the artificial sequence: the amino acid sequence
       of Fa1046 HCDR1

<400>  53

Asn Ser Gly Ile Asn
1               5

<210>  54
<211>  17
<212>  PRT
<213>  Artificial Sequence

<220>
<223>  description of the artificial sequence: the amino acid sequence
       of Fa1046 HCDR2

<400>  54

Trp Met Asn Pro Asn Ser Gly Asp Thr Gly Tyr Ala Gln Lys Phe Gln
1               5                   10                  15

Gly

<210>  55
<211>  13
<212>  PRT
<213>  Artificial Sequence

<220>
<223>  description of the artificial sequence: the amino acid sequence
       of Fa1046 HCDR3

<400>  55

Val Pro Val Val Gly Ala Thr Tyr Asn Trp Phe Asp Pro
1               5                   10

<210>  56
<211>  372
<212>  DNA
<213>  Artificial Sequence

<220>
<223>  description of the artificial sequence: the base sequence of
       Sibrotuzumab VH

<400>  56
caggtacagc ttgtgcagtc cggcgctgag gtcaaaaaac caggcgccag cgttaaggtg       60

tcatgcaaga ctagcaggta tacattcact gaatacacaa tacactgggt gcgacaggct      120

cccgggcaaa gactggagtg gattgggggc ataaacccca acaatgggat cccgaattat      180

aatcagaaat ttaagggtcg ggtgacaatc acagtagaca ctagcgcatc aaccgcctac      240

atggagctca gctcccttag gtctgaagac acagcagttt attactgcgc aaggcgccgc      300

atcgcctacg gctatgacga aggtcatgct atggactatt gggggcaagg gacactcgtg      360

actgtctcat ca                                                          372

<210>  57
<211>  124
<212>  PRT
<213>  Artificial Sequence

<220>
<223>  description of the artificial sequence: the amino acid sequence
       of Sibrotuzumab VH

<400>  57

Gln Val Gln Leu Val Gln Ser Gly Ala Glu Val Lys Lys Pro Gly Ala
1               5                   10                  15

Ser Val Lys Val Ser Cys Lys Thr Ser Arg Tyr Thr Phe Thr Glu Tyr
            20                  25                  30

Thr Ile His Trp Val Arg Gln Ala Pro Gly Gln Arg Leu Glu Trp Ile
        35                  40                  45

Gly Gly Ile Asn Pro Asn Asn Gly Ile Pro Asn Tyr Asn Gln Lys Phe
    50                  55                  60

Lys Gly Arg Val Thr Ile Thr Val Asp Thr Ser Ala Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Arg Ile Ala Tyr Gly Tyr Asp Glu Gly His Ala Met Asp
            100                 105                 110

Tyr Trp Gly Gln Gly Thr Leu Val Thr Val Ser Ser
        115                 120

<210>  58
<211>  339
<212>  DNA
<213>  Artificial Sequence

<220>
<223>  description of the artificial sequence: the base sequence of
       Sibrotuzumab VL

<400>  58
gacattgtca tgacgcagag tcctgacagt ctcgccgtgt ccttgggaga gcgggcaaca      60

atcaattgta aaagcagcca atctctcctg tacagccgga accagaagaa ctacctcgcc     120

tggtaccagc agaaacccgg tcagccgccc aagctcctga ttttttgggc cagcactcgg      180

gaaagcggtg tccccgacag attttccgga tctggcttcg ggacagattt taccctgacg     240

atctcatcac ttcaggcaga ggatgtggcc gtctactact gtcaacagta tttttcctac     300

cccctcactt tcggccaagg aaccaaggtg gagattaaa                            339

<210>  59
<211>  113
<212>  PRT
<213>  Artificial Sequence

<220>
<223>  description of the artificial sequence: the amino acid sequence
       of Sibrotuzumab VL

<400>  59

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Arg Asn Gln Lys Asn Tyr Leu Ala Trp Tyr Gln Gln Lys Pro Gly Gln
        35                  40                  45

Pro Pro Lys Leu Leu Ile Phe Trp Ala Ser Thr Arg Glu Ser Gly Val
    50                  55                  60

Pro Asp Arg Phe Ser Gly Ser Gly Phe Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Phe Ser Tyr Pro Leu Thr Phe Gly Gln Gly Thr Lys Val Glu Ile
            100                 105                 110

Lys

<210>  60
<211>  981
<212>  DNA
<213>  Artificial Sequence

<220>
<223>  description of the artificial sequence: the base sequence of
       IgG4PE R409K CH

<400>  60
gctagcacca agggcccatc cgtcttcccc ctggcgccct gctccaggag cacctccgag      60

agcacagccg ccctgggctg cctggtcaag gactacttcc ccgaaccggt gacggtgtcg     120

tggaactcag gcgccctgac cagcggcgtg cacaccttcc cggctgtcct acagtcctca     180

ggactctact ccctcagcag cgtggtgacc gtgccctcca gcagcttggg cacgaagacc     240

tacacctgca acgtagatca caagcccagc aacaccaagg tggacaagag agttgagtcc     300

aaatatggtc ccccatgccc accatgccca gcacctgagt tcgagggggg accatcagtc     360

ttcctgttcc ccccaaaacc caaggacact ctcatgatct cccggacccc tgaggtcacg     420

tgcgtggtgg tggacgtgag ccaggaagac cccgaggtcc agttcaactg gtacgtggat     480

ggcgtggagg tgcataatgc caagacaaag ccgcgggagg agcagttcaa cagcacgtac     540

cgtgtggtca gcgtcctcac cgtcctgcac caggactggc tgaacggcaa ggagtacaag     600

tgcaaggtct ccaacaaagg cctcccgtcc tccatcgaga aaaccatctc caaagccaaa     660

gggcagcccc gagagccaca ggtgtacacc ctgcccccat cccaggagga gatgaccaag     720

aaccaggtca gcctgacctg cctggtcaaa ggcttctacc ccagcgacat cgccgtggag 780

tgggagagca atgggcagcc ggagaacaac tacaagacca cgcctcccgt gctggactcc 840

gacggctcct tcttcctcta cagcaagcta accgtggaca agagcaggtg gcaggagggg 900

aatgtcttct catgctccgt gatgcatgag gctctgcaca accactacac acagaagagc 960

ctctccctgt ctctgggtaa a 981

<210> 61
<211> 327
<212> PRT
<213> Artificial Sequence

<220>
<223> description of the artificial sequence: the amino acid sequence
 of IgG4PE R409K CH

<400> 61

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Glu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe

165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Lys Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  62
<211>  294
<212>  DNA
<213>  Homo sapiens

<400>  62
gctagcacca aaggaccttc tgtatttcct cttgcgccat gctctcgctc tacgtcagaa      60

tcaactgccg ctctggggtg cctggttaaa gactacttcc cggagcctgt gacagtgagt     120

tggaactccg gcgccctgac atcaggagtg catacatttc ccgccgtgct tcagagcagc     180

ggactttata gcctcagcag tgtggtgacc gtgccatctt ccagcctggg gaccaagacc     240

tacacctgta acgtggacca caaacccagc aacaccaagg ttgataagag ggtc           294

<210>  63
<211>  98
<212>  PRT

<213>  Homo sapiens

<400>  63

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val

<210>  64
<211>  1983
<212>  DNA
<213>  Artificial Sequence

<220>
<223>  description of the artificial sequence: the base sequence of
       Ct-R1090-Fa018\201EH Chain

<400>  64
gaggtgcagc tggtgcagtc cggagcagag gtgaaaaagc ccggggagtc tctgaagatc      60

tcctgtaagg gttctggata cagctttatc atctactgga tcggctgggt gcgccagatg     120

cccgggaaag gcctggagtg gatggggatc atctatccta atgacgctga taccagatac     180

agcccgtcct tccaaggcca ggtcaccatc tcagccgaca agtccatcag caccgcctac     240

ctgcagtgga gcagcctgaa ggcctcggac accgccatgt attactgtgt gagactgggt     300

acgagggact ggttcgaccc ctggggccag ggaaccctgg tcaccgtctc ctcagcaagt     360

accaaggggc catccgtctt ccccctggcg ccctgctcca ggagcacctc cgagagcaca     420

gccgccctgg gctgcctggt caaggactac ttccccgaac cggtgacggt gtcgtggaac     480

tcaggcgccc tgaccagcgg cgtgcacacc ttcccggctg tcctacagtc ctcaggactc     540

tactccctca gcagcgtggt gaccgtgccc tccagcagct tgggcacgaa gacctacacc     600

tgcaacgtag atcacaagcc cagcaacacc aaggtggaca agagagttga gtccaaatat     660

ggtcccccat gcccaccatg cccagcacct gagttcgagg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaagaggt gcagctggtg cagtctgggg ctgaggtgaa gaagcctggg 1380

gcctcagtga aggtttcctg caaggcatct ggatacacct tcaccagtca ctatatacac 1440

tgggtgcgac aggcccctgg acaagggctt gagtggatgg gatggatcag cgctttcaat 1500

ggtgacacaa actatgcaca gaagttccag gacagagtca ccatgaccac agacacgtcc 1560

acgagcacag tgtacatgga gctgaggggc ctgagatctg aggacacggc cgtgtattac 1620

tgtgcgagag atagcaccag gtggttcatt gactactggg gccagggaac cacggtcacc 1680

gtctcctcag ctagcaccaa aggaccttct gtatttcctc ttgcgccatg ctctcgctct 1740

acgtcagaat caactgccgc tctggggtgc ctggttaaag actacttccc ggagcctgtg 1800

acagtgagtt ggaactccgg cgccctgaca tcaggagtgc atacatttcc cgccgtgctt 1860

cagagcagcg gactttatag cctcagcagt gtggtgaccg tgccatcttc cagcctgggg 1920

accaagacct acacctgtaa cgtggaccac aaacccagca acaccaaggt tgataagagg 1980

gtc 1983

<210> 65
<211> 661
<212> PRT
<213> Artificial Sequence

<220>
<223> description of the artificial sequence: the amino acid sequence
      of Ct-R1090-Fa018 H Chain

<400> 65

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Glu Val Gln
        435                 440                 445

Leu Val Gln Ser Gly Ala Glu Val Lys Lys Pro Gly Ala Ser Val Lys
    450                 455                 460

Val Ser Cys Lys Ala Ser Gly Tyr Thr Phe Thr Ser His Tyr Ile His
465                 470                 475                 480

Trp Val Arg Gln Ala Pro Gly Gln Gly Leu Glu Trp Met Gly Trp Ile
                485                 490                 495

Ser Ala Phe Asn Gly Asp Thr Asn Tyr Ala Gln Lys Phe Gln Asp Arg
            500                 505                 510

Val Thr Met Thr Thr Asp Thr Ser Thr Ser Thr Val Tyr Met Glu Leu
        515                 520                 525

Arg Gly Leu Arg Ser Glu Asp Thr Ala Val Tyr Tyr Cys Ala Arg Asp
    530                 535                 540

Ser Thr Arg Trp Phe Ile Asp Tyr Trp Gly Gln Gly Thr Thr Val Thr
545                 550                 555                 560

Val Ser Ser Ala Ser Thr Lys Gly Pro Ser Val Phe Pro Leu Ala Pro
                565                 570                 575

Cys Ser Arg Ser Thr Ser Glu Ser Thr Ala Ala Leu Gly Cys Leu Val
            580                 585                 590

Lys Asp Tyr Phe Pro Glu Pro Val Thr Val Ser Trp Asn Ser Gly Ala
        595                 600                 605

Leu Thr Ser Gly Val His Thr Phe Pro Ala Val Leu Gln Ser Ser Gly
    610                 615                 620

Leu Tyr Ser Leu Ser Ser Val Val Thr Val Pro Ser Ser Ser Leu Gly
625                 630                 635                 640

Thr Lys Thr Tyr Thr Cys Asn Val Asp His Lys Pro Ser Asn Thr Lys
                645                 650                 655

Val Asp Lys Arg Val
            660

<210>  66
<211>  1989
<212>  DNA
<213>  Artificial Sequence

<220>
<223>  description of the artificial sequence: the base sequence of
       Ct-R1090-Fa1007 H Chain

<400>  66
gaggtgcagc tggtgcagtc cggagcagag gtgaaaaagc ccggggagtc tctgaagatc      60

tcctgtaagg gttctggata cagctttatc atctactgga tcggctgggt gcgccagatg     120

cccgggaaag gcctggagtg gatggggatc atctatccta atgacgctga taccagatac     180

agcccgtcct tccaaggcca ggtcaccatc tcagccgaca agtccatcag caccgcctac     240

ctgcagtgga gcagcctgaa ggcctcggac accgccatgt attactgtgt gagactgggt     300

acgagggact ggttcgaccc ctggggccag ggaaccctgg tcaccgtctc ctcagcaagt     360

accaaggggc catccgtctt ccccctggcg ccctgctcca ggagcacctc cgagagcaca     420

gccgccctgg gctgcctggt caaggactac ttccccgaac cggtgacggt gtcgtggaac     480

tcaggcgccc tgaccagcgg cgtgcacacc ttcccggctg tcctacagtc ctcaggactc 540

tactccctca gcagcgtggt gaccgtgccc tccagcagct tgggcacgaa gacctacacc 600

tgcaacgtag atcacaagcc cagcaacacc aaggtggaca agagagttga gtccaaatat 660

ggtcccccat gcccaccatg cccagcacct gagttcgagg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaacaggt gcagctgcag gagtcgggcc caggactggt gaagccttcg 1380

gagaccctgt ccctcacctg cactgtctct ggtgactcca tcagcagtgg tagttactac 1440

tggggctgga tccgccagcc ccccgggaag gggctggagt ggattgggag tatctattct 1500

agtgggagca cctaccacga cccgtccctc aagaatcgag tcaccatgtc cgtagacacg 1560

tccaagaacc agttctccct gaggctgagc tctgtgaccg ctgcggacac ggccgtgtat 1620

tattgtgcga gagatagggg ctactactac aatatggacg tctggggcca agggaccacg 1680

gtcaccgtct cttcagctag caccaaagga ccttctgtat ttcctcttgc gccatgctct 1740

cgctctacgt cagaatcaac tgccgctctg gggtgcctgg ttaaagacta cttcccggag 1800

cctgtgacag tgagttggaa ctccggcgcc ctgacatcag gagtgcatac atttcccgcc 1860

gtgcttcaga gcagcggact ttatagcctc agcagtgtgg tgaccgtgcc atcttccagc 1920

ctggggacca agacctacac ctgtaacgtg gaccacaaac ccagcaacac caaggttgat 1980

aagagggtc 1989

<210> 67
<211> 663
<212> PRT
<213> Artificial Sequence

<220>
<223> description of the artificial sequence: the amino acid sequence
 of Ct-R1090-Fa1007 H Chain

<400>  67

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu

245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Gln Val Gln
        435                 440                 445

Leu Gln Glu Ser Gly Pro Gly Leu Val Lys Pro Ser Glu Thr Leu Ser
    450                 455                 460

Leu Thr Cys Thr Val Ser Gly Asp Ser Ile Ser Ser Gly Ser Tyr Tyr
465                 470                 475                 480

Trp Gly Trp Ile Arg Gln Pro Pro Gly Lys Gly Leu Glu Trp Ile Gly
                485                 490                 495

Ser Ile Tyr Ser Ser Gly Ser Thr Tyr His Asp Pro Ser Leu Lys Asn
            500                 505                 510

Arg Val Thr Met Ser Val Asp Thr Ser Lys Asn Gln Phe Ser Leu Arg
        515                 520                 525

Leu Ser Ser Val Thr Ala Ala Asp Thr Ala Val Tyr Tyr Cys Ala Arg
    530                 535                 540

Asp Arg Gly Tyr Tyr Tyr Asn Met Asp Val Trp Gly Gln Gly Thr Thr
545                 550                 555                 560

Val Thr Val Ser Ser Ala Ser Thr Lys Gly Pro Ser Val Phe Pro Leu
                565                 570                 575

Ala Pro Cys Ser Arg Ser Thr Ser Glu Ser Thr Ala Ala Leu Gly Cys
            580                 585                 590

Leu Val Lys Asp Tyr Phe Pro Glu Pro Val Thr Val Ser Trp Asn Ser
        595                 600                 605

Gly Ala Leu Thr Ser Gly Val His Thr Phe Pro Ala Val Leu Gln Ser
    610                 615                 620

Ser Gly Leu Tyr Ser Leu Ser Ser Val Val Thr Val Pro Ser Ser Ser
625                 630                 635                 640

Leu Gly Thr Lys Thr Tyr Thr Cys Asn Val Asp His Lys Pro Ser Asn
                645                 650                 655

Thr Lys Val Asp Lys Arg Val
            660

<210>  68
<211>  1989
<212>  DNA
<213>  Artificial Sequence

<220>
<223>  description of the artificial sequence: the base sequence of
       Ct-R1090-Fa1013\201EH Chain

<400>  68
   gaggtgcagc tggtgcagtc cggagcagag gtgaaaaagc ccggggagtc tctgaagatc      60
   tcctgtaagg gttctggata cagctttatc atctactgga tcggctgggt gcgccagatg     120
   cccgggaaag gcctggagtg gatggggatc atctatccta atgacgctga taccagatac     180
   agcccgtcct tccaaggcca ggtcaccatc tcagccgaca agtccatcag caccgcctac     240
   ctgcagtgga gcagcctgaa ggcctcggac accgccatgt attactgtgt gagactgggt     300
   acgagggact ggttcgaccc ctggggccag ggaaccctgg tcaccgtctc ctcagcaagt 360
   accaaggggc catccgtctt ccccctggcg ccctgctcca ggagcacctc cgagagcaca 420
   gccgccctgg gctgcctggt caaggactac ttccccgaac cggtgacggt gtcgtggaac 480
   tcaggcgccc tgaccagcgg cgtgcacacc ttcccggctg tcctacagtc ctcaggactc 540
   tactccctca gcagcgtggt gaccgtgccc tccagcagct tgggcacgaa gacctacacc 600
   tgcaacgtag atcacaagcc cagcaacacc aaggtggaca agagagttga gtccaaatat 660
   ggtcccccat gcccaccatg cccagcacct gagttcgagg ggggaccatc agtcttcctg 720
   ttccccccaa aacccaagga cactctcatg atctcccgga cccctgaggt cacgtgcgtg 780
   gtggtggacg tgagccagga agaccccgag gtccagttca actggtacgt ggatggcgtg 840
   gaggtgcata atgccaagac aaagccgcgg gaggagcagt tcaacagcac gtaccgtgtg 900
   gtcagcgtcc tcaccgtcct gcaccaggac tggctgaacg gcaaggagta caagtgcaag 960
   gtctccaaca aaggcctccc gtcctccatc gagaaaacca tctccaaagc caaagggcag 1020
   ccccgagagc cacaggtgta caccctgccc ccatcccagg aggagatgac caagaaccag 1080
   gtcagcctga cctgcctggt caaaggcttc taccccagcg acatcgccgt ggagtgggag 1140
   agcaatgggc agccggagaa caactacaag accacgcctc ccgtgctgga ctccgacggc 1200
   tccttcttcc tctacagcaa gctaaccgtg gacaagagca ggtggcagga ggggaatgtc 1260
   ttctcatgct ccgtgatgca tgaggctctg cacaaccact acacacagaa gagcctctcc 1320
   ctgtccctag gtaaacaggt gcagctgcag gagtcgggcc caggactggt gaagccttcg 1380
   gagaccctgt ccctcacctg cactgtctct ggtggctccg tcagcagtgg tagttactac 1440
   tggagctgga tccggcagtc cccagggaag ggactggagt ggattgggtt tatctattac 1500
   agtgggaaca ccaactacaa cccctccctc aagagtcgag tcaccatatc agtagacacg 1560
   tccaagaacc agttctccct gaagctgagc tctgtgaccg ctgcggacac ggccgtgtat 1620
   tactgtgcga gagatagggg ctactactac ggtatggacg tctggggcca agggaccacg 1680
   gtcaccgtct cctcagctag caccaaagga ccttctgtat ttcctcttgc gccatgctct 1740
   cgctctacgt cagaatcaac tgccgctctg ggtgcctggt ttaaagacta cttcccggag 1800
   cctgtgacag tgagttggaa ctccggcgcc ctgacatcag gagtgcatac atttcccgcc 1860
   gtgcttcaga gcagcggact ttatagcctc agcagtgtgg tgaccgtgcc atcttccagc 1920
   ctggggacca agacctacac ctgtaacgtg gaccacaaac ccagcaacac caaggttgat 1980
   aagagggtc 1989

<210> 69
<211> 663
<212> PRT

<213>  Artificial Sequence

<220>
<223>  description of the artificial sequence: the amino acid sequence
      of Ct-R1090-Fa1013 H Chain

<400>  69

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Gln Val Gln
        435                 440                 445

Leu Gln Glu Ser Gly Pro Gly Leu Val Lys Pro Ser Glu Thr Leu Ser
    450                 455                 460

Leu Thr Cys Thr Val Ser Gly Gly Ser Val Ser Ser Gly Ser Tyr Tyr
465                 470                 475                 480

Trp Ser Trp Ile Arg Gln Ser Pro Gly Lys Gly Leu Glu Trp Ile Gly
                485                 490                 495

Phe Ile Tyr Tyr Ser Gly Asn Thr Asn Tyr Asn Pro Ser Leu Lys Ser
            500                 505                 510

Arg Val Thr Ile Ser Val Asp Thr Ser Lys Asn Gln Phe Ser Leu Lys
        515                 520                 525

Leu Ser Ser Val Thr Ala Ala Asp Thr Ala Val Tyr Tyr Cys Ala Arg
    530                 535                 540

Asp Arg Gly Tyr Tyr Tyr Gly Met Asp Val Trp Gly Gln Gly Thr Thr
545                 550                 555                 560

Val Thr Val Ser Ser Ala Ser Thr Lys Gly Pro Ser Val Phe Pro Leu
                565                 570                 575

Ala Pro Cys Ser Arg Ser Thr Ser Glu Ser Thr Ala Ala Leu Gly Cys
            580                 585                 590

Leu Val Lys Asp Tyr Phe Pro Glu Pro Val Thr Val Ser Trp Asn Ser
        595                 600                 605

Gly Ala Leu Thr Ser Gly Val His Thr Phe Pro Ala Val Leu Gln Ser
    610                 615                 620

Ser Gly Leu Tyr Ser Leu Ser Ser Val Val Thr Val Pro Ser Ser Ser
625                 630                 635                 640

Leu Gly Thr Lys Thr Tyr Thr Cys Asn Val Asp His Lys Pro Ser Asn
                645                 650                 655

Thr Lys Val Asp Lys Arg Val
            660

<210>  70
<211>  1989
<212>  DNA
<213>  Artificial Sequence

<220>
<223>  description of the artificial sequence: the base sequence of
       Ct-R1090-Fa1014 H Chain

<400>  70
gaggtgcagc tggtgcagtc cggagcagag gtgaaaaagc ccggggagtc tctgaagatc       60

tcctgtaagg gttctggata cagctttatc atctactgga tcggctgggt gcgccagatg      120

cccgggaaag gcctggagtg gatggggatc atctatccta atgacgctga taccagatac 180

agcccgtcct tccaaggcca ggtcaccatc tcagccgaca agtccatcag caccgcctac 240

ctgcagtgga gcagcctgaa ggcctcggac accgccatgt attactgtgt gagactgggt 300

acgagggact ggttcgaccc ctggggccag ggaaccctgg tcaccgtctc ctcagcaagt 360

accaaggggc catccgtctt ccccctggcg ccctgctcca ggagcacctc cgagagcaca 420

gccgccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcacgaa gacctacacc 600

tgcaacgtag atcacaagcc cagcaacacc aaggtggaca agagagttga gtccaaatat 660

ggtcccccat gcccaccatg cccagcacct gagttcgagg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaacagct gcagctgcag gagtcgggcc caggactggt gaagccttcg 1380

gagaccctgt ccctcacctg cactgtctct ggtggctcca tcagcagtag tagttcctac 1440

tggggctgga tccgccagcc cccaggaaag ggactggagt ggattgggag tatctattat 1500

agtgggagca ccaactacaa cccctccctc aagagtcgag tcaccatatc agtagacacg 1560

tccaagaacc agttctccct gaagctgagg tctgtgaccg ctgcggacac ggccgtgtat 1620

tactgtgtga gagataaagg ctactactac ggtatggacg tctggggcca agggaccacg 1680

gtcaccgtct cctcagctag caccaaagga ccttctgtat ttcctcttgc gccatgctct 1740

cgctctacgt cagaatcaac tgccgctctg gggtgcctgg ttaaagacta cttcccggag 1800

cctgtgacag tgagttggaa ctccggcgcc ctgacatcag gagtgcatac atttcccgcc 1860

gtgcttcaga gcagcggact ttatagcctc agcagtgtgg tgaccgtgcc atcttccagc 1920

ctggggacca agacctacac ctgtaacgtg gaccacaaac ccagcaacac caaggttgat 1980

aagagggtc                                                              1989

<210>  71
<211>  663
<212>  PRT
<213>  Artificial Sequence

<220>
<223>  description of the artificial sequence: the amino acid sequence
       of Ct-R1090-Fa1014 H Chain

<400>  71

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser

195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Gln Leu Gln
        435                 440                 445

Leu Gln Glu Ser Gly Pro Gly Leu Val Lys Pro Ser Glu Thr Leu Ser
    450                 455                 460

Leu Thr Cys Thr Val Ser Gly Gly Ser Ile Ser Ser Ser Ser Ser Tyr
465                 470                 475                 480

Trp Gly Trp Ile Arg Gln Pro Pro Gly Lys Gly Leu Glu Trp Ile Gly
                485                 490                 495

Ser Ile Tyr Tyr Ser Gly Ser Thr Asn Tyr Asn Pro Ser Leu Lys Ser
            500                 505                 510

Arg Val Thr Ile Ser Val Asp Thr Ser Lys Asn Gln Phe Ser Leu Lys
        515                 520                 525

Leu Arg Ser Val Thr Ala Ala Asp Thr Ala Val Tyr Tyr Cys Val Arg
    530                 535                 540

Asp Lys Gly Tyr Tyr Tyr Gly Met Asp Val Trp Gly Gln Gly Thr Thr
545                 550                 555                 560

Val Thr Val Ser Ser Ala Ser Thr Lys Gly Pro Ser Val Phe Pro Leu
                565                 570                 575

Ala Pro Cys Ser Arg Ser Thr Ser Glu Ser Thr Ala Ala Leu Gly Cys
            580                 585                 590

Leu Val Lys Asp Tyr Phe Pro Glu Pro Val Thr Val Ser Trp Asn Ser
        595                 600                 605

Gly Ala Leu Thr Ser Gly Val His Thr Phe Pro Ala Val Leu Gln Ser
    610                 615                 620

Ser Gly Leu Tyr Ser Leu Ser Ser Val Val Thr Val Pro Ser Ser Ser
625                 630                 635                 640

Leu Gly Thr Lys Thr Tyr Thr Cys Asn Val Asp His Lys Pro Ser Asn
                645                 650                 655

Thr Lys Val Asp Lys Arg Val
            660

<210>  72
<211>  1995
<212>  DNA
<213>  Artificial Sequence

<220>
<223>  description of the artificial sequence: the base sequence of

Ct-R1090-Fa1046 H Chain

<400> 72
gaggtgcagc tggtgcagtc cggagcagag gtgaaaaagc ccggggagtc tctgaagatc 60

tcctgtaagg gttctggata cagctttatc atctactgga tcggctgggt gcgccagatg 120

cccgggaaag gcctggagtg gatggggatc atctatccta atgacgctga taccagatac 180

agcccgtcct tccaaggcca ggtcaccatc tcagccgaca agtccatcag caccgcctac 240

ctgcagtgga gcagcctgaa ggcctcggac accgccatgt attactgtgt gagactgggt 300

acgagggact ggttcgaccc ctggggccag ggaaccctgg tcaccgtctc ctcagcaagt 360

accaaggggc catccgtctt ccccctggcg ccctgctcca ggagcacctc cgagagcaca 420

gccgccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcacgaa gacctacacc 600

tgcaacgtag atcacaagcc cagcaacacc aaggtggaca agagagttga gtccaaatat 660

ggtcccccat gcccaccatg cccagcacct gagttcgagg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaagaggt gcagctggtg cagtctggag ctgaggtgaa gaagcctggg 1380

gcctcagtga aggtctcctg caaggcttct ggttacacct ttaccaactc tggtatcaac 1440

tgggtgcgac aggcccctgg acaagggctt gagtggatgg gatggatgaa ccctaacagt 1500

ggtgacacag gctatgcaca gaagttccag ggcagagtca ccatgaccag gaacacctcc 1560

ataagcacag cctacatgga gctgagcagc ctgagatctg aggacacggc cgtgtattac 1620

tgtgcgagag tcccagtagt gggagctacc tacaactggt tcgacccctg gggccaggga 1680

accacggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt     1860

cccgccgtgc ttcagagcag cggactttat agcctcagca gtgtggtgac cgtgccatct     1920

tccagcctgg ggaccaagac ctacacctgt aacgtggacc acaaacccag caacaccaag     1980

gttgataaga gggtc                                                      1995

<210>  73
<211>  665
<212>  PRT
<213>  Artificial Sequence

<220>
<223>  description of the artificial sequence: the amino acid sequence
       of Ct-R1090-Fa1046 H Chain

<400>  73

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
        180                 185                 190

Ser Leu Gly Thr Lys Thr Tyr Thr Cys Asn Val Asp His Lys Pro Ser
    195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
        260                 265                 270

Phe Asn Trp Tyr Val Asp Gly Val Glu Val His Asn Ala Lys Thr Lys
    275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
        340                 345                 350

Gln Glu Glu Met Thr Lys Asn Gln Val Ser Leu Thr Cys Leu Val Lys
    355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
        420                 425                 430

His Tyr Thr Gln Lys Ser Leu Ser Leu Ser Leu Gly Lys Glu Val Gln
        435                 440                 445

Leu Val Gln Ser Gly Ala Glu Val Lys Lys Pro Gly Ala Ser Val Lys
    450                 455                 460

Val Ser Cys Lys Ala Ser Gly Tyr Thr Phe Thr Asn Ser Gly Ile Asn
465                 470                 475                 480

Trp Val Arg Gln Ala Pro Gly Gln Gly Leu Glu Trp Met Gly Trp Met
                485                 490                 495

Asn Pro Asn Ser Gly Asp Thr Gly Tyr Ala Gln Lys Phe Gln Gly Arg
            500                 505                 510

Val Thr Met Thr Arg Asn Thr Ser Ile Ser Thr Ala Tyr Met Glu Leu
        515                 520                 525

Ser Ser Leu Arg Ser Glu Asp Thr Ala Val Tyr Tyr Cys Ala Arg Val
    530                 535                 540

Pro Val Val Gly Ala Thr Tyr Asn Trp Phe Asp Pro Trp Gly Gln Gly
545                 550                 555                 560

Thr Thr Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  74

<211>  366
<212>  DNA
<213>  Artificial Sequence

<220>
<223>  description of the artificial sequence: the base sequence of
       Fa1046-GTS VH

<400>  74
gaggtgcagc tggtgcagtc tggagctgag gtgaagaagc ctggggcctc agtgaaggtc        60

tcctgcaagg cttctggtta cacctttacc aactctggta tcaactgggt gcgacaggcc       120

cctggacaag ggcttgagtg gatgggatgg atgaacccta acagtggtga cacaggctat       180

gcacagaagt tccagggcag agtcaccatg accaggggga cctccataag cacagcctac       240

atggagctga gcagcctgag atctgaggac acggccgtgt attactgtgc gagagtccca       300

gtagtgggag ctacctacaa ctggttcgac ccctggggcc agggaaccac ggtcaccgtc       360

tcctca                                                                  366


<210>  75
<211>  122
<212>  PRT
<213>  Artificial Sequence

<220>
<223>  description of the artificial sequence: the amino acid sequence
       of Fa1046-GTS VH

<400>  75

Glu Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asn Ser
            20                  25                  30


Gly Ile Asn Trp Val Arg Gln Ala Pro Gly Gln Gly Leu Glu Trp Met
        35                  40                  45


Gly Trp Met Asn Pro Asn Ser Gly Asp Thr Gly Tyr Ala Gln Lys Phe
    50                  55                  60


Gln Gly Arg Val Thr Met Thr Arg Gly Thr Ser Ile Ser Thr Ala Tyr
65                  70                  75                  80


Met Glu Leu Ser Ser Leu Arg Ser Glu Asp Thr Ala Val Tyr Tyr Cys
                85                  90                  95


Ala Arg Val Pro Val Val Gly Ala Thr Tyr Asn Trp Phe Asp Pro Trp
            100                 105                 110

Gly Gln Gly Thr Thr Val Thr Val Ser Ser
        115                 120

<210>  76
<211>  366
<212>  DNA
<213>  Artificial Sequence

<220>
<223>  description of the artificial sequence: the base sequence of
       Fa1046-FTS VH

<400>  76
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggttca cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctca                                                                366

<210>  77
<211>  122
<212>  PRT
<213>  Artificial Sequence

<220>
<223>  description of the artificial sequence: the amino acid sequence
       of Fa1046-FTS VH

<400>  77

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe
    50                  55                  60

Gln Gly Arg Val Thr Met Thr Arg Phe Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser
        115                 120

<210>  78
<211>  366
<212>  DNA
<213>  Artificial Sequence

<220>
<223>  description of the artificial sequence: the base sequence of
       Fa1046-KTS VH

<400>  78
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggaaga cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctca                                                                366

<210>  79
<211>  122
<212>  PRT
<213>  Artificial Sequence

<220>
<223>  description of the artificial sequence: the amino acid sequence
       of Fa1046-KTS VH

<400>  79

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe
    50                  55                  60

Gln Gly Arg Val Thr Met Thr Arg Lys Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser
        115                 120

<210>  80
<211>  366
<212>  DNA
<213>  Artificial Sequence

<220>
<223>  description of the artificial sequence: the base sequence of
       Fa1046-WTS VH

<400>  80
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggtgga cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctca                                                                366

<210>  81
<211>  122
<212>  PRT
<213>  Artificial Sequence

<220>
<223>  description of the artificial sequence: the amino acid sequence
       of Fa1046-WTS VH

<400>  81

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe

50                  55                  60

Gln Gly Arg Val Thr Met Thr Arg Trp Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser
        115                 120

<210>  82
<211>  366
<212>  DNA
<213>  Artificial Sequence

<220>
<223>  description of the artificial sequence: the base sequence of
       Fa1046-TTS VH

<400>  82
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggacaa cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctca                                                                366

<210>  83
<211>  122
<212>  PRT
<213>  Artificial Sequence

<220>
<223>  description of the artificial sequence: the amino acid sequence
       of Fa1046-TTS VH

<400>  83

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe
    50                  55                  60

Gln Gly Arg Val Thr Met Thr Arg Thr Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser
        115                 120

<210>  84
<211>  1995
<212>  DNA
<213>  Artificial Sequence

<220>
<223>  description of the artificial sequence: the base sequence of
       Ct-R1090-Fa1046-GTS H Chain

<400>  84
gaggtgcagc tggtgcagtc cggagcagag gtgaaaaagc ccggggagtc tctgaagatc      60

tcctgtaagg gttctggata cagctttatc atctactgga tcggctgggt gcgccagatg     120

cccgggaaag gcctggagtg gatggggatc atctatccta atgacgctga taccagatac     180

agcccgtcct tccaaggcca ggtcaccatc tcagccgaca agtccatcag caccgcctac     240

ctgcagtgga gcagcctgaa ggcctcggac accgccatgt attactgtgt gagactgggt     300

acgagggact ggttcgaccc ctggggccag ggaaccctgg tcaccgtctc ctcagcaagt     360

accaaggggc catccgtctt ccccctggcg ccctgctcca ggagcacctc cgagagcaca     420

gccgccctgg gctgcctggt caaggactac ttccccgaac cggtgacggt gtcgtggaac     480

tcaggcgccc tgaccagcgg cgtgcacacc ttcccggctg tcctacagtc ctcaggactc     540

tactccctca gcagcgtggt gaccgtgccc tccagcagct tgggcacgaa gacctacacc     600

tgcaacgtag atcacaagcc cagcaacacc aaggtggaca agagagttga gtccaaatat     660

ggtcccccat gcccaccatg cccagcacct gagttcgagg ggggaccatc agtcttcctg     720

ttccccccaa aacccaagga cactctcatg atctcccgga cccctgaggt cacgtgcgtg     780

gtggtggacg tgagccagga agaccccgag gtccagttca actggtacgt ggatggcgtg     840

gaggtgcata atgccaagac aaagccgcgg gaggagcagt tcaacagcac gtaccgtgtg     900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaagaggt gcagctggtg cagtctggag ctgaggtgaa gaagcctggg 1380

gcctcagtga aggtctcctg caaggcttct ggttacacct ttaccaactc tggtatcaac 1440

tgggtgcgac aggcccctgg acaagggctt gagtggatgg gatggatgaa ccctaacagt 1500

ggtgacacag gctatgcaca gaagttccag ggcagagtca ccatgaccag ggggacctcc 1560

ataagcacag cctacatgga gctgagcagc ctgagatctg aggacacggc cgtgtattac 1620

tgtgcgagag tcccagtagt gggagctacc tacaactggt tcgacccctg gggccaggga 1680

accacggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 85
<211> 665
<212> PRT
<213> Artificial Sequence

<220>
<223> description of the artificial sequence: the amino acid sequence
of Ct-R1090-Fa1046-GTS H Chain

<400> 85

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Glu Val Gln
        435                 440                 445

Leu Val Gln Ser Gly Ala Glu Val Lys Lys Pro Gly Ala Ser Val Lys
    450                 455                 460

Val Ser Cys Lys Ala Ser Gly Tyr Thr Phe Thr Asn Ser Gly Ile Asn
465                 470                 475                 480

Trp Val Arg Gln Ala Pro Gly Gln Gly Leu Glu Trp Met Gly Trp Met
                485                 490                 495

Asn Pro Asn Ser Gly Asp Thr Gly Tyr Ala Gln Lys Phe Gln Gly Arg
            500                 505                 510

Val Thr Met Thr Arg Gly Thr Ser Ile Ser Thr Ala Tyr Met Glu Leu
        515                 520                 525

Ser Ser Leu Arg Ser Glu Asp Thr Ala Val Tyr Tyr Cys Ala Arg Val
    530                 535                 540

Pro Val Val Gly Ala Thr Tyr Asn Trp Phe Asp Pro Trp Gly Gln Gly
545                 550                 555                 560

Thr Thr Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  86
<211>  1995
<212>  DNA
<213>  Artificial Sequence

<220>
<223>  description of the artificial sequence: the base sequence of
       Ct-R1090-Fa1046-FTS H Chain

<400>  86
gaggtgcagc tggtgcagtc cggagcagag gtgaaaaagc ccggggagtc tctgaagatc      60

tcctgtaagg gttctggata cagctttatc atctactgga tcggctgggt gcgccagatg     120

cccgggaaag gcctggagtg gatggggatc atctatccta atgacgctga taccagatac     180

agcccgtcct tccaaggcca ggtcaccatc tcagccgaca agtccatcag caccgcctac     240

ctgcagtgga gcagcctgaa ggcctcggac accgccatgt attactgtgt gagactgggt     300

acgagggact ggttcgaccc ctggggccag ggaaccctgg tcaccgtctc ctcagcaagt     360

accaaggggc catccgtctt ccccctggcg ccctgctcca ggagcacctc cgagagcaca     420

gccgccctgg gctgcctggt caaggactac ttccccgaac cggtgacggt gtcgtggaac     480

tcaggcgccc tgaccagcgg cgtgcacacc ttcccggctg tcctacagtc ctcaggactc     540

tactccctca gcagcgtggt gaccgtgccc tccagcagct tgggcacgaa gacctacacc     600

tgcaacgtag atcacaagcc cagcaacacc aaggtggaca agagagttga gtccaaatat     660

ggtcccccat gcccaccatg cccagcacct gagttcgagg ggggaccatc agtcttcctg     720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaagaggt gcagctggtg cagtctggag ctgaggtgaa gaagcctggg 1380

gcctcagtga aggtctcctg caaggcttct ggttacacct ttaccaactc tggtatcaac 1440

tgggtgcgac aggcccctgg acaagggctt gagtggatgg gatggatgaa ccctaacagt 1500

ggtgacacag gctatgcaca gaagttccag ggcagagtca ccatgaccag gttcacctcc 1560

ataagcacag cctacatgga gctgagcagc ctgagatctg aggacacggc cgtgtattac 1620

tgtgcgagag tcccagtagt gggagctacc tacaactggt tcgacccctg gggccaggga 1680

accacggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 87
<211> 665
<212> PRT
<213> Artificial Sequence

<220>
<223> description of the artificial sequence: the amino acid sequence
of Ct-R1090-Fa1046-FTS H Chain

<400> 87

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys

275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Glu Val Gln
        435                 440                 445

Leu Val Gln Ser Gly Ala Glu Val Lys Lys Pro Gly Ala Ser Val Lys
    450                 455                 460

Val Ser Cys Lys Ala Ser Gly Tyr Thr Phe Thr Asn Ser Gly Ile Asn
465                 470                 475                 480

Trp Val Arg Gln Ala Pro Gly Gln Gly Leu Glu Trp Met Gly Trp Met
                485                 490                 495

Asn Pro Asn Ser Gly Asp Thr Gly Tyr Ala Gln Lys Phe Gln Gly Arg
            500                 505                 510

Val Thr Met Thr Arg Phe Thr Ser Ile Ser Thr Ala Tyr Met Glu Leu
        515                 520                 525

Ser Ser Leu Arg Ser Glu Asp Thr Ala Val Tyr Tyr Cys Ala Arg Val
    530                 535                 540

Pro Val Val Gly Ala Thr Tyr Asn Trp Phe Asp Pro Trp Gly Gln Gly
545                 550                 555                 560

Thr Thr Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  88
<211>  1995
<212>  DNA
<213>  Artificial Sequence

<220>
<223>  description of the artificial sequence: the base sequence of
       Ct-R1090-Fa1046-KTS H Chain

<400>  88
gaggtgcagc tggtgcagtc cggagcagag gtgaaaaagc ccggggagtc tctgaagatc      60

tcctgtaagg gttctggata cagctttatc atctactgga tcggctgggt gcgccagatg     120

cccgggaaag gcctggagtg gatggggatc atctatccta atgacgctga taccagatac     180

agcccgtcct tccaaggcca ggtcaccatc tcagccgaca agtccatcag caccgcctac     240

ctgcagtgga gcagcctgaa ggcctcggac accgccatgt attactgtgt gagactgggt     300

acgagggact ggttcgaccc ctggggccag ggaaccctgg tcaccgtctc ctcagcaagt     360

accaaggggc catccgtctt ccccctggcg ccctgctcca ggagcacctc cgagagcaca     420

gccgccctgg gctgcctggt caaggactac ttccccgaac cggtgacggt gtcgtggaac     480

tcaggcgccc tgaccagcgg cgtgcacacc ttcccggctg tcctacagtc ctcaggactc     540

tactccctca gcagcgtggt gaccgtgccc tccagcagct tgggcacgaa gacctacacc 600

tgcaacgtag atcacaagcc cagcaacacc aaggtggaca agagagttga gtccaaatat 660

ggtcccccat gcccaccatg cccagcacct gagttcgagg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaagaggt gcagctggtg cagtctggag ctgaggtgaa gaagcctggg 1380

gcctcagtga aggtctcctg caaggcttct ggttacacct ttaccaactc tggtatcaac 1440

tgggtgcgac aggcccctgg acaagggctt gagtggatgg gatggatgaa ccctaacagt 1500

ggtgacacag gctatgcaca gaagttccag ggcagagtca ccatgaccag gaagacctcc 1560

ataagcacag cctacatgga gctgagcagc ctgagatctg aggacacggc cgtgtattac 1620

tgtgcgagag tcccagtagt gggagctacc tacaactggt tcgacccctg gggccaggga 1680

accacggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 89
<211> 665
<212> PRT
<213> Artificial Sequence

<220>
<223> description of the artificial sequence: the amino acid sequence
      of Ct-R1090-Fa1046-KTS H Chain

<400> 89

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Glu Val Gln
        435                 440                 445

Leu Val Gln Ser Gly Ala Glu Val Lys Lys Pro Gly Ala Ser Val Lys
    450                 455                 460

Val Ser Cys Lys Ala Ser Gly Tyr Thr Phe Thr Asn Ser Gly Ile Asn
465                 470                 475                 480

Trp Val Arg Gln Ala Pro Gly Gln Gly Leu Glu Trp Met Gly Trp Met
                485                 490                 495

Asn Pro Asn Ser Gly Asp Thr Gly Tyr Ala Gln Lys Phe Gln Gly Arg
            500                 505                 510

Val Thr Met Thr Arg Lys Thr Ser Ile Ser Thr Ala Tyr Met Glu Leu
        515                 520                 525

Ser Ser Leu Arg Ser Glu Asp Thr Ala Val Tyr Tyr Cys Ala Arg Val
    530                 535                 540

Pro Val Val Gly Ala Thr Tyr Asn Trp Phe Asp Pro Trp Gly Gln Gly
545                 550                 555                 560

Thr Thr Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  90
<211>  1995
<212>  DNA
<213>  Artificial Sequence

<220>
<223>  description of the artificial sequence: the base sequence of
       Ct-R1090-Fa1046-WTS H Chain

<400>  90
gaggtgcagc tggtgcagtc cggagcagag gtgaaaaagc ccggggagtc tctgaagatc      60

tcctgtaagg gttctggata cagctttatc atctactgga tcggctgggt gcgccagatg     120

cccgggaaag gcctggagtg gatggggatc atctatccta atgacgctga taccagatac     180

agcccgtcct tccaaggcca ggtcaccatc tcagccgaca agtccatcag caccgcctac     240

ctgcagtgga gcagcctgaa ggcctcggac accgccatgt attactgtgt gagactgggt     300

acgagggact ggttcgaccc ctggggccag ggaaccctgg tcaccgtctc ctcagcaagt     360

accaaggggc catccgtctt ccccctggcg ccctgctcca ggagcacctc cgagagcaca 420

gccgccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcacgaa gacctacacc 600

tgcaacgtag atcacaagcc cagcaacacc aaggtggaca agagagttga gtccaaatat 660

ggtcccccat gcccaccatg cccagcacct gagttcgagg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaagaggt gcagctggtg cagtctggag ctgaggtgaa gaagcctggg 1380

gcctcagtga aggtctcctg caaggcttct ggttacacct ttaccaactc tggtatcaac 1440

tgggtgcgac aggcccctgg acaagggctt gagtggatgg gatggatgaa ccctaacagt 1500

ggtgacacag gctatgcaca gaagttccag ggcagagtca ccatgaccag gtggacctcc 1560

ataagcacag cctacatgga gctgagcagc ctgagatctg aggacacggc cgtgtattac 1620

tgtgcgagag tcccagtagt gggagctacc tacaactggt tcgacccctg gggccaggga 1680

accacggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 91
<211> 665
<212> PRT
<213> Artificial Sequence

<220>
<223>  description of the artificial sequence: the amino acid sequence
      of Ct-R1090-Fa1046-WTS H Chain

<400>  91

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu

225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Glu Val Gln
        435                 440                 445

Leu Val Gln Ser Gly Ala Glu Val Lys Lys Pro Gly Ala Ser Val Lys
    450                 455                 460

Val Ser Cys Lys Ala Ser Gly Tyr Thr Phe Thr Asn Ser Gly Ile Asn
465                 470                 475                 480

Trp Val Arg Gln Ala Pro Gly Gln Gly Leu Glu Trp Met Gly Trp Met
                485                 490                 495

Asn Pro Asn Ser Gly Asp Thr Gly Tyr Ala Gln Lys Phe Gln Gly Arg
            500                 505                 510

Val Thr Met Thr Arg Trp Thr Ser Ile Ser Thr Ala Tyr Met Glu Leu
        515                 520                 525

Ser Ser Leu Arg Ser Glu Asp Thr Ala Val Tyr Tyr Cys Ala Arg Val
    530                 535                 540

Pro Val Val Gly Ala Thr Tyr Asn Trp Phe Asp Pro Trp Gly Gln Gly
545                 550                 555                 560

Thr Thr Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  92
<211>  1995
<212>  DNA
<213>  Artificial Sequence

<220>
<223>  description of the artificial sequence: the base sequence of
       Ct-R1090-Fa1046-TTS H Chain

<400>  92
gaggtgcagc tggtgcagtc cggagcagag gtgaaaaagc ccggggagtc tctgaagatc      60

tcctgtaagg gttctggata cagctttatc atctactgga tcggctgggt gcgccagatg     120

cccgggaaag gcctggagtg gatggggatc atctatccta atgacgctga taccagatac     180

agcccgtcct tccaaggcca ggtcaccatc tcagccgaca agtccatcag caccgcctac 240

ctgcagtgga gcagcctgaa ggcctcggac accgccatgt attactgtgt gagactgggt 300

acgagggact ggttcgaccc ctggggccag ggaaccctgg tcaccgtctc ctcagcaagt 360

accaaggggc catccgtctt ccccctggcg ccctgctcca ggagcacctc cgagagcaca 420

gccgccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcacgaa gacctacacc 600

tgcaacgtag atcacaagcc cagcaacacc aaggtggaca agagagttga gtccaaatat 660

ggtcccccat gcccaccatg cccagcacct gagttcgagg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaagaggt gcagctggtg cagtctggag ctgaggtgaa gaagcctggg 1380

gcctcagtga aggtctcctg caaggcttct ggttacacct ttaccaactc tggtatcaac 1440

tgggtgcgac aggcccctgg acaagggctt gagtggatgg gatggatgaa ccctaacagt 1500

ggtgacacag gctatgcaca gaagttccag ggcagagtca ccatgaccag gacaacctcc 1560

ataagcacag cctacatgga gctgagcagc ctgagatctg aggacacggc cgtgtattac 1620

tgtgcgagag tcccagtagt gggagctacc tacaactggt tcgacccctg gggccaggga 1680

accacggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210>  93
<211>  665
<212>  PRT
<213>  Artificial Sequence

<220>
<223>  description of the artificial sequence: the amino acid sequence
       of Ct-R1090-Fa1046-TTS H Chain

<400>  93

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Ile Ile Tyr
            20                  25                  30

Trp Ile Gly Trp Val Arg Gln Met Pro Gly Lys Gly Leu Glu Trp Met
        35                  40                  45

Gly Ile Ile Tyr Pro Asn Asp Ala Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Val Arg Leu Gly Thr Arg Asp Trp Phe Asp Pro Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Glu Val Gln
        435                 440                 445

Leu Val Gln Ser Gly Ala Glu Val Lys Lys Pro Gly Ala Ser Val Lys
    450                 455                 460

Val Ser Cys Lys Ala Ser Gly Tyr Thr Phe Thr Asn Ser Gly Ile Asn
465                 470                 475                 480

Trp Val Arg Gln Ala Pro Gly Gln Gly Leu Glu Trp Met Gly Trp Met
                485                 490                 495

Asn Pro Asn Ser Gly Asp Thr Gly Tyr Ala Gln Lys Phe Gln Gly Arg
            500                 505                 510

Val Thr Met Thr Arg Thr Thr Ser Ile Ser Thr Ala Tyr Met Glu Leu
        515                 520                 525

Ser Ser Leu Arg Ser Glu Asp Thr Ala Val Tyr Tyr Cys Ala Arg Val
    530                 535                 540

Pro Val Val Gly Ala Thr Tyr Asn Trp Phe Asp Pro Trp Gly Gln Gly
545                 550                 555                 560

Thr Thr Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  94
<211>  1983
<212>  DNA
<213>  Artificial Sequence

<220>
<223>  description of the artificial sequence: the base sequence of
       Ct-Fa018-R1090 H Chain

<400> 94
gaggtgcagc tggtgcagtc tggggctgag gtgaagaagc ctggggcctc agtgaaggtt 60

tcctgcaagg catctggata caccttcacc agtcactata tacactgggt gcgacaggcc 120

cctggacaag ggcttgagtg gatgggatgg atcagcgctt tcaatggtga cacaaactat 180

gcacagaagt tccaggacag agtcaccatg accacagaca cgtccacgag cacagtgtac 240

atggagctga ggggcctgag atctgaggac acggccgtgt attactgtgc gagagatagc 300

accaggtggt tcattgacta ctggggccag ggaaccacgg tcaccgtctc ctcagcaagt 360

accaaggggc catccgtctt ccccctggcg ccctgctcta gaagcacctc cgagagcaca 420

gccgccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcacgaa gacctacacc 600

tgcaacgtag atcacaagcc cagcaacacc aaggtggaca agagagttga gtccaaatat 660

ggtcccccat gcccaccatg cccagcacct gagttcgagg ggggaccatc agtcttcctg 720

ttccccccaa aacccaagga cactctcatg atctcccgga cccctgaggt cacgtgcgtg 780

gtggtggacg tgagccagga agaccccgag gtccagttca actggtacgt ggatggcgtg 840

gaggtgcata atgccaagac aaagccgcgg gaggagcagt tcaacagcac gtaccgtgtg 900

gtcagcgtcc tcaccgtcct gcaccaggac tggctgaacg gcaaggagta caagtgcaag 960

gtctccaaca aaggcctccc gtcctccatc gagaaaacca tctccaaagc caaagggcag 1020

ccccgagagc cacaggtgta caccctgccc ccatcccagg aggagatgac caagaaccag 1080

gtcagcctga cctgcctggt caaaggcttc taccccagcg acatcgccgt ggagtgggag 1140

agcaatgggc agccggagaa caactacaag accacgcctc ccgtgctgga ctccgacggc 1200

tccttcttcc tctacagcaa gctaaccgtg gacaagagca ggtggcagga ggggaatgtc 1260

ttctcatgct ccgtgatgca tgaggctctg cacaaccact acacacagaa gagcctctcc 1320

ctgtccctag gtaaagaggt gcagctggtg cagtccggag cagaggtgaa aaagcccggg 1380

gagtctctga agatctcctg taagggttct ggatacagct ttatcatcta ctggatcggc 1440

tgggtgcgcc agatgcccgg gaaaggcctg gagtggatgg ggatcatcta tcctaatgac 1500

gctgatacca gatacagccc gtccttccaa ggccaggtca ccatctcagc cgacaagtcc 1560

atcagcaccg cctacctgca gtggagcagc ctgaaggcct cggacaccgc catgtattac 1620

tgtgtgagac tgggtacgag ggactggttc gacccctggg gccagggaac cctggtcacc 1680

gtctcctcag ctagcaccaa aggaccttct gtatttcctc ttgcgccatg ctctcgctct 1740

acgtcagaat caactgccgc tctggggtgc ctggttaaag actacttccc ggagcctgtg 1800

acagtgagtt ggaactccgg cgccctgaca tcaggagtgc atacatttcc cgccgtgctt 1860

cagagcagcg gactttatag cctcagcagt gtggtgaccg tgccatcttc cagcctgggg     1920

accaagacct acacctgtaa cgtggaccac aaacccagca acaccaaggt tgataagagg     1980

gtc                                                                   1983

<210>  95
<211>  661
<212>  PRT
<213>  Artificial Sequence

<220>
<223>  description of the artificial sequence: the amino acid sequence
       of Ct-Fa018-R1090 H Chain

<400>  95

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser His
            20                  25                  30

Tyr Ile His Trp Val Arg Gln Ala Pro Gly Gln Gly Leu Glu Trp Met
        35                  40                  45

Gly Trp Ile Ser Ala Phe Asn Gly Asp Thr Asn Tyr Ala Gln Lys Phe
    50                  55                  60

Gln Asp Arg Val Thr Met Thr Thr Asp Thr Ser Thr Ser Thr Val Tyr
65                  70                  75                  80

Met Glu Leu Arg Gly Leu Arg Ser Glu Asp Thr Ala Val Tyr Tyr Cys
                85                  90                  95

Ala Arg Asp Ser Thr Arg Trp Phe Ile Asp Tyr Trp Gly Gln Gly Thr
            100                 105                 110

Thr Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser

            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Glu Gly Gly Pro Ser Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Val Leu Asp Ser Asp Gly
385                 390                 395                 400

Ser Phe Phe Leu Tyr Ser Lys Leu Thr Val Asp Lys Ser Arg Trp Gln
                405                 410                 415

Glu Gly Asn Val Phe Ser Cys Ser Val Met His Glu Ala Leu His Asn
            420                 425                 430

His Tyr Thr Gln Lys Ser Leu Ser Leu Ser Leu Gly Lys Glu Val Gln
        435                 440                 445

Leu Val Gln Ser Gly Ala Glu Val Lys Lys Pro Gly Glu Ser Leu Lys
    450                 455                 460

Ile Ser Cys Lys Gly Ser Gly Tyr Ser Phe Ile Ile Tyr Trp Ile Gly
465                 470                 475                 480

Trp Val Arg Gln Met Pro Gly Lys Gly Leu Glu Trp Met Gly Ile Ile
                485                 490                 495

Tyr Pro Asn Asp Ala Asp Thr Arg Tyr Ser Pro Ser Phe Gln Gly Gln
            500                 505                 510

Val Thr Ile Ser Ala Asp Lys Ser Ile Ser Thr Ala Tyr Leu Gln Trp
        515                 520                 525

Ser Ser Leu Lys Ala Ser Asp Thr Ala Met Tyr Tyr Cys Val Arg Leu
    530                 535                 540

Gly Thr Arg Asp Trp Phe Asp Pro Trp Gly Gln Gly Thr Leu Val Thr
545                 550                 555                 560

Val Ser Ser Ala Ser Thr Lys Gly Pro Ser Val Phe Pro Leu Ala Pro
                565                 570                 575

Cys Ser Arg Ser Thr Ser Glu Ser Thr Ala Ala Leu Gly Cys Leu Val
            580                 585                 590

Lys Asp Tyr Phe Pro Glu Pro Val Thr Val Ser Trp Asn Ser Gly Ala
        595                 600                 605

Leu Thr Ser Gly Val His Thr Phe Pro Ala Val Leu Gln Ser Ser Gly
    610                 615                 620

Leu Tyr Ser Leu Ser Ser Val Val Thr Val Pro Ser Ser Ser Leu Gly
625                 630                 635                 640

Thr Lys Thr Tyr Thr Cys Asn Val Asp His Lys Pro Ser Asn Thr Lys
                645                 650                 655

Val Asp Lys Arg Val
            660

<210>  96
<211>  1989
<212>  DNA

<213>  Artificial Sequence

<220>
<223>  description of the artificial sequence: the base sequence of
      Ct-Fa1007-R1090 H Chain

<400>  96
caggtgcagc tgcaggagtc gggcccagga ctggtgaagc cttcggagac cctgtccctc      60

acctgcactg tctctggtga ctccatcagc agtggtagtt actactgggg ctggatccgc     120

cagccccccg ggaaggggct ggagtggatt gggagtatct attctagtgg gagcacctac     180

cacgacccgt ccctcaagaa tcgagtcacc atgtccgtag acacgtccaa gaaccagttc     240

tccctgaggc tgagctctgt gaccgctgcg gacacggccg tgtattattg tgcgagagat     300

aggggctact actacaatat ggacgtctgg ggccaaggga ccacggtcac cgtctcttca     360

gcaagtacca agggcccatc cgtcttcccc ctggcgccct gctctagaag cacctccgag     420

agcacagccg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacgaagacc     600

tacacctgca acgtagatca caagcccagc aacaccaagg tggacaagag agttgagtcc     660

aaatatggtc ccccatgccc accatgccca gcacctgagt tcgagggggg accatcagtc     720

ttcctgttcc ccccaaaacc caaggacact ctcatgatct cccggacccc tgaggtcacg     780

tgcgtggtgg tggacgtgag ccaggaagac cccgaggtcc agttcaactg gtacgtggat     840

ggcgtggagg tgcataatgc caagacaaag ccgcgggagg agcagttcaa cagcacgtac     900

cgtgtggtca gcgtcctcac cgtcctgcac caggactggc tgaacggcaa ggagtacaag     960

tgcaaggtct ccaacaaagg cctcccgtcc tccatcgaga aaaccatctc caaagccaaa    1020

gggcagcccc gagagccaca ggtgtacacc ctgcccccat cccaggagga gatgaccaag    1080

aaccaggtca gcctgacctg cctggtcaaa ggcttctacc ccagcgacat cgccgtggag    1140

tgggagagca atgggcagcc ggagaacaac tacaagacca cgcctcccgt gctggactcc    1200

gacggctcct tcttcctcta cagcaagcta accgtggaca agagcaggtg gcaggagggg    1260

aatgtcttct catgctccgt gatgcatgag gctctgcaca accactacac acagaagagc    1320

ctctccctgt ccctaggtaa agaggtgcag ctggtgcagt ccggagcaga ggtgaaaaag    1380

cccggggagt ctctgaagat ctcctgtaag ggttctggat acagctttat catctactgg    1440

atcggctggg tgcgccagat gcccgggaaa ggcctggagt ggatggggat catctatcct    1500

aatgacgctg ataccagata cagcccgtcc ttccaaggcc aggtcaccat ctcagccgac    1560

aagtccatca gcaccgccta cctgcagtgg agcagcctga aggcctcgga caccgccatg    1620

tattactgtg tgagactggg tacgagggac tggttcgacc cctggggcca gggaaccctg    1680

gtcaccgtct cctcagctag caccaaagga ccttctgtat ttcctcttgc gccatgctct 1740

cgctctacgt cagaatcaac tgccgctctg gggtgcctgg ttaaagacta cttcccggag 1800

cctgtgacag tgagttggaa ctccggcgcc ctgacatcag gagtgcatac atttcccgcc 1860

gtgcttcaga gcagcggact ttatagcctc agcagtgtgg tgaccgtgcc atcttccagc 1920

ctggggacca agacctacac ctgtaacgtg gaccacaaac ccagcaacac caaggttgat 1980

aagagggtc 1989

<210> 97
<211> 663
<212> PRT
<213> Artificial Sequence

<220>
<223> description of the artificial sequence: the amino acid sequence
      of Ct-Fa1007-R1090 H Chain

<400> 97

Gln Val Gln Leu Gln Glu Ser Gly Pro Gly Leu Val Lys Pro Ser Glu
1               5                   10                  15

Thr Leu Ser Leu Thr Cys Thr Val Ser Gly Asp Ser Ile Ser Ser Gly
            20                  25                  30

Ser Tyr Tyr Trp Gly Trp Ile Arg Gln Pro Pro Gly Lys Gly Leu Glu
        35                  40                  45

Trp Ile Gly Ser Ile Tyr Ser Ser Gly Ser Thr Tyr His Asp Pro Ser
    50                  55                  60

Leu Lys Asn Arg Val Thr Met Ser Val Asp Thr Ser Lys Asn Gln Phe
65                  70                  75                  80

Ser Leu Arg Leu Ser Ser Val Thr Ala Ala Asp Thr Ala Val Tyr Tyr
                85                  90                  95

Cys Ala Arg Asp Arg Gly Tyr Tyr Tyr Asn Met Asp Val Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Glu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys Glu
        435                 440                 445

Val Gln Leu Val Gln Ser Gly Ala Glu Val Lys Lys Pro Gly Glu Ser
    450                 455                 460

Leu Lys Ile Ser Cys Lys Gly Ser Gly Tyr Ser Phe Ile Ile Tyr Trp
465                 470                 475                 480

Ile Gly Trp Val Arg Gln Met Pro Gly Lys Gly Leu Glu Trp Met Gly
                485                 490                 495

Ile Ile Tyr Pro Asn Asp Ala Asp Thr Arg Tyr Ser Pro Ser Phe Gln
            500                 505                 510

Gly Gln Val Thr Ile Ser Ala Asp Lys Ser Ile Ser Thr Ala Tyr Leu
        515                 520                 525

Gln Trp Ser Ser Leu Lys Ala Ser Asp Thr Ala Met Tyr Tyr Cys Val
    530                 535                 540

Arg Leu Gly Thr Arg Asp Trp Phe Asp Pro Trp Gly Gln Gly Thr Leu
545                 550                 555                 560

Val Thr Val Ser Ser Ala Ser Thr Lys Gly Pro Ser Val Phe Pro Leu
                565                 570                 575

Ala Pro Cys Ser Arg Ser Thr Ser Glu Ser Thr Ala Ala Leu Gly Cys
            580                 585                 590

Leu Val Lys Asp Tyr Phe Pro Glu Pro Val Thr Val Ser Trp Asn Ser
        595                 600                 605

Gly Ala Leu Thr Ser Gly Val His Thr Phe Pro Ala Val Leu Gln Ser
    610                 615                 620

Ser Gly Leu Tyr Ser Leu Ser Ser Val Val Thr Val Pro Ser Ser Ser
625                 630                 635                 640

Leu Gly Thr Lys Thr Tyr Thr Cys Asn Val Asp His Lys Pro Ser Asn
                645                 650                 655

Thr Lys Val Asp Lys Arg Val

            660

<210> 98
<211> 1989
<212> DNA
<213> Artificial Sequence

<220>
<223> description of the artificial sequence: the base sequence of
      Ct-Fa1013-R1090 H Chain

<400> 98
caggtgcagc tgcaggagtc gggcccagga ctggtgaagc cttcggagac cctgtccctc      60

acctgcactg tctctggtgg ctccgtcagc agtggtagtt actactggag ctggatccgg     120

cagtccccag ggaagggact ggagtggatt gggtttatct attacagtgg gaacaccaac     180

tacaacccct ccctcaagag tcgagtcacc atatcagtag acacgtccaa gaaccagttc     240

tccctgaagc tgagctctgt gaccgctgcg gacacggccg tgtattactg tgcgagagat     300

aggggctact actacggtat ggacgtctgg ggccaaggga ccacggtcac cgtctcctca     360

gcaagtacca agggcccatc cgtcttcccc ctggcgccct gctctagaag cacctccgag     420

agcacagccg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacgaagacc     600

tacacctgca acgtagatca caagcccagc aacaccaagg tggacaagag agttgagtcc     660

aaatatggtc ccccatgccc accatgccca gcacctgagt tcgagggggg accatcagtc     720

ttcctgttcc ccccaaaacc caaggacact ctcatgatct cccggacccc tgaggtcacg     780

tgcgtggtgg tggacgtgag ccaggaagac cccgaggtcc agttcaactg gtacgtggat     840

ggcgtggagg tgcataatgc caagacaaag ccgcgggagg agcagttcaa cagcacgtac     900

cgtgtggtca gcgtcctcac cgtcctgcac caggactggc tgaacggcaa ggagtacaag     960

tgcaaggtct ccaacaaagg cctcccgtcc tccatcgaga aaaccatctc caaagccaaa    1020

gggcagcccc gagagccaca ggtgtacacc ctgcccccat cccaggagga gatgaccaag    1080

aaccaggtca gcctgacctg cctggtcaaa ggcttctacc ccagcgacat cgccgtggag    1140

tgggagagca atgggcagcc ggagaacaac tacaagacca cgcctcccgt gctggactcc    1200

gacggctcct tcttcctcta cagcaagcta accgtggaca agagcaggtg gcaggagggg    1260

aatgtcttct catgctccgt gatgcatgag gctctgcaca accactacac acagaagagc    1320

ctctccctgt ccctaggtaa agaggtgcag ctggtgcagt ccggagcaga ggtgaaaaag    1380

cccggggagt ctctgaagat ctcctgtaag ggttctggat acagctttat catctactgg    1440

atcggctggg tgcgccagat gcccgggaaa ggcctggagt ggatggggat catctatcct    1500

aatgacgctg ataccagata cagcccgtcc ttccaaggcc aggtcaccat ctcagccgac 1560

aagtccatca gcaccgccta cctgcagtgg agcagcctga aggcctcgga caccgccatg 1620

tattactgtg tgagactggg tacgagggac tggttcgacc cctggggcca gggaaccctg 1680

gtcaccgtct cctcagctag caccaaagga ccttctgtat ttcctcttgc gccatgctct 1740

cgctctacgt cagaatcaac tgccgctctg gggtgcctgg ttaaagacta cttcccggag 1800

cctgtgacag tgagttggaa ctccggcgcc ctgacatcag gagtgcatac atttcccgcc 1860

gtgcttcaga gcagcggact ttatagcctc agcagtgtgg tgaccgtgcc atcttccagc 1920

ctggggacca agacctacac ctgtaacgtg gaccacaaac ccagcaacac caaggttgat 1980

aagagggtc 1989

<210> 99
<211> 663
<212> PRT
<213> Artificial Sequence

<220>
<223> description of the artificial sequence: the amino acid sequence
      of Ct-Fa1013-R1090 H Chain

<400> 99

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Val Ser Ser Gly
            20                  25                  30

Ser Tyr Tyr Trp Ser Trp Ile Arg Gln Ser Pro Gly Lys Gly Leu Glu
        35                  40                  45

Trp Ile Gly Phe Ile Tyr Tyr Ser Gly Asn Thr Asn Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Asp Arg Gly Tyr Tyr Tyr Gly Met Asp Val Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala

    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Glu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys Glu
        435                 440                 445

Val Gln Leu Val Gln Ser Gly Ala Glu Val Lys Lys Pro Gly Glu Ser
    450                 455                 460

Leu Lys Ile Ser Cys Lys Gly Ser Gly Tyr Ser Phe Ile Ile Tyr Trp
465                 470                 475                 480

Ile Gly Trp Val Arg Gln Met Pro Gly Lys Gly Leu Glu Trp Met Gly
                485                 490                 495

Ile Ile Tyr Pro Asn Asp Ala Asp Thr Arg Tyr Ser Pro Ser Phe Gln
            500                 505                 510

Gly Gln Val Thr Ile Ser Ala Asp Lys Ser Ile Ser Thr Ala Tyr Leu
        515                 520                 525

Gln Trp Ser Ser Leu Lys Ala Ser Asp Thr Ala Met Tyr Tyr Cys Val
    530                 535                 540

Arg Leu Gly Thr Arg Asp Trp Phe Asp Pro Trp Gly Gln Gly Thr Leu
545                 550                 555                 560

Val Thr Val Ser Ser Ala Ser Thr Lys Gly Pro Ser Val Phe Pro Leu
                565                 570                 575

Ala Pro Cys Ser Arg Ser Thr Ser Glu Ser Thr Ala Ala Leu Gly Cys
            580                 585                 590

Leu Val Lys Asp Tyr Phe Pro Glu Pro Val Thr Val Ser Trp Asn Ser
        595                 600                 605

Gly Ala Leu Thr Ser Gly Val His Thr Phe Pro Ala Val Leu Gln Ser
    610                 615                 620

Ser Gly Leu Tyr Ser Leu Ser Ser Val Val Thr Val Pro Ser Ser Ser
625                 630                 635                 640

Leu Gly Thr Lys Thr Tyr Thr Cys Asn Val Asp His Lys Pro Ser Asn
                645                 650                 655

Thr Lys Val Asp Lys Arg Val
            660

<210>  100
<211>  1989
<212>  DNA
<213>  Artificial Sequence

<220>
<223>  description of the artificial sequence: the base sequence of
       Ct-Fa1014-R1090 H Chain

<400>  100
cagctgcagc tgcaggagtc gggcccagga ctggtgaagc cttcggagac cctgtccctc      60

acctgcactg tctctggtgg ctccatcagc agtagtagtt cctactgggg ctggatccgc     120

cagcccccag gaaagggact ggagtggatt gggagtatct attatagtgg gagcaccaac     180

tacaacccct ccctcaagag tcgagtcacc atatcagtag acacgtccaa gaaccagttc     240

tccctgaagc tgaggtctgt gaccgctgcg gacacggccg tgtattactg tgtgagagat     300

aaaggctact actacggtat ggacgtctgg ggccaaggga ccacggtcac cgtctcctca     360

gcaagtacca agggcccatc cgtcttcccc ctggcgccct gctctagaag cacctccgag     420

agcacagccg ccctgggctg cctggtcaag gactacttcc ccgaaccggt gacggtgtcg     480

tggaactcag gcgccctgac cagcggcgtg cacaccttcc cggctgtcct acagtcctca     540

ggactctact ccctcagcag cgtggtgacc gtgccctcca gcagcttggg cacgaagacc     600

tacacctgca acgtagatca caagcccagc aacaccaagg tggacaagag agttgagtcc     660

aaatatggtc ccccatgccc accatgccca gcacctgagt tcgagggggg accatcagtc     720

ttcctgttcc ccccaaaacc caaggacact ctcatgatct cccggacccc tgaggtcacg     780

tgcgtggtgg tggacgtgag ccaggaagac cccgaggtcc agttcaactg gtacgtggat     840

ggcgtggagg tgcataatgc caagacaaag ccgcgggagg agcagttcaa cagcacgtac     900

cgtgtggtca gcgtcctcac cgtcctgcac caggactggc tgaacggcaa ggagtacaag     960

tgcaaggtct ccaacaaagg cctcccgtcc tccatcgaga aaaccatctc caaagccaaa    1020

gggcagcccc gagagccaca ggtgtacacc ctgcccccat cccaggagga gatgaccaag    1080

aaccaggtca gcctgacctg cctggtcaaa ggcttctacc ccagcgacat cgccgtggag    1140

tgggagagca atgggcagcc ggagaacaac tacaagacca cgcctcccgt gctggactcc    1200

gacggctcct tcttcctcta cagcaagcta accgtggaca agagcaggtg gcaggagggg    1260

aatgtcttct catgctccgt gatgcatgag gctctgcaca accactacac acagaagagc    1320

ctctccctgt ccctaggtaa agaggtgcag ctggtgcagt ccggagcaga ggtgaaaaag 1380

cccggggagt ctctgaagat ctcctgtaag ggttctggat acagctttat catctactgg 1440

atcggctggg tgcgccagat gcccgggaaa ggcctggagt ggatggggat catctatcct 1500

aatgacgctg ataccagata cagcccgtcc ttccaaggcc aggtcaccat ctcagccgac 1560

aagtccatca gcaccgccta cctgcagtgg agcagcctga aggcctcgga caccgccatg 1620

tattactgtg tgagactggg tacgagggac tggttcgacc cctggggcca gggaaccctg 1680

gtcaccgtct cctcagctag caccaaagga ccttctgtat ttcctcttgc gccatgctct 1740

cgctctacgt cagaatcaac tgccgctctg ggttgcctgg ttaaagacta cttcccggag 1800

cctgtgacag tgagttggaa ctccggcgcc ctgacatcag gagtgcatac atttcccgcc 1860

gtgcttcaga gcagcggact ttatagcctc agcagtgtgg tgaccgtgcc atcttccagc 1920

ctggggacca agacctacac ctgtaacgtg gaccacaaac ccagcaacac caaggttgat 1980

aagagggtc 1989

<210> 101
<211> 663
<212> PRT
<213> Artificial Sequence

<220>
<223> description of the artificial sequence: the amino acid sequence
      of Ct-Fa1014-R1090 H Chain

<400> 101

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ser
            20                  25                  30

Ser Ser Tyr Trp Gly Trp Ile Arg Gln Pro Pro Gly Lys Gly Leu Glu
        35                  40                  45

Trp Ile Gly Ser Ile Tyr Tyr Ser Gly Ser Thr Asn Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Arg Ser Val Thr Ala Ala Asp Thr Ala Val Tyr Tyr
                85                  90                  95

Cys Val Arg Asp Lys Gly Tyr Tyr Tyr Gly Met Asp Val Trp Gly Gln
            100                 105                 110

Gly Thr Thr Val Thr Val Ser Ser Ala Ser Thr Lys Gly Pro Ser Val
        115                 120                 125

Phe Pro Leu Ala Pro Cys Ser Arg Ser Thr Ser Glu Ser Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Lys Thr Tyr Thr Cys Asn Val Asp His Lys
        195                 200                 205

Pro Ser Asn Thr Lys Val Asp Lys Arg Val Glu Ser Lys Tyr Gly Pro
    210                 215                 220

Pro Cys Pro Pro Cys Pro Ala Pro Glu Phe Glu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser Gln Glu Asp Pro Glu
            260                 265                 270

Val Gln Phe Asn Trp Tyr Val Asp Gly Val Glu Val His Asn Ala Lys
        275                 280                 285

Thr Lys Pro Arg Glu Glu Gln Phe Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Gly Leu Pro Ser Ser Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Gln Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400

Asp Gly Ser Phe Phe Leu Tyr Ser Lys Leu Thr Val Asp Lys Ser Arg
                405                 410                 415

Trp Gln Glu Gly Asn Val Phe Ser Cys Ser Val Met His Glu Ala Leu
            420                 425                 430

His Asn His Tyr Thr Gln Lys Ser Leu Ser Leu Ser Leu Gly Lys Glu
        435                 440                 445

Val Gln Leu Val Gln Ser Gly Ala Glu Val Lys Lys Pro Gly Glu Ser
    450                 455                 460

Leu Lys Ile Ser Cys Lys Gly Ser Gly Tyr Ser Phe Ile Ile Tyr Trp
465                 470                 475                 480

Ile Gly Trp Val Arg Gln Met Pro Gly Lys Gly Leu Glu Trp Met Gly
                485                 490                 495

Ile Ile Tyr Pro Asn Asp Ala Asp Thr Arg Tyr Ser Pro Ser Phe Gln
            500                 505                 510

Gly Gln Val Thr Ile Ser Ala Asp Lys Ser Ile Ser Thr Ala Tyr Leu
        515                 520                 525

Gln Trp Ser Ser Leu Lys Ala Ser Asp Thr Ala Met Tyr Tyr Cys Val
    530                 535                 540

Arg Leu Gly Thr Arg Asp Trp Phe Asp Pro Trp Gly Gln Gly Thr Leu
545                 550                 555                 560

Val Thr Val Ser Ser Ala Ser Thr Lys Gly Pro Ser Val Phe Pro Leu
                565                 570                 575

Ala Pro Cys Ser Arg Ser Thr Ser Glu Ser Thr Ala Ala Leu Gly Cys
            580                 585                 590

Leu Val Lys Asp Tyr Phe Pro Glu Pro Val Thr Val Ser Trp Asn Ser
        595                 600                 605

Gly Ala Leu Thr Ser Gly Val His Thr Phe Pro Ala Val Leu Gln Ser

610                 615                 620

Ser Gly Leu Tyr Ser Leu Ser Ser Val Val Thr Val Pro Ser Ser Ser
625                 630                 635                 640

Leu Gly Thr Lys Thr Tyr Thr Cys Asn Val Asp His Lys Pro Ser Asn
                645                 650                 655

Thr Lys Val Asp Lys Arg Val
            660

<210>  102
<211>  1995
<212>  DNA
<213>  Artificial Sequence

<220>
<223>  description of the artificial sequence: the base sequence of
       Ct-Fa1046-GTS-R1090 H Chain

<400>  102
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggggga cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctcagcaa gtaccaaggg gccatccgtc ttccccctgg cgccctgctc tagaagcacc     420

tccgagagca cagccgccct gggctgcctg gtcaaggact acttccccga accggtgacg     480

gtgtcgtgga actcaggcgc cctgaccagc ggcgtgcaca ccttcccggc tgtcctacag     540

tcctcaggac tctactccct cagcagcgtg gtgaccgtgc cctccagcag cttgggcacg     600

aagacctaca cctgcaacgt agatcacaag cccagcaaca ccaaggtgga caagagagtt     660

gagtccaaat atggtccccc atgcccacca tgcccagcac ctgagttcga ggggggacca     720

tcagtcttcc tgttcccccc aaaacccaag gacactctca tgatctcccg gacccctgag     780

gtcacgtgcg tggtggtgga cgtgagccag gaagaccccg aggtccagtt caactggtac     840

gtggatggcg tggaggtgca taatgccaag acaaagccgc gggaggagca gttcaacagc     900

acgtaccgtg tggtcagcgt cctcaccgtc ctgcaccagg actggctgaa cggcaaggag     960

tacaagtgca aggtctccaa caaaggcctc ccgtcctcca tcgagaaaac catctccaaa    1020

gccaaagggc agccccgaga gccacaggtg tacaccctgc ccccatccca ggaggagatg    1080

accaagaacc aggtcagcct gacctgcctg gtcaaaggct tctaccccag cgacatcgcc    1140

gtggagtggg agagcaatgg gcagccggag aacaactaca agaccacgcc tcccgtgctg 1200

gactccgacg gctccttctt cctctacagc aagctaaccg tggacaagag caggtggcag 1260

gaggggaatg tcttctcatg ctccgtgatg catgaggctc tgcacaacca ctacacacag 1320

aagagcctct ccctgtccct aggtaaagag gtgcagctgg tgcagtccgg agcagaggtg 1380

aaaaagcccg gggagtctct gaagatctcc tgtaagggtt ctggatacag ctttatcatc 1440

tactggatcg gctgggtgcg ccagatgccc gggaaaggcc tggagtggat ggggatcatc 1500

tatcctaatg acgctgatac cagatacagc ccgtccttcc aaggccaggt caccatctca 1560

gccgacaagt ccatcagcac cgcctacctg cagtggagca gcctgaaggc ctcggacacc 1620

gccatgtatt actgtgtgag actgggtacg agggactggt tcgacccctg gggccaggga 1680

accctggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 103
<211> 665
<212> PRT
<213> Artificial Sequence

<220>
<223> description of the artificial sequence: the amino acid sequence
of Ct-Fa1046-GTS-R1090 H Chain

<400> 103

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe
    50                  55                  60

Gln Gly Arg Val Thr Met Thr Arg Gly Thr Ser Ile Ser Thr Ala Tyr
65                  70                  75                  80

Met Glu Leu Ser Ser Leu Arg Ser Glu Asp Thr Ala Val Tyr Tyr Cys

85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Glu Ser Lys Tyr
    210                 215                 220

Gly Pro Pro Cys Pro Pro Cys Pro Ala Pro Glu Phe Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser Gln Glu Asp
            260                 265                 270

Pro Glu Val Gln Phe Asn Trp Tyr Val Asp Gly Val Glu Val His Asn
        275                 280                 285

Ala Lys Thr Lys Pro Arg Glu Glu Gln Phe Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Gly Leu Pro Ser Ser Ile Glu Lys
                325                 330                 335

Thr Ile Ser Lys Ala Lys Gly Gln Pro Arg Glu Pro Gln Val Tyr Thr
        340                 345                 350

Leu Pro Pro Ser Gln Glu Glu Met Thr Lys Asn Gln Val Ser Leu Thr
    355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Glu Gly Asn Val Phe Ser Cys Ser Val Met His Glu
            420                 425                 430

Ala Leu His Asn His Tyr Thr Gln Lys Ser Leu Ser Leu Ser Leu Gly
        435                 440                 445

Lys Glu Val Gln Leu Val Gln Ser Gly Ala Glu Val Lys Lys Pro Gly
    450                 455                 460

Glu Ser Leu Lys Ile Ser Cys Lys Gly Ser Gly Tyr Ser Phe Ile Ile
465                 470                 475                 480

Tyr Trp Ile Gly Trp Val Arg Gln Met Pro Gly Lys Gly Leu Glu Trp
                485                 490                 495

Met Gly Ile Ile Tyr Pro Asn Asp Ala Asp Thr Arg Tyr Ser Pro Ser
            500                 505                 510

Phe Gln Gly Gln Val Thr Ile Ser Ala Asp Lys Ser Ile Ser Thr Ala
        515                 520                 525

Tyr Leu Gln Trp Ser Ser Leu Lys Ala Ser Asp Thr Ala Met Tyr Tyr
    530                 535                 540

Cys Val Arg Leu Gly Thr Arg Asp Trp Phe Asp Pro Trp Gly Gln Gly
545                 550                 555                 560

Thr Leu Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210> 104
<211> 1995
<212> DNA
<213> Artificial Sequence

<220>
<223> description of the artificial sequence: the base sequence of
Ct-Fa1046-FTS-R1090 H Chain

<400> 104
gaggtgcagc tggtgcagtc tggagctgag gtgaagaagc ctggggcctc agtgaaggtc 60

tcctgcaagg cttctggtta cacctttacc aactctggta tcaactgggt gcgacaggcc 120

cctggacaag ggcttgagtg gatgggatgg atgaacccta acagtggtga cacaggctat 180

gcacagaagt tccagggcag agtcaccatg accaggttca cctccataag cacagcctac 240

atggagctga gcagcctgag atctgaggac acggccgtgt attactgtgc gagagtccca 300

gtagtgggag ctacctacaa ctggttcgac ccctggggcc agggaaccac ggtcaccgtc 360

tcctcagcaa gtaccaaggg gccatccgtc ttccccctgg cgccctgctc tagaagcacc 420

tccgagagca cagccgccct gggctgcctg gtcaaggact acttccccga accggtgacg 480

gtgtcgtgga actcaggcgc cctgaccagc ggcgtgcaca ccttcccggc tgtcctacag 540

tcctcaggac tctactccct cagcagcgtg gtgaccgtgc cctccagcag cttgggcacg 600

aagacctaca cctgcaacgt agatcacaag cccagcaaca ccaaggtgga caagagagtt 660

gagtccaaat atggtccccc atgcccacca tgcccagcac ctgagttcga ggggggacca 720

tcagtcttcc tgttcccccc aaaacccaag gacactctca tgatctcccg gacccctgag 780

gtcacgtgcg tggtggtgga cgtgagccag gaagaccccg aggtccagtt caactggtac 840

gtggatggcg tggaggtgca taatgccaag acaaagccgc gggaggagca gttcaacagc 900

acgtaccgtg tggtcagcgt cctcaccgtc ctgcaccagg actggctgaa cggcaaggag 960

tacaagtgca aggtctccaa caaaggcctc ccgtcctcca tcgagaaaac catctccaaa 1020

gccaaagggc agccccgaga gccacaggtg tacaccctgc ccccatccca ggaggagatg 1080

accaagaacc aggtcagcct gacctgcctg gtcaaaggct tctaccccag cgacatcgcc 1140

gtggagtggg agagcaatgg gcagccggag aacaactaca agaccacgcc tcccgtgctg 1200

gactccgacg gctccttctt cctctacagc aagctaaccg tggacaagag caggtggcag 1260

gaggggaatg tcttctcatg ctccgtgatg catgaggctc tgcacaacca ctacacacag 1320

aagagcctct ccctgtccct aggtaaagag gtgcagctgg tgcagtccgg agcagaggtg 1380

aaaaagcccg gggagtctct gaagatctcc tgtaagggtt ctggatacag ctttatcatc 1440

tactggatcg gctgggtgcg ccagatgccc gggaaaggcc tggagtggat ggggatcatc 1500

tatcctaatg acgctgatac cagatacagc ccgtccttcc aaggccaggt caccatctca 1560

gccgacaagt ccatcagcac cgcctacctg cagtggagca gcctgaaggc ctcggacacc 1620

gccatgtatt actgtgtgag actgggtacg agggactggt tcgacccctg gggccaggga 1680

accctggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 105
<211> 665
<212> PRT
<213> Artificial Sequence

<220>
<223> description of the artificial sequence: the amino acid sequence
      of Ct-Fa1046-FTS-R1090 H Chain

<400> 105

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe
    50                  55                  60

Gln Gly Arg Val Thr Met Thr Arg Phe Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Glu Ser Lys Tyr
    210                 215                 220

Gly Pro Pro Cys Pro Pro Cys Pro Ala Pro Glu Phe Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser Gln Glu Asp
            260                 265                 270

Pro Glu Val Gln Phe Asn Trp Tyr Val Asp Gly Val Glu Val His Asn
        275                 280                 285

Ala Lys Thr Lys Pro Arg Glu Glu Gln Phe Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Gly Leu Pro Ser Ser Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Gln Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Glu Gly Asn Val Phe Ser Cys Ser Val Met His Glu
            420                 425                 430

Ala Leu His Asn His Tyr Thr Gln Lys Ser Leu Ser Leu Ser Leu Gly
        435                 440                 445

Lys Glu Val Gln Leu Val Gln Ser Gly Ala Glu Val Lys Lys Pro Gly
    450                 455                 460

Glu Ser Leu Lys Ile Ser Cys Lys Gly Ser Gly Tyr Ser Phe Ile Ile
465                 470                 475                 480

Tyr Trp Ile Gly Trp Val Arg Gln Met Pro Gly Lys Gly Leu Glu Trp
                485                 490                 495

Met Gly Ile Ile Tyr Pro Asn Asp Ala Asp Thr Arg Tyr Ser Pro Ser
            500                 505                 510

Phe Gln Gly Gln Val Thr Ile Ser Ala Asp Lys Ser Ile Ser Thr Ala
        515                 520                 525

Tyr Leu Gln Trp Ser Ser Leu Lys Ala Ser Asp Thr Ala Met Tyr Tyr
    530                 535                 540

Cys Val Arg Leu Gly Thr Arg Asp Trp Phe Asp Pro Trp Gly Gln Gly
545                 550                 555                 560

Thr Leu Val Thr Val Ser Ser Ala Ser Thr Lys Gly Pro Ser Val Phe

565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  106
<211>  1995
<212>  DNA
<213>  Artificial Sequence

<220>
<223>  description of the artificial sequence: the base sequence of
       Ct-Fa1046-KTS-R1090 H Chain

<400>  106
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggaaga cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctcagcaa gtaccaaggg gccatccgtc ttccccctgg cgccctgctc tagaagcacc     420

tccgagagca cagccgccct gggctgcctg gtcaaggact acttccccga accggtgacg     480

gtgtcgtgga actcaggcgc cctgaccagc ggcgtgcaca ccttcccggc tgtcctacag     540

tcctcaggac tctactccct cagcagcgtg gtgaccgtgc cctccagcag cttgggcacg     600

aagacctaca cctgcaacgt agatcacaag cccagcaaca ccaaggtgga caagagagtt     660

gagtccaaat atggtccccc atgcccacca tgcccagcac ctgagttcga ggggggacca     720

tcagtcttcc tgttcccccc aaaacccaag gacactctca tgatctcccg gacccctgag     780

gtcacgtgcg tggtggtgga cgtgagccag gaagaccccg aggtccagtt caactggtac 840

gtggatggcg tggaggtgca taatgccaag acaaagccgc gggaggagca gttcaacagc 900

acgtaccgtg tggtcagcgt cctcaccgtc ctgcaccagg actggctgaa cggcaaggag 960

tacaagtgca aggtctccaa caaaggcctc ccgtcctcca tcgagaaaac catctccaaa 1020

gccaaagggc agccccgaga gccacaggtg tacaccctgc ccccatccca ggaggagatg 1080

accaagaacc aggtcagcct gacctgcctg gtcaaaggct tctaccccag cgacatcgcc 1140

gtggagtggg agagcaatgg gcagccggag aacaactaca agaccacgcc tcccgtgctg 1200

gactccgacg gctccttctt cctctacagc aagctaaccg tggacaagag caggtggcag 1260

gaggggaatg tcttctcatg ctccgtgatg catgaggctc tgcacaacca ctacacacag 1320

aagagcctct ccctgtccct aggtaaagag gtgcagctgg tgcagtccgg agcagaggtg 1380

aaaaagcccg gggagtctct gaagatctcc tgtaagggtt ctggatacag ctttatcatc 1440

tactggatcg gctgggtgcg ccagatgccc gggaaaggcc tggagtggat ggggatcatc 1500

tatcctaatg acgctgatac cagatacagc ccgtccttcc aaggccaggt caccatctca 1560

gccgacaagt ccatcagcac cgcctacctg cagtggagca gcctgaaggc ctcggacacc 1620

gccatgtatt actgtgtgag actgggtacg agggactggt tcgacccctg gggccaggga 1680

accctggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 107
<211> 665
<212> PRT
<213> Artificial Sequence

<220>
<223> description of the artificial sequence: the amino acid sequence
      of Ct-Fa1046-KTS-R1090 H Chain

<400> 107

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met

35                  40                  45

Gly Trp Met Asn Pro Asn Ser Gly Asp Thr Gly Tyr Ala Gln Lys Phe
    50                  55                  60

Gln Gly Arg Val Thr Met Thr Arg Lys Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Glu Ser Lys Tyr
    210                 215                 220

Gly Pro Pro Cys Pro Pro Cys Pro Ala Pro Glu Phe Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser Gln Glu Asp
            260                 265                 270

Pro Glu Val Gln Phe Asn Trp Tyr Val Asp Gly Val Glu Val His Asn
        275                 280                 285

Ala Lys Thr Lys Pro Arg Glu Glu Gln Phe Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Gly Leu Pro Ser Ser Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Gln Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Glu Gly Asn Val Phe Ser Cys Ser Val Met His Glu
            420                 425                 430

Ala Leu His Asn His Tyr Thr Gln Lys Ser Leu Ser Leu Ser Leu Gly
        435                 440                 445

Lys Glu Val Gln Leu Val Gln Ser Gly Ala Glu Val Lys Lys Pro Gly
    450                 455                 460

Glu Ser Leu Lys Ile Ser Cys Lys Gly Ser Gly Tyr Ser Phe Ile Ile
465                 470                 475                 480

Tyr Trp Ile Gly Trp Val Arg Gln Met Pro Gly Lys Gly Leu Glu Trp
                485                 490                 495

Met Gly Ile Ile Tyr Pro Asn Asp Ala Asp Thr Arg Tyr Ser Pro Ser
            500                 505                 510

Phe Gln Gly Gln Val Thr Ile Ser Ala Asp Lys Ser Ile Ser Thr Ala
        515                 520                 525

Tyr Leu Gln Trp Ser Ser Leu Lys Ala Ser Asp Thr Ala Met Tyr Tyr
    530                 535                 540

Cys Val Arg Leu Gly Thr Arg Asp Trp Phe Asp Pro Trp Gly Gln Gly
545                 550                 555                 560

Thr Leu Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  108
<211>  1995
<212>  DNA
<213>  Artificial Sequence

<220>
<223>  description of the artificial sequence: the base sequence of
       Ct-Fa1046-WTS-R1090 H Chain

<400>  108
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggtgga cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctcagcaa gtaccaaggg gccatccgtc ttccccctgg cgccctgctc tagaagcacc     420

tccgagagca cagccgccct gggctgcctg gtcaaggact acttccccga accggtgacg     480

gtgtcgtgga actcaggcgc cctgaccagc ggcgtgcaca ccttcccggc tgtcctacag     540

tcctcaggac tctactccct cagcagcgtg gtgaccgtgc cctccagcag cttgggcacg     600

aagacctaca cctgcaacgt agatcacaag cccagcaaca ccaaggtgga caagagagtt 660

gagtccaaat atggtccccc atgcccacca tgcccagcac ctgagttcga ggggggacca 720

tcagtcttcc tgttcccccc aaaacccaag gacactctca tgatctcccg gacccctgag 780

gtcacgtgcg tggtggtgga cgtgagccag gaagaccccg aggtccagtt caactggtac 840

gtggatggcg tggaggtgca taatgccaag acaaagccgc gggaggagca gttcaacagc 900

acgtaccgtg tggtcagcgt cctcaccgtc ctgcaccagg actggctgaa cggcaaggag 960

tacaagtgca aggtctccaa caaaggcctc ccgtcctcca tcgagaaaac catctccaaa 1020

gccaaagggc agccccgaga gccacaggtg tacaccctgc ccccatccca ggaggagatg 1080

accaagaacc aggtcagcct gacctgcctg gtcaaaggct tctaccccag cgacatcgcc 1140

gtggagtggg agagcaatgg gcagccggag aacaactaca agaccacgcc tcccgtgctg 1200

gactccgacg gctccttctt cctctacagc aagctaaccg tggacaagag caggtggcag 1260

gaggggaatg tcttctcatg ctccgtgatg catgaggctc tgcacaacca ctacacacag 1320

aagagcctct ccctgtccct aggtaaagag gtgcagctgg tgcagtccgg agcagaggtg 1380

aaaaagcccg gggagtctct gaagatctcc tgtaagggtt ctggatacag ctttatcatc 1440

tactggatcg gctgggtgcg ccagatgccc gggaaaggcc tggagtggat ggggatcatc 1500

tatcctaatg acgctgatac cagatacagc ccgtccttcc aaggccaggt caccatctca 1560

gccgacaagt ccatcagcac cgcctacctg cagtggagca gcctgaaggc ctcggacacc 1620

gccatgtatt actgtgtgag actgggtacg agggactggt tcgacccctg gggccaggga 1680

accctggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 109
<211> 665
<212> PRT
<213> Artificial Sequence

<220>
<223> description of the artificial sequence: the amino acid sequence
      of Ct-Fa1046-WTS-R1090 H Chain

<400> 109

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe
    50                  55                  60

Gln Gly Arg Val Thr Met Thr Arg Trp Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Glu Ser Lys Tyr
    210                 215                 220

Gly Pro Pro Cys Pro Pro Cys Pro Ala Pro Glu Phe Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser Gln Glu Asp
            260                 265                 270

Pro Glu Val Gln Phe Asn Trp Tyr Val Asp Gly Val Glu Val His Asn
        275                 280                 285

Ala Lys Thr Lys Pro Arg Glu Glu Gln Phe Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Gly Leu Pro Ser Ser Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Gln Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Glu Gly Asn Val Phe Ser Cys Ser Val Met His Glu
            420                 425                 430

Ala Leu His Asn His Tyr Thr Gln Lys Ser Leu Ser Leu Ser Leu Gly
        435                 440                 445

Lys Glu Val Gln Leu Val Gln Ser Gly Ala Glu Val Lys Lys Pro Gly
    450                 455                 460

Glu Ser Leu Lys Ile Ser Cys Lys Gly Ser Gly Tyr Ser Phe Ile Ile
465                 470                 475                 480

Tyr Trp Ile Gly Trp Val Arg Gln Met Pro Gly Lys Gly Leu Glu Trp
                485                 490                 495

Met Gly Ile Ile Tyr Pro Asn Asp Ala Asp Thr Arg Tyr Ser Pro Ser
            500                 505                 510

Phe Gln Gly Gln Val Thr Ile Ser Ala Asp Lys Ser Ile Ser Thr Ala

515                 520                 525

Tyr Leu Gln Trp Ser Ser Leu Lys Ala Ser Asp Thr Ala Met Tyr Tyr
    530                 535                 540

Cys Val Arg Leu Gly Thr Arg Asp Trp Phe Asp Pro Trp Gly Gln Gly
545                 550                 555                 560

Thr Leu Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  110
<211>  1995
<212>  DNA
<213>  Artificial Sequence

<220>
<223>  description of the artificial sequence: the base sequence of
       Ct-Fa1046-TTS-R1090 H Chain

<400>  110
gaggtgcagc tggtgcagtc tggagctgag gtgaagaagc ctggggcctc agtgaaggtc      60

tcctgcaagg cttctggtta cacctttacc aactctggta tcaactgggt gcgacaggcc     120

cctggacaag ggcttgagtg gatgggatgg atgaacccta acagtggtga cacaggctat     180

gcacagaagt tccagggcag agtcaccatg accaggacaa cctccataag cacagcctac     240

atggagctga gcagcctgag atctgaggac acggccgtgt attactgtgc gagagtccca     300

gtagtgggag ctacctacaa ctggttcgac ccctggggcc agggaaccac ggtcaccgtc     360

tcctcagcaa gtaccaaggg gccatccgtc ttccccctgg cgccctgctc tagaagcacc     420

tccgagagca cagccgccct gggctgcctg gtcaaggact acttccccga accggtgacg 480

gtgtcgtgga actcaggcgc cctgaccagc ggcgtgcaca ccttcccggc tgtcctacag 540

tcctcaggac tctactccct cagcagcgtg gtgaccgtgc cctccagcag cttgggcacg 600

aagacctaca cctgcaacgt agatcacaag cccagcaaca ccaaggtgga caagagagtt 660

gagtccaaat atggtccccc atgcccacca tgcccagcac ctgagttcga ggggggacca 720

tcagtcttcc tgttcccccc aaaacccaag gacactctca tgatctcccg gacccctgag 780

gtcacgtgcg tggtggtgga cgtgagccag gaagaccccg aggtccagtt caactggtac 840

gtggatggcg tggaggtgca taatgccaag acaaagccgc gggaggagca gttcaacagc 900

acgtaccgtg tggtcagcgt cctcaccgtc ctgcaccagg actggctgaa cggcaaggag 960

tacaagtgca aggtctccaa caaaggcctc ccgtcctcca tcgagaaaac catctccaaa 1020

gccaaagggc agccccgaga gccacaggtg tacaccctgc ccccatccca ggaggagatg 1080

accaagaacc aggtcagcct gacctgcctg gtcaaaggct tctaccccag cgacatcgcc 1140

gtggagtggg agagcaatgg gcagccggag aacaactaca agaccacgcc tcccgtgctg 1200

gactccgacg gctccttctt cctctacagc aagctaaccg tggacaagag caggtggcag 1260

gaggggaatg tcttctcatg ctccgtgatg catgaggctc tgcacaacca ctacacacag 1320

aagagcctct ccctgtccct aggtaaagag gtgcagctgg tgcagtccgg agcagaggtg 1380

aaaaagcccg gggagtctct gaagatctcc tgtaagggtt ctggatacag ctttatcatc 1440

tactggatcg gctgggtgcg ccagatgccc gggaaaggcc tggagtggat ggggatcatc 1500

tatcctaatg acgctgatac cagatacagc ccgtccttcc aaggccaggt caccatctca 1560

gccgacaagt ccatcagcac cgcctacctg cagtggagca gcctgaaggc ctcggacacc 1620

gccatgtatt actgtgtgag actgggtacg agggactggt tcgacccctg gggccaggga 1680

accctggtca ccgtctcctc agctagcacc aaaggacctt ctgtatttcc tcttgcgcca 1740

tgctctcgct ctacgtcaga atcaactgcc gctctggggt gcctggttaa agactacttc 1800

ccggagcctg tgacagtgag ttggaactcc ggcgccctga catcaggagt gcatacattt 1860

cccgccgtgc ttcagagcag cggactttat agcctcagca gtgtggtgac cgtgccatct 1920

tccagcctgg ggaccaagac ctacacctgt aacgtggacc acaaacccag caacaccaag 1980

gttgataaga gggtc 1995

<210> 111
<211> 665
<212> PRT
<213> Artificial Sequence

<220>
<223> description of the artificial sequence: the amino acid sequence
of Ct-Fa1046-TTS-R1090 H Chain

<400>  111

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Ser
            20                  25                  30

Gly Ile Asn Trp Val Arg Gln Ala Pro Gly Gln Gly Leu Glu Trp Met
        35                  40                  45

Gly Trp Met Asn Pro Asn Ser Gly Asp Thr Gly Tyr Ala Gln Lys Phe
    50                  55                  60

Gln Gly Arg Val Thr Met Thr Arg Thr Thr Ser Ile Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Val Pro Val Val Gly Ala Thr Tyr Asn Trp Phe Asp Pro Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Cys Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Glu Ser Lys Tyr
    210                 215                 220

Gly Pro Pro Cys Pro Pro Cys Pro Ala Pro Glu Phe Glu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser Gln Glu Asp
            260                 265                 270

Pro Glu Val Gln Phe Asn Trp Tyr Val Asp Gly Val Glu Val His Asn
        275                 280                 285

Ala Lys Thr Lys Pro Arg Glu Glu Gln Phe Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Gly Leu Pro Ser Ser Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Gln Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Glu Gly Asn Val Phe Ser Cys Ser Val Met His Glu
            420                 425                 430

Ala Leu His Asn His Tyr Thr Gln Lys Ser Leu Ser Leu Ser Leu Gly
        435                 440                 445

Lys Glu Val Gln Leu Val Gln Ser Gly Ala Glu Val Lys Lys Pro Gly
    450                 455                 460

Glu Ser Leu Lys Ile Ser Cys Lys Gly Ser Gly Tyr Ser Phe Ile Ile
465                 470                 475                 480

Tyr Trp Ile Gly Trp Val Arg Gln Met Pro Gly Lys Gly Leu Glu Trp
                485                 490                 495

Met Gly Ile Ile Tyr Pro Asn Asp Ala Asp Thr Arg Tyr Ser Pro Ser
            500                 505                 510

Phe Gln Gly Gln Val Thr Ile Ser Ala Asp Lys Ser Ile Ser Thr Ala
        515                 520                 525

Tyr Leu Gln Trp Ser Ser Leu Lys Ala Ser Asp Thr Ala Met Tyr Tyr
    530                 535                 540

Cys Val Arg Leu Gly Thr Arg Asp Trp Phe Asp Pro Trp Gly Gln Gly
545                 550                 555                 560

Thr Leu Val Thr Val Ser Ser Ala Ser Thr Lys Gly Pro Ser Val Phe
                565                 570                 575

Pro Leu Ala Pro Cys Ser Arg Ser Thr Ser Glu Ser Thr Ala Ala Leu
            580                 585                 590

Gly Cys Leu Val Lys Asp Tyr Phe Pro Glu Pro Val Thr Val Ser Trp
        595                 600                 605

Asn Ser Gly Ala Leu Thr Ser Gly Val His Thr Phe Pro Ala Val Leu
    610                 615                 620

Gln Ser Ser Gly Leu Tyr Ser Leu Ser Ser Val Val Thr Val Pro Ser
625                 630                 635                 640

Ser Ser Leu Gly Thr Lys Thr Tyr Thr Cys Asn Val Asp His Lys Pro
                645                 650                 655

Ser Asn Thr Lys Val Asp Lys Arg Val
            660                 665

<210>  112
<211>  676
<212>  PRT
<213>  Artificial Sequence

<220>
<223>  description of the artificial sequence: the amino acid sequence
       of P1AA9663 compartment 1

<400>  112

Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Ser Phe Thr Gly Tyr
            20                  25                  30

Tyr Ile His Trp Val Arg Gln Ala Pro Gly Lys Gly Leu Glu Trp Val
        35                  40                  45

Ala Arg Val Ile Pro Asn Ala Gly Gly Thr Ser Tyr Asn Gln Lys Phe
    50                  55                  60

Lys Gly Arg Phe Thr Leu Ser Val Asp Asn Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Glu Gly Ile Tyr Trp Trp Gly Gln Gly Thr Leu Val Thr Val
            100                 105                 110

Ser Ser Ala Ser Thr Lys Gly Pro Ser Val Phe Pro Leu Ala Pro Ser
        115                 120                 125

Ser Lys Ser Thr Ser Gly Gly Thr Ala Ala Leu Gly Cys Leu Val Glu
    130                 135                 140

Asp Tyr Phe Pro Glu Pro Val Thr Val Ser Trp Asn Ser Gly Ala Leu
145                 150                 155                 160

Thr Ser Gly Val His Thr Phe Pro Ala Val Leu Gln Ser Ser Gly Leu
                165                 170                 175

Tyr Ser Leu Ser Ser Val Val Thr Val Pro Ser Ser Ser Leu Gly Thr
            180                 185                 190

Gln Thr Tyr Ile Cys Asn Val Asn His Lys Pro Ser Asn Thr Lys Val
        195                 200                 205

Asp Glu Lys Val Glu Pro Lys Ser Cys Asp Lys Thr His Thr Cys Pro
    210                 215                 220

Pro Cys Pro Ala Pro Glu Ala Ala Gly Gly Pro Ser Val Phe Leu Phe
225                 230                 235                 240

Pro Pro Lys Pro Lys Asp Thr Leu Met Ile Ser Arg Thr Pro Glu Val
                245                 250                 255

Thr Cys Val Val Val Asp Val Ser His Glu Asp Pro Glu Val Lys Phe
            260                 265                 270

Asn Trp Tyr Val Asp Gly Val Glu Val His Asn Ala Lys Thr Lys Pro
        275                 280                 285

Arg Glu Glu Gln Tyr Asn Ser Thr Tyr Arg Val Val Ser Val Leu Thr
    290                 295                 300

Val Leu His Gln Asp Trp Leu Asn Gly Lys Glu Tyr Lys Cys Lys Val
305                 310                 315                 320

Ser Asn Lys Ala Leu Gly Ala Pro Ile Glu Lys Thr Ile Ser Lys Ala
                325                 330                 335

Lys Gly Gln Pro Arg Glu Pro Gln Val Tyr Thr Leu Pro Pro Ser Arg
            340                 345                 350

Asp Glu Leu Thr Lys Asn Gln Val Ser Leu Thr Cys Leu Val Lys Gly
        355                 360                 365

Phe Tyr Pro Ser Asp Ile Ala Val Glu Trp Glu Ser Asn Gly Gln Pro
    370                 375                 380

Glu Asn Asn Tyr Lys Thr Thr Pro Pro Val Leu Asp Ser Asp Gly Ser
385                 390                 395                 400

Phe Phe Leu Tyr Ser Lys Leu Thr Val Asp Lys Ser Arg Trp Gln Gln
                405                 410                 415

Gly Asn Val Phe Ser Cys Ser Val Met His Glu Ala Leu His Asn His
            420                 425                 430

Tyr Thr Gln Lys Ser Leu Ser Leu Ser Pro Gly Gly Gly Gly Gly Ser
        435                 440                 445

Gly Gly Gly Gly Ser Gly Gly Gly Gly Ser Gly Gly Gly Gly Ser Glu
    450                 455                 460

Ile Val Leu Thr Gln Ser Pro Gly Thr Leu Ser Leu Ser Pro Gly Glu
465                 470                 475                 480

Arg Ala Thr Leu Ser Cys Arg Ala Ser Gln Ser Val Ser Arg Ser Tyr
                485                 490                 495

Leu Ala Trp Tyr Gln Gln Lys Pro Gly Gln Ala Pro Arg Leu Leu Ile
            500                 505                 510

Ile Gly Ala Ser Thr Arg Ala Thr Gly Ile Pro Asp Arg Phe Ser Gly
        515                 520                 525

Ser Gly Ser Gly Thr Asp Phe Thr Leu Thr Ile Ser Arg Leu Glu Pro

530                 535                 540

Glu Asp Phe Ala Val Tyr Tyr Cys Gln Gln Gly Gln Val Ile Pro Pro
545                 550                 555                 560

Thr Phe Gly Gln Gly Thr Lys Val Glu Ile Lys Ser Ser Ala Ser Thr
                565                 570                 575

Lys Gly Pro Ser Val Phe Pro Leu Ala Pro Ser Ser Lys Ser Thr Ser
            580                 585                 590

Gly Gly Thr Ala Ala Leu Gly Cys Leu Val Lys Asp Tyr Phe Pro Glu
        595                 600                 605

Pro Val Thr Val Ser Trp Asn Ser Gly Ala Leu Thr Ser Gly Val His
    610                 615                 620

Thr Phe Pro Ala Val Leu Gln Ser Ser Gly Leu Tyr Ser Leu Ser Ser
625                 630                 635                 640

Val Val Thr Val Pro Ser Ser Ser Leu Gly Thr Gln Thr Tyr Ile Cys
                645                 650                 655

Asn Val Asn His Lys Pro Ser Asn Thr Lys Val Asp Lys Lys Val Glu
            660                 665                 670

Pro Lys Ser Cys
        675

<210>  113
<211>  219
<212>  PRT
<213>  Artificial Sequence

<220>
<223>  description of the artificial sequence: the amino acid sequence
       of P1AA9663 compartment 2

<400>  113

Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Ser Leu Val His Ser
            20                  25                  30

Asn Gly Asn Thr Phe Leu His Trp Tyr Gln Gln Lys Pro Gly Lys Ala
        35                  40                  45

Pro Lys Leu Leu Ile Tyr Thr Val Ser Asn Arg Phe Ser Gly Val Pro

50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Phe Cys Ser Gln Thr
                85                  90                  95

Thr His Val Pro Trp Thr Phe Gly Gln Gly Thr Lys Val Glu Ile Lys
            100                 105                 110

Arg Thr Val Ala Ala Pro Ser Val Phe Ile Phe Pro Pro Ser Asp Arg
        115                 120                 125

Lys Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  114
<211>  223
<212>  PRT
<213>  Artificial Sequence

<220>
<223>  description of the artificial sequence: the amino acid sequence
       of P1AA9663 compartment 3

<400>  114

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser His
            20                  25                  30

Ala Met Ser Trp Val Arg Gln Ala Pro Gly Lys Gly Leu Glu Trp Val

35                  40                  45

Ser Ala Ile Trp Ala Ser Gly Glu Gln Tyr Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Gly Trp Leu Gly Asn Phe Asp Tyr Trp Gly Gln Gly Thr Leu Val
            100                 105                 110

Thr Val Ser Ser Ala Ser Val Ala Ala Pro Ser Val Phe Ile Phe Pro
        115                 120                 125

Pro Ser Asp Glu Gln Leu Lys Ser Gly Thr Ala Ser Val Val Cys Leu
    130                 135                 140

Leu Asn Asn Phe Tyr Pro Arg Glu Ala Lys Val Gln Trp Lys Val Asp
145                 150                 155                 160

Asn Ala Leu Gln Ser Gly Asn Ser Gln Glu Ser Val Thr Glu Gln Asp
                165                 170                 175

Ser Lys Asp Ser Thr Tyr Ser Leu Ser Ser Thr Leu Thr Leu Ser Lys
            180                 185                 190

Ala Asp Tyr Glu Lys His Lys Val Tyr Ala Cys Glu Val Thr His Gln
        195                 200                 205

Gly Leu Ser Ser Pro Val Thr Lys Ser Phe Asn Arg Gly Glu Cys
    210                 215                 220

<210>  115
<211>  2028
<212>  DNA
<213>  Artificial Sequence

<220>
<223>  description of the artificial sequence: the base sequence of
       P1AA9663 compartment 1

<400>  115
gaagttcagt tggtcgaatc tggtggaggt ttggtccagc cggggggttc tcttaggttg      60

tcatgtgcgg caagcgggta ttcatttacc ggctattata tacactgggt tcggcaggca      120

cccggtaaag gcctcgaatg ggtagccaga gtcatcccaa atgccggagg gacttcctat      180

aatcaaaagt tcaaaggccg gtttactctt agcgtggata attctaaaaa tacggcctac 240

ctgcaaatga acagtctgcg agcggaagat accgccgtgt actattgcgc tcgggagggc 300

atctattggt ggggtcaggg aacgctcgtg actgtatcca gtgcttcaac taaagggccc 360

agcgtctttc ctctggcacc aagttcaaag tccacatcag gaggtacagc tgctttgggt 420

tgccttgtag aagattattt tccagaaccc gtcacagtct cctggaattc cggtgccctg 480

acgagcggtg tacacacctt tccggcggtg ctccagagct caggtctcta ttccttgtcc 540

agcgtagtca cagtaccgag ctccagtctg ggtactcaga catacatctg caacgtcaat 600

cacaaaccgt ccaatacaaa ggttgacgag aaggtggagc cgaagagttg tgacaaaaca 660

cacacctgtc caccttgtcc ggcaccagaa gctgctggtg gcccctccgt attcttgttt 720

cctccaaaac cgaaagatac tctcatgatt tctcgcaccc ccgaggtaac gtgcgtagtg 780

gtcgatgtat cacatgaaga ccccgaagta aaatttaatt ggtatgttga cggcgtagaa 840

gtgcataacg ccaagactaa gccacgcgag gagcaataca actccactta tagggtagtg 900

tctgttttga ctgtccttca ccaagactgg ttgaatggaa aggagtataa atgtaaggtc 960

tctaataagg ctctcggcgc acctatagag aagaccatct caaaagcaaa agggcaacca 1020

cgggagcctc aagtttatac cttgccccct agtcgcgatg agttgaccaa gaaccaggtg 1080

tcccttacct gtcttgttaa aggtttctat cccagtgaca tagcggtgga gtgggaaagt 1140

aacggtcagc ctgagaacaa ctataagacg acacctcctg ttctcgattc cgatggcagt 1200

tttttcctgt attctaaact taccgttgat aaatcacgct ggcagcaggg gaatgtcttt 1260

tcttgttcag ttatgcatga ggctttgcac aatcactaca cgcaaaaatc actcagtctc 1320

tctccgggag gcggaggggg gagtggggga ggggggtctg gaggaggagg aagtggaggt 1380

ggaggatctg aaattgtatt gacccaatct cccggaacgc tttctctgag ccctggagag 1440

cgggctaccc tgtcatgtcg agcaagccaa tcagtaagca gatcctacct cgcatggtac 1500

cagcagaaac ccggacaggc accacgcctc ctcataattg gcgccagcac cagggccacg 1560

ggcatacccg accgcttctc cggttctgga tcagggaccg atttcaccct caccatatct 1620

agactggaac cggaggattt tgccgtatat tactgccagc aaggacaggt catacctccc 1680

acgtttggac aagggacgaa agtcgaaatc aaaagctccg catcaaccaa gggaccgagt 1740

gtcttccccc tcgccccgag ctctaaaagc acgtctgggg gtactgcagc gctcggatgc 1800

cttgtgaagg attactttcc agaaccggtc acggtgagtt ggaatagtgg agcgctcacg 1860

tcaggagtac atacattccc tgcagtactg cagagtagtg gactttacag tttgagcagc 1920

gtggtcacag tccccagttc atccctgggt acccaaacgt atatatgtaa cgtcaaccat 1980

aaacccagta ataccaaggt ggataagaag gtggaaccga agtcatgc 2028

<210>  116
<211>  657
<212>  DNA
<213>  Artificial Sequence

<220>
<223>  description of the artificial sequence: the base sequence of
       P1AA9663 compartment 2

<400>  116
gatattcaaa tgactcaatc cccatcctct ctttccgcat ctgttggtga cagggtgact       60

atcacttgta ggtcctctca gagcctggta cattctaacg gcaatacatt tttgcactgg      120

tatcagcaaa agccaggcaa ggctcccaaa ctgcttatct acaccgtaag taaccgcttc      180

tccggcgtcc ctagtaggtt ctccggtagc ggcagtggca ctgatttcac cctcactatc      240

agcagtcttc aaccggaaga ctttgccact tacttctgta gccagactac gcacgtccct      300

tggaccttcg gccagggcac gaaggtcgag ataaagcgca cagtggctgc tccgtctgta      360

ttcatcttcc ccccatcaga tcgaaaactg aagtcaggca cagccagtgt cgtttgcttg      420

ctgaataatt tctatccgcg cgaggccaaa gtccaatgga aagtcgataa tgctctccaa      480

tccggtaatt cccaagagtc tgtcaccgag caagattcaa aagatagcac ttactccttg      540

agctctactt tgacgctcag caaagcggac tacgagaagc ataaggtgta cgcttgcgaa      600

gttacacacc agggcttgag cagtccggtc accaaaagtt ttaatcgcgg cgagtgc         657


<210>  117
<211>  669
<212>  DNA
<213>  Artificial Sequence

<220>
<223>  description of the artificial sequence: the base sequence of
       P1AA9663 compartment 3

<400>  117
gaggtccaat tgctcgaatc aggaggcggc cttgttcaac cgggcggatc actcaggctg       60

agttgcgccg cgtcaggctt tacctttagc tcacacgcca tgtcttgggt caggcaggct      120

cccggcaaag gcctcgaatg ggtatctgca atctgggctt caggtgagca gtactacgcc      180

gactctgtaa aaggacggtt tacaatttca agagacaaca gtaaaaatac actgtacctt      240

cagatgaata gtctgcgagc cgaggacact gccgtctact actgcgcaaa gggttggctt      300

ggcaacttcg attattgggg tcaggggaca ttggtgacgg tcagctccgc gagtgtagca      360

gcaccctccg tatttatatt cccgccatcc gacgagcagc tcaaaagtgg aacggcttct      420

gtagtttgtc ttcttaacaa tttttacccg cgagaagcca aggtccaatg gaaagtggat      480

aacgcgttgc agagcggcaa ttcccaagag agcgtaactg agcaagattc taaggattct      540

acctatagtc tttcttcaac actgaccttg agtaaagcag actacgagaa gcacaaagtt      600

tacgcctgcg aagttacgca tcaaggattg tcctctccag taactaaaag ttttaatagg 660

ggtgaatgc 669